# EUROPEAN PATENT SPECIFICATION

(11) **EP 1 586 331 B1**
(45) Date of publication and mention of the grant of the patent: **28.05.2008**
(21) Application number: 05013732.2
(22) Date of filing: 24.09.2002
(51) Int. Cl.: A61K 39/12, A61K 39/295, C07K 14/08, C12P 21/06, C12N 5/00, C12N 15/00, C12N 15/861, C12N 15/63

(54) **Development of a preventive vaccine for filovirus infection in primates**
Entwicklung einer Vakzine zur Prävention von Infektionen mit Filovirus bei Primaten
Mise au point d'un vaccin préventif contre l'infection par filovirus chez les primates

(30) Priority: 01.10.2001 US 326476
(43) Date of publication of application: 19.10.2005
(62) Divisional of application: 02761814.9
(73) Proprietor: The Government of the U.S.A., as repr. by the Secretary, Dept. of Health & Human Services, the Nat. Inst. of Health, Rockville, MD 20852 (US)
(72) Inventor: Nabel, Gary, J., Washington DC 20008 (US); Yang, Zhi-yong, Potomac MD 20854 (US); Sullivan, Nancy, Kensington MD 20895 (US); Sanchez, Anthony, Lilburn GA 30047 (US)
(74) Representative: Vossius & Partner

(56) References cited:
- WO-A-97/42320
- SULLIVAN N J ET AL: "Development of a preventive vaccine for Ebola virus infection in primates" NATURE, MACMILLAN JOURNALS LTD. LONDON, GB, vol. 408, no. 6812, 30 November 2000 (2000-11-30), pages 605-609, XP002242909 ISSN: 0028-0836
- XU L ET AL: "Immunization for Ebola virus infection" NATURE MEDICINE, NATURE PUBLISHING, CO, US, vol. 4, no. 1, January 1998 (1998-01), pages 37-42, XP002131515 ISSN: 1078-8956
- VANDERZANDEN L ET AL: "DNA Vaccines Expressing either the GP or NP Genes of Ebola Virus Protect Mice from Lethal Challenge" VIROLOGY, ACADEMIC PRESS,ORLANDO, US, vol. 246, no. 1, 20 June 1998 (1998-06-20), pages 134-144, XP004445782 ISSN: 0042-6822

### SEQUENCE LISTING

<110> The Government of the United States of America as represented by Secretary, Health and Human Services
   NABEL, GARY
   YANG, ZHI-YONG
   SULLIVAN, NANCY
   SANCHEZ, ANTHONY
<120> Development of a Preventive Vaccine for Filovirus Infection in Primates
<130> K1565 EP/1 S3
<150> US 60/326476
   <151> 2001-10-01
<160> 52
<170> FastSEQ for Windows Version 4.0
<210> 1
   <211> 7154
   <212> DNA
   <213> Artificial Sequence
<220>
   <223> pVR1012 -GP(Z)
<400> 1
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaaggt gctgcaatcg gactggcctg gataccatat 3540
   ttcgggccag cagccgaggg aatttacata gaggggctaa tgcacaatca agatggttta 3600
   atctgtgggt tgagacagct ggccaacgag acgactcaag ctcttcaact gttcctgaga 3660
   gccacaactg agctacgcac cttttcaatc ctcaaccgta aggcaattga tttcttgctg 3720
   cagcgatggg gcggcacatg ccacattctg ggaccggact gctgtatcga accacatgat 3780
   tggaccaaga acataacaga caaaattgat cagattattc atgattttgt tgataaaacc 3840
   cttccggacc agggggacaa tgacaattgg tggacaggat ggagacaatg gataccggca 3900
   ggtattggag ttacaggcgt tgtaattgca gttatcgctt tattctgtat atgcaaattt 3960
   gtcttttagt ttttcttcag attgcttcat ggaaaagctc agcctcaaat caatgaaacc 4020
   aggatttaat tatatggatt acttgaatct aagattactt gacaaatgat aatataatac 4080
   actggagctt taaacatagc caatgtgatt ctaactcctt taaactcaca gttaatcata 4140
   aacaaggttt gaggtaccga gctcgaattg atctgctgtg ccttctagtt gccagccatc 4200
   tgttgtttgc ccctcccccg tgccttcctt gaccctggaa ggtgccactc ccactgtcct 4260
   ttcctaataa aatgaggaaa ttgcatcgca ttgtctgagt aggtgtcatt ctattctggg 4320
   gggtggggtg gggcaggaca gcaaggggga ggattgggaa gacaatagca ggcatgctgg 4380
   ggatgcggtg ggctctatgg gtacccaggt gctgaagaat tgacccggtt cctcctgggc 4440
   cagaaagaag caggcacatc cccttctctg tgacacaccc tgtccacgcc cctggttctt 4500
   agttccagcc ccactcatag gacactcata gctcaggagg gctccgcctt caatcccacc 4560
   cgctaaagta cttggagcgg tctctccctc cctcatcagc ccaccaaacc aaacctagcc 4620
   tccaagagtg ggaagaaatt aaagcaagat aggctattaa gtgcagaggg agagaaaatg 4680
   cctccaacat gtgaggaagt aatgagagaa atcatagaat ttcttccgct tcctcgctca 4740
   ctgactcgct gcgctcggtc gttcggctgc ggcgagcggt atcagctcac tcaaaggcgg 4800
   taatacggtt atccacagaa tcaggggata acgcaggaaa gaacatgtga gcaaaaggcc 4860
   agcaaaaggc caggaaccgt aaaaaggccg cgttgctggc gtttttccat aggctccgcc 4920
   cccctgacga gcatcacaaa aatcgacgct caagtcagag gtggcgaaac ccgacaggac 4980
   tataaagata ccaggcgttt ccccctggaa gctccctcgt gcgctctcct gttccgaccc 5040
   tgccgcttac cggatacctg tccgcctttc tcccttcggg aagcgtggcg ctttctcata 5100
   gctcacgctg taggtatctc agttcggtgt aggtcgttcg ctccaagctg ggctgtgtgc 5160
   acgaaccccc cgttcagccc gaccgctgcg ccttatccgg taactatcgt cttgagtcca 5220
   acccggtaag acacgactta tcgccactgg cagcagccac tggtaacagg attagcagag 5280
   cgaggtatgt aggcggtgct acagagttct tgaagtggtg gcctaactac ggctacacta 5340
   gaagaacagt atttggtatc tgcgctctgc tgaagccagt taccttcgga aaaagagttg 5400
   gtagctcttg atccggcaaa caaaccaccg ctggtagcgg tggttttttt gtttgcaagc 5460
   agcagattac gcgcagaaaa aaaggatctc aagaagatcc tttgatcttt tctacggggt 5520
   ctgacgctca gtggaacgaa aactcacgtt aagggatttt ggtcatgaga ttatcaaaaa 5580
   ggatcttcac ctagatcctt ttaaattaaa aatgaagttt taaatcaatc taaagtatat 5640
   atgagtaaac ttggtctgac agttaccaat gcttaatcag tgaggcacct atctcagcga 5700
   tctgtctatt tcgttcatcc atagttgcct gactcggggg gggggggcgc tgaggtctgc 5760
   ctcgtgaaga aggtgttgct gactcatacc aggcctgaat cgccccatca tccagccaga 5820
   aagtgaggga gccacggttg atgagagctt tgttgtaggt ggaccagttg gtgattttga 5880
   acttttgctt tgccacggaa cggtctgcgt tgtcgggaag atgcgtgatc tgatccttca 5940
   actcagcaaa agttcgattt attcaacaaa gccgccgtcc cgtcaagtca gcgtaatgct 6000
   ctgccagtgt tacaaccaat taaccaattc tgattagaaa aactcatcga gcatcaaatg 6060
   aaactgcaat ttattcatat caggattatc aataccatat ttttgaaaaa gccgtttctg 6120
   taatgaagga gaaaactcac cgaggcagtt ccataggatg gcaagatcct ggtatcggtc 6180
   tgcgattccg actcgtccaa catcaataca acctattaat ttcccctcgt caaaaataag 6240
   gttatcaagt gagaaatcac catgagtgac gactgaatcc ggtgagaatg gcaaaagctt 6300
   atgcatttct ttccagactt gttcaacagg ccagccatta cgctcgtcat caaaatcact 6360
   cgcatcaacc aaaccgttat tcattcgtga ttgcgcctga gcgagacgaa atacgcgatc 6420
   gctgttaaaa ggacaattac aaacaggaat cgaatgcaac cggcgcagga acactgccag 6480
   cgcatcaaca atattttcac ctgaatcagg atattcttct aatacctgga atgctgtttt 6540
   cccggggatc gcagtggtga gtaaccatgc atcatcagga gtacggataa aatgcttgat 6600
   ggtcggaaga ggcataaatt ccgtcagcca gtttagtctg accatctcat ctgtaacatc 6660
   attggcaacg ctacctttgc catgtttcag aaacaactct ggcgcatcgg gcttcccata 6720
   caatcgatag attgtcgcac ctgattgccc gacattatcg cgagcccatt tatacccata 6780
   taaatcagca tccatgttgg aatttaatcg cggcctcgag caagacgttt cccgttgaat 6840
   atggctcata acaccccttg tattactgtt tatgtaagca gacagtttta ttgttcatga 6900
   tgatatattt ttatcttgtg caatgtaaca tcagagattt tgagacacaa cgtggctttc 6960
   cccccccccc cattattgaa gcatttatca gggttattgt ctcatgagcg gatacatatt 7020
   tgaatgtatt tagaaaaata aacaaatagg ggttccgcgc acatttcccc gaaaagtgcc 7080
   acctgacgtc taagaaacca ttattatcat gacattaacc tataaaaata ggcgtatcac 7140
   gaggcccttt cgtc                                                   7154
<210> 2
   <211> 7188
   <212> DNA
   <213> Artificial Sequence
<220>
   <223> pVR1012x/s Ebola GP(Z)
<400> 2
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaaggt gctgcaatcg gactggcctg gataccatat 3540
   ttcgggccag cagccgaggg aatttacata gaggggctaa tgcacaatca agatggttta 3600
   atctgtgggt tgagacagct ggccaacgag acgactcaag ctcttcaact gttcctgaga 3660
   gccacaactg agctacgcac cttttcaatc ctcaaccgta aggcaattga tttcttgctg 3720
   cagcgatggg gcggcacatg ccacattctg ggaccggact gctgtatcga accacatgat 3780
   tggaccaaga acataacaga caaaattgat cagattattc atgattttgt tgataaaacc 3840
   cttccggacc agggggacaa tgacaattgg tggacaggat ggagacaatg gataccggca 3900
   ggtattggag ttacaggcgt tgtaattgca gttatcgctt tattctgtat atgcaaattt 3960
   gtcttttagt ttttcttcag attgcttcat ggaaaagctc agcctcaaat caatgaaacc 4020
   aggatttaat tatatggatt acttgaatct aagattactt gacaaatgat aatataatac 4080
   actggagctt taaacatagc caatgtgatt ctaactcctt taaactcaca gttaatcata 4140
   aacaaggttt gaggtaccga gctcgaattg atctgctgtg ccttctagtt gccagccatc 4200
   tgttgtttgc ccctcccccg tgccttcctt gaccctggaa ggtgccactc ccactgtcct 4260
   ttcctaataa aatgaggaaa ttgcatcgca ttgtctgagt aggtgtcatt ctattctggg 4320
   gggtggggtg gggcaggaca gcaaggggga ggattgggaa gacaatagca ggcatgctgg 4380
   ggatgcggtg ggctctatgg gtacccaggt gctgaagaat tgacccggtt cctcctgggc 4440
   cagaaagaag caggcacatc cccttctctg tgacacaccc tgtccacgcc cctggttctt 4500
   agttccagcc ccactcatag gacactcata gctcaggagg gctccgcctt caatcccacc 4560
   cgctaaagta cttggagcgg tctctccctc cctcatcagc ccaccaaacc aaacctagcc 4620
   tccaagagtg ggaagaaatt aaagcaagat aggctattaa gtgcagaggg agagaaaatg 4680
   cctccaacat gtgaggaagt aatgagagaa atcatagaat tttaaggcca tgatttaagg 4740
   ccatcatggc cttaatcttc cgcttcctcg ctcactgact cgctgcgctc ggtcgttcgg 4800
   ctgcggcgag cggtatcagc tcactcaaag gcggtaatac ggttatccac agaatcaggg 4860
   gataacgcag gaaagaacat gtgagcaaaa ggccagcaaa aggccaggaa ccgtaaaaag 4920
   gccgcgttgc tggcgttttt ccataggctc cgcccccctg acgagcatca caaaaatcga 4980
   cgctcaagtc agaggtggcg aaacccgaca ggactataaa gataccaggc gtttccccct 5040
   ggaagctccc tcgtgcgctc tcctgttccg accctgccgc ttaccggata cctgtccgcc 5100
   tttctccctt cgggaagcgt ggcgctttct catagctcac gctgtaggta tctcagttcg 5160
   gtgtaggtcg ttcgctccaa gctgggctgt gtgcacgaac cccccgttca gcccgaccgc 5220
   tgcgccttat ccggtaacta tcgtcttgag tccaacccgg taagacacga cttatcgcca 5280
   ctggcagcag ccactggtaa caggattagc agagcgaggt atgtaggcgg tgctacagag 5340
   ttcttgaagt ggtggcctaa ctacggctac actagaagaa cagtatttgg tatctgcgct 5400
   ctgctgaagc cagttacctt cggaaaaaga gttggtagct cttgatccgg caaacaaacc 5460
   accgctggta gcggtggttt ttttgtttgc aagcagcaga ttacgcgcag aaaaaaagga 5520
   tctcaagaag atcctttgat cttttctacg gggtctgacg ctcagtggaa cgaaaactca 5580
   cgttaaggga ttttggtcat gagattatca aaaaggatct tcacctagat ccttttaaat 5640
   taaaaatgaa gttttaaatc aatctaaagt atatatgagt aaacttggtc tgacagttac 5700
   caatgcttaa tcagtgaggc acctatctca gcgatctgtc tatttcgttc atccatagtt 5760
   gcctgactcg gggggggggg gcgctgaggt ctgcctcgtg aagaaggtgt tgctgactca 5820
   taccaggcct gaatcgcccc atcatccagc cagaaagtga gggagccacg gttgatgaga 5880
   gctttgttgt aggtggacca gttggtgatt ttgaactttt gctttgccac ggaacggtct 5940
   gcgttgtcgg gaagatgcgt gatctgatcc ttcaactcag caaaagttcg atttattcaa 6000
   caaagccgcc gtcccgtcaa gtcagcgtaa tgctctgcca gtgttacaac caattaacca 6060
   attctgatta gaaaaactca tcgagcatca aatgaaactg caatttattc atatcaggat 6120
   tatcaatacc atatttttga aaaagccgtt tctgtaatga aggagaaaac tcaccgaggc 6180
   agttccatag gatggcaaga tcctggtatc ggtctgcgat tccgactcgt ccaacatcaa 6240
   tacaacctat taatttcccc tcgtcaaaaa taaggttatc aagtgagaaa tcaccatgag 6300
   tgacgactga atccggtgag aatggcaaaa gcttatgcat ttctttccag acttgttcaa 6360
   caggccagcc attacgctcg tcatcaaaat cactcgcatc aaccaaaccg ttattcattc 6420
   gtgattgcgc ctgagcgaga cgaaatacgc gatcgctgtt aaaaggacaa ttacaaacag 6480
   gaatcgaatg caaccggcgc aggaacactg ccagcgcatc aacaatattt tcacctgaat 6540
   caggatattc ttctaatacc tggaatgctg ttttcccggg gatcgcagtg gtgagtaacc 6600
   atgcatcatc aggagtacgg ataaaatgct tgatggtcgg aagaggcata aattccgtca 6660
   gccagtttag tctgaccatc tcatctgtaa catcattggc aacgctacct ttgccatgtt 6720
   tcagaaacaa ctctggcgca tcgggcttcc catacaatcg atagattgtc gcacctgatt 6780
   gcccgacatt atcgcgagcc catttatacc catataaatc agcatccatg ttggaattta 6840
   atcgcggcct cgagcaagac gtttcccgtt gaatatggct cataacaccc cttgtattac 6900
   tgtttatgta agcagacagt tttattgttc atgatgatat atttttatct tgtgcaatgt 6960
   aacatcagag attttgagac acaacgtggc tttccccccc cccccattat tgaagcattt 7020
   atcagggtta ttgtctcatg agcggataca tatttgaatg tatttagaaa aataaacaaa 7080
   taggggttcc gcgcacattt ccccgaaaag tgccacctga cgtctaagaa accattatta 7140
   tcatgacatt aacctataaa aataggcgta tcacgaggcc ctttcgtc              7188
<210> 3
   <211> 6624
   <212> DNA
   <213> Artificial Sequence
<220>
   <223> pVR1012-GP(Z) delta MUC
<400> 3
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgtaggctt aattaccaat actattgctg gagtcgcagg 2880
   actgatcaca ggcgggagaa gaactcgaag agaagcaatt gtcaatgctc aacccaaatg 2940
   caaccctaat ttacattact ggactactca ggatgaaggt gctgcaatcg gactggcctg 3000
   gataccatat ttcgggccag cagccgaggg aatttacata gaggggctaa tgcacaatca 3060
   agatggttta atctgtgggt tgagacagct ggccaacgag acgactcaag ctcttcaact 3120
   gttcctgaga gccacaactg agctacgcac cttttcaatc ctcaaccgta aggcaattga 3180
   tttcttgctg cagcgatggg gcggcacatg ccacattctg ggaccggact gctgtatcga 3240
   accacatgat tggaccaaga acataacaga caaaattgat cagattattc atgattttgt 3300
   tgataaaacc cttccggacc agggggacaa tgacaattgg tggacaggat ggagacaatg 3360
   gataccggca ggtattggag ttacaggcgt tgtaattgca gttatcgctt tattctgtat 3420
   atgcaaattt gtcttttagt ttttcttcag attgcttcat ggaaaagctc agcctcaaat 3480
   caatgaaacc aggatttaat tatatggatt acttgaatct aagattactt gacaaatgat 3540
   aatataatac actggagctt taaacatagc caatgtgatt ctaactcctt taaactcaca 3600
   gttaatcata aacaaggttt gaggtaccga gctcgaattg atctgctgtg ccttctagtt 3660
   gccagccatc tgttgtttgc ccctcccccg tgccttcctt gaccctggaa ggtgccactc 3720
   ccactgtcct ttcctaataa aatgaggaaa ttgcatcgca ttgtctgagt aggtgtcatt 3780
   ctattctggg gggtggggtg gggcaggaca gcaaggggga ggattgggaa gacaatagca 3840
   ggcatgctgg ggatgcggtg ggctctatgg gtacccaggt gctgaagaat tgacccggtt 3900
   cctcctgggc cagaaagaag caggcacatc cccttctctg tgacacaccc tgtccacgcc 3960
   cctggttctt agttccagcc ccactcatag gacactcata gctcaggagg gctccgcctt 4020
   caatcccacc cgctaaagta cttggagcgg tctctccctc cctcatcagc ccaccaaacc 4080
   aaacctagcc tccaagagtg ggaagaaatt aaagcaagat aggctattaa gtgcagaggg 4140
   agagaaaatg cctccaacat gtgaggaagt aatgagagaa atcatagaat ttcttccgct 4200
   tcctcgctca ctgactcgct gcgctcggtc gttcggctgc ggcgagcggt atcagctcac 4260
   tcaaaggcgg taatacggtt atccacagaa tcaggggata acgcaggaaa gaacatgtga 4320
   gcaaaaggcc agcaaaaggc caggaaccgt aaaaaggccg cgttgctggc gtttttccat 4380
   aggctccgcc cccctgacga gcatcacaaa aatcgacgct caagtcagag gtggcgaaac 4440
   ccgacaggac tataaagata ccaggcgttt ccccctggaa gctccctcgt gcgctctcct 4500
   gttccgaccc tgccgcttac cggatacctg tccgcctttc tcccttcggg aagcgtggcg 4560
   ctttctcata gctcacgctg taggtatctc agttcggtgt aggtcgttcg ctccaagctg 4620
   ggctgtgtgc acgaaccccc cgttcagccc gaccgctgcg ccttatccgg taactatcgt 4680
   cttgagtcca acccggtaag acacgactta tcgccactgg cagcagccac tggtaacagg 4740
   attagcagag cgaggtatgt aggcggtgct acagagttct tgaagtggtg gcctaactac 4800
   ggctacacta gaagaacagt atttggtatc tgcgctctgc tgaagccagt taccttcgga 4860
   aaaagagttg gtagctcttg atccggcaaa caaaccaccg ctggtagcgg tggttttttt 4920
   gtttgcaagc agcagattac gcgcagaaaa aaaggatctc aagaagatcc tttgatcttt 4980
   tctacggggt ctgacgctca gtggaacgaa aactcacgtt aagggatttt ggtcatgaga 5040
   ttatcaaaaa ggatcttcac ctagatcctt ttaaattaaa aatgaagttt taaatcaatc 5100
   taaagtatat atgagtaaac ttggtctgac agttaccaat gcttaatcag tgaggcacct 5160
   atctcagcga tctgtctatt tcgttcatcc atagttgcct gactcggggg gggggggcgc 5220
   tgaggtctgc ctcgtgaaga aggtgttgct gactcatacc aggcctgaat cgccccatca 5280
   tccagccaga aagtgaggga gccacggttg atgagagctt tgttgtaggt ggaccagttg 5340
   gtgattttga acttttgctt tgccacggaa cggtctgcgt tgtcgggaag atgcgtgatc 5400
   tgatccttca actcagcaaa agttcgattt attcaacaaa gccgccgtcc cgtcaagtca 5460
   gcgtaatgct ctgccagtgt tacaaccaat taaccaattc tgattagaaa aactcatcga 5520
   gcatcaaatg aaactgcaat ttattcatat caggattatc aataccatat ttttgaaaaa 5580
   gccgtttctg taatgaagga gaaaactcac cgaggcagtt ccataggatg gcaagatcct 5640
   ggtatcggtc tgcgattccg actcgtccaa catcaataca acctattaat ttcccctcgt 5700
   caaaaataag gttatcaagt gagaaatcac catgagtgac gactgaatcc ggtgagaatg 5760
   gcaaaagctt atgcatttct ttccagactt gttcaacagg ccagccatta cgctcgtcat 5820
   caaaatcact cgcatcaacc aaaccgttat tcattcgtga ttgcgcctga gcgagacgaa 5880
   atacgcgatc gctgttaaaa ggacaattac aaacaggaat cgaatgcaac cggcgcagga 5940
   acactgccag cgcatcaaca atattttcac ctgaatcagg atattcttct aatacctgga 6000
   atgctgtttt cccggggatc gcagtggtga gtaaccatgc atcatcagga gtacggataa 6060
   aatgcttgat ggtcggaaga ggcataaatt ccgtcagcca gtttagtctg accatctcat 6120
   ctgtaacatc attggcaacg ctacctttgc catgtttcag aaacaactct ggcgcatcgg 6180
   gcttcccata caatcgatag attgtcgcac ctgattgccc gacattatcg cgagcccatt 6240
   tatacccata taaatcagca tccatgttgg aatttaatcg cggcctcgag caagacgttt 6300
   cccgttgaat atggctcata acaccccttg tattactgtt tatgtaagca gacagtttta 6360
   ttgttcatga tgatatattt ttatcttgtg caatgtaaca tcagagattt tgagacacaa 6420
   cgtggctttc cccccccccc cattattgaa gcatttatca gggttattgt ctcatgagcg 6480
   gatacatatt tgaatgtatt tagaaaaata aacaaatagg ggttccgcgc acatttcccc 6540
   gaaaagtgcc acctgacgtc taagaaacca ttattatcat gacattaacc tataaaaata 6600
   ggcgtatcac gaggcccttt cgtc 6624
<210> 4
   <211> 6561
   <212> DNA
   <213> Artificial Sequence
<220>
   <223> pVR1012-GP(Z) deltaMUC delta FUR
<400> 4
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcggaagaga agcaattgtc aatgctcaac ccaaatgcaa 2880
   ccctaattta cattactgga ctactcagga tgaaggtgct gcaatcggac tggcctggat 2940
   accatatttc gggccagcag ccgagggaat ttacatagag gggctaatgc acaatcaaga 3000
   tggtttaatc tgtgggttga gacagctggc caacgagacg actcaagctc ttcaactgtt 3060
   cctgagagcc acaactgagc tacgcacctt ttcaatcctc aaccgtaagg caattgattt 3120
   cttgctgcag cgatggggcg gcacatgcca cattctggga ccggactgct gtatcgaacc 3180
   acatgattgg accaagaaca taacagacaa aattgatcag attattcatg attttgttga 3240
   taaaaccctt ccggaccagg gggacaatga caattggtgg acaggatgga gacaatggat 3300
   accggcaggt attggagtta caggcgttgt aattgcagtt atcgctttat tctgtatatg 3360
   caaatttgtc ttttagtttt tcttcagatt gcttcatgga aaagctcagc ctcaaatcaa 3420
   tgaaaccagg atttaattat atggattact tgaatctaag attacttgac aaatgataat 3480
   ataatacact ggagctttaa acatagccaa tgtgattcta actcctttaa actcacagtt 3540
   aatcataaac aaggtttgag gtaccgagct cgaattgatc tgctgtgcct tctagttgcc 3600
   agccatctgt tgtttgcccc tcccccgtgc cttccttgac cctggaaggt gccactccca 3660
   ctgtcctttc ctaataaaat gaggaaattg catcgcattg tctgagtagg tgtcattcta 3720
   ttctgggggg tggggtgggg caggacagca agggggagga ttgggaagac aatagcaggc 3780
   atgctgggga tgcggtgggc tctatgggta cccaggtgct gaagaattga cccggttcct 3840
   cctgggccag aaagaagcag gcacatcccc ttctctgtga cacaccctgt ccacgcccct 3900
   ggttcttagt tccagcccca ctcataggac actcatagct caggagggct ccgccttcaa 3960
   tcccacccgc taaagtactt ggagcggtct ctccctccct catcagccca ccaaaccaaa 4020
   cctagcctcc aagagtggga agaaattaaa gcaagatagg ctattaagtg cagagggaga 4080
   gaaaatgcct ccaacatgtg aggaagtaat gagagaaatc atagaatttc ttccgcttcc 4140
   tcgctcactg actcgctgcg ctcggtcgtt cggctgcggc gagcggtatc agctcactca 4200
   aaggcggtaa tacggttatc cacagaatca ggggataacg caggaaagaa catgtgagca 4260
   aaaggccagc aaaaggccag gaaccgtaaa aaggccgcgt tgctggcgtt tttccatagg 4320
   ctccgccccc ctgacgagca tcacaaaaat cgacgctcaa gtcagaggtg gcgaaacccg 4380
   acaggactat aaagatacca ggcgtttccc cctggaagct ccctcgtgcg ctctcctgtt 4440
   ccgaccctgc cgcttaccgg atacctgtcc gcctttctcc cttcgggaag cgtggcgctt 4500
   tctcatagct cacgctgtag gtatctcagt tcggtgtagg tcgttcgctc caagctgggc 4560
   tgtgtgcacg aaccccccgt tcagcccgac cgctgcgcct tatccggtaa ctatcgtctt 4620
   gagtccaacc cggtaagaca cgacttatcg ccactggcag cagccactgg taacaggatt 4680
   agcagagcga ggtatgtagg cggtgctaca gagttcttga agtggtggcc taactacggc 4740
   tacactagaa gaacagtatt tggtatctgc gctctgctga agccagttac cttcggaaaa 4800
   agagttggta gctcttgatc cggcaaacaa accaccgctg gtagcggtgg tttttttgtt 4860
   tgcaagcagc agattacgcg cagaaaaaaa ggatctcaag aagatccttt gatcttttct 4920
   acggggtctg acgctcagtg gaacgaaaac tcacgttaag ggattttggt catgagatta 4980
   tcaaaaagga tcttcaccta gatcctttta aattaaaaat gaagttttaa atcaatctaa 5040
   agtatatatg agtaaacttg gtctgacagt taccaatgct taatcagtga ggcacctatc 5100
   tcagcgatct gtctatttcg ttcatccata gttgcctgac tcgggggggg ggggcgctga 5160
   ggtctgcctc gtgaagaagg tgttgctgac tcataccagg cctgaatcgc cccatcatcc 5220
   agccagaaag tgagggagcc acggttgatg agagctttgt tgtaggtgga ccagttggtg 5280
   attttgaact tttgctttgc cacggaacgg tctgcgttgt cgggaagatg cgtgatctga 5340
   tccttcaact cagcaaaagt tcgatttatt caacaaagcc gccgtcccgt caagtcagcg 5400
   taatgctctg ccagtgttac aaccaattaa ccaattctga ttagaaaaac tcatcgagca 5460
   tcaaatgaaa ctgcaattta ttcatatcag gattatcaat accatatttt tgaaaaagcc 5520
   gtttctgtaa tgaaggagaa aactcaccga ggcagttcca taggatggca agatcctggt 5580
   atcggtctgc gattccgact cgtccaacat caatacaacc tattaatttc ccctcgtcaa 5640
   aaataaggtt atcaagtgag aaatcaccat gagtgacgac tgaatccggt gagaatggca 5700
   aaagcttatg catttctttc cagacttgtt caacaggcca gccattacgc tcgtcatcaa 5760
   aatcactcgc atcaaccaaa ccgttattca ttcgtgattg cgcctgagcg agacgaaata 5820
   cgcgatcgct gttaaaagga caattacaaa caggaatcga atgcaaccgg cgcaggaaca 5880
   ctgccagcgc atcaacaata ttttcacctg aatcaggata ttcttctaat acctggaatg 5940
   ctgttttccc ggggatcgca gtggtgagta accatgcatc atcaggagta cggataaaat 6000
   gcttgatggt cggaagaggc ataaattccg tcagccagtt tagtctgacc atctcatctg 6060
   taacatcatt ggcaacgcta cctttgccat gtttcagaaa caactctggc gcatcgggct 6120
   tcccatacaa tcgatagatt gtcgcacctg attgcccgac attatcgcga gcccatttat 6180
   acccatataa atcagcatcc atgttggaat ttaatcgcgg cctcgagcaa gacgtttccc 6240
   gttgaatatg gctcataaca ccccttgtat tactgtttat gtaagcagac agttttattg 6300
   ttcatgatga tatattttta tcttgtgcaa tgtaacatca gagattttga gacacaacgt 6360
   ggctttcccc ccccccccat tattgaagca tttatcaggg ttattgtctc atgagcggat 6420
   acatatttga atgtatttag aaaaataaac aaataggggt tccgcgcaca tttccccgaa 6480
   aagtgccacc tgacgtctaa gaaaccatta ttatcatgac attaacctat aaaaataggc 6540
   gtatcacgag gccctttcgt c 6561
<210> 5
   <211> 6724
   <212> DNA
   <213> Artificial Sequence
<220>
   <223> pVR1012-GP(Z) delta GP2
<400> 5
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actccggacc 3420
   agggggacaa tgacaattgg tggacaggat ggagacaatg gataccggca ggtattggag 3480
   ttacaggcgt tgtaattgca gttatcgctt tattctgtat atgcaaattt gtcttttagt 3540
   ttttcttcag attgcttcat ggaaaagctc agcctcaaat caatgaaacc aggatttaat 3600
   tatatggatt acttgaatct aagattactt gacaaatgat aatataatac actggagctt 3660
   taaacatagc caatgtgatt ctaactcctt taaactcaca gttaatcata aacaaggttt 3720
   gaggtaccga gctcgaattg atctgctgtg ccttctagtt gccagccatc tgttgtttgc 3780
   ccctcccccg tgccttcctt gaccctggaa ggtgccactc ccactgtcct ttcctaataa 3840
   aatgaggaaa ttgcatcgca ttgtctgagt aggtgtcatt ctattctggg gggtggggtg 3900
   gggcaggaca gcaaggggga ggattgggaa gacaatagca ggcatgctgg ggatgcggtg 3960
   ggctctatgg gtacccaggt gctgaagaat tgacccggtt cctcctgggc cagaaagaag 4020
   caggcacatc cccttctctg tgacacaccc tgtccacgcc cctggttctt agttccagcc 4080
   ccactcatag gacactcata gctcaggagg gctccgcctt caatcccacc cgctaaagta 4140
   cttggagcgg tctctccctc cctcatcagc ccaccaaacc aaacctagcc tccaagagtg 4200
   ggaagaaatt aaagcaagat aggctattaa gtgcagaggg agagaaaatg cctccaacat 4260
   gtgaggaagt aatgagagaa atcatagaat ttcttccgct tcctcgctca ctgactcgct 4320
   gcgctcggtc gttcggctgc ggcgagcggt atcagctcac tcaaaggcgg taatacggtt 4380
   atccacagaa tcaggggata acgcaggaaa gaacatgtga gcaaaaggcc agcaaaaggc 4440
   caggaaccgt aaaaaggccg cgttgctggc gtttttccat aggctccgcc cccctgacga 4500
   gcatcacaaa aatcgacgct caagtcagag gtggcgaaac ccgacaggac tataaagata 4560
   ccaggcgttt ccccctggaa gctccctcgt gcgctctcct gttccgaccc tgccgcttac 4620
   cggatacctg tccgcctttc tcccttcggg aagcgtggcg ctttctcata gctcacgctg 4680
   taggtatctc agttcggtgt aggtcgttcg ctccaagctg ggctgtgtgc acgaaccccc 4740
   cgttcagccc gaccgctgcg ccttatccgg taactatcgt cttgagtcca acccggtaag 4800
   acacgactta tcgccactgg cagcagccac tggtaacagg attagcagag cgaggtatgt 4860
   aggcggtgct acagagttct tgaagtggtg gcctaactac ggctacacta gaagaacagt 4920
   atttggtatc tgcgctctgc tgaagccagt taccttcgga aaaagagttg gtagctcttg 4980
   atccggcaaa caaaccaccg ctggtagcgg tggttttttt gtttgcaagc agcagattac 5040
   gcgcagaaaa aaaggatctc aagaagatcc tttgatcttt tctacggggt ctgacgctca 5100
   gtggaacgaa aactcacgtt aagggatttt ggtcatgaga ttatcaaaaa ggatcttcac 5160
   ctagatcctt ttaaattaaa aatgaagttt taaatcaatc taaagtatat atgagtaaac 5220
   ttggtctgac agttaccaat gcttaatcag tgaggcacct atctcagcga tctgtctatt 5280
   tcgttcatcc atagttgcct gactcggggg gggggggcgc tgaggtctgc ctcgtgaaga 5340
   aggtgttgct gactcatacc aggcctgaat cgccccatca tccagccaga aagtgaggga 5400
   gccacggttg atgagagctt tgttgtaggt ggaccagttg gtgattttga acttttgctt 5460
   tgccacggaa cggtctgcgt tgtcgggaag atgcgtgatc tgatccttca actcagcaaa 5520
   agttcgattt attcaacaaa gccgccgtcc cgtcaagtca gcgtaatgct ctgccagtgt 5580
   tacaaccaat taaccaattc tgattagaaa aactcatcga gcatcaaatg aaactgcaat 5640
   ttattcatat caggattatc aataccatat ttttgaaaaa gccgtttctg taatgaagga 5700
   gaaaactcac cgaggcagtt ccataggatg gcaagatcct ggtatcggtc tgcgattccg 5760
   actcgtccaa catcaataca acctattaat ttcccctcgt caaaaataag gttatcaagt 5820
   gagaaatcac catgagtgac gactgaatcc ggtgagaatg gcaaaagctt atgcatttct 5880
   ttccagactt gttcaacagg ccagccatta cgctcgtcat caaaatcact cgcatcaacc 5940
   aaaccgttat tcattcgtga ttgcgcctga gcgagacgaa atacgcgatc gctgttaaaa 6000
   ggacaattac aaacaggaat cgaatgcaac cggcgcagga acactgccag cgcatcaaca 6060
   atattttcac ctgaatcagg atattcttct aatacctgga atgctgtttt cccggggatc 6120
   gcagtggtga gtaaccatgc atcatcagga gtacggataa aatgcttgat ggtcggaaga 6180
   ggcataaatt ccgtcagcca gtttagtctg accatctcat ctgtaacatc attggcaacg 6240
   ctacctttgc catgtttcag aaacaactct ggcgcatcgg gcttcccata caatcgatag 6300
   attgtcgcac ctgattgccc gacattatcg cgagcccatt tatacccata taaatcagca 6360
   tccatgttgg aatttaatcg cggcctcgag caagacgttt cccgttgaat atggctcata 6420
   acaccccttg tattactgtt tatgtaagca gacagtttta ttgttcatga tgatatattt 6480
   ttatcttgtg caatgtaaca tcagagattt tgagacacaa cgtggctttc cccccccccc 6540
   cattattgaa gcatttatca gggttattgt ctcatgagcg gatacatatt tgaatgtatt 6600
   tagaaaaata aacaaatagg ggttccgcgc acatttcccc gaaaagtgcc acctgacgtc 6660
   taagaaacca ttattatcat gacattaacc tataaaaata ggcgtatcac gaggcccttt 6720
   cgtc                                                              6724
<210> 6
   <211> 6887
   <212> DNA
   <213> Artificial Sequence
<220>
   <223> pVR1012-GP(Z) delta GP2 delta C-term A
<400> 6
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaaggt gctgcaatcg gactggcctg gataccatat 3540
   ttcgggccag cagccgaggg aatttacata gaggggctaa tgcacaatca agatggttta 3600
   atctgtgggt tgagacagct ggggataccg gcaggtattg gagttacagg cgttgtaatt 3660
   gcagttatcg ctttattctg tatatgcaaa tttgtctttt agtttttctt cagattgctt 3720
   catggaaaag ctcagcctca aatcaatgaa accaggattt aattatatgg attacttgaa 3780
   tctaagatta cttgacaaat gataatataa tacactggag ctttaaacat agccaatgtg 3840
   attctaactc ctttaaactc acagttaatc ataaacaagg tttgaggtac cgagctcgaa 3900
   ttgatctgct gtgccttcta gttgccagcc atctgttgtt tgcccctccc ccgtgccttc 3960
   cttgaccctg gaaggtgcca ctcccactgt cctttcctaa taaaatgagg aaattgcatc 4020
   gcattgtctg agtaggtgtc attctattct ggggggtggg gtggggcagg acagcaaggg 4080
   ggaggattgg gaagacaata gcaggcatgc tggggatgcg gtgggctcta tgggtaccca 4140
   ggtgctgaag aattgacccg gttcctcctg ggccagaaag aagcaggcac atccccttct 4200
   ctgtgacaca ccctgtccac gcccctggtt cttagttcca gccccactca taggacactc 4260
   atagctcagg agggctccgc cttcaatccc acccgctaaa gtacttggag cggtctctcc 4320
   ctccctcatc agcccaccaa accaaaccta gcctccaaga gtgggaagaa attaaagcaa 4380
   gataggctat taagtgcaga gggagagaaa atgcctccaa catgtgagga agtaatgaga 4440
   gaaatcatag aatttcttcc gcttcctcgc tcactgactc gctgcgctcg gtcgttcggc 4500
   tgcggcgagc ggtatcagct cactcaaagg cggtaatacg gttatccaca gaatcagggg 4560
   ataacgcagg aaagaacatg tgagcaaaag gccagcaaaa ggccaggaac cgtaaaaagg 4620
   ccgcgttgct ggcgtttttc cataggctcc gcccccctga cgagcatcac aaaaatcgac 4680
   gctcaagtca gaggtggcga aacccgacag gactataaag ataccaggcg tttccccctg 4740
   gaagctccct cgtgcgctct cctgttccga ccctgccgct taccggatac ctgtccgcct 4800
   ttctcccttc gggaagcgtg gcgctttctc atagctcacg ctgtaggtat ctcagttcgg 4860
   tgtaggtcgt tcgctccaag ctgggctgtg tgcacgaacc ccccgttcag cccgaccgct 4920
   gcgccttatc cggtaactat cgtcttgagt ccaacccggt aagacacgac ttatcgccac 4980
   tggcagcagc cactggtaac aggattagca gagcgaggta tgtaggcggt gctacagagt 5040
   tcttgaagtg gtggcctaac tacggctaca ctagaagaac agtatttggt atctgcgctc 5100
   tgctgaagcc agttaccttc ggaaaaagag ttggtagctc ttgatccggc aaacaaacca 5160
   ccgctggtag cggtggtttt tttgtttgca agcagcagat tacgcgcaga aaaaaaggat 5220
   ctcaagaaga tcctttgatc ttttctacgg ggtctgacgc tcagtggaac gaaaactcac 5280
   gttaagggat tttggtcatg agattatcaa aaaggatctt cacctagatc cttttaaatt 5340
   aaaaatgaag ttttaaatca atctaaagta tatatgagta aacttggtct gacagttacc 5400
   aatgcttaat cagtgaggca cctatctcag cgatctgtct atttcgttca tccatagttg 5460
   cctgactcgg gggggggggg cgctgaggtc tgcctcgtga agaaggtgtt gctgactcat 5520
   accaggcctg aatcgcccca tcatccagcc agaaagtgag ggagccacgg ttgatgagag 5580
   ctttgttgta ggtggaccag ttggtgattt tgaacttttg ctttgccacg gaacggtctg 5640
   cgttgtcggg aagatgcgtg atctgatcct tcaactcagc aaaagttcga tttattcaac 5700
   aaagccgccg tcccgtcaag tcagcgtaat gctctgccag tgttacaacc aattaaccaa 5760
   ttctgattag aaaaactcat cgagcatcaa atgaaactgc aatttattca tatcaggatt 5820
   atcaatacca tatttttgaa aaagccgttt ctgtaatgaa ggagaaaact caccgaggca 5880
   gttccatagg atggcaagat cctggtatcg gtctgcgatt ccgactcgtc caacatcaat 5940
   acaacctatt aatttcccct cgtcaaaaat aaggttatca agtgagaaat caccatgagt 6000
   gacgactgaa tccggtgaga atggcaaaag cttatgcatt tctttccaga cttgttcaac 6060
   aggccagcca ttacgctcgt catcaaaatc actcgcatca accaaaccgt tattcattcg 6120
   tgattgcgcc tgagcgagac gaaatacgcg atcgctgtta aaaggacaat tacaaacagg 6180
   aatcgaatgc aaccggcgca ggaacactgc cagcgcatca acaatatttt cacctgaatc 6240
   aggatattct tctaatacct ggaatgctgt tttcccgggg atcgcagtgg tgagtaacca 6300
   tgcatcatca ggagtacgga taaaatgctt gatggtcgga agaggcataa attccgtcag 6360
   ccagtttagt ctgaccatct catctgtaac atcattggca acgctacctt tgccatgttt 6420
   cagaaacaac tctggcgcat cgggcttccc atacaatcga tagattgtcg cacctgattg 6480
   cccgacatta tcgcgagccc atttataccc atataaatca gcatccatgt tggaatttaa 6540
   tcgcggcctc gagcaagacg tttcccgttg aatatggctc ataacacccc ttgtattact 6600
   gtttatgtaa gcagacagtt ttattgttca tgatgatata tttttatctt gtgcaatgta 6660
   acatcagaga ttttgagaca caacgtggct ttcccccccc ccccattatt gaagcattta 6720
   tcagggttat tgtctcatga gcggatacat atttgaatgt atttagaaaa ataaacaaat 6780
   aggggttccg cgcacatttc cccgaaaagt gccacctgac gtctaagaaa ccattattat 6840
   catgacatta acctataaaa ataggcgtat cacgaggccc tttcgtc              6887
<210> 7
   <211> 7044
   <212> DNA
   <213> Artificial Sequence
<220>
   <223> pVR1012-GP(Z) delta GP2 Delta C-term B
<400> 7
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaaggt gctgcaatcg gactggcctg gataccatat 3540
   ttcgggccag cagccgaggg aatttacata gaggggctaa tgcacaatca agatggttta 3600
   atctgtgggt tgagacagct ggccaacgag acgactcaag ctcttcaact gttcctgaga 3660
   gccacaactg agctacgcac cttttcaatc ctcaaccgta aggcaattga tttcttgctg 3720
   cagcgatggg gcggcacatg ccacattctg ggaccggact gctgtatcga accacatgag 3780
   gataccggca ggtattggag ttacaggcgt tgtaattgca gttatcgctt tattctgtat 3840
   atgcaaattt gtcttttagt ttttcttcag attgcttcat ggaaaagctc agcctcaaat 3900
   caatgaaacc aggatttaat tatatggatt acttgaatct aagattactt gacaaatgat 3960
   aatataatac actggagctt taaacatagc caatgtgatt ctaactcctt taaactcaca 4020
   gttaatcata aacaaggttt gaggtaccga gctcgaattg atctgctgtg ccttctagtt 4080
   gccagccatc tgttgtttgc ccctcccccg tgccttcctt gaccctggaa ggtgccactc 4140
   ccactgtcct ttcctaataa aatgaggaaa ttgcatcgca ttgtctgagt aggtgtcatt 4200
   ctattctggg gggtggggtg gggcaggaca gcaaggggga ggattgggaa gacaatagca 4260
   ggcatgctgg ggatgcggtg ggctctatgg gtacccaggt gctgaagaat tgacccggtt 4320
   cctcctgggc cagaaagaag caggcacatc cccttctctg tgacacaccc tgtccacgcc 4380
   cctggttctt agttccagcc ccactcatag gacactcata gctcaggagg gctccgcctt 4440
   caatcccacc cgctaaagta cttggagcgg tctctccctc cctcatcagc ccaccaaacc 4500
   aaacctagcc tccaagagtg ggaagaaatt aaagcaagat aggctattaa gtgcagaggg 4560
   agagaaaatg cctccaacat gtgaggaagt aatgagagaa atcatagaat ttcttccgct 4620
   tcctcgctca ctgactcgct gcgctcggtc gttcggctgc ggcgagcggt atcagctcac 4680
   tcaaaggcgg taatacggtt atccacagaa tcaggggata acgcaggaaa gaacatgtga 4740
   gcaaaaggcc agcaaaaggc caggaaccgt aaaaaggccg cgttgctggc gtttttccat 4800
   aggctccgcc cccctgacga gcatcacaaa aatcgacgct caagtcagag gtggcgaaac 4860
   ccgacaggac tataaagata ccaggcgttt ccccctggaa gctccctcgt gcgctctcct 4920
   gttccgaccc tgccgcttac cggatacctg tccgcctttc tcccttcggg aagcgtggcg 4980
   ctttctcata gctcacgctg taggtatctc agttcggtgt aggtcgttcg ctccaagctg 5040
   ggctgtgtgc acgaaccccc cgttcagccc gaccgctgcg ccttatccgg taactatcgt 5100
   cttgagtcca acccggtaag acacgactta tcgccactgg cagcagccac tggtaacagg 5160
   attagcagag cgaggtatgt aggcggtgct acagagttct tgaagtggtg gcctaactac 5220
   ggctacacta gaagaacagt atttggtatc tgcgctctgc tgaagccagt taccttcgga 5280
   aaaagagttg gtagctcttg atccggcaaa caaaccaccg ctggtagcgg tggttttttt 5340
   gtttgcaagc agcagattac gcgcagaaaa aaaggatctc aagaagatcc tttgatcttt 5400
   tctacggggt ctgacgctca gtggaacgaa aactcacgtt aagggatttt ggtcatgaga 5460
   ttatcaaaaa ggatcttcac ctagatcctt ttaaattaaa aatgaagttt taaatcaatc 5520
   taaagtatat atgagtaaac ttggtctgac agttaccaat gcttaatcag tgaggcacct 5580
   atctcagcga tctgtctatt tcgttcatcc atagttgcct gactcggggg gggggggcgc 5640
   tgaggtctgc ctcgtgaaga aggtgttgct gactcatacc aggcctgaat cgccccatca 5700
   tccagccaga aagtgaggga gccacggttg atgagagctt tgttgtaggt ggaccagttg 5760
   gtgattttga acttttgctt tgccacggaa cggtctgcgt tgtcgggaag atgcgtgatc 5820
   tgatccttca actcagcaaa agttcgattt attcaacaaa gccgccgtcc cgtcaagtca 5880
   gcgtaatgct ctgccagtgt tacaaccaat taaccaattc tgattagaaa aactcatcga 5940
   gcatcaaatg aaactgcaat ttattcatat caggattatc aataccatat ttttgaaaaa 6000
   gccgtttctg taatgaagga gaaaactcac cgaggcagtt ccataggatg gcaagatcct 6060
   ggtatcggtc tgcgattccg actcgtccaa catcaataca acctattaat ttcccctcgt 6120
   caaaaataag gttatcaagt gagaaatcac catgagtgac gactgaatcc ggtgagaatg 6180
   gcaaaagctt atgcatttct ttccagactt gttcaacagg ccagccatta cgctcgtcat 6240
   caaaatcact cgcatcaacc aaaccgttat tcattcgtga ttgcgcctga gcgagacgaa 6300
   atacgcgatc gctgttaaaa ggacaattac aaacaggaat cgaatgcaac cggcgcagga 6360
   acactgccag cgcatcaaca atattttcac ctgaatcagg atattcttct aatacctgga 6420
   atgctgtttt cccggggatc gcagtggtga gtaaccatgc atcatcagga gtacggataa 6480
   aatgcttgat ggtcggaaga ggcataaatt ccgtcagcca gtttagtctg accatctcat 6540
   ctgtaacatc attggcaacg ctacctttgc catgtttcag aaacaactct ggcgcatcgg 6600
   gcttcccata caatcgatag attgtcgcac ctgattgccc gacattatcg cgagcccatt 6660
   tatacccata taaatcagca tccatgttgg aatttaatcg cggcctcgag caagacgttt 6720
   cccgttgaat atggctcata acaccccttg tattactgtt tatgtaagca gacagtttta 6780
   ttgttcatga tgatatattt ttatcttgtg caatgtaaca tcagagattt tgagacacaa 6840
   cgtggctttc cccccccccc cattattgaa gcatttatca gggttattgt ctcatgagcg 6900
   gatacatatt tgaatgtatt tagaaaaata aacaaatagg ggttccgcgc acatttcccc 6960
   gaaaagtgcc acctgacgtc taagaaacca ttattatcat gacattaacc tataaaaata 7020
   ggcgtatcac gaggcccttt cgtc                                        7044
<210> 8
   <211> 7106
   <212> DNA
   <213> Artificial Sequence
<220>
   <223> pVR1012-GP(Z) delta GP2 delta FUS
<400> 8
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaagag ggaatttaca tagaggggct aatgcacaat 3540
   caagatggtt taatctgtgg gttgagacag ctggccaacg agacgactca agctcttcaa 3600
   ctgttcctga gagccacaac tgagctacgc accttttcaa tcctcaaccg taaggcaatt 3660
   gatttcttgc tgcagcgatg gggcggcaca tgccacattc tgggaccgga ctgctgtatc 3720
   gaaccacatg attggaccaa gaacataaca gacaaaattg atcagattat tcatgatttt 3780
   gttgataaaa cccttccgga ccagggggac aatgacaatt ggtggacagg atggagacaa 3840
   tggataccgg caggtattgg agttacaggc gttgtaattg cagttatcgc tttattctgt 3900
   atatgcaaat ttgtctttta gtttttcttc agattgcttc atggaaaagc tcagcctcaa 3960
   atcaatgaaa ccaggattta attatatgga ttacttgaat ctaagattac ttgacaaatg 4020
   ataatataat acactggagc tttaaacata gccaatgtga ttctaactcc tttaaactca 4080
   cagttaatca taaacaaggt ttgaggtacc gagctcgaat tgatctgctg tgccttctag 4140
   ttgccagcca tctgttgttt gcccctcccc cgtgccttcc ttgaccctgg aaggtgccac 4200
   tcccactgtc ctttcctaat aaaatgagga aattgcatcg cattgtctga gtaggtgtca 4260
   ttctattctg gggggtgggg tggggcagga cagcaagggg gaggattggg aagacaatag 4320
   caggcatgct ggggatgcgg tgggctctat gggtacccag gtgctgaaga attgacccgg 4380
   ttcctcctgg gccagaaaga agcaggcaca tccccttctc tgtgacacac cctgtccacg 4440
   cccctggttc ttagttccag ccccactcat aggacactca tagctcagga gggctccgcc 4500
   ttcaatccca cccgctaaag tacttggagc ggtctctccc tccctcatca gcccaccaaa 4560
   ccaaacctag cctccaagag tgggaagaaa ttaaagcaag ataggctatt aagtgcagag 4620
   ggagagaaaa tgcctccaac atgtgaggaa gtaatgagag aaatcataga atttcttccg 4680
   cttcctcgct cactgactcg ctgcgctcgg tcgttcggct gcggcgagcg gtatcagctc 4740
   actcaaaggc ggtaatacgg ttatccacag aatcagggga taacgcagga aagaacatgt 4800
   gagcaaaagg ccagcaaaag gccaggaacc gtaaaaaggc cgcgttgctg gcgtttttcc 4860
   ataggctccg cccccctgac gagcatcaca aaaatcgacg ctcaagtcag aggtggcgaa 4920
   acccgacagg actataaaga taccaggcgt ttccccctgg aagctccctc gtgcgctctc 4980
   ctgttccgac cctgccgctt accggatacc tgtccgcctt tctcccttcg ggaagcgtgg 5040
   cgctttctca tagctcacgc tgtaggtatc tcagttcggt gtaggtcgtt cgctccaagc 5100
   tgggctgtgt gcacgaaccc cccgttcagc ccgaccgctg cgccttatcc ggtaactatc 5160
   gtcttgagtc caacccggta agacacgact tatcgccact ggcagcagcc actggtaaca 5220
   ggattagcag agcgaggtat gtaggcggtg ctacagagtt cttgaagtgg tggcctaact 5280
   acggctacac tagaagaaca gtatttggta tctgcgctct gctgaagcca gttaccttcg 5340
   gaaaaagagt tggtagctct tgatccggca aacaaaccac cgctggtagc ggtggttttt 5400
   ttgtttgcaa gcagcagatt acgcgcagaa aaaaaggatc tcaagaagat cctttgatct 5460
   tttctacggg gtctgacgct cagtggaacg aaaactcacg ttaagggatt ttggtcatga 5520
   gattatcaaa aaggatcttc acctagatcc ttttaaatta aaaatgaagt tttaaatcaa 5580
   tctaaagtat atatgagtaa acttggtctg acagttacca atgcttaatc agtgaggcac 5640
   ctatctcagc gatctgtcta tttcgttcat ccatagttgc ctgactcggg gggggggggc 5700
   gctgaggtct gcctcgtgaa gaaggtgttg ctgactcata ccaggcctga atcgccccat 5760
   catccagcca gaaagtgagg gagccacggt tgatgagagc tttgttgtag gtggaccagt 5820
   tggtgatttt gaacttttgc tttgccacgg aacggtctgc gttgtcggga agatgcgtga 5880
   tctgatcctt caactcagca aaagttcgat ttattcaaca aagccgccgt cccgtcaagt 5940
   cagcgtaatg ctctgccagt gttacaacca attaaccaat tctgattaga aaaactcatc 6000
   gagcatcaaa tgaaactgca atttattcat atcaggatta tcaataccat atttttgaaa 6060
   aagccgtttc tgtaatgaag gagaaaactc accgaggcag ttccatagga tggcaagatc 6120
   ctggtatcgg tctgcgattc cgactcgtcc aacatcaata caacctatta atttcccctc 6180
   gtcaaaaata aggttatcaa gtgagaaatc accatgagtg acgactgaat ccggtgagaa 6240
   tggcaaaagc ttatgcattt ctttccagac ttgttcaaca ggccagccat tacgctcgtc 6300
   atcaaaatca ctcgcatcaa ccaaaccgtt attcattcgt gattgcgcct gagcgagacg 6360
   aaatacgcga tcgctgttaa aaggacaatt acaaacagga atcgaatgca accggcgcag 6420
   gaacactgcc agcgcatcaa caatattttc acctgaatca ggatattctt ctaatacctg 6480
   gaatgctgtt ttcccgggga tcgcagtggt gagtaaccat gcatcatcag gagtacggat 6540
   aaaatgcttg atggtcggaa gaggcataaa ttccgtcagc cagtttagtc tgaccatctc 6600
   atctgtaaca tcattggcaa cgctaccttt gccatgtttc agaaacaact ctggcgcatc 6660
   gggcttccca tacaatcgat agattgtcgc acctgattgc ccgacattat cgcgagccca 6720
   tttataccca tataaatcag catccatgtt ggaatttaat cgcggcctcg agcaagacgt 6780
   ttcccgttga atatggctca taacacccct tgtattactg tttatgtaag cagacagttt 6840
   tattgttcat gatgatatat ttttatcttg tgcaatgtaa catcagagat tttgagacac 6900
   aacgtggctt tccccccccc cccattattg aagcatttat cagggttatt gtctcatgag 6960
   cggatacata tttgaatgta tttagaaaaa taaacaaata ggggttccgc gcacatttcc 7020
   ccgaaaagtg ccacctgacg tctaagaaac cattattatc atgacattaa cctataaaaa 7080
   taggcgtatc acgaggccct ttcgtc                                      7106
<210> 9
   <211> 6914
   <212> DNA
   <213> Artificial Sequence
<220>
   <223> pVR1012-GP(Z) delta TM
<400> 9
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcgacaaact agtttgtcgt 2100
   gacaaactgt catccacaaa tcaattgaga tcagttggac tgaatctcga agggaatgga 2160
   gtggcaactg acgtgccatc tgcaactaaa agatggggct tcaggtccgg tgtcccacca 2220
   aaggtggtca attatgaagc tggtgaatgg gctgaaaact gctacaatct tgaaatcaaa 2280
   aaacctgacg ggagtgagtg tctaccagca gcgccagacg ggattcgggg cttcccccgg 2340
   tgccggtatg tgcacaaagt atcaggaacg ggaccgtgtg ccggagactt tgccttccat 2400
   aaagagggtg ctttcttcct gtatgatcga cttgcttcca cagttatcta ccgaggaacg 2460
   actttcgctg aaggtgtcgt tgcatttctg atactgcccc aagctaagaa ggacttcttc 2520
   agctcacacc ccttgagaga gccggtcaat gcaacggagg acccgtctag tggctactat 2580
   tctaccacaa ttagatatca ggctaccggt tttggaacca atgagacaga gtacttgttc 2640
   gaggttgaca atttgaccta cgtccaactt gaatcaagat tcacaccaca gtttctgctc 2700
   cagctgaatg agacaatata tacaagtggg aaaaggagca ataccacggg aaaactaatt 2760
   tggaaggtca accccgaaat tgatacaaca atcggggagt gggccttctg ggaaactaaa 2820
   aaaaacctca ctagaaaaat tcgcagtgaa gagttgtctt tcacagttgt atcaaacgga 2880
   gccaaaaaca tcagtggtca gagtccggcg cgaacttctt ccgacccagg gaccaacaca 2940
   acaactgaag accacaaaat catggcttca gaaaattcct ctgcaatggt tcaagtgcac 3000
   agtcaaggaa gggaagctgc agtgtcgcat ctaacaaccc ttgccacaat ctccacgagt 3060
   ccccaatccc tcacaaccaa accaggtccg gacaacagca cccataatac acccgtgtat 3120
   aaacttgaca tctctgaggc aactcaagtt gaacaacatc accgcagaac agacaacgac 3180
   agcacagcct ccgacactcc ctctgccacg accgcagccg gacccccaaa agcagagaac 3240
   accaacacga gcaagagcac tgacttcctg gaccccgcca ccacaacaag tccccaaaac 3300
   cacagcgaga ccgctggcaa caacaacact catcaccaag ataccggaga agagagtgcc 3360
   agcagcggga agctaggctt aattaccaat actattgctg gagtcgcagg actgatcaca 3420
   ggcgggagaa gaactcgaag agaagcaatt gtcaatgctc aacccaaatg caaccctaat 3480
   ttacattact ggactactca ggatgaaggt gctgcaatcg gactggcctg gataccatat 3540
   ttcgggccag cagccgaggg aatttacata gaggggctaa tgcacaatca agatggttta 3600
   atctgtgggt tgagacagct ggccaacgag acgactcaag ctcttcaact gttcctgaga 3660
   gccacaactg agctacgcac cttttcaatc ctcaaccgta aggcaattga tttcttgctg 3720
   cagcgatggg gcggcacatg ccacattctg ggaccggact gctgtatcga accacatgat 3780
   tggaccaaga acataacaga caaaattgat cagattattc atgattttgt tgataaaacc 3840
   cttccggacc agggggacaa tgacaattgg tggacaggat ggagacaatg gatggccgca 3900
   tcgtgactga ctgacgatct gcctcgcgag atctgctgtg ccttctagtt gccagccatc 3960
   tgttgtttgc ccctcccccg tgccttcctt gaccctggaa ggtgccactc ccactgtcct 4020
   ttcctaataa aatgaggaaa ttgcatcgca ttgtctgagt aggtgtcatt ctattctggg 4080
   gggtggggtg gggcaggaca gcaaggggga ggattgggaa gacaatagca ggcatgctgg 4140
   ggatgcggtg ggctctatgg gtacccaggt gctgaagaat tgacccggtt cctcctgggc 4200
   cagaaagaag caggcacatc cccttctctg tgacacaccc tgtccacgcc cctggttctt 4260
   agttccagcc ccactcatag gacactcata gctcaggagg gctccgcctt caatcccacc 4320
   cgctaaagta cttggagcgg tctctccctc cctcatcagc ccaccaaacc aaacctagcc 4380
   tccaagagtg ggaagaaatt aaagcaagat aggctattaa gtgcagaggg agagaaaatg 4440
   cctccaacat gtgaggaagt aatgagagaa atcatagaat ttcttccgct tcctcgctca 4500
   ctgactcgct gcgctcggtc gttcggctgc ggcgagcggt atcagctcac tcaaaggcgg 4560
   taatacggtt atccacagaa tcaggggata acgcaggaaa gaacatgtga gcaaaaggcc 4620
   agcaaaaggc caggaaccgt aaaaaggccg cgttgctggc gtttttccat aggctccgcc 4680
   cccctgacga gcatcacaaa aatcgacgct caagtcagag gtggcgaaac ccgacaggac 4740
   tataaagata ccaggcgttt ccccctggaa gctccctcgt gcgctctcct gttccgaccc 4800
   tgccgcttac cggatacctg tccgcctttc tcccttcggg aagcgtggcg ctttctcata 4860
   gctcacgctg taggtatctc agttcggtgt aggtcgttcg ctccaagctg ggctgtgtgc 4920
   acgaaccccc cgttcagccc gaccgctgcg ccttatccgg taactatcgt cttgagtcca 4980
   acccggtaag acacgactta tcgccactgg cagcagccac tggtaacagg attagcagag 5040
   cgaggtatgt aggcggtgct acagagttct tgaagtggtg gcctaactac ggctacacta 5100
   gaagaacagt atttggtatc tgcgctctgc tgaagccagt taccttcgga aaaagagttg 5160
   gtagctcttg atccggcaaa caaaccaccg ctggtagcgg tggttttttt gtttgcaagc 5220
   agcagattac gcgcagaaaa aaaggatctc aagaagatcc tttgatcttt tctacggggt 5280
   ctgacgctca gtggaacgaa aactcacgtt aagggatttt ggtcatgaga ttatcaaaaa 5340
   ggatcttcac ctagatcctt ttaaattaaa aatgaagttt taaatcaatc taaagtatat 5400
   atgagtaaac ttggtctgac agttaccaat gcttaatcag tgaggcacct atctcagcga 5460
   tctgtctatt tcgttcatcc atagttgcct gactcggggg gggggggcgc tgaggtctgc 5520
   ctcgtgaaga aggtgttgct gactcatacc aggcctgaat cgccccatca tccagccaga 5580
   aagtgaggga gccacggttg atgagagctt tgttgtaggt ggaccagttg gtgattttga 5640
   acttttgctt tgccacggaa cggtctgcgt tgtcgggaag atgcgtgatc tgatccttca 5700
   actcagcaaa agttcgattt attcaacaaa gccgccgtcc cgtcaagtca gcgtaatgct 5760
   ctgccagtgt tacaaccaat taaccaattc tgattagaaa aactcatcga gcatcaaatg 5820
   aaactgcaat ttattcatat caggattatc aataccatat ttttgaaaaa gccgtttctg 5880
   taatgaagga gaaaactcac cgaggcagtt ccataggatg gcaagatcct ggtatcggtc 5940
   tgcgattccg actcgtccaa catcaataca acctattaat ttcccctcgt caaaaataag 6000
   gttatcaagt gagaaatcac catgagtgac gactgaatcc ggtgagaatg gcaaaagctt 6060
   atgcatttct ttccagactt gttcaacagg ccagccatta cgctcgtcat caaaatcact 6120
   cgcatcaacc aaaccgttat tcattcgtga ttgcgcctga gcgagacgaa atacgcgatc 6180
   gctgttaaaa ggacaattac aaacaggaat cgaatgcaac cggcgcagga acactgccag 6240
   cgcatcaaca atattttcac ctgaatcagg atattcttct aatacctgga atgctgtttt 6300
   cccggggatc gcagtggtga gtaaccatgc atcatcagga gtacggataa aatgcttgat 6360
   ggtcggaaga ggcataaatt ccgtcagcca gtttagtctg accatctcat ctgtaacatc 6420
   attggcaacg ctacctttgc catgtttcag aaacaactct ggcgcatcgg gcttcccata 6480
   caatcgatag attgtcgcac ctgattgccc gacattatcg cgagcccatt tatacccata 6540
   taaatcagca tccatgttgg aatttaatcg cggcctcgag caagacgttt cccgttgaat 6600
   atggctcata acaccccttg tattactgtt tatgtaagca gacagtttta ttgttcatga 6660
   tgatatattt ttatcttgtg caatgtaaca tcagagattt tgagacacaa cgtggctttc 6720
   cccccccccc cattattgaa gcatttatca gggttattgt ctcatgagcg gatacatatt 6780
   tgaatgtatt tagaaaaata aacaaatagg ggttccgcgc acatttcccc gaaaagtgcc 6840
   acctgacgtc taagaaacca ttattatcat gacattaacc tataaaaata ggcgtatcac 6900
   gaggcccttt cgtc 6914
<210> 10
   <211> 6467
   <212> DNA
   <213> Artificial Sequence
<220>
   <223> pVR1012-GP(Z) delta SGP
<400> 10
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatcgatcca acaacacaat gggcgttaca ggaatattgc agttacctcg tgatcgattc 1980
   aagaggacat cattctttct ttgggtaatt atccttttcc aaagaacatt ttccatccca 2040
   cttggagtca tccacaatag cacattacag gttagtgatg tcaaccccga aattgataca 2100
   acaatcgggg agtgggcctt ctgggaaact aaaaaaaacc tcactagaaa aattcgcagt 2160
   gaagagttgt ctttcacagt tgtatcaaac ggagccaaaa acatcagtgg tcagagtccg 2220
   gcgcgaactt cttccgaccc agggaccaac acaacaactg aagaccacaa aatcatggct 2280
   tcagaaaatt cctctgcaat ggttcaagtg cacagtcaag gaagggaagc tgcagtgtcg 2340
   catctaacaa cccttgccac aatctccacg agtccccaat ccctcacaac caaaccaggt 2400
   ccggacaaca gcacccataa tacacccgtg tataaacttg acatctctga ggcaactcaa 2460
   gttgaacaac atcaccgcag aacagacaac gacagcacag cctccgacac tccctctgcc 2520
   acgaccgcag ccggaccccc aaaagcagag aacaccaaca cgagcaagag cactgacttc 2580
   ctggaccccg ccaccacaac aagtccccaa aaccacagcg agaccgctgg caacaacaac 2640
   actcatcacc aagataccgg agaagagagt gccagcagcg ggaagctagg cttaattacc 2700
   aatactattg ctggagtcgc aggactgatc acaggcggga gaagaactcg aagagaagca 2760
   attgtcaatg ctcaacccaa atgcaaccct aatttacatt actggactac tcaggatgaa 2820
   ggtgctgcaa tcggactggc ctggatacca tatttcgggc cagcagccga gggaatttac 2880
   atagaggggc taatgcacaa tcaagatggt ttaatctgtg ggttgagaca gctggccaac 2940
   gagacgactc aagctcttca actgttcctg agagccacaa ctgagctacg caccttttca 3000
   atcctcaacc gtaaggcaat tgatttcttg ctgcagcgat ggggcggcac atgccacatt 3060
   ctgggaccgg actgctgtat cgaaccacat gattggacca agaacataac agacaaaatt 3120
   gatcagatta ttcatgattt tgttgataaa acccttccgg accaggggga caatgacaat 3180
   tggtggacag gatggagaca atggataccg gcaggtattg gagttacagg cgttgtaatt 3240
   gcagttatcg ctttattctg tatatgcaaa tttgtctttt agtttttctt cagattgctt 3300
   catggaaaag ctcagcctca aatcaatgaa accaggattt aattatatgg attacttgaa 3360
   tctaagatta cttgacaaat gataatataa tacactggag ctttaaacat agccaatgtg 3420
   attctaactc ctttaaactc acagttaatc ataaacaagg tttgaggtac cgagctcgaa 3480
   ttgatctgct gtgccttcta gttgccagcc atctgttgtt tgcccctccc ccgtgccttc 3540
   cttgaccctg gaaggtgcca ctcccactgt cctttcctaa taaaatgagg aaattgcatc 3600
   gcattgtctg agtaggtgtc attctattct ggggggtggg gtggggcagg acagcaaggg 3660
   ggaggattgg gaagacaata gcaggcatgc tggggatgcg gtgggctcta tgggtaccca 3720
   ggtgctgaag aattgacccg gttcctcctg ggccagaaag aagcaggcac atccccttct 3780
   ctgtgacaca ccctgtccac gcccctggtt cttagttcca gccccactca taggacactc 3840
   atagctcagg agggctccgc cttcaatccc acccgctaaa gtacttggag cggtctctcc 3900
   ctccctcatc agcccaccaa accaaaccta gcctccaaga gtgggaagaa attaaagcaa 3960
   gataggctat taagtgcaga gggagagaaa atgcctccaa catgtgagga agtaatgaga 4020
   gaaatcatag aatttcttcc gcttcctcgc tcactgactc gctgcgctcg gtcgttcggc 4080
   tgcggcgagc ggtatcagct cactcaaagg cggtaatacg gttatccaca gaatcagggg 4140
   ataacgcagg aaagaacatg tgagcaaaag gccagcaaaa ggccaggaac cgtaaaaagg 4200
   ccgcgttgct ggcgtttttc cataggctcc gcccccctga cgagcatcac aaaaatcgac 4260
   gctcaagtca gaggtggcga aacccgacag gactataaag ataccaggcg tttccccctg 4320
   gaagctccct cgtgcgctct cctgttccga ccctgccgct taccggatac ctgtccgcct 4380
   ttctcccttc gggaagcgtg gcgctttctc atagctcacg ctgtaggtat ctcagttcgg 4440
   tgtaggtcgt tcgctccaag ctgggctgtg tgcacgaacc ccccgttcag cccgaccgct 4500
   gcgccttatc cggtaactat cgtcttgagt ccaacccggt aagacacgac ttatcgccac 4560
   tggcagcagc cactggtaac aggattagca gagcgaggta tgtaggcggt gctacagagt 4620
   tcttgaagtg gtggcctaac tacggctaca ctagaagaac agtatttggt atctgcgctc 4680
   tgctgaagcc agttaccttc ggaaaaagag ttggtagctc ttgatccggc aaacaaacca 4740
   ccgctggtag cggtggtttt tttgtttgca agcagcagat tacgcgcaga aaaaaaggat 4800
   ctcaagaaga tcctttgatc ttttctacgg ggtctgacgc tcagtggaac gaaaactcac 4860
   gttaagggat tttggtcatg agattatcaa aaaggatctt cacctagatc cttttaaatt 4920
   aaaaatgaag ttttaaatca atctaaagta tatatgagta aacttggtct gacagttacc 4980
   aatgcttaat cagtgaggca cctatctcag cgatctgtct atttcgttca tccatagttg 5040
   cctgactcgg gggggggggg cgctgaggtc tgcctcgtga agaaggtgtt gctgactcat 5100
   accaggcctg aatcgcccca tcatccagcc agaaagtgag ggagccacgg ttgatgagag 5160
   ctttgttgta ggtggaccag ttggtgattt tgaacttttg ctttgccacg gaacggtctg 5220
   cgttgtcggg aagatgcgtg atctgatcct tcaactcagc aaaagttcga tttattcaac 5280
   aaagccgccg tcccgtcaag tcagcgtaat gctctgccag tgttacaacc aattaaccaa 5340
   ttctgattag aaaaactcat cgagcatcaa atgaaactgc aatttattca tatcaggatt 5400
   atcaatacca tatttttgaa aaagccgttt ctgtaatgaa ggagaaaact caccgaggca 5460
   gttccatagg atggcaagat cctggtatcg gtctgcgatt ccgactcgtc caacatcaat 5520
   acaacctatt aatttcccct cgtcaaaaat aaggttatca agtgagaaat caccatgagt 5580
   gacgactgaa tccggtgaga atggcaaaag cttatgcatt tctttccaga cttgttcaac 5640
   aggccagcca ttacgctcgt catcaaaatc actcgcatca accaaaccgt tattcattcg 5700
   tgattgcgcc tgagcgagac gaaatacgcg atcgctgtta aaaggacaat tacaaacagg 5760
   aatcgaatgc aaccggcgca ggaacactgc cagcgcatca acaatatttt cacctgaatc 5820
   aggatattct tctaatacct ggaatgctgt tttcccgggg atcgcagtgg tgagtaacca 5880
   tgcatcatca ggagtacgga taaaatgctt gatggtcgga agaggcataa attccgtcag 5940
   ccagtttagt ctgaccatct catctgtaac atcattggca acgctacctt tgccatgttt 6000
   cagaaacaac tctggcgcat cgggcttccc atacaatcga tagattgtcg cacctgattg 6060
   cccgacatta tcgcgagccc atttataccc atataaatca gcatccatgt tggaatttaa 6120
   tcgcggcctc gagcaagacg tttcccgttg aatatggctc ataacacccc ttgtattact 6180
   gtttatgtaa gcagacagtt ttattgttca tgatgatata tttttatctt gtgcaatgta 6240
   acatcagaga ttttgagaca caacgtggct ttcccccccc ccccattatt gaagcattta 6300
   tcagggttat tgtctcatga gcggatacat atttgaatgt atttagaaaa ataaacaaat 6360
   aggggttccg cgcacatttc cccgaaaagt gccacctgac gtctaagaaa ccattattat 6420
   catgacatta acctataaaa ataggcgtat cacgaggccc tttcgtc 6467
<210> 11
   <211> 6913
   <212> DNA
   <213> Artificial Sequence
<220>
   <223> pVR1012x/s Ebola GP(R)(dTM)
<400> 11
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatccccaaa ttacctatac aacatggggt 1920
   caggatatca acttctccaa ttgcctcggg aacgttttcg taaaacttcg ttcttagtat 1980
   gggtaatcat cctcttccag cgagcaatct ccatgccgct tggtatagtg acaaatagca 2040
   ctctcaaagc aacagaaatt gatcaattgg tttgtcggga caaactgtca tcaaccagtc 2100
   agctcaagtc tgtggggctg aatctggaag gaaatggaat tgcaaccgat gtcccatcag 2160
   caacaaaacg ctggggattt cgttcaggtg tgcctcccaa ggtggtcagc tatgaagccg 2220
   gagaatgggc agaaaattgc tacaatctgg agatcaaaaa gtcagacgga agtgaatgcc 2280
   tccctctccc tcccgacggt gtacgaggat tccctagatg tcgctatgtc cacaaagttc 2340
   aaggaacagg tccttgtccc ggtgacttag ctttccataa aaatggggct tttttcttgt 2400
   atgatagatt ggcctcaact gtcatctacc gagggacaac ttttgctgaa ggtgtcgtag 2460
   cttttttaat tctgtcagag cccaagaagc atttttggaa ggctacacca gctcatgaac 2520
   cggtgaacac aacagatgat tccacaagct actacatgac cctgacactc agctacgaga 2580
   tgtcaaattt tgggggcaat gaaagtaaca ccctttttaa ggtagacaac cacacatatg 2640
   tgcaactaga tcgtccacac actccgcagt tccttgttca gctcaatgaa acacttcgaa 2700
   gaaataatcg ccttagcaac agtacaggga gattgacttg gacattggat cctaaaattg 2760
   aaccagatgt tggtgagtgg gccttctggg aaactaaaaa aacttttccc aacaacttca 2820
   tggagaaaac ttgcatttcc aaattctatc aacccacacc aacaactcct cagatcagag 2880
   cccggcggga actgtccaag gaaaaattag ctaccaccca cccgccaaca actccgagct 2940
   ggttccaacg gattcccctc cagtggtttc agtgctcact gcaggacgga cagaggaaat 3000
   gtcgacccaa ggtctaacca acggagagac aatcacaggt ttcaccgcga acccaatgac 3060
   aaccaccatt gccccaagtc caaccatgac aagcgaggtt gataacaatg taccaagtga 3120
   acaaccgaac aacacagcat ccattgaaga ctccccccca tcggcaagca acgagacaat 3180
   ttaccactcc gagatggatc cgatccaagg ctcgaacaac tccgcccaga gcccacagac 3240
   caagaccacg ccagcaccca caacatcccc gatgacccag gacccgcaag agacggccaa 3300
   cagcagcaaa ccaggaacca gcccaggaag cgcagccgga ccaagtcagc ccggactcac 3360
   tataaataca gtaagtaagg tagctgattc actgagtccc accaggaaac aaaagcgatc 3420
   ggttcgacaa aacaccgcta ataaatgtaa cccagatctt tactattgga cagctgttga 3480
   tgagggggca gcagtaggat tggcatggat tccatatttc ggacctgcag cagaaggcat 3540
   ctacattgag ggtgtaatgc ataatcagaa tgggcttatt tgcgggctac gtcagctagc 3600
   caatgaaact acccaggctc ttcaattatt tctgcgggcc acaacagaac tgaggactta 3660
   ctcacttctt aacagaaaag ctattgattt tcttcttcaa cgatggggag gtacctgtcg 3720
   aatcctagga ccatcttgtt gcattgagcc acatgattgg acaaaaaata ttactgatga 3780
   aattaaccaa attaaacatg actttattga caatccccta ccagaccacg gagatgatct 3840
   taatctatgg acaggttgga gacaatggtg aatctagacc aggccctgga tccagatctg 3900
   ctgtgccttc tagttgccag ccatctgttg tttgcccctc ccccgtgcct tccttgaccc 3960
   tggaaggtgc cactcccact gtcctttcct aataaaatga ggaaattgca tcgcattgtc 4020
   tgagtaggtg tcattctatt ctggggggtg gggtggggca ggacagcaag ggggaggatt 4080
   gggaagacaa tagcaggcat gctggggatg cggtgggctc tatgggtacc caggtgctga 4140
   agaattgacc cggttcctcc tgggccagaa agaagcaggc acatcccctt ctctgtgaca 4200
   caccctgtcc acgcccctgg ttcttagttc cagccccact cataggacac tcatagctca 4260
   ggagggctcc gccttcaatc ccacccgcta aagtacttgg agcggtctct ccctccctca 4320
   tcagcccacc aaaccaaacc tagcctccaa gagtgggaag aaattaaagc aagataggct 4380
   attaagtgca gagggagaga aaatgcctcc aacatgtgag gaagtaatga gagaaatcat 4440
   agaattttaa ggccatgatt taaggccatc atggccttaa tcttccgctt cctcgctcac 4500
   tgactcgctg cgctcggtcg ttcggctgcg gcgagcggta tcagctcact caaaggcggt 4560
   aatacggtta tccacagaat caggggataa cgcaggaaag aacatgtgag caaaaggcca 4620
   gcaaaaggcc aggaaccgta aaaaggccgc gttgctggcg tttttccata ggctccgccc 4680
   ccctgacgag catcacaaaa atcgacgctc aagtcagagg tggcgaaacc cgacaggact 4740
   ataaagatac caggcgtttc cccctggaag ctccctcgtg cgctctcctg ttccgaccct 4800
   gccgcttacc ggatacctgt ccgcctttct cccttcggga agcgtggcgc tttctcatag 4860
   ctcacgctgt aggtatctca gttcggtgta ggtcgttcgc tccaagctgg gctgtgtgca 4920
   cgaacccccc gttcagcccg accgctgcgc cttatccggt aactatcgtc ttgagtccaa 4980
   cccggtaaga cacgacttat cgccactggc agcagccact ggtaacagga ttagcagagc 5040
   gaggtatgta ggcggtgcta cagagttctt gaagtggtgg cctaactacg gctacactag 5100
   aagaacagta tttggtatct gcgctctgct gaagccagtt accttcggaa aaagagttgg 5160
   tagctcttga tccggcaaac aaaccaccgc tggtagcggt ggtttttttg tttgcaagca 5220
   gcagattacg cgcagaaaaa aaggatctca agaagatcct ttgatctttt ctacggggtc 5280
   tgacgctcag tggaacgaaa actcacgtta agggattttg gtcatgagat tatcaaaaag 5340
   gatcttcacc tagatccttt taaattaaaa atgaagtttt aaatcaatct aaagtatata 5400
   tgagtaaact tggtctgaca gttaccaatg cttaatcagt gaggcaccta tctcagcgat 5460
   ctgtctattt cgttcatcca tagttgcctg actcgggggg ggggggcgct gaggtctgcc 5520
   tcgtgaagaa ggtgttgctg actcatacca ggcctgaatc gccccatcat ccagccagaa 5580
   agtgagggag ccacggttga tgagagcttt gttgtaggtg gaccagttgg tgattttgaa 5640
   cttttgcttt gccacggaac ggtctgcgtt gtcgggaaga tgcgtgatct gatccttcaa 5700
   ctcagcaaaa gttcgattta ttcaacaaag ccgccgtccc gtcaagtcag cgtaatgctc 5760
   tgccagtgtt acaaccaatt aaccaattct gattagaaaa actcatcgag catcaaatga 5820
   aactgcaatt tattcatatc aggattatca ataccatatt tttgaaaaag ccgtttctgt 5880
   aatgaaggag aaaactcacc gaggcagttc cataggatgg caagatcctg gtatcggtct 5940
   gcgattccga ctcgtccaac atcaatacaa cctattaatt tcccctcgtc aaaaataagg 6000
   ttatcaagtg agaaatcacc atgagtgacg actgaatccg gtgagaatgg caaaagctta 6060
   tgcatttctt tccagacttg ttcaacaggc cagccattac gctcgtcatc aaaatcactc 6120
   gcatcaacca aaccgttatt cattcgtgat tgcgcctgag cgagacgaaa tacgcgatcg 6180
   ctgttaaaag gacaattaca aacaggaatc gaatgcaacc ggcgcaggaa cactgccagc 6240
   gcatcaacaa tattttcacc tgaatcagga tattcttcta atacctggaa tgctgttttc 6300
   ccggggatcg cagtggtgag taaccatgca tcatcaggag tacggataaa atgcttgatg 6360
   gtcggaagag gcataaattc cgtcagccag tttagtctga ccatctcatc tgtaacatca 6420
   ttggcaacgc tacctttgcc atgtttcaga aacaactctg gcgcatcggg cttcccatac 6480
   aatcgataga ttgtcgcacc tgattgcccg acattatcgc gagcccattt atacccatat 6540
   aaatcagcat ccatgttgga atttaatcgc ggcctcgagc aagacgtttc ccgttgaata 6600
   tggctcataa caccccttgt attactgttt atgtaagcag acagttttat tgttcatgat 6660
   gatatatttt tatcttgtgc aatgtaacat cagagatttt gagacacaac gtggctttcc 6720
   cccccccccc attattgaag catttatcag ggttattgtc tcatgagcgg atacatattt 6780
   gaatgtattt agaaaaataa acaaataggg gttccgcgca catttccccg aaaagtgcca 6840
   cctgacgtct aagaaaccat tattatcatg acattaacct ataaaaatag gcgtatcacg 6900
   aggccctttc gtc                                                    6913
<210> 12
   <211> 8131
   <212> DNA
   <213> Artificial Sequence
<220>
   <223> pAdApt Ebola GP(R)(dTM)
<400> 12
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcaacttct ccaattgcct cgggaacgtt 1320
   ttcgtaaaac ttcgttctta gtatgggtaa tcatcctctt ccagcgagca atctccatgc 1380
   cgcttggtat agtgacaaat agcactctca aagcaacaga aattgatcaa ttggtttgtc 1440
   gggacaaact gtcatcaacc agtcagctca agtctgtggg gctgaatctg gaaggaaatg 1500
   gaattgcaac cgatgtccca tcagcaacaa aacgctgggg atttcgttca ggtgtgcctc 1560
   ccaaggtggt cagctatgaa gccggagaat gggcagaaaa ttgctacaat ctggagatca 1620
   aaaagtcaga cggaagtgaa tgcctccctc tccctcccga cggtgtacga ggattcccta 1680
   gatgtcgcta tgtccacaaa gttcaaggaa caggtccttg tcccggtgac ttagctttcc 1740
   ataaaaatgg ggcttttttc ttgtatgata gattggcctc aactgtcatc taccgaggga 1800
   caacttttgc tgaaggtgtc gtagcttttt taattctgtc agagcccaag aagcattttt 1860
   ggaaggctac accagctcat gaaccggtga acacaacaga tgattccaca agctactaca 1920
   tgaccctgac actcagctac gagatgtcaa attttggggg caatgaaagt aacacccttt 1980
   ttaaggtaga caaccacaca tatgtgcaac tagatcgtcc acacactccg cagttccttg 2040
   ttcagctcaa tgaaacactt cgaagaaata atcgccttag caacagtaca gggagattga 2100
   cttggacatt ggatcctaaa attgaaccag atgttggtga gtgggccttc tgggaaacta 2160
   aaaaaacttt tcccaacaac ttcatggaga aaacttgcat ttccaaattc tatcaaccca 2220
   caccaacaac tcctcagatc agagcccggc gggaactgtc caaggaaaaa ttagctacca 2280
   cccacccgcc aacaactccg agctggttcc aacggattcc cctccagtgg tttcagtgct 2340
   cactgcagga cggacagagg aaatgtcgac ccaaggtcta accaacggag agacaatcac 2400
   aggtttcacc gcgaacccaa tgacaaccac cattgcccca agtccaacca tgacaagcga 2460
   ggttgataac aatgtaccaa gtgaacaacc gaacaacaca gcatccattg aagactcccc 2520
   cccatcggca agcaacgaga caatttacca ctccgagatg gatccgatcc aaggctcgaa 2580
   caactccgcc cagagcccac agaccaagac cacgccagca cccacaacat ccccgatgac 2640
   ccaggacccg caagagacgg ccaacagcag caaaccagga accagcccag gaagcgcagc 2700
   cggaccaagt cagcccggac tcactataaa tacagtaagt aaggtagctg attcactgag 2760
   tcccaccagg aaacaaaagc gatcggttcg acaaaacacc gctaataaat gtaacccaga 2820
   tctttactat tggacagctg ttgatgaggg ggcagcagta ggattggcat ggattccata 2880
   tttcggacct gcagcagaag gcatctacat tgagggtgta atgcataatc agaatgggct 2940
   tatttgcggg ctacgtcagc tagccaatga aactacccag gctcttcaat tatttctgcg 3000
   ggccacaaca gaactgagga cttactcact tcttaacaga aaagctattg attttcttct 3060
   tcaacgatgg ggaggtacct gtcgaatcct aggaccatct tgttgcattg agccacatga 3120
   ttggacaaaa aatattactg atgaaattaa ccaaattaaa catgacttta ttgacaatcc 3180
   cctaccagac cacggagatg atcttaatct atggacaggt tggagacaat ggtgaatcta 3240
   gaccaggccc tggatccaga tctgctgtgc cttctagttg ccagccatct gttgtttgcc 3300
   cctcccccgt gccttccttg accctggaag gtgccactcc cactgtcctt tcctaataaa 3360
   atgaggaaat tgcatcgcat tgtctgagta ggtgtcattc tattctgggg ggtggggtgg 3420
   ggcagcacag caagggggag gattgggaag acaatagcag gcatgctggg gatgcggtgg 3480
   gctctatggg tacccagggc cgcataactt cgtataatgt atgctatacg aagttataag 3540
   atctgtactg aaatgtgtgg gcgtggctta agggtgggaa agaatatata aggtgggggt 3600
   cttatgtagt tttgtatctg ttttgcagca gccgccgccg ccatgagcac caactcgttt 3660
   gatggaagca ttgtgagctc atatttgaca acgcgcatgc ccccatgggc cggggtgcgt 3720
   cagaatgtga tgggctccag cattgatggt cgccccgtcc tgcccgcaaa ctctactacc 3780
   ttgacctacg agaccgtgtc tggaacgccg ttggagactg cagcctccgc cgccgcttca 3840
   gccgctgcag ccaccgcccg cgggattgtg actgactttg ctttcctgag cccgcttgca 3900
   agcagtgcag cttcccgttc atccgcccgc gatgacaagt tgacggctct tttggcacaa 3960
   ttggattctt tgacccggga acttaatgtc gtttctcagc agctgttgga tctgcgccag 4020
   caggtttctg ccctgaaggc ttcctcccct cccaatgcgg tttaaaacat aaataaaaaa 4080
   ccagactctg tttggatttg gatcaagcaa gtgtcttgct gtctttattt aggggttttg 4140
   cgcgcgcggt aggcccggga ccagcggtct cggtcgttga gggtcctgtg tattttttcc 4200
   aggacgtggt aaaggtgact ctggatgttc agatacatgg gcataagccc gtctctgggg 4260
   tggaggtagc accactgcag agcttcatgc tgcggggtgg tgttgtagat gatccagtcg 4320
   tagcaggagc gctgggcgtg gtgcctaaaa atgtctttca gtagcaagct gattgccagg 4380
   ggcaggccct tggtgtaagt gtttacaaag cggttaagct gggatgggtg catacgtggg 4440
   gatatgagat gcatcttgga ctgtattttt aggttggcta tgttcccagc catatccctc 4500
   cggggattca tgttgtgcag aaccaccagc acagtgtatc cggtgcactt gggaaatttg 4560
   tcatgtagct tagaaggaaa tgcgtggaag aacttggaga cgcccttgtg acctccaaga 4620
   ttttccatgc attcgtccat aatgatggca atgggcccac gggcggcggc ctgggcgaag 4680
   atatttctgg gatcactaac gtcatagttg tgttccagga tgagatcgtc ataggccatt 4740
   tttacaaagc gcgggcggag ggtgccagac tgcggtataa tggttccatc cggcccaggg 4800
   gcgtagttac cctcacagat ttgcatttcc cacgctttga gttcagatgg ggggatcatg 4860
   tctacctgcg gggcgatgaa gaaaacggtt tccggggtag gggagatcag ctgggaagaa 4920
   agcaggttcc tgagcagctg cgacttaccg cagccggtgg gcccgtaaat cacacctatt 4980
   accggctgca actggtagtt aagagagctg cagctgccgt catccctgag caggggggcc 5040
   acttcgttaa gcatgtccct gactcgcatg ttttccctga ccaaatccgc cagaaggcgc 5100
   tcgccgccca gcgatagcag ttcttgcaag gaagcaaagt ttttcaacgg tttgagaccg 5160
   tccgccgtag gcatgctttt gagcgtttga ccaagcagtt ccaggcggtc ccacagctcg 5220
   gtcacctgct ctacggcatc tcgatccagc atatctcctc gtttcgcggg ttggggcggc 5280
   tttcgctgta cggcagtagt cggtgctcgt ccagacgggc cagggtcatg tctttccacg 5340
   ggcgcagggt cctcgtcagc gtagtctggg tcacggtgaa ggggtgcgct ccgggctgcg 5400
   cgctggccag ggtgcgcttg aggctggtcc tgctggtgct gaagcgctgc cggtcttcgc 5460
   cctgcgcgtc ggccaggtag catttgacca tggtgtcata gtccagcccc tccgcggcgt 5520
   ggcccttggc gcgcagcttg cccttggagg aggcgccgca cgaggggcag tgcagacttt 5580
   tgagggcgta gagcttgggc gcgagaaata ccgattccgg ggagtaggca tccgcgccgc 5640
   aggccccgca gacggtctcg cattccacga gccaggtgag ctctggccgt tcggggtcaa 5700
   aaaccaggtt tcccccatgc tttttgatgc gtttcttacc tctggtttcc atgagccggt 5760
   gtccacgctc ggtgacgaaa aggctgtccg tgtccccgta tacagacttg agaggcctgt 5820
   cctcgagcgg tgttccgcgg tcctcctcgt atagaaactc ggaccactct gagacaaagg 5880
   ctcgcgtcca ggccagcacg aaggaggcta agtgggaggg gtagcggtcg ttgtccacta 5940
   gggggtccac tcgctccagg gtgtgaagac acatgtcgcc ctcttcggca tcaaggaagg 6000
   tgattggttt gtaggtgtag gccacgtgac cgggtgttcc tgaagggggg ctataaaagg 6060
   gggtgggggc gcgttcgtcc tcactctctt ccgcatcgct gtctgcgagg gccagctgtt 6120
   ggggtgagtc gacgcgaggc tggatggcct tccccattat gattcttctc gcttccggcg 6180
   gcatcgggat gcccgcgttg caggccatgc tgtccaggca ggtagatgac gaccatcagg 6240
   gacagcttca aggccagcaa aaggccagga accgtaaaaa ggccgcgttg ctggcgtttt 6300
   tccataggct ccgcccccct gacgagcatc acaaaaatcg acgctcaagt cagaggtggc 6360
   gaaacccgac aggactataa agataccagg cgtttccccc tggaagctcc ctcgtgcgct 6420
   ctcctgttcc gaccctgccg cttaccggat acctgtccgc ctttctccct tcgggaagcg 6480
   tggcgctttc tcatagctca cgctgtaggt atctcagttc ggtgtaggtc gttcgctcca 6540
   agctgggctg tgtgcacgaa ccccccgttc agcccgaccg ctgcgcctta tccggtaact 6600
   atcgtcttga gtccaacccg gtaagacacg acttatcgcc actggcagca gccactggta 6660
   acaggattag cagagcgagg tatgtaggcg gtgctacaga gttcttgaag tggtggccta 6720
   actacggcta cactagaagg acagtatttg gtatctgcgc tctgctgaag ccagttacct 6780
   tcggaaaaag agttggtagc tcttgatccg gcaaacaaac caccgctggt agcggtggtt 6840
   tttttgtttg caagcagcag attacgcgca gaaaaaaagg atctcaagaa gatcctttga 6900
   tcttttctac ggggtctgac gctcagtgga acgaaaactc acgttaaggg attttggtca 6960
   tgagattatc aaaaaggatc ttcacctaga tccttttaaa ttaaaaatga agttttaaat 7020
   caatctaaag tatatatgag taaacttggt ctgacagtta ccaatgctta atcagtgagg 7080
   cacctatctc agcgatctgt ctatttcgtt catccatagt tgcctgactc cccgtcgtgt 7140
   agataactac gatacgggag ggcttaccat ctggccccag tgctgcaatg ataccgcgag 7200
   acccacgctc accggctcca gatttatcag caataaacca gccagccgga agggccgagc 7260
   gcagaagtgg tcctgcaact ttatccgcct ccatccagtc tattaattgt tgccgggaag 7320
   ctagagtaag tagttcgcca gttaatagtt tgcgcaacgt tgttgccatt gctgcaggca 7380
   tcgtggtgtc acgctcgtcg tttggtatgg cttcattcag ctccggttcc caacgatcaa 7440
   ggcgagttac atgatccccc atgttgtgca aaaaagcggt tagctccttc ggtcctccga 7500
   tcgttgtcag aagtaagttg gccgcagtgt tatcactcat ggttatggca gcactgcata 7560
   attctcttac tgtcatgcca tccgtaagat gcttttctgt gactggtgag tactcaacca 7620
   agtcattctg agaatagtgt atgcggcgac cgagttgctc ttgcccggcg tcaacacggg 7680
   ataataccgc gccacatagc agaactttaa aagtgctcat cattggaaaa cgttcttcgg 7740
   ggcgaaaact ctcaaggatc ttaccgctgt tgagatccag ttcgatgtaa cccactcgtg 7800
   cacccaactg atcttcagca tcttttactt tcaccagcgt ttctgggtga gcaaaaacag 7860
   gaaggcaaaa tgccgcaaaa aagggaataa gggcgacacg gaaatgttga atactcatac 7920
   tcttcctttt tcaatattat tgaagcattt atcagggtta ttgtctcatg agcggataca 7980
   tatttgaatg tatttagaaa aataaacaaa taggggttcc gcgcacattt ccccgaaaag 8040
   tgccacctga cgtctaagaa accattatta tcatgacatt aacctataaa aataggcgta 8100
   tcacgaggcc ctttcgtctt caagaattgt t 8131
<210> 13
   <211> 7082
   <212> DNA
   <213> Artificial Sequence
<220>
   <223> pVR1012-GP(S)
<400> 13
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctagc tagatgcatg 1920
   ctcgagcggc cgccagtgtg atggatatct gcagaattcg gcttatcttc aggatctcgc 1980
   catggagggt cttagcctac tccaattgcc cagagataaa tttcgaaaaa gctctttctt 2040
   tgtttgggtc atcatcttat ttcaaaaggc cttttccatg cctttgggtg ttgtgaccaa 2100
   cagcacttta gaagtaacag agattgacca gctagtctgc aaggatcatc ttgcatccac 2160
   tgaccagctg aaatcagttg gtctcaacct cgaggggagc ggagtatcta ctgatatccc 2220
   atctgcgaca aagcgttggg gcttcagatc tggtgtgcct cccaaggtgg tcagctatga 2280
   agcaggagaa tgggctgaaa attgctacaa tcttgaaata aagaagccgg acgggagcga 2340
   atgcttaccc ccaccgccgg atggtgtcag aggctttcca aggtgccgct atgttcacaa 2400
   agcccaagga accgggccct gcccgggtga ctatgccttt cacaaggatg gagctttctt 2460
   cctctatgac aggctggctt caactgtaat ttacagagga gtcaattttg ctgagggggt 2520
   aattgcattc ttgatattgg ctaaaccaaa ggaaacgttc cttcaatcac cccccattcg 2580
   agaggcagta aactacactg aaaatacatc aagttactat gccacatcct acttggagta 2640
   cgaaatcgaa aattttggtg ctcaacactc cacgaccctt ttcaaaatta acaataatac 2700
   ttttgttctt ctggacaggc cccacacgcc tcagttcctt ttccagctga atgataccat 2760
   tcaccttcac caacagttga gcaacacaac tgggaaacta atttggacac tagatgctaa 2820
   tatcaatgct gatattggtg aatgggcttt ttgggaaaat aaaaaaaatc tctccgaaca 2880
   actacgtgga gaagagctgt ctttcgaaac tttatcgctc aacgagacag aagacgatga 2940
   tgcgacatcg tcgagaacta caaagggaag aatctccgac cgggccacca ggaagtattc 3000
   ggacctggtt ccaaaggatt cccctgggat ggtttcattg cacgtaccag aaggggaaac 3060
   aacattgccg tctcagaatt cgacagaagg tcgaagagta gatgtgaata ctcaggaaac 3120
   tatcacagag acaactgcaa caatcatagg cactaacggt aacaacatgc agatctccac 3180
   catcgggaca ggactgagct ccagccaaat cctgagttcc tcaccgacca tggcaccaag 3240
   ccctgagact cagacctcca caacctacac accaaaacta ccagtgatga ccaccgagga 3300
   accaacaaca ccaccgagaa actctcctgg ctcaacaaca gaagcaccca ctctcaccac 3360
   cccagagaat ataacaacag cggttaaaac tgttttgcca caagagtcca caagcaacgg 3420
   tctaataact tcaacagtaa cagggattct tgggagcctt ggacttcgaa aacgcagcag 3480
   aagacaagtt aacaccaggg ccacgggtaa atgcaatccc aacttacact actggactgc 3540
   acaagaacaa cataatgctg ctgggattgc ctggatcccg tactttggac cgggtgcaga 3600
   aggcatatac actgaaggcc ttatgcacaa ccaaaatgcc ttagtctgtg gactcagaca 3660
   acttgcaaat gaaacaactc aagctctgca gcttttctta agggccacga cggagctgcg 3720
   gacatatacc atactcaata ggaaggccat agatttcctt ctgcgacgat ggggcgggac 3780
   atgtaggatc ctgggaccag attgttgcat tgagccacat gattggacca aaaacatcac 3840
   tgataaaatc aaccaaatca tccatgattt catcgacaac cctttaccca atcaggataa 3900
   tgatgataat tggtggacgg gctggagaca gtggatccct gcaggaatag gcattactgg 3960
   aattattatt gcaatcattg ctcttctttg cgtctgcaag ctgctttgtt gaatatcaag 4020
   ccgaattcca gcacactggc ggccgttact agtggatccg agctcggatc caagctctag 4080
   accaggccct ggatccagat ctgctgtgcc ttctagttgc cagccatctg ttgtttgccc 4140
   ctcccccgtg ccttccttga ccctggaagg tgccactccc actgtccttt cctaataaaa 4200
   tgaggaaatt gcatcgcatt gtctgagtag gtgtcattct attctggggg gtggggtggg 4260
   gcaggacagc aagggggagg attgggaaga caatagcagg catgctgggg atgcggtggg 4320
   ctctatgggt acccaggtgc tgaagaattg acccggttcc tcctgggcca gaaagaagca 4380
   ggcacatccc cttctctgtg acacaccctg tccacgcccc tggttcttag ttccagcccc 4440
   actcatagga cactcatagc tcaggagggc tccgccttca atcccacccg ctaaagtact 4500
   tggagcggtc tctccctccc tcatcagccc accaaaccaa acctagcctc caagagtggg 4560
   aagaaattaa agcaagatag gctattaagt gcagagggag agaaaatgcc tccaacatgt 4620
   gaggaagtaa tgagagaaat catagaattt cttccgcttc ctcgctcact gactcgctgc 4680
   gctcggtcgt tcggctgcgg cgagcggtat cagctcactc aaaggcggta atacggttat 4740
   ccacagaatc aggggataac gcaggaaaga acatgtgagc aaaaggccag caaaaggcca 4800
   ggaaccgtaa aaaggccgcg ttgctggcgt ttttccatag gctccgcccc cctgacgagc 4860
   atcacaaaaa tcgacgctca agtcagaggt ggcgaaaccc gacaggacta taaagatacc 4920
   aggcgtttcc ccctggaagc tccctcgtgc gctctcctgt tccgaccctg ccgcttaccg 4980
   gatacctgtc cgcctttctc ccttcgggaa gcgtggcgct ttctcatagc tcacgctgta 5040
   ggtatctcag ttcggtgtag gtcgttcgct ccaagctggg ctgtgtgcac gaaccccccg 5100
   ttcagcccga ccgctgcgcc ttatccggta actatcgtct tgagtccaac ccggtaagac 5160
   acgacttatc gccactggca gcagccactg gtaacaggat tagcagagcg aggtatgtag 5220
   gcggtgctac agagttcttg aagtggtggc ctaactacgg ctacactaga agaacagtat 5280
   ttggtatctg cgctctgctg aagccagtta ccttcggaaa aagagttggt agctcttgat 5340
   ccggcaaaca aaccaccgct ggtagcggtg gtttttttgt ttgcaagcag cagattacgc 5400
   gcagaaaaaa aggatctcaa gaagatcctt tgatcttttc tacggggtct gacgctcagt 5460
   ggaacgaaaa ctcacgttaa gggattttgg tcatgagatt atcaaaaagg atcttcacct 5520
   agatcctttt aaattaaaaa tgaagtttta aatcaatcta aagtatatat gagtaaactt 5580
   ggtctgacag ttaccaatgc ttaatcagtg aggcacctat ctcagcgatc tgtctatttc 5640
   gttcatccat agttgcctga ctcggggggg gggggcgctg aggtctgcct cgtgaagaag 5700
   gtgttgctga ctcataccag gcctgaatcg ccccatcatc cagccagaaa gtgagggagc 5760
   cacggttgat gagagctttg ttgtaggtgg accagttggt gattttgaac ttttgctttg 5820
   ccacggaacg gtctgcgttg tcgggaagat gcgtgatctg atccttcaac tcagcaaaag 5880
   ttcgatttat tcaacaaagc cgccgtcccg tcaagtcagc gtaatgctct gccagtgtta 5940
   caaccaatta accaattctg attagaaaaa ctcatcgagc atcaaatgaa actgcaattt 6000
   attcatatca ggattatcaa taccatattt ttgaaaaagc cgtttctgta atgaaggaga 6060
   aaactcaccg aggcagttcc ataggatggc aagatcctgg tatcggtctg cgattccgac 6120
   tcgtccaaca tcaatacaac ctattaattt cccctcgtca aaaataaggt tatcaagtga 6180
   gaaatcacca tgagtgacga ctgaatccgg tgagaatggc aaaagcttat gcatttcttt 6240
   ccagacttgt tcaacaggcc agccattacg ctcgtcatca aaatcactcg catcaaccaa 6300
   accgttattc attcgtgatt gcgcctgagc gagacgaaat acgcgatcgc tgttaaaagg 6360
   acaattacaa acaggaatcg aatgcaaccg gcgcaggaac actgccagcg catcaacaat 6420
   attttcacct gaatcaggat attcttctaa tacctggaat gctgttttcc cggggatcgc 6480
   agtggtgagt aaccatgcat catcaggagt acggataaaa tgcttgatgg tcggaagagg 6540
   cataaattcc gtcagccagt ttagtctgac catctcatct gtaacatcat tggcaacgct 6600
   acctttgcca tgtttcagaa acaactctgg cgcatcgggc ttcccataca atcgatagat 6660
   tgtcgcacct gattgcccga cattatcgcg agcccattta tacccatata aatcagcatc 6720
   catgttggaa tttaatcgcg gcctcgagca agacgtttcc cgttgaatat ggctcataac 6780
   accccttgta ttactgttta tgtaagcaga cagttttatt gttcatgatg atatattttt 6840
   atcttgtgca atgtaacatc agagattttg agacacaacg tggctttccc ccccccccca 6900
   ttattgaagc atttatcagg gttattgtct catgagcgga tacatatttg aatgtattta 6960
   gaaaaataaa caaatagggg ttccgcgcac atttccccga aaagtgccac ctgacgtcta 7020
   agaaaccatt attatcatga cattaaccta taaaaatagg cgtatcacga ggccctttcg 7080
   tc                                                                7082
<210> 14
   <211> 7087
   <212> DNA
   <213> Artificial Sequence
<220>
   <223> pVR1012x/s Ebola GP(S)
<400> 14
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgccagtgt gatggatatc 1920
   tgcagaattc ggcttatctt caggatctcg ccatggaggg tcttagccta ctccaattgc 1980
   ccagagataa atttcgaaaa agctctttct ttgtttgggt catcatctta tttcaaaagg 2040
   ccttttccat gcctttgggt gttgtgacca acagcacttt agaagtaaca gagattgacc 2100
   agctagtctg caaggatcat cttgcatcca ctgaccagct gaaatcagtt ggtctcaacc 2160
   tcgaggggag cggagtatct actgatatcc catctgcgac aaagcgttgg ggcttcagat 2220
   ctggtgtgcc tcccaaggtg gtcagctatg aagcaggaga atgggctgaa aattgctaca 2280
   atcttgaaat aaagaagccg gacgggagcg aatgcttacc cccaccgccg gatggtgtca 2340
   gaggctttcc aaggtgccgc tatgttcaca aagcccaagg aaccgggccc tgcccgggtg 2400
   actatgcctt tcacaaggat ggagctttct tcctctatga caggctggct tcaactgtaa 2460
   tttacagagg agtcaatttt gctgaggggg taattgcatt cttgatattg gctaaaccaa 2520
   aggaaacgtt ccttcaatca ccccccattc gagaggcagt aaactacact gaaaatacat 2580
   caagttacta tgccacatcc tacttggagt acgaaatcga aaattttggt gctcaacact 2640
   ccacgaccct tttcaaaatt aacaataata cttttgttct tctggacagg ccccacacgc 2700
   ctcagttcct tttccagctg aatgatacca ttcaccttca ccaacagttg agcaacacaa 2760
   ctgggaaact aatttggaca ctagatgcta atatcaatgc tgatattggt gaatgggctt 2820
   tttgggaaaa taaaaaaaat ctctccgaac aactacgtgg agaagagctg tctttcgaaa 2880
   ctttatcgct caacgagaca gaagacgatg atgcgacatc gtcgagaact acaaagggaa 2940
   gaatctccga ccgggccacc aggaagtatt cggacctggt tccaaaggat tcccctggga 3000
   tggtttcatt gcacgtacca gaaggggaaa caacattgcc gtctcagaat tcgacagaag 3060
   gtcgaagagt agatgtgaat actcaggaaa ctatcacaga gacaactgca acaatcatag 3120
   gcactaacgg taacaacatg cagatctcca ccatcgggac aggactgagc tccagccaaa 3180
   tcctgagttc ctcaccgacc atggcaccaa gccctgagac tcagacctcc acaacctaca 3240
   caccaaaact accagtgatg accaccgagg aatcaacaac accaccgaga aactctcctg 3300
   gctcaacaac agaagcaccc actctcacca ccccagagaa tataacaaca gcggttaaaa 3360
   ctgttttgcc acaagagtcc acaagcaacg gtctaataac ttcaacagta acagggattc 3420
   ttgggagcct tggacttcga aaacgcagca gaagacaagt taacaccagg gccacgggta 3480
   aatgcaatcc caacttacac tactggactg cacaagaaca acataatgct gctgggattg 3540
   cctggatccc gtactttgga ccgggtgcag aaggcatata cactgaaggc cttatgcaca 3600
   accaaaatgc cttagtctgt ggactcagac aacttgcaaa tgaaacaact caagctctgc 3660
   agcttttctt aagggccacg acggagctgc ggacatatac catactcaat aggaaggcca 3720
   tagatttcct tctgcgacga tggggcggga catgtaggat cctgggacca gattgttgca 3780
   ttgagccaca tgattggacc aaaaacatca ctgataaaat caaccaaatc atccatgatt 3840
   tcatcgacaa ccctttaccc aatcaggata atgatgataa ttggtggacg ggctggagac 3900
   agtggatccc tgcaggaata ggcattactg gaattattat tgcaatcatt gctcttcttt 3960
   gcgtctgcaa gctgctttgt tgaatatcaa gccgaattcc agcacactgg cggccgttac 4020
   tagtggatcc gagctcggta ccaagctcta gaccaggccc tggatccaga tctgctgtgc 4080
   cttctagttg ccagccatct gttgtttgcc cctcccccgt gccttccttg accctggaag 4140
   gtgccactcc cactgtcctt tcctaataaa atgaggaaat tgcatcgcat tgtctgagta 4200
   ggtgtcattc tattctgggg ggtggggtgg ggcaggacag caagggggag gattgggaag 4260
   acaatagcag gcatgctggg gatgcggtgg gctctatggg tacccaggtg ctgaagaatt 4320
   gacccggttc ctcctgggcc agaaagaagc aggcacatcc ccttctctgt gacacaccct 4380
   gtccacgccc ctggttctta gttccagccc cactcatagg acactcatag ctcaggaggg 4440
   ctccgccttc aatcccaccc gctaaagtac ttggagcggt ctctccctcc ctcatcagcc 4500
   caccaaacca aacctagcct ccaagagtgg gaagaaatta aagcaagata ggctattaag 4560
   tgcagaggga gagaaaatgc ctccaacatg tgaggaagta atgagagaaa tcatagaatt 4620
   ttaaggccat gatttaaggc catcatggcc ttaatcttcc gcttcctcgc tcactgactc 4680
   gctgcgctcg gtcgttcggc tgcggcgagc ggtatcagct cactcaaagg cggtaatacg 4740
   gttatccaca gaatcagggg ataacgcagg aaagaacatg tgagcaaaag gccagcaaaa 4800
   ggccaggaac cgtaaaaagg ccgcgttgct ggcgtttttc cataggctcc gcccccctga 4860
   cgagcatcac aaaaatcgac gctcaagtca gaggtggcga aacccgacag gactataaag 4920
   ataccaggcg tttccccctg gaagctccct cgtgcgctct cctgttccga ccctgccgct 4980
   taccggatac ctgtccgcct ttctcccttc gggaagcgtg gcgctttctc atagctcacg 5040
   ctgtaggtat ctcagttcgg tgtaggtcgt tcgctccaag ctgggctgtg tgcacgaacc 5100
   ccccgttcag cccgaccgct gcgccttatc cggtaactat cgtcttgagt ccaacccggt 5160
   aagacacgac ttatcgccac tggcagcagc cactggtaac aggattagca gagcgaggta 5220
   tgtaggcggt gctacagagt tcttgaagtg gtggcctaac tacggctaca ctagaagaac 5280
   agtatttggt atctgcgctc tgctgaagcc agttaccttc ggaaaaagag ttggtagctc 5340
   ttgatccggc aaacaaacca ccgctggtag cggtggtttt tttgtttgca agcagcagat 5400
   tacgcgcaga aaaaaaggat ctcaagaaga tcctttgatc ttttctacgg ggtctgacgc 5460
   tcagtggaac gaaaactcac gttaagggat tttggtcatg agattatcaa aaaggatctt 5520
   cacctagatc cttttaaatt aaaaatgaag ttttaaatca atctaaagta tatatgagta 5580
   aacttggtct gacagttacc aatgcttaat cagtgaggca cctatctcag cgatctgtct 5640
   atttcgttca tccatagttg cctgactcgg gggggggggg cgctgaggtc tgcctcgtga 5700
   agaaggtgtt gctgactcat accaggcctg aatcgcccca tcatccagcc agaaagtgag 5760
   ggagccacgg ttgatgagag ctttgttgta ggtggaccag ttggtgattt tgaacttttg 5820
   ctttgccacg gaacggtctg cgttgtcggg aagatgcgtg atctgatcct tcaactcagc 5880
   aaaagttcga tttattcaac aaagccgccg tcccgtcaag tcagcgtaat gctctgccag 5940
   tgttacaacc aattaaccaa ttctgattag aaaaactcat cgagcatcaa atgaaactgc 6000
   aatttattca tatcaggatt atcaatacca tatttttgaa aaagccgttt ctgtaatgaa 6060
   ggagaaaact caccgaggca gttccatagg atggcaagat cctggtatcg gtctgcgatt 6120
   ccgactcgtc caacatcaat acaacctatt aatttcccct cgtcaaaaat aaggttatca 6180
   agtgagaaat caccatgagt gacgactgaa tccggtgaga atggcaaaag cttatgcatt 6240
   tctttccaga cttgttcaac aggccagcca ttacgctcgt catcaaaatc actcgcatca 6300
   accaaaccgt tattcattcg tgattgcgcc tgagcgagac gaaatacgcg atcgctgtta 6360
   aaaggacaat tacaaacagg aatcgaatgc aaccggcgca ggaacactgc cagcgcatca 6420
   acaatatttt cacctgaatc aggatattct tctaatacct ggaatgctgt tttcccgggg 6480
   atcgcagtgg tgagtaacca tgcatcatca ggagtacgga taaaatgctt gatggtcgga 6540
   agaggcataa attccgtcag ccagtttagt ctgaccatct catctgtaac atcattggca 6600
   acgctacctt tgccatgttt cagaaacaac tctggcgcat cgggcttccc atacaatcga 6660
   tagattgtcg cacctgattg cccgacatta tcgcgagccc atttataccc atataaatca 6720
   gcatccatgt tggaatttaa tcgcggcctc gagcaagacg tttcccgttg aatatggctc 6780
   ataacacccc ttgtattact gtttatgtaa gcagacagtt ttattgttca tgatgatata 6840
   tttttatctt gtgcaatgta acatcagaga ttttgagaca caacgtggct ttcccccccc 6900
   ccccattatt gaagcattta tcagggttat tgtctcatga gcggatacat atttgaatgt 6960
   atttagaaaa ataaacaaat aggggttccg cgcacatttc cccgaaaagt gccacctgac 7020
   gtctaagaaa ccattattat catgacatta acctataaaa ataggcgtat cacgaggccc 7080
   tttcgtc 7087
<210> 15
   <211> 6940
   <212> DNA
   <213> Artificial Sequence
<220>
   <223> pVR1012-GP(S) delta TM
<400> 15
<210> 16
   <211> 7002
   <212> DNA
   <213> Artificial Sequence
<220>
   <223> pVR1012-GP(IC)
<400> 16
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga attcggcttc 1920
   taatcacagt caccatggga gcgtcaggga ttctgcaatt gccccgtgag cgcttcagga 1980
   aaacatcttt ctttgtttgg gtaataatcc tattccataa agtcttttca atcccgttgg 2040
   gggttgtaca caacaatacc ctacaagtga gtgatattga caagtttgtg tgccgagaca 2100
   aactctcttc aactagccaa ttgaagtcag tcgggttgaa cttggagggc aatggagtag 2160
   caactgatgt accaacggca accaaaagat ggggttttcg agctggtgtt ccaccaaagg 2220
   tggtaaattg cgaagctgga gaatgggctg agaactgtta taacctggct ataaagaaag 2280
   ttgatggtag tgagtgccta ccagaagccc ctgagggagt gagggatttt ccccgttgcc 2340
   gctatgtaca caaagtctca ggaactggac catgcccagg aggactcgcc tttcacaaag 2400
   aaggagcctt cttcctgtat gaccgactcg catcaacaat catttatcgg ggtacaacct 2460
   ttgccgaagg agttattgca tttctgatct tgcctaaggc gcgaaaggat tttttccagt 2520
   ctcctccatt gcatgagcct gccaacatga ccacggatcc ctccagttac tatcacacga 2580
   caacaataaa ctacgtggtt gataattttg gaaccaacac cacagagttt ctgttccaag 2640
   tcgatcattt gacgtatgtg cagctcgagg caagattcac accacaattc cttgtcctcc 2700
   taaatgaaac catctactct gataaccgca gaagtaacac aacaggaaaa ctaatctgga 2760
   aaataaatcc cactgttgat accagcatgg gtgagtgggc tttctgggaa aataaaaaaa 2820
   acttcacaaa aaccctttca agtgaagagt tgtctttcgt acctgtacca gaaacccaga 2880
   accaggtcct tgacacgaca gcgacggtct ctcctcccat ctccgcccac aaccacgcag 2940
   ccgaagacca caaagaattg gtttcagagg attccactcc agtggttcag atgcaaaaca 3000
   tcaagggaaa ggacacaatg ccaaccacag tgacgggtgt accaacaacc acaccctctc 3060
   catttccaat caatgctcgc aacactgatc ataccaaatc atttatcggc ctggaggggc 3120
   cccaagaaga ccacagcacc acacagcctg ccaagaccac cagccaacca accaacagca 3180
   cagaatcgac gacactaaac ccaacatcag agccctccag tagaggcacg ggaccatcca 3240
   gccccacggt ccccaacacc acagaaagcc acgccgaact tggcaagaca accccaacca 3300
   cactcccaga acagcacact gccgccagtg ccattccaag agccgtgcac cccgacgaac 3360
   tcagtggacc tggcttcctg acgaacacaa tacggggggt tacaaatctc ctgacaggat 3420
   ccagaagaaa gcgaagggat gtcactccca atacacaacc caaatgcaac ccaaacctgc 3480
   actattggac agccttggat gagggtgctg ccataggttt agcctggata ccatacttcg 3540
   ggccagcagc tgagggaatt tacactgaag gcataatgga gaatcaaaat ggattgatct 3600
   gtggattgag gcagctggcc aacgaaacga cacaagctct tcaattgttc ttaagggcaa 3660
   ctactgagtt gcgtacattc tctatactaa atcggaaagc aatagacttc ttgctccaaa 3720
   gatggggagg aacatgtcac attctagggc ctgattgttg cattgaaccc caagattgga 3780
   ccaaaaatat cactgataaa attgatcaaa taatccatga ctttgtcgat aataatcttc 3840
   caaatcagaa tgatggcagc aactggtgga ctggatggaa acaatgggtt cctgctggaa 3900
   taggaatcac aggagtaatc attgctatta ttgctttgct gtgcatttgc aaattcatgc 3960
   tttgaactaa tatagcatca tactttaagc cgaattctag accaggccct ggatccagat 4020
   ctgctgtgcc ttctagttgc cagccatctg ttgtttgccc ctcccccgtg ccttccttga 4080
   ccctggaagg tgccactccc actgtccttt cctaataaaa tgaggaaatt gcatcgcatt 4140
   gtctgagtag gtgtcattct attctggggg gtggggtggg gcaggacagc aagggggagg 4200
   attgggaaga caatagcagg catgctgggg atgcggtggg ctctatgggt acccaggtgc 4260
   tgaagaattg acccggttcc tcctgggcca gaaagaagca ggcacatccc cttctctgtg 4320
   acacaccctg tccacgcccc tggttcttag ttccagcccc actcatagga cactcatagc 4380
   tcaggagggc tccgccttca atcccacccg ctaaagtact tggagcggtc tctccctccc 4440
   tcatcagccc accaaaccaa acctagcctc caagagtggg aagaaattaa agcaagatag 4500
   gctattaagt gcagagggag agaaaatgcc tccaacatgt gaggaagtaa tgagagaaat 4560
   catagaattt cttccgcttc ctcgctcact gactcgctgc gctcggtcgt tcggctgcgg 4620
   cgagcggtat cagctcactc aaaggcggta atacggttat ccacagaatc aggggataac 4680
   gcaggaaaga acatgtgagc aaaaggccag caaaaggcca ggaaccgtaa aaaggccgcg 4740
   ttgctggcgt ttttccatag gctccgcccc cctgacgagc atcacaaaaa tcgacgctca 4800
   agtcagaggt ggcgaaaccc gacaggacta taaagatacc aggcgtttcc ccctggaagc 4860
   tccctcgtgc gctctcctgt tccgaccctg ccgcttaccg gatacctgtc cgcctttctc 4920
   ccttcgggaa gcgtggcgct ttctcatagc tcacgctgta ggtatctcag ttcggtgtag 4980
   gtcgttcgct ccaagctggg ctgtgtgcac gaaccccccg ttcagcccga ccgctgcgcc 5040
   ttatccggta actatcgtct tgagtccaac ccggtaagac acgacttatc gccactggca 5100
   gcagccactg gtaacaggat tagcagagcg aggtatgtag gcggtgctac agagttcttg 5160
   aagtggtggc ctaactacgg ctacactaga agaacagtat ttggtatctg cgctctgctg 5220
   aagccagtta ccttcggaaa aagagttggt agctcttgat ccggcaaaca aaccaccgct 5280
   ggtagcggtg gtttttttgt ttgcaagcag cagattacgc gcagaaaaaa aggatctcaa 5340
   gaagatcctt tgatcttttc tacggggtct gacgctcagt ggaacgaaaa ctcacgttaa 5400
   gggattttgg tcatgagatt atcaaaaagg atcttcacct agatcctttt aaattaaaaa 5460
   tgaagtttta aatcaatcta aagtatatat gagtaaactt ggtctgacag ttaccaatgc 5520
   ttaatcagtg aggcacctat ctcagcgatc tgtctatttc gttcatccat agttgcctga 5580
   ctcggggggg gggggcgctg aggtctgcct cgtgaagaag gtgttgctga ctcataccag 5640
   gcctgaatcg ccccatcatc cagccagaaa gtgagggagc cacggttgat gagagctttg 5700
   ttgtaggtgg accagttggt gattttgaac ttttgctttg ccacggaacg gtctgcgttg 5760
   tcgggaagat gcgtgatctg atccttcaac tcagcaaaag ttcgatttat tcaacaaagc 5820
   cgccgtcccg tcaagtcagc gtaatgctct gccagtgtta caaccaatta accaattctg 5880
   attagaaaaa ctcatcgagc atcaaatgaa actgcaattt attcatatca ggattatcaa 5940
   taccatattt ttgaaaaagc cgtttctgta atgaaggaga aaactcaccg aggcagttcc 6000
   ataggatggc aagatcctgg tatcggtctg cgattccgac tcgtccaaca tcaatacaac 6060
   ctattaattt cccctcgtca aaaataaggt tatcaagtga gaaatcacca tgagtgacga 6120
   ctgaatccgg tgagaatggc aaaagcttat gcatttcttt ccagacttgt tcaacaggcc 6180
   agccattacg ctcgtcatca aaatcactcg catcaaccaa accgttattc attcgtgatt 6240
   gcgcctgagc gagacgaaat acgcgatcgc tgttaaaagg acaattacaa acaggaatcg 6300
   aatgcaaccg gcgcaggaac actgccagcg catcaacaat attttcacct gaatcaggat 6360
   attcttctaa tacctggaat gctgttttcc cggggatcgc agtggtgagt aaccatgcat 6420
   catcaggagt acggataaaa tgcttgatgg tcggaagagg cataaattcc gtcagccagt 6480
   ttagtctgac catctcatct gtaacatcat tggcaacgct acctttgcca tgtttcagaa 6540
   acaactctgg cgcatcgggc ttcccataca atcgatagat tgtcgcacct gattgcccga 6600
   cattatcgcg agcccattta tacccatata aatcagcatc catgttggaa tttaatcgcg 6660
   gcctcgagca agacgtttcc cgttgaatat ggctcataac accccttgta ttactgttta 6720
   tgtaagcaga cagttttatt gttcatgatg atatattttt atcttgtgca atgtaacatc 6780
   agagattttg agacacaacg tggctttccc ccccccccca ttattgaagc atttatcagg 6840
   gttattgtct catgagcgga tacatatttg aatgtattta gaaaaataaa caaatagggg 6900
   ttccgcgcac atttccccga aaagtgccac ctgacgtcta agaaaccatt attatcatga 6960
   cattaaccta taaaaatagg cgtatcacga ggccctttcg tc                    7002
<210> 17
   <211> 7036
   <212> DNA
   <213> Artificial Sequence
<220>
   <223> pVR1012 x/s Ebola GP(IC)
<400> 17
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga attcggcttc 1920
   taatcacagt caccatggga gcgtcaggga ttctgcaatt gccccgtgag cgcttcagga 1980
   aaacatcttt ctttgtttgg gtaataatcc tattccataa agtcttttca atcccgttgg 2040
   gggttgtaca caacaatacc ctacaagtga gtgatattga caagtttgtg tgccgagaca 2100
   aactctcttc aactagccaa ttgaagtcag tcgggttgaa cttggagggc aatggagtag 2160
   caactgatgt accaacggca accaaaagat ggggttttcg agctggtgtt ccaccaaagg 2220
   tggtaaattg cgaagctgga gaatgggctg agaactgtta taacctggct ataaagaaag 2280
   ttgatggtag tgagtgccta ccagaagccc ctgagggagt gagggatttt ccccgttgcc 2340
   gctatgtaca caaagtctca ggaactggac catgcccagg aggactcgcc tttcacaaag 2400
   aaggagcctt cttcctgtat gaccgactcg catcaacaat catttatcgg ggtacaacct 2460
   ttgccgaagg agttattgca tttctgatct tgcctaaggc gcgaaaggat tttttccagt 2520
   ctcctccatt gcatgagcct gccaacatga ccacggatcc ctccagttac tatcacacga 2580
   caacaataaa ctacgtggtt gataattttg gaaccaacac cacagagttt ctgttccaag 2640
   tcgatcattt gacgtatgtg cagctcgagg caagattcac accacaattc cttgtcctcc 2700
   taaatgaaac catctactct gataaccgca gaagtaacac aacaggaaaa ctaatctgga 2760
   aaataaatcc cactgttgat accagcatgg gtgagtgggc tttctgggaa aataaaaaaa 2820
   acttcacaaa aaccctttca agtgaagagt tgtctttcgt acctgtacca gaaacccaga 2880
   accaggtcct tgacacgaca gcgacggtct ctcctcccat ctccgcccac aaccacgcag 2940
   ccgaagacca caaagaattg gtttcagagg attccactcc agtggttcag atgcaaaaca 3000
   tcaagggaaa ggacacaatg ccaaccacag tgacgggtgt accaacaacc acaccctctc 3060
   catttccaat caatgctcgc aacactgatc ataccaaatc atttatcggc ctggaggggc 3120
   cccaagaaga ccacagcacc acacagcctg ccaagaccac cagccaacca accaacagca 3180
   cagaatcgac gacactaaac ccaacatcag agccctccag tagaggcacg ggaccatcca 3240
   gccccacggt ccccaacacc acagaaagcc acgccgaact tggcaagaca accccaacca 3300
   cactcccaga acagcacact gccgccagtg ccattccaag agccgtgcac cccgacgaac 3360
   tcagtggacc tggcttcctg acgaacacaa tacggggggt tacaaatctc ctgacaggat 3420
   ccagaagaaa gcgaagggat gtcactccca atacacaacc caaatgcaac ccaaacctgc 3480
   actattggac agccttggat gagggtgctg ccataggttt agcctggata ccatacttcg 3540
   ggccagcagc tgagggaatt tacactgaag gcataatgga gaatcaaaat ggattgatct 3600
   gtggattgag gcagctggcc aacgaaacga cacaagctct tcaattgttc ttaagggcaa 3660
   ctactgagtt gcgtacattc tctatactaa atcggaaagc aatagacttc ttgctccaaa 3720
   gatggggagg aacatgtcac attctagggc ctgattgttg cattgaaccc caagattgga 3780
   ccaaaaatat cactgataaa attgatcaaa taatccatga ctttgtcgat aataatcttc 3840
   caaatcagaa tgatggcagc aactggtgga ctggatggaa acaatgggtt cctgctggaa 3900
   taggaatcac aggagtaatc attgctatta ttgctttgct gtgcatttgc aaattcatgc 3960
   tttgaactaa tatagcatca tactttaagc cgaattctag accaggccct ggatccagat 4020
   ctgctgtgcc ttctagttgc cagccatctg ttgtttgccc ctcccccgtg ccttccttga 4080
   ccctggaagg tgccactccc actgtccttt cctaataaaa tgaggaaatt gcatcgcatt 4140
   gtctgagtag gtgtcattct attctggggg gtggggtggg gcaggacagc aagggggagg 4200
   attgggaaga caatagcagg catgctgggg atgcggtggg ctctatgggt acccaggtgc 4260
   tgaagaattg acccggttcc tcctgggcca gaaagaagca ggcacatccc cttctctgtg 4320
   acacaccctg tccacgcccc tggttcttag ttccagcccc actcatagga cactcatagc 4380
   tcaggagggc tccgccttca atcccacccg ctaaagtact tggagcggtc tctccctccc 4440
   tcatcagccc accaaaccaa acctagcctc caagagtggg aagaaattaa agcaagatag 4500
   gctattaagt gcagagggag agaaaatgcc tccaacatgt gaggaagtaa tgagagaaat 4560
   catagaattt taaggccatg atttaaggcc atcatggcct taatcttccg cttcctcgct 4620
   cactgactcg ctgcgctcgg tcgttcggct gcggcgagcg gtatcagctc actcaaaggc 4680
   ggtaatacgg ttatccacag aatcagggga taacgcagga aagaacatgt gagcaaaagg 4740
   ccagcaaaag gccaggaacc gtaaaaaggc cgcgttgctg gcgtttttcc ataggctccg 4800
   cccccctgac gagcatcaca aaaatcgacg ctcaagtcag aggtggcgaa acccgacagg 4860
   actataaaga taccaggcgt ttccccctgg aagctccctc gtgcgctctc ctgttccgac 4920
   cctgccgctt accggatacc tgtccgcctt tctcccttcg ggaagcgtgg cgctttctca 4980
   tagctcacgc tgtaggtatc tcagttcggt gtaggtcgtt cgctccaagc tgggctgtgt 5040
   gcacgaaccc cccgttcagc ccgaccgctg cgccttatcc ggtaactatc gtcttgagtc 5100
   caacccggta agacacgact tatcgccact ggcagcagcc actggtaaca ggattagcag 5160
   agcgaggtat gtaggcggtg ctacagagtt cttgaagtgg tggcctaact acggctacac 5220
   tagaagaaca gtatttggta tctgcgctct gctgaagcca gttaccttcg gaaaaagagt 5280
   tggtagctct tgatccggca aacaaaccac cgctggtagc ggtggttttt ttgtttgcaa 5340
   gcagcagatt acgcgcagaa aaaaaggatc tcaagaagat cctttgatct tttctacggg 5400
   gtctgacgct cagtggaacg aaaactcacg ttaagggatt ttggtcatga gattatcaaa 5460
   aaggatcttc acctagatcc ttttaaatta aaaatgaagt tttaaatcaa tctaaagtat 5520
   atatgagtaa acttggtctg acagttacca atgcttaatc agtgaggcac ctatctcagc 5580
   gatctgtcta tttcgttcat ccatagttgc ctgactcggg gggggggggc gctgaggtct 5640
   gcctcgtgaa gaaggtgttg ctgactcata ccaggcctga atcgccccat catccagcca 5700
   gaaagtgagg gagccacggt tgatgagagc tttgttgtag gtggaccagt tggtgatttt 5760
   gaacttttgc tttgccacgg aacggtctgc gttgtcggga agatgcgtga tctgatcctt 5820
   caactcagca aaagttcgat ttattcaaca aagccgccgt cccgtcaagt cagcgtaatg 5880
   ctctgccagt gttacaacca attaaccaat tctgattaga aaaactcatc gagcatcaaa 5940
   tgaaactgca atttattcat atcaggatta tcaataccat atttttgaaa aagccgtttc 6000
   tgtaatgaag gagaaaactc accgaggcag ttccatagga tggcaagatc ctggtatcgg 6060
   tctgcgattc cgactcgtcc aacatcaata caacctatta atttcccctc gtcaaaaata 6120
   aggttatcaa gtgagaaatc accatgagtg acgactgaat ccggtgagaa tggcaaaagc 6180
   ttatgcattt ctttccagac ttgttcaaca ggccagccat tacgctcgtc atcaaaatca 6240
   ctcgcatcaa ccaaaccgtt attcattcgt gattgcgcct gagcgagacg aaatacgcga 6300
   tcgctgttaa aaggacaatt acaaacagga atcgaatgca accggcgcag gaacactgcc 6360
   agcgcatcaa caatattttc acctgaatca ggatattctt ctaatacctg gaatgctgtt 6420
   ttcccgggga tcgcagtggt gagtaaccat gcatcatcag gagtacggat aaaatgcttg 6480
   atggtcggaa gaggcataaa ttccgtcagc cagtttagtc tgaccatctc atctgtaaca 6540
   tcattggcaa cgctaccttt gccatgtttc agaaacaact ctggcgcatc gggcttccca 6600
   tacaatcgat agattgtcgc acctgattgc ccgacattat cgcgagccca tttataccca 6660
   tataaatcag catccatgtt ggaatttaat cgcggcctcg agcaagacgt ttcccgttga 6720
   atatggctca taacacccct tgtattactg tttatgtaag cagacagttt tattgttcat 6780
   gatgatatat ttttatcttg tgcaatgtaa catcagagat tttgagacac aacgtggctt 6840
   tccccccccc cccattattg aagcatttat cagggttatt gtctcatgag cggatacata 6900
   tttgaatgta tttagaaaaa taaacaaata ggggttccgc gcacatttcc ccgaaaagtg 6960
   ccacctgacg tctaagaaac cattattatc atgacattaa cctataaaaa taggcgtatc 7020
   acgaggccct ttcgtc                                                 7036
<210> 18
   <211> 6885
   <212> DNA
   <213> Artificial Sequence
<220>
   <223> pVR1012-GP(IC) delta TM
<400> 18
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga attcggcttc 1920
   taatcacagt caccatggga gcgtcaggga ttctgcaatt gccccgtgag cgcttcagga 1980
   aaacatcttt ctttgtttgg gtaataatcc tattccataa agtcttttca atcccgttgg 2040
   gggttgtaca caacaatacc ctacaagtga gtgatattga caagtttgtg tgccgagaca 2100
   aactctcttc aactagccaa ttgaagtcag tcgggttgaa cttggagggc aatggagtag 2160
   caactgatgt accaacggca accaaaagat ggggttttcg agctggtgtt ccaccaaagg 2220
   tggtaaattg cgaagctgga gaatgggctg agaactgtta taacctggct ataaagaaag 2280
   ttgatggtag tgagtgccta ccagaagccc ctgagggagt gagggatttt ccccgttgcc 2340
   gctatgtaca caaagtctca ggaactggac catgcccagg aggactcgcc tttcacaaag 2400
   aaggagcctt cttcctgtat gaccgactcg catcaacaat catttatcgg ggtacaacct 2460
   ttgccgaagg agttattgca tttctgatct tgcctaaggc gcgaaaggat tttttccagt 2520
   ctcctccatt gcatgagcct gccaacatga ccacggatcc ctccagttac tatcacacga 2580
   caacaataaa ctacgtggtt gataattttg gaaccaacac cacagagttt ctgttccaag 2640
   tcgatcattt gacgtatgtg cagctcgagg caagattcac accacaattc cttgtcctcc 2700
   taaatgaaac catctactct gataaccgca gaagtaacac aacaggaaaa ctaatctgga 2760
   aaataaatcc cactgttgat accagcatgg gtgagtgggc tttctgggaa aataaaaaaa 2820
   acttcacaaa aaccctttca agtgaagagt tgtctttcgt acctgtacca gaaacccaga 2880
   accaggtcct tgacacgaca gcgacggtct ctcctcccat ctccgcccac aaccacgcag 2940
   ccgaagacca caaagaattg gtttcagagg attccactcc agtggttcag atgcaaaaca 3000
   tcaagggaaa ggacacaatg ccaaccacag tgacgggtgt accaacaacc acaccctctc 3060
   catttccaat caatgctcgc aacactgatc ataccaaatc atttatcggc ctggaggggc 3120
   cccaagaaga ccacagcacc acacagcctg ccaagaccac cagccaacca accaacagca 3180
   cagaatcgac gacactaaac ccaacatcag agccctccag tagaggcacg ggaccatcca 3240
   gccccacggt ccccaacacc acagaaagcc acgccgaact tggcaagaca accccaacca 3300
   cactcccaga acagcacact gccgccagtg ccattccaag agccgtgcac cccgacgaac 3360
   tcagtggacc tggcttcctg acgaacacaa tacggggggt tacaaatctc ctgacaggat 3420
   ccagaagaaa gcgaagggat gtcactccca atacacaacc caaatgcaac ccaaacctgc 3480
   actattggac agccttggat gagggtgctg ccataggttt agcctggata ccatacttcg 3540
   ggccagcagc tgagggaatt tacactgaag gcataatgga gaatcaaaat ggattgatct 3600
   gtggattgag gcagctggcc aacgaaacga cacaagctct tcaattgttc ttaagggcaa 3660
   ctactgagtt gcgtacattc tctatactaa atcggaaagc aatagacttc ttgctccaaa 3720
   gatggggagg aacatgtcac attctagggc ctgattgttg cattgaaccc caagattgga 3780
   ccaaaaatat cactgataaa attgatcaaa taatccatga ctttgtcgat aataatcttc 3840
   caaatcagaa tgatggcagg gccgcatcgt gactgactga cgatctgcct cgcggatcca 3900
   gatctgctgt gccttctagt tgccagccat ctgttgtttg cccctccccc gtgccttcct 3960
   tgaccctgga aggtgccact cccactgtcc tttcctaata aaatgaggaa attgcatcgc 4020
   attgtctgag taggtgtcat tctattctgg ggggtggggt ggggcaggac agcaaggggg 4080
   aggattggga agacaatagc aggcatgctg gggatgcggt gggctctatg ggtacccagg 4140
   tgctgaagaa ttgacccggt tcctcctggg ccagaaagaa gcaggcacat ccccttctct 4200
   gtgacacacc ctgtccacgc ccctggttct tagttccagc cccactcata ggacactcat 4260
   agctcaggag ggctccgcct tcaatcccac ccgctaaagt acttggagcg gtctctccct 4320
   ccctcatcag cccaccaaac caaacctagc ctccaagagt gggaagaaat taaagcaaga 4380
   taggctatta agtgcagagg gagagaaaat gcctccaaca tgtgaggaag taatgagaga 4440
   aatcatagaa tttcttccgc ttcctcgctc actgactcgc tgcgctcggt cgttcggctg 4500
   cggcgagcgg tatcagctca ctcaaaggcg gtaatacggt tatccacaga atcaggggat 4560
   aacgcaggaa agaacatgtg agcaaaaggc cagcaaaagg ccaggaaccg taaaaaggcc 4620
   gcgttgctgg cgtttttcca taggctccgc ccccctgacg agcatcacaa aaatcgacgc 4680
   tcaagtcaga ggtggcgaaa cccgacagga ctataaagat accaggcgtt tccccctgga 4740
   agctccctcg tgcgctctcc tgttccgacc ctgccgctta ccggatacct gtccgccttt 4800
   ctcccttcgg gaagcgtggc gctttctcat agctcacgct gtaggtatct cagttcggtg 4860
   taggtcgttc gctccaagct gggctgtgtg cacgaacccc ccgttcagcc cgaccgctgc 4920
   gccttatccg gtaactatcg tcttgagtcc aacccggtaa gacacgactt atcgccactg 4980
   gcagcagcca ctggtaacag gattagcaga gcgaggtatg taggcggtgc tacagagttc 5040
   ttgaagtggt ggcctaacta cggctacact agaagaacag tatttggtat ctgcgctctg 5100
   ctgaagccag ttaccttcgg aaaaagagtt ggtagctctt gatccggcaa acaaaccacc 5160
   gctggtagcg gtggtttttt tgtttgcaag cagcagatta cgcgcagaaa aaaaggatct 5220
   caagaagatc ctttgatctt ttctacgggg tctgacgctc agtggaacga aaactcacgt 5280
   taagggattt tggtcatgag attatcaaaa aggatcttca cctagatcct tttaaattaa 5340
   aaatgaagtt ttaaatcaat ctaaagtata tatgagtaaa cttggtctga cagttaccaa 5400
   tgcttaatca gtgaggcacc tatctcagcg atctgtctat ttcgttcatc catagttgcc 5460
   tgactcgggg ggggggggcg ctgaggtctg cctcgtgaag aaggtgttgc tgactcatac 5520
   caggcctgaa tcgccccatc atccagccag aaagtgaggg agccacggtt gatgagagct 5580
   ttgttgtagg tggaccagtt ggtgattttg aacttttgct ttgccacgga acggtctgcg 5640
   ttgtcgggaa gatgcgtgat ctgatccttc aactcagcaa aagttcgatt tattcaacaa 5700
   agccgccgtc ccgtcaagtc agcgtaatgc tctgccagtg ttacaaccaa ttaaccaatt 5760
   ctgattagaa aaactcatcg agcatcaaat gaaactgcaa tttattcata tcaggattat 5820
   caataccata tttttgaaaa agccgtttct gtaatgaagg agaaaactca ccgaggcagt 5880
   tccataggat ggcaagatcc tggtatcggt ctgcgattcc gactcgtcca acatcaatac 5940
   aacctattaa tttcccctcg tcaaaaataa ggttatcaag tgagaaatca ccatgagtga 6000
   cgactgaatc cggtgagaat ggcaaaagct tatgcatttc tttccagact tgttcaacag 6060
   gccagccatt acgctcgtca tcaaaatcac tcgcatcaac caaaccgtta ttcattcgtg 6120
   attgcgcctg agcgagacga aatacgcgat cgctgttaaa aggacaatta caaacaggaa 6180
   tcgaatgcaa ccggcgcagg aacactgcca gcgcatcaac aatattttca cctgaatcag 6240
   gatattcttc taatacctgg aatgctgttt tcccggggat cgcagtggtg agtaaccatg 6300
   catcatcagg agtacggata aaatgcttga tggtcggaag aggcataaat tccgtcagcc 6360
   agtttagtct gaccatctca tctgtaacat cattggcaac gctacctttg ccatgtttca 6420
   gaaacaactc tggcgcatcg ggcttcccat acaatcgata gattgtcgca cctgattgcc 6480
   cgacattatc gcgagcccat ttatacccat ataaatcagc atccatgttg gaatttaatc 6540
   gcggcctcga gcaagacgtt tcccgttgaa tatggctcat aacacccctt gtattactgt 6600
   ttatgtaagc agacagtttt attgttcatg atgatatatt tttatcttgt gcaatgtaac 6660
   atcagagatt ttgagacaca acgtggcttt cccccccccc ccattattga agcatttatc 6720
   agggttattg tctcatgagc ggatacatat ttgaatgtat ttagaaaaat aaacaaatag 6780
   gggttccgcg cacatttccc cgaaaagtgc cacctgacgt ctaagaaacc attattatca 6840
   tgacattaac ctataaaaat aggcgtatca cgaggccctt tcgtc                 6885
<210> 19
   <211> 6889
   <212> DNA
   <213> Artificial Sequence
<220>
   <223> pVR1012x/s Ebola GP(IC)(dTM)
<400> 19
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gataattctc taatcacagt catcatggga 1920
   gcgtcaggga ttctgcaatt gccccgtgag cgcttcagga aaacatcttt ctttgtttgg 1980
   gtaataatcc tattccataa agtcttttca atcccgttgg gggttgtaca caacaatacc 2040
   ctacaagtga gtgatattga caagtttgtg tgccgagaca aactctcttc aactagccaa 2100
   ttgaagtcag tcgggttgaa cttggagggc aatggagtag caactgatgt accaacggca 2160
   accaaaagat ggggttttcg agctggtgtt ccaccaaagg tggtaaatta cgaagctgga 2220
   gaatgggctg agaactgtta taacctggct ataaagaaag ttgatggtag tgagtgccta 2280
   ccagaagccc ctgagggagt gagggatttt ccccgttgcc gctatgtaca caaagtctca 2340
   ggaactggac catgcccagg aggactcgcc tttcacaaag aaggagcctt cttcctgtat 2400
   gaccgactcg catcaacaat catttatcgg ggtacaacct ttgccgaagg agttattgca 2460
   tttctgatct tgcctaaggc gcgaaaggat tttttccagt ctcctccatt gcatgagcct 2520
   gccaacatga ccacggatcc ctccagttac tatcacacga caacaataaa ctacgtggtt 2580
   gataattttg gaaccaacac cacagagttt ctgttccaag tcgatcattt gacgtatgtg 2640
   cagctcgagg caagattcac accacaattc cttgtcctcc taaatgaaac catctactct 2700
   gataaccgca gaagtaacac aacaggaaaa ctaatctgga aaataaatcc cactgttgat 2760
   accagcatgg gtgagtgggc tttctgggaa aataaaaaaa cttcacaaaa accctttcaa 2820
   gtgaagagtt gtctttcgta cctgtaccag aaacccagaa ccaggtcctt gacacgacag 2880
   cgacggtctc tcctcccatc tccgcccaca accacgcagg cgaagaccac aaagaattgg 2940
   tttcagagga ttccactcca gtggttcaga tgcaaaacat caagggaaag gacacaatgc 3000
   caaccacagt gacgggtgta ccaacaacca caccctctcc atttccaatc aatgctcgca 3060
   acactgatca taccaaatca tttatcggcc tggaggggcc ccaagaagac cacagcacca 3120
   cacagcctgc caagaccacc agccaaccaa ccaacagcac agaatcgacg acactaaacc 3180
   caacatcaga gccctccagt agaggcacgg gaccatccag ccccacggtc cccaacacca 3240
   cagaaagcca cgccgaactt ggcaagacaa ccccaaccac actcccagaa cagcacactg 3300
   ccgccagtgc cattccaaga gccgtgcacc ccgacgaact cagtggacct ggcttcctga 3360
   cgaacacaat acggggggtg acaaatctcc tgacaggatc cagaagaaag cgaagggatg 3420
   tcactcccaa tacacaaccc aaatgcaacc caaacctgca ctattggaca gccttggatg 3480
   agggtgctgc cataggttta gcctggatac catacttcgg gccagcagct gagggaattt 3540
   acactgaagg cataatggag aatcaaaatg gattgatctg tggattgagg cagctggcca 3600
   acgaaacgac acaagctctt caattgttct taagggcaac tactgagttg cgtacattct 3660
   ctatactaaa tcggaaagca atagacttct tgctccaaag atggggagga acatgtcaca 3720
   ttctagggcc tgattgttgc attgaacccc aagattggac caaaaatatc actgataaaa 3780
   ttgatcaaat aatccatgac tttgtcgata ataatcttcc aaatcagaat gatggcagca 3840
   actggtggac tggatggaaa caatggtgaa gatctgctgt gccttctagt tgccagccat 3900
   ctgttgtttg cccctccccc gtgccttcct tgaccctgga aggtgccact cccactgtcc 3960
   tttcctaata aaatgaggaa attgcatcgc attgtctgag taggtgtcat tctattctgg 4020
   ggggtggggt ggggcaggac agcaaggggg aggattggga agacaatagc aggcatgctg 4080
   gggatgcggt gggctctatg ggtacccagg tgctgaagaa ttgacccggt tcctcctggg 4140
   ccagaaagaa gcaggcacat ccccttctct gtgacacacc ctgtccacgc ccctggttct 4200
   tagttccagc cccactcata ggacactcat agctcaggag ggctccgcct tcaatcccac 4260
   ccgctaaagt acttggagcg gtctctccct ccctcatcag cccaccaaac caaacctagc 4320
   ctccaagagt gggaagaaat taaagcaaga taggctatta agtgcagagg gagagaaaat 4380
   gcctccaaca tgtgaggaag taatgagaga aatcatagaa ttttaaggcc atgatttaag 4440
   gccatcatgg ccttaatctt ccgcttcctc gctcactgac tcgctgcgct cggtcgttcg 4500
   gctgcggcga gcggtatcag ctcactcaaa ggcggtaata cggttatcca cagaatcagg 4560
   ggataacgca ggaaagaaca tgtgagcaaa aggccagcaa aaggccagga accgtaaaaa 4620
   ggccgcgttg ctggcgtttt tccataggct ccgcccccct gacgagcatc acaaaaatcg 4680
   acgctcaagt cagaggtggc gaaacccgac aggactataa agataccagg cgtttccccc 4740
   tggaagctcc ctcgtgcgct ctcctgttcc gaccctgccg cttaccggat acctgtccgc 4800
   ctttctccct tcgggaagcg tggcgctttc tcatagctca cgctgtaggt atctcagttc 4860
   ggtgtaggtc gttcgctcca agctgggctg tgtgcacgaa ccccccgttc agcccgaccg 4920
   ctgcgcctta tccggtaact atcgtcttga gtccaacccg gtaagacacg acttatcgcc 4980
   actggcagca gccactggta acaggattag cagagcgagg tatgtaggcg gtgctacaga 5040
   gttcttgaag tggtggccta actacggcta cactagaaga acagtatttg gtatctgcgc 5100
   tctgctgaag ccagttacct tcggaaaaag agttggtagc tcttgatccg gcaaacaaac 5160
   caccgctggt agcggtggtt tttttgtttg caagcagcag attacgcgca gaaaaaaagg 5220
   atctcaagaa gatcctttga tcttttctac ggggtctgac gctcagtgga acgaaaactc 5280
   acgttaaggg attttggtca tgagattatc aaaaaggatc ttcacctaga tccttttaaa 5340
   ttaaaaatga agttttaaat caatctaaag tatatatgag taaacttggt ctgacagtta 5400
   ccaatgctta atcagtgagg cacctatctc agcgatctgt ctatttcgtt catccatagt 5460
   tgcctgactc gggggggggg ggcgctgagg tctgcctcgt gaagaaggtg ttgctgactc 5520
   ataccaggcc tgaatcgccc catcatccag ccagaaagtg agggagccac ggttgatgag 5580
   agctttgttg taggtggacc agttggtgat tttgaacttt tgctttgcca cggaacggtc 5640
   tgcgttgtcg ggaagatgcg tgatctgatc cttcaactca gcaaaagttc gatttattca 5700
   acaaagccgc cgtcccgtca agtcagcgta atgctctgcc agtgttacaa ccaattaacc 5760
   aattctgatt agaaaaactc atcgagcatc aaatgaaact gcaatttatt catatcagga 5820
   ttatcaatac catatttttg aaaaagccgt ttctgtaatg aaggagaaaa ctcaccgagg 5880
   cagttccata ggatggcaag atcctggtat cggtctgcga ttccgactcg tccaacatca 5940
   atacaaccta ttaatttccc ctcgtcaaaa ataaggttat caagtgagaa atcaccatga 6000
   gtgacgactg aatccggtga gaatggcaaa agcttatgca tttctttcca gacttgttca 6060
   acaggccagc cattacgctc gtcatcaaaa tcactcgcat caaccaaacc gttattcatt 6120
   cgtgattgcg cctgagcgag acgaaatacg cgatcgctgt taaaaggaca attacaaaca 6180
   ggaatcgaat gcaaccggcg caggaacact gccagcgcat caacaatatt ttcacctgaa 6240
   tcaggatatt cttctaatac ctggaatgct gttttcccgg ggatcgcagt ggtgagtaac 6300
   catgcatcat caggagtacg gataaaatgc ttgatggtcg gaagaggcat aaattccgtc 6360
   agccagttta gtctgaccat ctcatctgta acatcattgg caacgctacc tttgccatgt 6420
   ttcagaaaca actctggcgc atcgggcttc ccatacaatc gatagattgt cgcacctgat 6480
   tgcccgacat tatcgcgagc ccatttatac ccatataaat cagcatccat gttggaattt 6540
   aatcgcggcc tcgagcaaga cgtttcccgt tgaatatggc tcataacacc ccttgtatta 6600
   ctgtttatgt aagcagacag ttttattgtt catgatgata tatttttatc ttgtgcaatg 6660
   taacatcaga gattttgaga cacaacgtgg ctttcccccc ccccccatta ttgaagcatt 6720
   tatcagggtt attgtctcat gagcggatac atatttgaat gtatttagaa aaataaacaa 6780
   ataggggttc cgcgcacatt tccccgaaaa gtgccacctg acgtctaaga aaccattatt 6840
   atcatgacat taacctataa aaataggcgt atcacgaggc cctttcgtc 6889
<210> 20
   <211> 8146
   <212> DNA
   <213> Artificial Sequence
<220>
   <223> pAdApt EbolaGP(IC)(dTM)
<400> 20
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat aattctctaa tcacagtcat catgggagcg 1320
   tcagggattc tgcaattgcc ccgtgagcgc ttcaggaaaa catctttctt tgtttgggta 1380
   ataatcctat tccataaagt cttttcaatc ccgttggggg ttgtacacaa caatacccta 1440
   caagtgagtg atattgacaa gtttgtgtgc cgagacaaac tctcttcaac tagccaattg 1500
   aagtcagtcg ggttgaactt ggagggcaat ggagtagcaa ctgatgtacc aacggcaacc 1560
   aaaagatggg gttttcgagc tggtgttcca ccaaaggtgg taaattacga agctggagaa 1620
   tgggctgaga actgttataa cctggctata aagaaagttg atggtagtga gtgcctacca 1680
   gaagcccctg agggagtgag ggattttccc cgttgccgct atgtacacaa agtctcagga 1740
   actggaccat gcccaggagg actcgccttt cacaaagaag gagccttctt cctgtatgac 1800
   cgactcgcat caacaatcat ttatcggggt acaacctttg ccgaaggagt tattgcattt 1860
   ctgatcttgc ctaaggcgcg aaaggatttt ttccagtctc ctccattgca tgagcctgcc 1920
   aacatgacca cggatccctc cagttactat cacacgacaa caataaacta cgtggttgat 1980
   aattttggaa ccaacaccac agagtttctg ttccaagtcg atcatttgac gtatgtgcag 2040
   ctcgaggcaa gattcacacc acaattcctt gtcctcctaa atgaaaccat ctactctgat 2100
   aaccgcagaa gtaacacaac aggaaaacta atctggaaaa taaatcccac tgttgatacc 2160
   agcatgggtg agtgggcttt ctgggaaaat aaaaaaactt cacaaaaacc ctttcaagtg 2220
   aagagttgtc tttcgtacct gtaccagaaa cccagaacca ggtccttgac acgacagcga 2280
   cggtctctcc tcccatctcc gcccacaacc acgcaggcga agaccacaaa gaattggttt 2340
   cagaggattc cactccagtg gttcagatgc aaaacatcaa gggaaaggac acaatgccaa 2400
   ccacagtgac gggtgtacca acaaccacac cctctccatt tccaatcaat gctcgcaaca 2460
   ctgatcatac caaatcattt atcggcctgg aggggcccca agaagaccac agcaccacac 2520
   agcctgccaa gaccaccagc caaccaacca acagcacaga atcgacgaca ctaaacccaa 2580
   catcagagcc ctccagtaga ggcacgggac catccagccc cacggtcccc aacaccacag 2640
   aaagccacgc cgaacttggc aagacaaccc caaccacact cccagaacag cacactgccg 2700
   ccagtgccat tccaagagcc gtgcaccccg acgaactcag tggacctggc ttcctgacga 2760
   acacaatacg gggggtgaca aatctcctga caggatccag aagaaagcga agggatgtca 2820
   ctcccaatac acaacccaaa tgcaacccaa acctgcacta ttggacagcc ttggatgagg 2880
   gtgctgccat aggtttagcc tggataccat acttcgggcc agcagctgag ggaatttaca 2940
   ctgaaggcat aatggagaat caaaatggat tgatctgtgg attgaggcag ctggccaacg 3000
   aaacgacaca agctcttcaa ttgttcttaa gggcaactac tgagttgcgt acattctcta 3060
   tactaaatcg gaaagcaata gacttcttgc tccaaagatg gggaggaaca tgtcacattc 3120
   tagggcctga ttgttgcatt gaaccccaag attggaccaa aaatatcact gataaaattg 3180
   atcaaataat ccatgacttt gtcgataata atcttccaaa tcagaatgat ggcagcaact 3240
   ggtggactgg atggaaacaa tggtgaagat ccagatctgc tgtgccttct agttgccagc 3300
   catctgttgt ttgcccctcc cccgtgcctt ccttgaccct ggaaggtgcc actcccactg 3360
   tcctttccta ataaaatgag gaaattgcat cgcattgtct gagtaggtgt cattctattc 3420
   tggggggtgg ggtggggcag cacagcaagg gggaggattg ggaagacaat agcaggcatg 3480
   ctggggatgc ggtgggctct atgggtaccc agggccgcat aacttcgtat aatgtatgct 3540
   atacgaagtt ataagatctg tactgaaatg tgtgggcgtg gcttaagggt gggaaagaat 3600
   atataaggtg ggggtcttat gtagttttgt atctgttttg cagcagccgc cgccgccatg 3660
   agcaccaact cgtttgatgg aagcattgtg agctcatatt tgacaacgcg catgccccca 3720
   tgggccgggg tgcgtcagaa tgtgatgggc tccagcattg atggtcgccc cgtcctgccc 3780
   gcaaactcta ctaccttgac ctacgagacc gtgtctggaa cgccgttgga gactgcagcc 3840
   tccgccgccg cttcagccgc tgcagccacc gcccgcggga ttgtgactga ctttgctttc 3900
   ctgagcccgc ttgcaagcag tgcagcttcc cgttcatccg cccgcgatga caagttgacg 3960
   gctcttttgg cacaattgga ttctttgacc cgggaactta atgtcgtttc tcagcagctg 4020
   ttggatctgc gccagcaggt ttctgccctg aaggcttcct cccctcccaa tgcggtttaa 4080
   aacataaata aaaaaccaga ctctgtttgg atttggatca agcaagtgtc ttgctgtctt 4140
   tatttagggg ttttgcgcgc gcggtaggcc cgggaccagc ggtctcggtc gttgagggtc 4200
   ctgtgtattt tttccaggac gtggtaaagg tgactctgga tgttcagata catgggcata 4260
   agcccgtctc tggggtggag gtagcaccac tgcagagctt catgctgcgg ggtggtgttg 4320
   tagatgatcc agtcgtagca ggagcgctgg gcgtggtgcc taaaaatgtc tttcagtagc 4380
   aagctgattg ccaggggcag gcccttggtg taagtgttta caaagcggtt aagctgggat 4440
   gggtgcatac gtggggatat gagatgcatc ttggactgta tttttaggtt ggctatgttc 4500
   ccagccatat ccctccgggg attcatgttg tgcagaacca ccagcacagt gtatccggtg 4560
   cacttgggaa atttgtcatg tagcttagaa ggaaatgcgt ggaagaactt ggagacgccc 4620
   ttgtgacctc caagattttc catgcattcg tccataatga tggcaatggg cccacgggcg 4680
   gcggcctggg cgaagatatt tctgggatca ctaacgtcat agttgtgttc caggatgaga 4740
   tcgtcatagg ccatttttac aaagcgcggg cggagggtgc cagactgcgg tataatggtt 4800
   ccatccggcc caggggcgta gttaccctca cagatttgca tttcccacgc tttgagttca 4860
   gatgggggga tcatgtctac ctgcggggcg atgaagaaaa cggtttccgg ggtaggggag 4920
   atcagctggg aagaaagcag gttcctgagc agctgcgact taccgcagcc ggtgggcccg 4980
   taaatcacac ctattaccgg ctgcaactgg tagttaagag agctgcagct gccgtcatcc 5040
   ctgagcaggg gggccacttc gttaagcatg tccctgactc gcatgttttc cctgaccaaa 5100
   tccgccagaa ggcgctcgcc gcccagcgat agcagttctt gcaaggaagc aaagtttttc 5160
   aacggtttga gaccgtccgc cgtaggcatg cttttgagcg tttgaccaag cagttccagg 5220
   cggtcccaca gctcggtcac ctgctctacg gcatctcgat ccagcatatc tcctcgtttc 5280
   gcgggttggg gcggctttcg ctgtacggca gtagtcggtg ctcgtccaga cgggccaggg 5340
   tcatgtcttt ccacgggcgc agggtcctcg tcagcgtagt ctgggtcacg gtgaaggggt 5400
   gcgctccggg ctgcgcgctg gccagggtgc gcttgaggct ggtcctgctg gtgctgaagc 5460
   gctgccggtc ttcgccctgc gcgtcggcca ggtagcattt gaccatggtg tcatagtcca 5520
   gcccctccgc ggcgtggccc ttggcgcgca gcttgccctt ggaggaggcg ccgcacgagg 5580
   ggcagtgcag acttttgagg gcgtagagct tgggcgcgag aaataccgat tccggggagt 5640
   aggcatccgc gccgcaggcc ccgcagacgg tctcgcattc cacgagccag gtgagctctg 5700
   gccgttcggg gtcaaaaacc aggtttcccc catgcttttt gatgcgtttc ttacctctgg 5760
   tttccatgag ccggtgtcca cgctcggtga cgaaaaggct gtccgtgtcc ccgtatacag 5820
   acttgagagg cctgtcctcg agcggtgttc cgcggtcctc ctcgtataga aactcggacc 5880
   actctgagac aaaggctcgc gtccaggcca gcacgaagga ggctaagtgg gaggggtagc 5940
   ggtcgttgtc cactaggggg tccactcgct ccagggtgtg aagacacatg tcgccctctt 6000
   cggcatcaag gaaggtgatt ggtttgtagg tgtaggccac gtgaccgggt gttcctgaag 6060
   gggggctata aaagggggtg ggggcgcgtt cgtcctcact ctcttccgca tcgctgtctg 6120
   cgagggccag ctgttggggt gagtcgacgc gaggctggat ggccttcccc attatgattc 6180
   ttctcgcttc cggcggcatc gggatgcccg cgttgcaggc catgctgtcc aggcaggtag 6240
   atgacgacca tcagggacag cttcaaggcc agcaaaaggc caggaaccgt aaaaaggccg 6300
   cgttgctggc gtttttccat aggctccgcc cccctgacga gcatcacaaa aatcgacgct 6360
   caagtcagag gtggcgaaac ccgacaggac tataaagata ccaggcgttt ccccctggaa 6420
   gctccctcgt gcgctctcct gttccgaccc tgccgcttac cggatacctg tccgcctttc 6480
   tcccttcggg aagcgtggcg ctttctcata gctcacgctg taggtatctc agttcggtgt 6540
   aggtcgttcg ctccaagctg ggctgtgtgc acgaaccccc cgttcagccc gaccgctgcg 6600
   ccttatccgg taactatcgt cttgagtcca acccggtaag acacgactta tcgccactgg 6660
   cagcagccac tggtaacagg attagcagag cgaggtatgt aggcggtgct acagagttct 6720
   tgaagtggtg gcctaactac ggctacacta gaaggacagt atttggtatc tgcgctctgc 6780
   tgaagccagt taccttcgga aaaagagttg gtagctcttg atccggcaaa caaaccaccg 6840
   ctggtagcgg tggttttttt gtttgcaagc agcagattac gcgcagaaaa aaaggatctc 6900
   aagaagatcc tttgatcttt tctacggggt ctgacgctca gtggaacgaa aactcacgtt 6960
   aagggatttt ggtcatgaga ttatcaaaaa ggatcttcac ctagatcctt ttaaattaaa 7020
   aatgaagttt taaatcaatc taaagtatat atgagtaaac ttggtctgac agttaccaat 7080
   gcttaatcag tgaggcacct atctcagcga tctgtctatt tcgttcatcc atagttgcct 7140
   gactccccgt cgtgtagata actacgatac gggagggctt accatctggc cccagtgctg 7200
   caatgatacc gcgagaccca cgctcaccgg ctccagattt atcagcaata aaccagccag 7260
   ccggaagggc cgagcgcaga agtggtcctg caactttatc cgcctccatc cagtctatta 7320
   attgttgccg ggaagctaga gtaagtagtt cgccagttaa tagtttgcgc aacgttgttg 7380
   ccattgctgc aggcatcgtg gtgtcacgct cgtcgtttgg tatggcttca ttcagctccg 7440
   gttcccaacg atcaaggcga gttacatgat cccccatgtt gtgcaaaaaa gcggttagct 7500
   ccttcggtcc tccgatcgtt gtcagaagta agttggccgc agtgttatca ctcatggtta 7560
   tggcagcact gcataattct cttactgtca tgccatccgt aagatgcttt tctgtgactg 7620
   gtgagtactc aaccaagtca ttctgagaat agtgtatgcg gcgaccgagt tgctcttgcc 7680
   cggcgtcaac acgggataat accgcgccac atagcagaac tttaaaagtg ctcatcattg 7740
   gaaaacgttc ttcggggcga aaactctcaa ggatcttacc gctgttgaga tccagttcga 7800
   tgtaacccac tcgtgcaccc aactgatctt cagcatcttt tactttcacc agcgtttctg 7860
   ggtgagcaaa aacaggaagg caaaatgccg caaaaaaggg aataagggcg acacggaaat 7920
   gttgaatact catactcttc ctttttcaat attattgaag catttatcag ggttattgtc 7980
   tcatgagcgg atacatattt gaatgtattt agaaaaataa acaaataggg gttccgcgca 8040
   catttccccg aaaagtgcca cctgacgtct aagaaaccat tattatcatg acattaacct 8100
   ataaaaatag gcgtatcacg aggccctttc gtcttcaaga attgtt                8146
<210> 21
   <211> 7023
   <212> DNA
   <213> Artificial Sequence
<220>
   <223> pVR1012x/s-SGP(IC)
<400> 21
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga attcggcttc 1920
   taatcacagt caccatggga gcgtcaggga ttctgcaatt gccccgtgag cgcttcagga 1980
   aaacatcttt ctttgtttgg gtaataatcc tattccataa agtcttttca atcccgttgg 2040
   gggttgtaca caacaatacc ctacaagtga gtgatattga caagtttgtg tgccgagaca 2100
   aactctcttc aactagccaa ttgaagtcag tcgggttgaa cttggagggc aatggagtag 2160
   caactgatgt accaacggca accaaaagat ggggttttcg agctggtgtt ccaccaaagg 2220
   tggtaaattg cgaagctgga gaatgggctg agaactgtta taacctggct ataaagaaag 2280
   ttgatggtag tgagtgccta ccagaagccc ctgagggagt gagggatttt ccccgttgcc 2340
   gctatgtaca caaagtctca ggaactggac catgcccagg aggactcgcc tttcacaaag 2400
   aaggagcctt cttcctgtat gaccgactcg catcaacaat catttatcgg ggtacaacct 2460
   ttgccgaagg agttattgca tttctgatct tgcctaaggc gcgaaaggat tttttccagt 2520
   ctcctccatt gcatgagcct gccaacatga ccacggatcc ctccagttac tatcacacga 2580
   caacaataaa ctacgtggtt gataattttg gaaccaacac cacagagttt ctgttccaag 2640
   tcgatcattt gacgtatgtg cagctcgagg caagattcac accacaattc cttgtcctcc 2700
   taaatgaaac catctactct gataaccgca gaagtaacac aacaggaaaa ctaatctgga 2760
   aaataaatcc cactgttgat accagcatgg gtgagtgggc tttctgggaa aataaaaaaa 2820
   acttcacaaa aaccctttca agtgaagagt tgtctttcgt acctgtacca gaaacccaga 2880
   accaggtcct tgacacgaca gcgacggtct ctcctcccat ctccgcccac aaccacgcag 2940
   ccgaagacca caaagaattg gtttcagagg attccactcc agtggttcag atgcaaaaca 3000
   tcaagggaaa ggacacaatg ccaaccacag tgacgggtgt accaacaacc acaccctctc 3060
   catttccaat caatgctcgc aacactgatc ataccaaatc atttatcggc ctggaggggc 3120
   cccaagaaga ccacagcacc acacagcctg ccaagaccac cagccaacca accaacagca 3180
   cagaatcgac gacactaaac ccaacatcag agccctccag tagaggcacg ggaccatcca 3240
   gccccacggt ccccaacacc acagaaagcc acgccgaact tggcaagaca accccaacca 3300
   cactcccaga acagcacact gccgccagtg ccattccaag agccgtgcac cccgacgaac 3360
   tcagtggacc tggcttcctg acgaacacaa tacggggggt tacaaatctc ctgacaggat 3420
   ccagaagaaa gcgaagggat gtcactccca atacacaacc caaatgcaac ccaaacctgc 3480
   actattggac agccttggat gagggtgctg ccataggttt agcctggata ccatacttcg 3540
   ggccagcagc tgagggaatt tacactgaag gcataatgga gaatcaaaat ggattgatct 3600
   gtggattgag gcagctggcc aacgaaacga cacaagctct tcaattgttc ttaagggcaa 3660
   ctactgagtt gcgtacattc tctatactaa atcggaaagc aatagacttc ttgctccaaa 3720
   gatggggagg aacatgtcac attctagggc ctgattgttg cattgaaccc caagattgga 3780
   ccaaaaatat cactgataaa attgatcaaa taatccatga ctttgtcgat aataatcttc 3840
   caaatcagaa tgatggcagc aactggtgga ctggatggaa acaatgggtt cctgctggaa 3900
   taggaatcac aggagtaatc attgctatta ttgctttgct gtgcatttgc aaattcatgc 3960
   tttgaactaa tatagcatca tactttaagc cgaattctag accaggccct ggatccagat 4020
   ctgctgtgcc ttctagttgc cagccatctg ttgtttgccc ctcccccgtg ccttccttga 4080
   ccctggaagg tgccactccc actgtccttt cctaataaaa tgaggaaatt gcatcgcatt 4140
   gtctgagtag gtgtcattct attctggggg gtggggtggg gcaggacagc aagggggagg 4200
   attgggaaga caatagcagg catgctgggg atgcggtggg ctctatgggt acccaggtgc 4260
   tgaagaattg acccggttcc tcctgggcca gaaagaagca ggcacatccc cttctctgtg 4320
   acacaccctg tccacgcccc tggttcttag ttccagcccc actcatagga cactcatagc 4380
   tcaggagggc tccgccttca atcccacccg ctaaagtact tggagcggtc tctccctccc 4440
   tcatcagccc accaaaccaa acctagcctc caagagtggg aagaaattaa agcaagatag 4500
   gctattaagt gcagagggag agaaaatgcc tccaacatgt gaggaagtaa tgagagaaat 4560
   catagaattt taaggccatc atggccttaa tcttccgctt cctcgctcac tgactcgctg 4620
   cgctcggtcg ttcggctgcg gcgagcggta tcagctcact caaaggcggt aatacggtta 4680
   tccacagaat caggggataa cgcaggaaag aacatgtgag caaaaggcca gcaaaaggcc 4740
   aggaaccgta aaaaggccgc gttgctggcg tttttccata ggctccgccc ccctgacgag 4800
   catcacaaaa atcgacgctc aagtcagagg tggcgaaacc cgacaggact ataaagatac 4860
   caggcgtttc cccctggaag ctccctcgtg cgctctcctg ttccgaccct gccgcttacc 4920
   ggatacctgt ccgcctttct cccttcggga agcgtggcgc tttctcatag ctcacgctgt 4980
   aggtatctca gttcggtgta ggtcgttcgc tccaagctgg gctgtgtgca cgaacccccc 5040
   gttcagcccg accgctgcgc cttatccggt aactatcgtc ttgagtccaa cccggtaaga 5100
   cacgacttat cgccactggc agcagccact ggtaacagga ttagcagagc gaggtatgta 5160
   ggcggtgcta cagagttctt gaagtggtgg cctaactacg gctacactag aagaacagta 5220
   tttggtatct gcgctctgct gaagccagtt accttcggaa aaagagttgg tagctcttga 5280
   tccggcaaac aaaccaccgc tggtagcggt ggtttttttg tttgcaagca gcagattacg 5340
   cgcagaaaaa aaggatctca agaagatcct ttgatctttt ctacggggtc tgacgctcag 5400
   tggaacgaaa actcacgtta agggattttg gtcatgagat tatcaaaaag gatcttcacc 5460
   tagatccttt taaattaaaa atgaagtttt aaatcaatct aaagtatata tgagtaaact 5520
   tggtctgaca gttaccaatg cttaatcagt gaggcaccta tctcagcgat ctgtctattt 5580
   cgttcatcca tagttgcctg actcgggggg ggggggcgct gaggtctgcc tcgtgaagaa 5640
   ggtgttgctg actcatacca ggcctgaatc gccccatcat ccagccagaa agtgagggag 5700
   ccacggttga tgagagcttt gttgtaggtg gaccagttgg tgattttgaa cttttgcttt 5760
   gccacggaac ggtctgcgtt gtcgggaaga tgcgtgatct gatccttcaa ctcagcaaaa 5820
   gttcgattta ttcaacaaag ccgccgtccc gtcaagtcag cgtaatgctc tgccagtgtt 5880
   acaaccaatt aaccaattct gattagaaaa actcatcgag catcaaatga aactgcaatt 5940
   tattcatatc aggattatca ataccatatt tttgaaaaag ccgtttctgt aatgaaggag 6000
   aaaactcacc gaggcagttc cataggatgg caagatcctg gtatcggtct gcgattccga 6060
   ctcgtccaac atcaatacaa cctattaatt tcccctcgtc aaaaataagg ttatcaagtg 6120
   agaaatcacc atgagtgacg actgaatccg gtgagaatgg caaaagctta tgcatttctt 6180
   tccagacttg ttcaacaggc cagccattac gctcgtcatc aaaatcactc gcatcaacca 6240
   aaccgttatt cattcgtgat tgcgcctgag cgagacgaaa tacgcgatcg ctgttaaaag 6300
   gacaattaca aacaggaatc gaatgcaacc ggcgcaggaa cactgccagc gcatcaacaa 6360
   tattttcacc tgaatcagga tattcttcta atacctggaa tgctgttttc ccggggatcg 6420
   cagtggtgag taaccatgca tcatcaggag tacggataaa atgcttgatg gtcggaagag 6480
   gcataaattc cgtcagccag tttagtctga ccatctcatc tgtaacatca ttggcaacgc 6540
   tacctttgcc atgtttcaga aacaactctg gcgcatcggg cttcccatac aatcgataga 6600
   ttgtcgcacc tgattgcccg acattatcgc gagcccattt atacccatat aaatcagcat 6660
   ccatgttgga atttaatcgc ggcctcgagc aagacgtttc ccgttgaata tggctcataa 6720
   caccccttgt attactgttt atgtaagcag acagttttat tgttcatgat gatatatttt 6780
   tatcttgtgc aatgtaacat cagagatttt gagacacaac gtggctttcc cccccccccc 6840
   attattgaag catttatcag ggttattgtc tcatgagcgg atacatattt gaatgtattt 6900
   agaaaaataa acaaataggg gttccgcgca catttccccg aaaagtgcca cctgacgtct 6960
   aagaaaccat tattatcatg acattaacct ataaaaatag gcgtatcacg aggccctttc 7020
   gtc                                                               7023
<210> 22
   <211> 7295
   <212> DNA
   <213> Artificial Sequence
<220>
   <223> pVR1012-NP
<400> 22
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatccagatc gatccgagta tggattctcg tcctcagaaa atctggatgg cgccgagtct 1980
   cactgaatct gacatggatt accacaagat cttgacagca ggtctgtccg ttcaacaggg 2040
   gattgttcgg caaagagtca tcccagtgta tcaagtaaac aatcttgaag aaatttgcca 2100
   acttatcata caggcctttg aagcaggtgt tgattttcaa gagagtgcgg acagtttcct 2160
   tctcatgctt tgtcttcatc atgcgtacca gggagattac aaacttttct tggaaagtgg 2220
   cgcagtcaag tatttggaag ggcacgggtt ccgttttgaa gtcaagaagc gtgatggagt 2280
   gaagcgcctt gaggaattgc tgccagcagt atctagtgga aaaaacatta agagaacact 2340
   tgctgccatg ccggaagagg agacaactga agctaatgcc ggtcagtttc tctcctttgc 2400
   aagtctattc cttccgaaat tggtagtagg agaaaaggct tgccttgaga aggttcaaag 2460
   gcaaattcaa gtacatgcag agcaaggact gatacaatat ccaacagctt ggcaatcagt 2520
   aggacacatg atggtgattt tccgtttgat gcgaacaaat tttctgatca aatttctcct 2580
   aatacaccaa gggatgcaca tggttgccgg gcatgatgcc aacgatgctg tgatttcaaa 2640
   ttcagtggct caagctcgtt tttcaggctt attgattgtc aaaacagtac ttgatcatat 2700
   cctacaaaag acagaacgag gagttcgtct ccatcctctt gcaaggaccg ccaaggtaaa 2760
   aaatgaggtg aactccttta aggctgcact cagctccctg gccaagcatg gagagtatgc 2820
   tcctttcgcc cgacttttga acctttctgg agtaaataat cttgagcatg gtcttttccc 2880
   tcaactatcg gcaattgcac tcggagtcgc cacagcacac gggagtaccc tcgcaggagt 2940
   aaatgttgga gaacagtatc aacaactcag agaggctgcc actgaggctg agaagcaact 3000
   ccaacaatat gcagagtctc gcgaacttga ccatcttgga cttgatgatc aggaaaagaa 3060
   aattcttatg aacttccatc agaaaaagaa cgaaatcagc ttccagcaaa caaacgctat 3120
   ggtaactcta agaaaagagc gcctggccaa gctgacagaa gctatcactg ctgcgtcact 3180
   gcccaaaaca agtggacatt acgatgatga tgacgacatt ccctttccag gacccatcaa 3240
   tgatgacgac aatcctggcc atcaagatga tgatccgact gactcacagg atacgaccat 3300
   tcccgatgtg gtggttgatc ccgatgatgg aagctacggc gaataccaga gttactcgga 3360
   aaacggcatg aatgcaccag atgacttggt cctattcgat ctagacgagg acgacgagga 3420
   cactaagcca gtgcctaata gatcgaccaa gggtggacaa cagaagaaca gtcaaaaggg 3480
   ccagcatata gagggcagac agacacaatc caggccaatt caaaatgtcc caggccctca 3540
   cagaacaatc caccacgcca gtgcgccact cacggacaat gacagaagaa atgaaccctc 3600
   cggctcaacc agccctcgca tgctgacacc aattaacgaa gaggcagacc cactggacga 3660
   tgccgacgac gagacgtcta gccttccgcc cttggagtca gatgatgaag agcaggacag 3720
   ggacggaact tccaaccgca cacccactgt cgccccaccg gctcccgtat acagagatca 3780
   ctctgaaaag aaagaactcc cgcaagacga gcaacaagat caggaccaca ctcaagaggc 3840
   caggaaccag gacagtgaca acacccagtc agaacactct tttgaggaga tgtatcgcca 3900
   cattctaaga tcacaggggc catttgatgc tgttttgtat tatcatatga tgaaggatga 3960
   gcctgtagtt ttcagtacca gtgatggcaa agagtacacg tatccagact cccttgaaga 4020
   ggaatatcca ccatggctca ctgaaaaaga ggctatgaat gaagagaata gatttgttac 4080
   attggatggt caacaatttt attggccggt gatgaatcac aagaataaat tcatggcaat 4140
   cctgcaacat catcagtgaa tgagcatgga acaatgggat gattcaaccg acaaatagct 4200
   aacattaagt agtcaaggaa cgaaaacagg aagaattttt gatgtctaag gtgtgaatta 4260
   ttatcacaat aaaagtgatt cttatttttg aatttgggcg agctcgaatt gatctgctgt 4320
   gccttctagt tgccagccat ctgttgtttg cccctccccc gtgccttcct tgaccctgga 4380
   aggtgccact cccactgtcc tttcctaata aaatgaggaa attgcatcgc attgtctgag 4440
   taggtgtcat tctattctgg ggggtggggt ggggcaggac agcaaggggg aggattggga 4500
   agacaatagc aggcatgctg gggatgcggt gggctctatg ggtacccagg tgctgaagaa 4560
   ttgacccggt tcctcctggg ccagaaagaa gcaggcacat ccccttctct gtgacacacc 4620
   ctgtccacgc ccctggttct tagttccagc cccactcata ggacactcat agctcaggag 4680
   ggctccgcct tcaatcccac ccgctaaagt acttggagcg gtctctccct ccctcatcag 4740
   cccaccaaac caaacctagc ctccaagagt gggaagaaat taaagcaaga taggctatta 4800
   agtgcagagg gagagaaaat gcctccaaca tgtgaggaag taatgagaga aatcatagaa 4860
   tttcttccgc ttcctcgctc actgactcgc tgcgctcggt cgttcggctg cggcgagcgg 4920
   tatcagctca ctcaaaggcg gtaatacggt tatccacaga atcaggggat aacgcaggaa 4980
   agaacatgtg agcaaaaggc cagcaaaagg ccaggaaccg taaaaaggcc gcgttgctgg 5040
   cgtttttcca taggctccgc ccccctgacg agcatcacaa aaatcgacgc tcaagtcaga 5100
   ggtggcgaaa cccgacagga ctataaagat accaggcgtt tccccctgga agctccctcg 5160
   tgcgctctcc tgttccgacc ctgccgctta ccggatacct gtccgccttt ctcccttcgg 5220
   gaagcgtggc gctttctcat agctcacgct gtaggtatct cagttcggtg taggtcgttc 5280
   gctccaagct gggctgtgtg cacgaacccc ccgttcagcc cgaccgctgc gccttatccg 5340
   gtaactatcg tcttgagtcc aacccggtaa gacacgactt atcgccactg gcagcagcca 5400
   ctggtaacag gattagcaga gcgaggtatg taggcggtgc tacagagttc ttgaagtggt 5460
   ggcctaacta cggctacact agaagaacag tatttggtat ctgcgctctg ctgaagccag 5520
   ttaccttcgg aaaaagagtt ggtagctctt gatccggcaa acaaaccacc gctggtagcg 5580
   gtggtttttt tgtttgcaag cagcagatta cgcgcagaaa aaaaggatct caagaagatc 5640
   ctttgatctt ttctacgggg tctgacgctc agtggaacga aaactcacgt taagggattt 5700
   tggtcatgag attatcaaaa aggatcttca cctagatcct tttaaattaa aaatgaagtt 5760
   ttaaatcaat ctaaagtata tatgagtaaa cttggtctga cagttaccaa tgcttaatca 5820
   gtgaggcacc tatctcagcg atctgtctat ttcgttcatc catagttgcc tgactcgggg 5880
   ggggggggcg ctgaggtctg cctcgtgaag aaggtgttgc tgactcatac caggcctgaa 5940
   tcgccccatc atccagccag aaagtgaggg agccacggtt gatgagagct ttgttgtagg 6000
   tggaccagtt ggtgattttg aacttttgct ttgccacgga acggtctgcg ttgtcgggaa 6060
   gatgcgtgat ctgatccttc aactcagcaa aagttcgatt tattcaacaa agccgccgtc 6120
   ccgtcaagtc agcgtaatgc tctgccagtg ttacaaccaa ttaaccaatt ctgattagaa 6180
   aaactcatcg agcatcaaat gaaactgcaa tttattcata tcaggattat caataccata 6240
   tttttgaaaa agccgtttct gtaatgaagg agaaaactca ccgaggcagt tccataggat 6300
   ggcaagatcc tggtatcggt ctgcgattcc gactcgtcca acatcaatac aacctattaa 6360
   tttcccctcg tcaaaaataa ggttatcaag tgagaaatca ccatgagtga cgactgaatc 6420
   cggtgagaat ggcaaaagct tatgcatttc tttccagact tgttcaacag gccagccatt 6480
   acgctcgtca tcaaaatcac tcgcatcaac caaaccgtta ttcattcgtg attgcgcctg 6540
   agcgagacga aatacgcgat cgctgttaaa aggacaatta caaacaggaa tcgaatgcaa 6600
   ccggcgcagg aacactgcca gcgcatcaac aatattttca cctgaatcag gatattcttc 6660
   taatacctgg aatgctgttt tcccggggat cgcagtggtg agtaaccatg catcatcagg 6720
   agtacggata aaatgcttga tggtcggaag aggcataaat tccgtcagcc agtttagtct 6780
   gaccatctca tctgtaacat cattggcaac gctacctttg ccatgtttca gaaacaactc 6840
   tggcgcatcg ggcttcccat acaatcgata gattgtcgca cctgattgcc cgacattatc 6900
   gcgagcccat ttatacccat ataaatcagc atccatgttg gaatttaatc gcggcctcga 6960
   gcaagacgtt tcccgttgaa tatggctcat aacacccctt gtattactgt ttatgtaagc 7020
   agacagtttt attgttcatg atgatatatt tttatcttgt gcaatgtaac atcagagatt 7080
   ttgagacaca acgtggcttt cccccccccc ccattattga agcatttatc agggttattg 7140
   tctcatgagc ggatacatat ttgaatgtat ttagaaaaat aaacaaatag gggttccgcg 7200
   cacatttccc cgaaaagtgc cacctgacgt ctaagaaacc attattatca tgacattaac 7260
   ctataaaaat aggcgtatca cgaggccctt tcgtc                            7295
<210> 23
   <211> 7329
   <212> DNA
   <213> Artificial Sequence
<220>
   <223> pVR1012x/s Ebola-NP
<400> 23
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga ccaggccctg 1920
   gatccagatc gatccgagta tggattctcg tcctcagaaa atctggatgg cgccgagtct 1980
   cactgaatct gacatggatt accacaagat cttgacagca ggtctgtccg ttcaacaggg 2040
   gattgttcgg caaagagtca tcccagtgta tcaagtaaac aatcttgaag aaatttgcca 2100
   acttatcata caggcctttg aagcaggtgt tgattttcaa gagagtgcgg acagtttcct 2160
   tctcatgctt tgtcttcatc atgcgtacca gggagattac aaacttttct tggaaagtgg 2220
   cgcagtcaag tatttggaag ggcacgggtt ccgttttgaa gtcaagaagc gtgatggagt 2280
   gaagcgcctt gaggaattgc tgccagcagt atctagtgga aaaaacatta agagaacact 2340
   tgctgccatg ccggaagagg agacaactga agctaatgcc ggtcagtttc tctcctttgc 2400
   aagtctattc cttccgaaat tggtagtagg agaaaaggct tgccttgaga aggttcaaag 2460
   gcaaattcaa gtacatgcag agcaaggact gatacaatat ccaacagctt ggcaatcagt 2520
   aggacacatg atggtgattt tccgtttgat gcgaacaaat tttctgatca aatttctcct 2580
   aatacaccaa gggatgcaca tggttgccgg gcatgatgcc aacgatgctg tgatttcaaa 2640
   ttcagtggct caagctcgtt tttcaggctt attgattgtc aaaacagtac ttgatcatat 2700
   cctacaaaag acagaacgag gagttcgtct ccatcctctt gcaaggaccg ccaaggtaaa 2760
   aaatgaggtg aactccttta aggctgcact cagctccctg gccaagcatg gagagtatgc 2820
   tcctttcgcc cgacttttga acctttctgg agtaaataat cttgagcatg gtcttttccc 2880
   tcaactatcg gcaattgcac tcggagtcgc cacagcacac gggagtaccc tcgcaggagt 2940
   aaatgttgga gaacagtatc aacaactcag agaggctgcc actgaggctg agaagcaact 3000
   ccaacaatat gcagagtctc gcgaacttga ccatcttgga cttgatgatc aggaaaagaa 3060
   aattcttatg aacttccatc agaaaaagaa cgaaatcagc ttccagcaaa caaacgctat 3120
   ggtaactcta agaaaagagc gcctggccaa gctgacagaa gctatcactg ctgcgtcact 3180
   gcccaaaaca agtggacatt acgatgatga tgacgacatt ccctttccag gacccatcaa 3240
   tgatgacgac aatcctggcc atcaagatga tgatccgact gactcacagg atacgaccat 3300
   tcccgatgtg gtggttgatc ccgatgatgg aagctacggc gaataccaga gttactcgga 3360
   aaacggcatg aatgcaccag atgacttggt cctattcgat ctagacgagg acgacgagga 3420
   cactaagcca gtgcctaata gatcgaccaa gggtggacaa cagaagaaca gtcaaaaggg 3480
   ccagcatata gagggcagac agacacaatc caggccaatt caaaatgtcc caggccctca 3540
   cagaacaatc caccacgcca gtgcgccact cacggacaat gacagaagaa atgaaccctc 3600
   cggctcaacc agccctcgca tgctgacacc aattaacgaa gaggcagacc cactggacga 3660
   tgccgacgac gagacgtcta gccttccgcc cttggagtca gatgatgaag agcaggacag 3720
   ggacggaact tccaaccgca cacccactgt cgccccaccg gctcccgtat acagagatca 3780
   ctctgaaaag aaagaactcc cgcaagacga gcaacaagat caggaccaca ctcaagaggc 3840
   caggaaccag gacagtgaca acacccagtc agaacactct tttgaggaga tgtatcgcca 3900
   cattctaaga tcacaggggc catttgatgc tgttttgtat tatcatatga tgaaggatga 3960
   gcctgtagtt ttcagtacca gtgatggcaa agagtacacg tatccagact cccttgaaga 4020
   ggaatatcca ccatggctca ctgaaaaaga ggctatgaat gaagagaata gatttgttac 4080
   attggatggt caacaatttt attggccggt gatgaatcac aagaataaat tcatggcaat 4140
   cctgcaacat catcagtgaa tgagcatgga acaatgggat gattcaaccg acaaatagct 4200
   aacattaagt agtcaaggaa cgaaaacagg aagaattttt gatgtctaag gtgtgaatta 4260
   ttatcacaat aaaagtgatt cttatttttg aatttgggcg agctcgaatt gatctgctgt 4320
   gccttctagt tgccagccat ctgttgtttg cccctccccc gtgccttcct tgaccctgga 4380
   aggtgccact cccactgtcc tttcctaata aaatgaggaa attgcatcgc attgtctgag 4440
   taggtgtcat tctattctgg ggggtggggt ggggcaggac agcaaggggg aggattggga 4500
   agacaatagc aggcatgctg gggatgcggt gggctctatg ggtacccagg tgctgaagaa 4560
   ttgacccggt tcctcctggg ccagaaagaa gcaggcacat ccccttctct gtgacacacc 4620
   ctgtccacgc ccctggttct tagttccagc cccactcata ggacactcat agctcaggag 4680
   ggctccgcct tcaatcccac ccgctaaagt acttggagcg gtctctccct ccctcatcag 4740
   cccaccaaac caaacctagc ctccaagagt gggaagaaat taaagcaaga taggctatta 4800
   agtgcagagg gagagaaaat gcctccaaca tgtgaggaag taatgagaga aatcatagaa 4860
   ttttaaggcc atgatttaag gccatcatgg ccttaatctt ccgcttcctc gctcactgac 4920
   tcgctgcgct cggtcgttcg gctgcggcga gcggtatcag ctcactcaaa ggcggtaata 4980
   cggttatcca cagaatcagg ggataacgca ggaaagaaca tgtgagcaaa aggccagcaa 5040
   aaggccagga accgtaaaaa ggccgcgttg ctggcgtttt tccataggct ccgcccccct 5100
   gacgagcatc acaaaaatcg acgctcaagt cagaggtggc gaaacccgac aggactataa 5160
   agataccagg cgtttccccc tggaagctcc ctcgtgcgct ctcctgttcc gaccctgccg 5220
   cttaccggat acctgtccgc ctttctccct tcgggaagcg tggcgctttc tcatagctca 5280
   cgctgtaggt atctcagttc ggtgtaggtc gttcgctcca agctgggctg tgtgcacgaa 5340
   ccccccgttc agcccgaccg ctgcgcctta tccggtaact atcgtcttga gtccaacccg 5400
   gtaagacacg acttatcgcc actggcagca gccactggta acaggattag cagagcgagg 5460
   tatgtaggcg gtgctacaga gttcttgaag tggtggccta actacggcta cactagaaga 5520
   acagtatttg gtatctgcgc tctgctgaag ccagttacct tcggaaaaag agttggtagc 5580
   tcttgatccg gcaaacaaac caccgctggt agcggtggtt tttttgtttg caagcagcag 5640
   attacgcgca gaaaaaaagg atctcaagaa gatcctttga tcttttctac ggggtctgac 5700
   gctcagtgga acgaaaactc acgttaaggg attttggtca tgagattatc aaaaaggatc 5760
   ttcacctaga tccttttaaa ttaaaaatga agttttaaat caatctaaag tatatatgag 5820
   taaacttggt ctgacagtta ccaatgctta atcagtgagg cacctatctc agcgatctgt 5880
   ctatttcgtt catccatagt tgcctgactc gggggggggg ggcgctgagg tctgcctcgt 5940
   gaagaaggtg ttgctgactc ataccaggcc tgaatcgccc catcatccag ccagaaagtg 6000
   agggagccac ggttgatgag agctttgttg taggtggacc agttggtgat tttgaacttt 6060
   tgctttgcca cggaacggtc tgcgttgtcg ggaagatgcg tgatctgatc cttcaactca 6120
   gcaaaagttc gatttattca acaaagccgc cgtcccgtca agtcagcgta atgctctgcc 6180
   agtgttacaa ccaattaacc aattctgatt agaaaaactc atcgagcatc aaatgaaact 6240
   gcaatttatt catatcagga ttatcaatac catatttttg aaaaagccgt ttctgtaatg 6300
   aaggagaaaa ctcaccgagg cagttccata ggatggcaag atcctggtat cggtctgcga 6360
   ttccgactcg tccaacatca atacaaccta ttaatttccc ctcgtcaaaa ataaggttat 6420
   caagtgagaa atcaccatga gtgacgactg aatccggtga gaatggcaaa agcttatgca 6480
   tttctttcca gacttgttca acaggccagc cattacgctc gtcatcaaaa tcactcgcat 6540
   caaccaaacc gttattcatt cgtgattgcg cctgagcgag acgaaatacg cgatcgctgt 6600
   taaaaggaca attacaaaca ggaatcgaat gcaaccggcg caggaacact gccagcgcat 6660
   caacaatatt ttcacctgaa tcaggatatt cttctaatac ctggaatgct gttttcccgg 6720
   ggatcgcagt ggtgagtaac catgcatcat caggagtacg gataaaatgc ttgatggtcg 6780
   gaagaggcat aaattccgtc agccagttta gtctgaccat ctcatctgta acatcattgg 6840
   caacgctacc tttgccatgt ttcagaaaca actctggcgc atcgggcttc ccatacaatc 6900
   gatagattgt cgcacctgat tgcccgacat tatcgcgagc ccatttatac ccatataaat 6960
   cagcatccat gttggaattt aatcgcggcc tcgagcaaga cgtttcccgt tgaatatggc 7020
   tcataacacc ccttgtatta ctgtttatgt aagcagacag ttttattgtt catgatgata 7080
   tatttttatc ttgtgcaatg taacatcaga gattttgaga cacaacgtgg ctttcccccc 7140
   ccccccatta ttgaagcatt tatcagggtt attgtctcat gagcggatac atatttgaat 7200
   gtatttagaa aaataaacaa ataggggttc cgcgcacatt tccccgaaaa gtgccacctg 7260
   acgtctaaga aaccattatt atcatgacat taacctataa aaataggcgt atcacgaggc 7320
   cctttcgtc                                                         7329
<210> 24
   <211> 6148
   <212> DNA
   <213> Artificial Sequence
<220>
   <223> pVR1012-VP35
<400> 24
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgaattct ctagcactcg aagcttattg tcttcaatgt aaaagaaaag 1920
   ctggtctaac aagatgacaa ctagaacaaa gggcaggggc catactgcgg ccacgactca 1980
   aaacgacaga atgccaggcc ctgagctttc gggctggatc tctgagcagc taatgaccgg 2040
   aagaattcct gtaagcgaca tcttctgtga tattgagaac aatccaggat tatgctacgc 2100
   atcccaaatg caacaaacga agccaaaccc gaagacgcgc aacagtcaaa cccaaacgga 2160
   cccaatttgc aatcatagtt ttgaggaggt agtacaaaca ttggcttcat tggctactgt 2220
   tgtgcaacaa caaaccatcg catcagaatc attagaacaa cgcattacga gtcttgagaa 2280
   tggtctaaag ccagtttatg atatggcaaa aacaatctcc tcattgaaca gggtttgtgc 2340
   tgagatggtt gcaaaatatg atcttctggt gatgacaacc ggtcgggcaa cagcaaccgc 2400
   tgcggcaact gaggcttatt gggccgaaca tggtcaacca ccacctggac catcacttta 2460
   tgaagaaagt gcgattcggg gtaagattga atctagagat gagaccgtcc ctcaaagtgt 2520
   tagggaggca ttcaacaatc taaacagtac cacttcacta actgaggaaa attttgggaa 2580
   acctgacatt tcggcaaagg atttgagaaa cattatgtat gatcacttgc ctggttttgg 2640
   aactgctttc caccaattag tacaagtgat ttgtaaattg ggaaaagata gcaactcatt 2700
   ggacatcatt catgctgagt tccaggccag cctggctgaa ggagactctc ctcaatgtgc 2760
   cctaattcaa attacaaaaa gagttccaat cttccaagat gctgctccat ctgtcatcca 2820
   catccgcttt cgaggtgaca ttccccgagc ttgccagaaa agcttgcgtc cagtcccacc 2880
   atcgcccaag attgatcgag gttgggatgt gtttttcagc ttcaagatgg taaaacactt 2940
   ggactcaaaa tttgagccaa tctcccttcc ctccgaaaga ggcgaataat agcagaggct 3000
   tcaactgctg aactataggg tacgttacat taatgataca cttgtgagta tcagccctgg 3060
   ataatataag tcaattaaac gaccaagata aaattgttca tatctcgcta gcagcttaaa 3120
   atataaatgt aataggagct atatctctga cagggggatc cagatctgct gtgccttcta 3180
   gttgccagcc atctgttgtt tgcccctccc ccgtgccttc cttgaccctg gaaggtgcca 3240
   ctcccactgt cctttcctaa taaaatgagg aaattgcatc gcattgtctg agtaggtgtc 3300
   attctattct ggggggtggg gtggggcagc acagcaaggg ggaggattgg gaagacaata 3360
   gcaggcatgc tggggatgcg gtgggctcta tgggtaccca ggtgctgaag aattgacccg 3420
   gttcctcctg ggccagaaag aagcaggcac atccccttct ctgtgacaca ccctgtccac 3480
   gcccctggtt cttagttcca gccccactca taggacactc atagctcagg agggctccgc 3540
   cttcaatccc acccgctaaa gtacttggag cggtctctcc ctccctcatc agcccaccaa 3600
   accaaaccta gcctccaaga gtgggaagaa attaaagcaa gataggctat taagtgcaga 3660
   gggagagaaa atgcctccaa catgtgagga agtaatgaga gaaatcatag aatttcttcc 3720
   gcttcctcgc tcactgactc gctgcgctcg gtcgttcggc tgcggcgagc ggtatcagct 3780
   cactcaaagg cggtaatacg gttatccaca gaatcagggg ataacgcagg aaagaacatg 3840
   tgagcaaaag gccagcaaaa ggccaggaac cgtaaaaagg ccgcgttgct ggcgtttttc 3900
   cataggctcc gcccccctga cgagcatcac aaaaatcgac gctcaagtca gaggtggcga 3960
   aacccgacag gactataaag ataccaggcg tttccccctg gaagctccct cgtgcgctct 4020
   cctgttccga ccctgccgct taccggatac ctgtccgcct ttctcccttc gggaagcgtg 4080
   gcgctttctc aatgctcacg ctgtaggtat ctcagttcgg tgtaggtcgt tcgctccaag 4140
   ctgggctgtg tgcacgaacc ccccgttcag cccgaccgct gcgccttatc cggtaactat 4200
   cgtcttgagt ccaacccggt aagacacgac ttatcgccac tggcagcagc cactggtaac 4260
   aggattagca gagcgaggta tgtaggcggt gctacagagt tcttgaagtg gtggcctaac 4320
   tacggctaca ctagaaggac agtatttggt atctgcgctc tgctgaagcc agttaccttc 4380
   ggaaaaagag ttggtagctc ttgatccggc aaacaaacca ccgctggtag cggtggtttt 4440
   tttgtttgca agcagcagat tacgcgcaga aaaaaaggat ctcaagaaga tcctttgatc 4500
   ttttctacgg ggtctgacgc tcagtggaac gaaaactcac gttaagggat tttggtcatg 4560
   agattatcaa aaaggatctt cacctagatc cttttaaatt aaaaatgaag ttttaaatca 4620
   atctaaagta tatatgagta aacttggtct gacagttacc aatgcttaat cagtgaggca 4680
   cctatctcag cgatctgtct atttcgttca tccatagttg cctgactccg gggggggggg 4740
   gcgctgaggt ctgcctcgtg aagaaggtgt tgctgactca taccaggcct gaatcgcccc 4800
   atcatccagc cagaaagtga gggagccacg gttgatgaga gctttgttgt aggtggacca 4860
   gttggtgatt ttgaactttt gctttgccac ggaacggtct gcgttgtcgg gaagatgcgt 4920
   gatctgatcc ttcaactcag caaaagttcg atttattcaa caaagccgcc gtcccgtcaa 4980
   gtcagcgtaa tgctctgcca gtgttacaac caattaacca attctgatta gaaaaactca 5040
   tcgagcatca aatgaaactg caatttattc atatcaggat tatcaatacc atatttttga 5100
   aaaagccgtt tctgtaatga aggagaaaac tcaccgaggc agttccatag gatggcaaga 5160
   tcctggtatc ggtctgcgat tccgactcgt ccaacatcaa tacaacctat taatttcccc 5220
   tcgtcaaaaa taaggttatc aagtgagaaa tcaccatgag tgacgactga atccggtgag 5280
   aatggcaaaa gcttatgcat ttctttccag acttgttcaa caggccagcc attacgctcg 5340
   tcatcaaaat cactcgcatc aaccaaaccg ttattcattc gtgattgcgc ctgagcgaga 5400
   cgaaatacgc gatcgctgtt aaaaggacaa ttacaaacag gaatcgaatg caaccggcgc 5460
   aggaacactg ccagcgcatc aacaatattt tcacctgaat caggatattc ttctaatacc 5520
   tggaatgctg ttttcccggg gatcgcagtg gtgagtaacc atgcatcatc aggagtacgg 5580
   ataaaatgct tgatggtcgg aagaggcata aattccgtca gccagtttag tctgaccatc 5640
   tcatctgtaa catcattggc aacgctacct ttgccatgtt tcagaaacaa ctctggcgca 5700
   tcgggcttcc catacaatcg atagattgtc gcacctgatt gcccgacatt atcgcgagcc 5760
   catttatacc catataaatc agcatccatg ttggaattta atcgcggcct cgagcaagac 5820
   gtttcccgtt gaatatggct cataacaccc cttgtattac tgtttatgta agcagacagt 5880
   tttattgttc atgatgatat atttttatct tgtgcaatgt aacatcagag attttgagac 5940
   acaacgtggc tttccccccc cccccattat tgaagcattt atcagggtta ttgtctcatg 6000
   agcggataca tatttgaatg tatttagaaa aataaacaaa taggggttcc gcgcacattt 6060
   ccccgaaaag tgccacctga cgtctaagaa accattatta tcatgacatt aacctataaa 6120
   aataggcgta tcacgaggcc ctttcgtc                                    6148
<210> 25
   <211> 10783
   <212> DNA
   <213> Artificial Sequence
<220>
   <223> pAD/CMV-GP(dTM)(Z-CITE-S)
<400> 25
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat ctagaccagg ccctggatcg atccaacaac 1320
   acaatgggcg ttacaggaat attgcagtta cctcgtgatc gattcaagag gacatcattc 1380
   tttctttggg taattatcct tttccaaaga acattttcca tcccacttgg agtcatccac 1440
   aatagcacat tacaggttag tgatgtcgac aaactagttt gtcgtgacaa actgtcatcc 1500
   acaaatcaat tgagatcagt tggactgaat ctcgaaggga atggagtggc aactgacgtg 1560
   ccatctgcaa ctaaaagatg gggcttcagg tccggtgtcc caccaaaggt ggtcaattat 1620
   gaagctggtg aatgggctga aaactgctac aatcttgaaa tcaaaaaacc tgacgggagt 1680
   gagtgtctac cagcagcgcc agacgggatt cggggcttcc cccggtgccg gtatgtgcac 1740
   aaagtatcag gaacgggacc gtgtgccgga gactttgcct tccataaaga gggtgctttc 1800
   ttcctgtatg atcgacttgc ttccacagtt atctaccgag gaacgacttt cgctgaaggt 1860
   gtcgttgcat ttctgatact gccccaagct aagaaggact tcttcagctc acaccccttg 1920
   agagagccgg tcaatgcaac ggaggacccg tctagtggct actattctac cacaattaga 1980
   tatcaggcta ccggttttgg aaccaatgag acagagtact tgttcgaggt tgacaatttg 2040
   acctacgtcc aacttgaatc aagattcaca ccacagtttc tgctccagct gaatgagaca 2100
   atatatacaa gtgggaaaag gagcaatacc acgggaaaac taatttggaa ggtcaacccc 2160
   gaaattgata caacaatcgg ggagtgggcc ttctgggaaa ctaaaaaaaa cctcactaga 2220
   aaaattcgca gtgaagagtt gtctttcaca gttgtatcaa acggagccaa aaacatcagt 2280
   ggtcagagtc cggcgcgaac ttcttccgac ccagggacca acacaacaac tgaagaccac 2340
   aaaatcatgg cttcagaaaa ttcctctgca atggttcaag tgcacagtca aggaagggaa 2400
   gctgcagtgt cgcatctaac aacccttgcc acaatctcca cgagtcccca atccctcaca 2460
   accaaaccag gtccggacaa cagcacccat aatacacccg tgtataaact tgacatctct 2520
   gaggcaactc aagttgaaca acatcaccgc agaacagaca acgacagcac agcctccgac 2580
   actccctctg ccacgaccgc agccggaccc ccaaaagcag agaacaccaa cacgagcaag 2640
   agcactgact tcctggaccc cgccaccaca acaagtcccc aaaaccacag cgagaccgct 2700
   ggcaacaaca acactcatca ccaagatacc ggagaagaga gtgccagcag cgggaagcta 2760
   ggcttaatta ccaatactat tgctggagtc gcaggactga tcacaggcgg gagaagaact 2820
   cgaagagaag caattgtcaa tgctcaaccc aaatgcaacc ctaatttaca ttactggact 2880
   actcaggatg aaggtgctgc aatcggactg gcctggatac catatttcgg gccagcagcc 2940
   gagggaattt acatagaggg gctaatgcac aatcaagatg gtttaatctg tgggttgaga 3000
   cagctggcca acgagacgac tcaagctctt caactgttcc tgagagccac aactgagcta 3060
   cgcacctttt caatcctcaa ccgtaaggca attgatttct tgctgcagcg atggggcggc 3120
   acatgccaca ttctgggacc ggactgctgt atcgaaccac atgattggac caagaacata 3180
   acagacaaaa ttgatcagat tattcatgat tttgttgata aaacccttcc ggaccagggg 3240
   gacaatgaca attggtggac aggatggaga caatggatgg ccgcatcgtg actgactgac 3300
   gatctgcctc gcgagatcaa ttccgcccct ctccctcccc cccccctaac gttactggcc 3360
   gaagccgctt ggaataaggc cggtgtgcgt ttgtctatat gttattttcc accatattgc 3420
   cgtcttttgg caatgtgagg gcccggaaac ctggccctgt cttcttgacg agcattccta 3480
   ggggtctttc ccctctcgcc aaaggaatgc aaggtctgtt gaatgtcgtg aaggaagcag 3540
   ttcctctgga agcttcttga agacaaacaa cgtctgtagc gaccctttgc aggcagcgga 3600
   accccccacc tggcgacagg tgcctctgcg gccaaaagcc acgtgtataa gatacacctg 3660
   caaaggcggc acaaccccag tgccacgttg tgagttggat agttgtggaa agagtcaaat 3720
   ggctctcctc aagcgtattc aacaaggggc tgaaggatgc ccagaaggta ccccattgta 3780
   tgggatctga tctggggcct cggtgcacat gctttacatg tgtttagtcg aggttaaaaa 3840
   acgtctaggc cccccgaacc acggggacgt ggttttcctt tgaaaaacac gatgataata 3900
   tggccacaac catggagggt cttagcctac tccaattgcc cagagataaa tttcgaaaaa 3960
   gctctttctt tgtttgggtc atcatcttat ttcaaaaggc cttttccatg cctttgggtg 4020
   ttgtgaccaa cagcacttta gaagtaacag agattgacca gctagtctgc aaggatcatc 4080
   ttgcatccac tgaccagctg aaatcagttg gtctcaacct cgaggggagc ggagtatcta 4140
   ctgatatccc atctgcgaca aagcgttggg gcttcagatc tggtgtgcct cccaaggtgg 4200
   tcagctatga agcaggagaa tgggctgaaa attgctacaa tcttgaaata aagaagccgg 4260
   acgggagcga atgcttaccc ccaccgccgg atggtgtcag aggctttcca aggtgccgct 4320
   atgttcacaa agcccaagga accgggccct gcccgggtga ctatgccttt cacaaggatg 4380
   gagctttctt cctctatgac aggctggctt caactgtaat ttacagagga gtcaattttg 4440
   ctgagggggt aattgcattc ttgatattgg ctaaaccaaa ggaaacgttc cttcaatcac 4500
   cccccattcg agaggcagta aactacactg aaaatacatc aagttactat gccacatcct 4560
   acttggagta cgaaatcgaa aattttggtg ctcaacactc cacgaccctt ttcaaaatta 4620
   acaataatac ttttgttctt ctggacaggc cccacacgcc tcagttcctt ttccagctga 4680
   atgataccat tcaccttcac caacagttga gcaacacaac tgggaaacta atttggacac 4740
   tagatgctaa tatcaatgct gatattggtg aatgggcttt ttgggaaaat aaaaaaaatc 4800
   tctccgaaca actacgtgga gaagagctgt ctttcgaaac tttatcgctc aacgagacag 4860
   aagacgatga tgcgacatcg tcgagaacta caaagggaag aatctccgac cgggccacca 4920
   ggaagtattc ggacctggtt ccaaaggatt cccctgggat ggtttcattg cacgtaccag 4980
   aaggggaaac aacattgccg tctcagaatt cgacagaagg tcgaagagta gatgtgaata 5040
   ctcaggaaac tatcacagag acaactgcaa caatcatagg cactaacggt aacaacatgc 5100
   agatctccac catcgggaca ggactgagct ccagccaaat cctgagttcc tcaccgacca 5160
   tggcaccaag ccctgagact cagacctcca caacctacac accaaaacta ccagtgatga 5220
   ccaccgagga accaacaaca ccaccgagaa actctcctgg ctcaacaaca gaagcaccca 5280
   ctctcaccac cccagagaat ataacaacag cggttaaaac tgttttgcca caagagtcca 5340
   caagcaacgg tctaataact tcaacagtaa cagggattct tgggagcctt ggacttcgaa 5400
   aacgcagcag aagacaagtt aacaccaggg ccacgggtaa atgcaatccc aacttacact 5460
   actggactgc acaagaacaa cataatgctg ctgggattgc ctggatcccg tactttggac 5520
   cgggtgcaga aggcatatac actgaaggcc ttatgcacaa ccaaaatgcc ttagtctgtg 5580
   gactcagaca acttgcaaat gaaacaactc aagctctgca gcttttctta agggccacga 5640
   cggagctgcg gacatatacc atactcaata ggaaggccat agatttcctt ctgcgacgat 5700
   ggggcgggac atgtaggatc ctgggaccag attgttgcat tgagccacat gattggacca 5760
   aaaacatcac tgataaaatc aaccaaatca tccatgattt catcgacaac cctttaccca 5820
   atcaggataa tgatgataat tggtggacgg gctggagaca gtggatcccg gccgcatcgt 5880
   gactgactga cgatctgcct cgcggatcca gatctgctgt gccttctagt tgccagccat 5940
   ctgttgtttg cccctccccc gtgccttcct tgaccctgga aggtgccact cccactgtcc 6000
   tttcctaata aaatgaggaa attgcatcgc attgtctgag taggtgtcat tctattctgg 6060
   ggggtggggt ggggcagcac agcaaggggg aggattggga agacaatagc aggcatgctg 6120
   gggatgcggt gggctctatg ggtacccagg gccgcataac ttcgtataat gtatgctata 6180
   cgaagttata agatctgtac tgaaatgtgt gggcgtggct taagggtggg aaagaatata 6240
   taaggtgggg gtcttatgta gttttgtatc tgttttgcag cagccgccgc cgccatgagc 6300
   accaactcgt ttgatggaag cattgtgagc tcatatttga caacgcgcat gcccccatgg 6360
   gccggggtgc gtcagaatgt gatgggctcc agcattgatg gtcgccccgt cctgcccgca 6420
   aactctacta ccttgaccta cgagaccgtg tctggaacgc cgttggagac tgcagcctcc 6480
   gccgccgctt cagccgctgc agccaccgcc cgcgggattg tgactgactt tgctttcctg 6540
   agcccgcttg caagcagtgc agcttcccgt tcatccgccc gcgatgacaa gttgacggct 6600
   cttttggcac aattggattc tttgacccgg gaacttaatg tcgtttctca gcagctgttg 6660
   gatctgcgcc agcaggtttc tgccctgaag gcttcctccc ctcccaatgc ggtttaaaac 6720
   ataaataaaa aaccagactc tgtttggatt tggatcaagc aagtgtcttg ctgtctttat 6780
   ttaggggttt tgcgcgcgcg gtaggcccgg gaccagcggt ctcggtcgtt gagggtcctg 6840
   tgtatttttt ccaggacgtg gtaaaggtga ctctggatgt tcagatacat gggcataagc 6900
   ccgtctctgg ggtggaggta gcaccactgc agagcttcat gctgcggggt ggtgttgtag 6960
   atgatccagt cgtagcagga gcgctgggcg tggtgcctaa aaatgtcttt cagtagcaag 7020
   ctgattgcca ggggcaggcc cttggtgtaa gtgtttacaa agcggttaag ctgggatggg 7080
   tgcatacgtg gggatatgag atgcatcttg gactgtattt ttaggttggc tatgttccca 7140
   gccatatccc tccggggatt catgttgtgc agaaccacca gcacagtgta tccggtgcac 7200
   ttgggaaatt tgtcatgtag cttagaagga aatgcgtgga agaacttgga gacgcccttg 7260
   tgacctccaa gattttccat gcattcgtcc ataatgatgg caatgggccc acgggcggcg 7320
   gcctgggcga agatatttct gggatcacta acgtcatagt tgtgttccag gatgagatcg 7380
   tcataggcca tttttacaaa gcgcgggcgg agggtgccag actgcggtat aatggttcca 7440
   tccggcccag gggcgtagtt accctcacag atttgcattt cccacgcttt gagttcagat 7500
   ggggggatca tgtctacctg cggggcgatg aagaaaacgg tttccggggt aggggagatc 7560
   agctgggaag aaagcaggtt cctgagcagc tgcgacttac cgcagccggt gggcccgtaa 7620
   atcacaccta ttaccggctg caactggtag ttaagagagc tgcagctgcc gtcatccctg 7680
   agcagggggg ccacttcgtt aagcatgtcc ctgactcgca tgttttccct gaccaaatcc 7740
   gccagaaggc gctcgccgcc cagcgatagc agttcttgca aggaagcaaa gtttttcaac 7800
   ggtttgagac cgtccgccgt aggcatgctt ttgagcgttt gaccaagcag ttccaggcgg 7860
   tcccacagct cggtcacctg ctctacggca tctcgatcca gcatatctcc tcgtttcgcg 7920
   ggttggggcg gctttcgctg tacggcagta gtcggtgctc gtccagacgg gccagggtca 7980
   tgtctttcca cgggcgcagg gtcctcgtca gcgtagtctg ggtcacggtg aaggggtgcg 8040
   ctccgggctg cgcgctggcc agggtgcgct tgaggctggt cctgctggtg ctgaagcgct 8100
   gccggtcttc gccctgcgcg tcggccaggt agcatttgac catggtgtca tagtccagcc 8160
   cctccgcggc gtggcccttg gcgcgcagct tgcccttgga ggaggcgccg cacgaggggc 8220
   agtgcagact tttgagggcg tagagcttgg gcgcgagaaa taccgattcc ggggagtagg 8280
   catccgcgcc gcaggccccg cagacggtct cgcattccac gagccaggtg agctctggcc 8340
   gttcggggtc aaaaaccagg tttcccccat gctttttgat gcgtttctta cctctggttt 8400
   ccatgagccg gtgtccacgc tcggtgacga aaaggctgtc cgtgtccccg tatacagact 8460
   tgagaggcct gtcctcgagc ggtgttccgc ggtcctcctc gtatagaaac tcggaccact 8520
   ctgagacaaa ggctcgcgtc caggccagca cgaaggaggc taagtgggag gggtagcggt 8580
   cgttgtccac tagggggtcc actcgctcca gggtgtgaag acacatgtcg ccctcttcgg 8640
   catcaaggaa ggtgattggt ttgtaggtgt aggccacgtg accgggtgtt cctgaagggg 8700
   ggctataaaa gggggtgggg gcgcgttcgt cctcactctc ttccgcatcg ctgtctgcga 8760
   gggccagctg ttggggtgag tcgacgcgag gctggatggc cttccccatt atgattcttc 8820
   tcgcttccgg cggcatcggg atgcccgcgt tgcaggccat gctgtccagg caggtagatg 8880
   acgaccatca gggacagctt caaggccagc aaaaggccag gaaccgtaaa aaggccgcgt 8940
   tgctggcgtt tttccatagg ctccgccccc ctgacgagca tcacaaaaat cgacgctcaa 9000
   gtcagaggtg gcgaaacccg acaggactat aaagatacca ggcgtttccc cctggaagct 9060
   ccctcgtgcg ctctcctgtt ccgaccctgc cgcttaccgg atacctgtcc gcctttctcc 9120
   cttcgggaag cgtggcgctt tctcatagct cacgctgtag gtatctcagt tcggtgtagg 9180
   tcgttcgctc caagctgggc tgtgtgcacg aaccccccgt tcagcccgac cgctgcgcct 9240
   tatccggtaa ctatcgtctt gagtccaacc cggtaagaca cgacttatcg ccactggcag 9300
   cagccactgg taacaggatt agcagagcga ggtatgtagg cggtgctaca gagttcttga 9360
   agtggtggcc taactacggc tacactagaa ggacagtatt tggtatctgc gctctgctga 9420
   agccagttac cttcggaaaa agagttggta gctcttgatc cggcaaacaa accaccgctg 9480
   gtagcggtgg tttttttgtt tgcaagcagc agattacgcg cagaaaaaaa ggatctcaag 9540
   aagatccttt gatcttttct acggggtctg acgctcagtg gaacgaaaac tcacgttaag 9600
   ggattttggt catgagatta tcaaaaagga tcttcaccta gatcctttta aattaaaaat 9660
   gaagttttaa atcaatctaa agtatatatg agtaaacttg gtctgacagt taccaatgct 9720
   taatcagtga ggcacctatc tcagcgatct gtctatttcg ttcatccata gttgcctgac 9780
   tccccgtcgt gtagataact acgatacggg agggcttacc atctggcccc agtgctgcaa 9840
   tgataccgcg agacccacgc tcaccggctc cagatttatc agcaataaac cagccagccg 9900
   gaagggccga gcgcagaagt ggtcctgcaa ctttatccgc ctccatccag tctattaatt 9960
   gttgccggga agctagagta agtagttcgc cagttaatag tttgcgcaac gttgttgcca 10020
   ttgctgcagg catcgtggtg tcacgctcgt cgtttggtat ggcttcattc agctccggtt 10080
   cccaacgatc aaggcgagtt acatgatccc ccatgttgtg caaaaaagcg gttagctcct 10140
   tcggtcctcc gatcgttgtc agaagtaagt tggccgcagt gttatcactc atggttatgg 10200
   cagcactgca taattctctt actgtcatgc catccgtaag atgcttttct gtgactggtg 10260
   agtactcaac caagtcattc tgagaatagt gtatgcggcg accgagttgc tcttgcccgg 10320
   cgtcaacacg ggataatacc gcgccacata gcagaacttt aaaagtgctc atcattggaa 10380
   aacgttcttc ggggcgaaaa ctctcaagga tcttaccgct gttgagatcc agttcgatgt 10440
   aacccactcg tgcacccaac tgatcttcag catcttttac tttcaccagc gtttctgggt 10500
   gagcaaaaac aggaaggcaa aatgccgcaa aaaagggaat aagggcgaca cggaaatgtt 10560
   gaatactcat actcttcctt tttcaatatt attgaagcat ttatcagggt tattgtctca 10620
   tgagcggata catatttgaa tgtatttaga aaaataaaca aataggggtt ccgcgcacat 10680
   ttccccgaaa agtgccacct gacgtctaag aaaccattat tatcatgaca ttaacctata 10740
   aaaataggcg tatcacgagg ccctttcgtc ttcaagaatt gtt                  10783
<210> 26
   <211> 8338
   <212> DNA
   <213> Artificial Sequence
<220>
   <223> pAdApt Ebola GP(S)
<400> 26
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ccagtgtgat ggatatctgc 1320
   agaattcggc ttatcttcag gatctcgcca tggagggtct tagcctactc caattgccca 1380
   gagataaatt tcgaaaaagc tctttctttg tttgggtcat catcttattt caaaaggcct 1440
   tttccatgcc tttgggtgtt gtgaccaaca gcactttaga agtaacagag attgaccagc 1500
   tagtctgcaa ggatcatctt gcatccactg accagctgaa atcagttggt ctcaacctcg 1560
   aggggagcgg agtatctact gatatcccat ctgcgacaaa gcgttggggc ttcagatctg 1620
   gtgtgcctcc caaggtggtc agctatgaag caggagaatg ggctgaaaat tgctacaatc 1680
   ttgaaataaa gaagccggac gggagcgaat gcttaccccc accgccggat ggtgtcagag 1740
   gctttccaag gtgccgctat gttcacaaag cccaaggaac cgggccctgc ccgggtgact 1800
   atgcctttca caaggatgga gctttcttcc tctatgacag gctggcttca actgtaattt 1860
   acagaggagt caattttgct gagggggtaa ttgcattctt gatattggct aaaccaaagg 1920
   aaacgttcct tcaatcaccc cccattcgag aggcagtaaa ctacactgaa aatacatcaa 1980
   gttactatgc cacatcctac ttggagtacg aaatcgaaaa ttttggtgct caacactcca 2040
   cgaccctttt caaaattaac aataatactt ttgttcttct ggacaggccc cacacgcctc 2100
   agttcctttt ccagctgaat gataccattc accttcacca acagttgagc aacacaactg 2160
   ggaaactaat ttggacacta gatgctaata tcaatgctga tattggtgaa tgggcttttt 2220
   gggaaaataa aaaaaatctc tccgaacaac tacgtggaga agagctgtct ttcgaaactt 2280
   tatcgctcaa cgagacagaa gacgatgatg cgacatcgtc gagaactaca aagggaagaa 2340
   tctccgaccg ggccaccagg aagtattcgg acctggttcc aaaggattcc cctgggatgg 2400
   tttcattgca cgtaccagaa ggggaaacaa cattgccgtc tcagaattcg acagaaggtc 2460
   gaagagtaga tgtgaatact caggaaacta tcacagagac aactgcaaca atcataggca 2520
   ctaacggtaa caacatgcag atctccacca tcgggacagg actgagctcc agccaaatcc 2580
   tgagttcctc accgaccatg gcaccaagcc ctgagactca gacctccaca acctacacac 2640
   caaaactacc agtgatgacc accgaggaac caacaacacc accgagaaac tctcctggct 2700
   caacaacaga agcacccact ctcaccaccc cagagaatat aacaacagcg gttaaaactg 2760
   ttttgccaca agagtccaca agcaacggtc taataacttc aacagtaaca gggattcttg 2820
   ggagccttgg acttcgaaaa cgcagcagaa gacaagttaa caccagggcc acgggtaaat 2880
   gcaatcccaa cttacactac tggactgcac aagaacaaca taatgctgct gggattgcct 2940
   ggatcccgta ctttggaccg ggtgcagaag gcatatacac tgaaggcctt atgcacaacc 3000
   aaaatgcctt agtctgtgga ctcagacaac ttgcaaatga aacaactcaa gctctgcagc 3060
   ttttcttaag ggccacgacg gagctgcgga catataccat actcaatagg aaggccatag 3120
   atttccttct gcgacgatgg ggcgggacat gtaggatcct gggaccagat tgttgcattg 3180
   agccacatga ttggaccaaa aacatcactg ataaaatcaa ccaaatcatc catgatttca 3240
   tcgacaaccc tttacccaat caggataatg atgataattg gtggacgggc tggagacagt 3300
   ggatccctgc aggaataggc attactggaa ttattattgc aatcattgct cttctttgcg 3360
   tctgcaagct gctttgttga atatcaagcc gaattccagc acactggcgg ccgttactag 3420
   tggatccgag ctcggatcca agctctagac caggccctgg atccagatct gctgtgcctt 3480
   ctagttgcca gccatctgtt gtttgcccct cccccgtgcc ttccttgacc ctggaaggtg 3540
   ccactcccac tgtcctttcc taataaaatg aggaaattgc atcgcattgt ctgagtaggt 3600
   gtcattctat tctggggggt ggggtggggc aggacagcaa gggggaggat tgggaagaca 3660
   atagcaggca tgctggggat gcggtgggct ctatgggtac ccagggccgc ataacttcgt 3720
   ataatgtatg ctatacgaag ttataagatc tgtactgaaa tgtgtgggcg tggcttaagg 3780
   gtgggaaaga atatataagg tgggggtctt atgtagtttt gtatctgttt tgcagcagcc 3840
   gccgccgcca tgagcaccaa ctcgtttgat ggaagcattg tgagctcata tttgacaacg 3900
   cgcatgcccc catgggccgg ggtgcgtcag aatgtgatgg gctccagcat tgatggtcgc 3960
   cccgtcctgc ccgcaaactc tactaccttg acctacgaga ccgtgtctgg aacgccgttg 4020
   gagactgcag cctccgccgc cgcttcagcc gctgcagcca ccgcccgcgg gattgtgact 4080
   gactttgctt tcctgagccc gcttgcaagc agtgcagctt cccgttcatc cgcccgcgat 4140
   gacaagttga cggctctttt ggcacaattg gattctttga cccgggaact taatgtcgtt 4200
   tctcagcagc tgttggatct gcgccagcag gtttctgccc tgaaggcttc ctcccctccc 4260
   aatgcggttt aaaacataaa taaaaaacca gactctgttt ggatttggat caagcaagtg 4320
   tcttgctgtc tttatttagg ggttttgcgc gcgcggtagg cccgggacca gcggtctcgg 4380
   tcgttgaggg tcctgtgtat tttttccagg acgtggtaaa ggtgactctg gatgttcaga 4440
   tacatgggca taagcccgtc tctggggtgg aggtagcacc actgcagagc ttcatgctgc 4500
   ggggtggtgt tgtagatgat ccagtcgtag caggagcgct gggcgtggtg cctaaaaatg 4560
   tctttcagta gcaagctgat tgccaggggc aggcccttgg tgtaagtgtt tacaaagcgg 4620
   ttaagctggg atgggtgcat acgtggggat atgagatgca tcttggactg tatttttagg 4680
   ttggctatgt tcccagccat atccctccgg ggattcatgt tgtgcagaac caccagcaca 4740
   gtgtatccgg tgcacttggg aaatttgtca tgtagcttag aaggaaatgc gtggaagaac 4800
   ttggagacgc ccttgtgacc tccaagattt tccatgcatt cgtccataat gatggcaatg 4860
   ggcccacggg cggcggcctg ggcgaagata tttctgggat cactaacgtc atagttgtgt 4920
   tccaggatga gatcgtcata ggccattttt acaaagcgcg ggcggagggt gccagactgc 4980
   ggtataatgg ttccatccgg cccaggggcg tagttaccct cacagatttg catttcccac 5040
   gctttgagtt cagatggggg gatcatgtct acctgcgggg cgatgaagaa aacggtttcc 5100
   ggggtagggg agatcagctg ggaagaaagc aggttcctga gcagctgcga cttaccgcag 5160
   ccggtgggcc cgtaaatcac acctattacc ggctgcaact ggtagttaag agagctgcag 5220
   ctgccgtcat ccctgagcag gggggccact tcgttaagca tgtccctgac tcgcatgttt 5280
   tccctgacca aatccgccag aaggcgctcg ccgcccagcg atagcagttc ttgcaaggaa 5340
   gcaaagtttt tcaacggttt gagaccgtcc gccgtaggca tgcttttgag cgtttgacca 5400
   agcagttcca ggcggtccca cagctcggtc acctgctcta cggcatctcg atccagcata 5460
   tctcctcgtt tcgcgggttg gggcggcttt cgctgtacgg cagtagtcgg tgctcgtcca 5520
   gacgggccag ggtcatgtct ttccacgggc gcagggtcct cgtcagcgta gtctgggtca 5580
   cggtgaaggg gtgcgctccg ggctgcgcgc tggccagggt gcgcttgagg ctggtcctgc 5640
   tggtgctgaa gcgctgccgg tcttcgccct gcgcgtcggc caggtagcat ttgaccatgg 5700
   tgtcatagtc cagcccctcc gcggcgtggc ccttggcgcg cagcttgccc ttggaggagg 5760
   cgccgcacga ggggcagtgc agacttttga gggcgtagag cttgggcgcg agaaataccg 5820
   attccgggga gtaggcatcc gcgccgcagg ccccgcagac ggtctcgcat tccacgagcc 5880
   aggtgagctc tggccgttcg gggtcaaaaa ccaggtttcc cccatgcttt ttgatgcgtt 5940
   tcttacctct ggtttccatg agccggtgtc cacgctcggt gacgaaaagg ctgtccgtgt 6000
   ccccgtatac agacttgaga ggcctgtcct cgagcggtgt tccgcggtcc tcctcgtata 6060
   gaaactcgga ccactctgag acaaaggctc gcgtccaggc cagcacgaag gaggctaagt 6120
   gggaggggta gcggtcgttg tccactaggg ggtccactcg ctccagggtg tgaagacaca 6180
   tgtcgccctc ttcggcatca aggaaggtga ttggtttgta ggtgtaggcc acgtgaccgg 6240
   gtgttcctga aggggggcta taaaaggggg tgggggcgcg ttcgtcctca ctctcttccg 6300
   catcgctgtc tgcgagggcc agctgttggg gtgagtcgac gcgaggctgg atggccttcc 6360
   ccattatgat tcttctcgct tccggcggca tcgggatgcc cgcgttgcag gccatgctgt 6420
   ccaggcaggt agatgacgac catcagggac agcttcaagg ccagcaaaag gccaggaacc 6480
   gtaaaaaggc cgcgttgctg gcgtttttcc ataggctccg cccccctgac gagcatcaca 6540
   aaaatcgacg ctcaagtcag aggtggcgaa acccgacagg actataaaga taccaggcgt 6600
   ttccccctgg aagctccctc gtgcgctctc ctgttccgac cctgccgctt accggatacc 6660
   tgtccgcctt tctcccttcg ggaagcgtgg cgctttctca tagctcacgc tgtaggtatc 6720
   tcagttcggt gtaggtcgtt cgctccaagc tgggctgtgt gcacgaaccc cccgttcagc 6780
   ccgaccgctg cgccttatcc ggtaactatc gtcttgagtc caacccggta agacacgact 6840
   tatcgccact ggcagcagcc actggtaaca ggattagcag agcgaggtat gtaggcggtg 6900
   ctacagagtt cttgaagtgg tggcctaact acggctacac tagaaggaca gtatttggta 6960
   tctgcgctct gctgaagcca gttaccttcg gaaaaagagt tggtagctct tgatccggca 7020
   aacaaaccac cgctggtagc ggtggttttt ttgtttgcaa gcagcagatt acgcgcagaa 7080
   aaaaaggatc tcaagaagat cctttgatct tttctacggg gtctgacgct cagtggaacg 7140
   aaaactcacg ttaagggatt ttggtcatga gattatcaaa aaggatcttc acctagatcc 7200
   ttttaaatta aaaatgaagt tttaaatcaa tctaaagtat atatgagtaa acttggtctg 7260
   acagttacca atgcttaatc agtgaggcac ctatctcagc gatctgtcta tttcgttcat 7320
   ccatagttgc ctgactcccc gtcgtgtaga taactacgat acgggagggc ttaccatctg 7380
   gccccagtgc tgcaatgata ccgcgagacc cacgctcacc ggctccagat ttatcagcaa 7440
   taaaccagcc agccggaagg gccgagcgca gaagtggtcc tgcaacttta tccgcctcca 7500
   tccagtctat taattgttgc cgggaagcta gagtaagtag ttcgccagtt aatagtttgc 7560
   gcaacgttgt tgccattgct gcaggcatcg tggtgtcacg ctcgtcgttt ggtatggctt 7620
   cattcagctc cggttcccaa cgatcaaggc gagttacatg atcccccatg ttgtgcaaaa 7680
   aagcggttag ctccttcggt cctccgatcg ttgtcagaag taagttggcc gcagtgttat 7740
   cactcatggt tatggcagca ctgcataatt ctcttactgt catgccatcc gtaagatgct 7800
   tttctgtgac tggtgagtac tcaaccaagt cattctgaga atagtgtatg cggcgaccga 7860
   gttgctcttg cccggcgtca acacgggata ataccgcgcc acatagcaga actttaaaag 7920
   tgctcatcat tggaaaacgt tcttcggggc gaaaactctc aaggatctta ccgctgttga 7980
   gatccagttc gatgtaaccc actcgtgcac ccaactgatc ttcagcatct tttactttca 8040
   ccagcgtttc tgggtgagca aaaacaggaa ggcaaaatgc cgcaaaaaag ggaataaggg 8100
   cgacacggaa atgttgaata ctcatactct tcctttttca atattattga agcatttatc 8160
   agggttattg tctcatgagc ggatacatat ttgaatgtat ttagaaaaat aaacaaatag 8220
   gggttccgcg cacatttccc cgaaaagtgc cacctgacgt ctaagaaacc attattatca 8280
   tgacattaac ctataaaaat aggcgtatca cgaggccctt tcgtcttcaa gaattgtt 8338
<210> 27
   <211> 8221
   <212> DNA
   <213> Artificial Sequence
<220>
   <223> pAdApt Ebola GP(S)(dTM)
<400> 27
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ccagtgtgat ggatatctgc 1320
   agaattcggc ttatcttcag gatctcgcca tggagggtct tagcctactc caattgccca 1380
   gagataaatt tcgaaaaagc tctttctttg tttgggtcat catcttattt caaaaggcct 1440
   tttccatgcc tttgggtgtt gtgaccaaca gcactttaga agtaacagag attgaccagc 1500
   tagtctgcaa ggatcatctt gcatccactg accagctgaa atcagttggt ctcaacctcg 1560
   aggggagcgg agtatctact gatatcccat ctgcgacaaa gcgttggggc ttcagatctg 1620
   gtgtgcctcc caaggtggtc agctatgaag caggagaatg ggctgaaaat tgctacaatc 1680
   ttgaaataaa gaagccggac gggagcgaat gcttaccccc accgccggat ggtgtcagag 1740
   gctttccaag gtgccgctat gttcacaaag cccaaggaac cgggccctgc ccgggtgact 1800
   atgcctttca caaggatgga gctttcttcc tctatgacag gctggcttca actgtaattt 1860
   acagaggagt caattttgct gagggggtaa ttgcattctt gatattggct aaaccaaagg 1920
   aaacgttcct tcaatcaccc cccattcgag aggcagtaaa ctacactgaa aatacatcaa 1980
   gttactatgc cacatcctac ttggagtacg aaatcgaaaa ttttggtgct caacactcca 2040
   cgaccctttt caaaattaac aataatactt ttgttcttct ggacaggccc cacacgcctc 2100
   agttcctttt ccagctgaat gataccattc accttcacca acagttgagc aacacaactg 2160
   ggaaactaat ttggacacta gatgctaata tcaatgctga tattggtgaa tgggcttttt 2220
   gggaaaataa aaaaaatctc tccgaacaac tacgtggaga agagctgtct ttcgaaactt 2280
   tatcgctcaa cgagacagaa gacgatgatg cgacatcgtc gagaactaca aagggaagaa 2340
   tctccgaccg ggccaccagg aagtattcgg acctggttcc aaaggattcc cctgggatgg 2400
   tttcattgca cgtaccagaa ggggaaacaa cattgccgtc tcagaattcg acagaaggtc 2460
   gaagagtaga tgtgaatact caggaaacta tcacagagac aactgcaaca atcataggca 2520
   ctaacggtaa caacatgcag atctccacca tcgggacagg actgagctcc agccaaatcc 2580
   tgagttcctc accgaccatg gcaccaagcc ctgagactca gacctccaca acctacacac 2640
   caaaactacc agtgatgacc accgaggaac caacaacacc accgagaaac tctcctggct 2700
   caacaacaga agcacccact ctcaccaccc cagagaatat aacaacagcg gttaaaactg 2760
   ttttgccaca agagtccaca agcaacggtc taataacttc aacagtaaca gggattcttg 2820
   ggagccttgg acttcgaaaa cgcagcagaa gacaagttaa caccagggcc acgggtaaat 2880
   gcaatcccaa cttacactac tggactgcac aagaacaaca taatgctgct gggattgcct 2940
   ggatcccgta ctttggaccg ggtgcagaag gcatatacac tgaaggcctt atgcacaacc 3000
   aaaatgcctt agtctgtgga ctcagacaac ttgcaaatga aacaactcaa gctctgcagc 3060
   ttttcttaag ggccacgacg gagctgcgga catataccat actcaatagg aaggccatag 3120
   atttccttct gcgacgatgg ggcgggacat gtaggatcct gggaccagat tgttgcattg 3180
   agccacatga ttggaccaaa aacatcactg ataaaatcaa ccaaatcatc catgatttca 3240
   tcgacaaccc tttacccaat caggataatg atgataattg gtggacgggc tggagacagt 3300
   ggatcccggc cgcatcgtga ctgactgacg atctgcctcg cggatccaga tctgctgtgc 3360
   cttctagttg ccagccatct gttgtttgcc cctcccccgt gccttccttg accctggaag 3420
   gtgccactcc cactgtcctt tcctaataaa atgaggaaat tgcatcgcat tgtctgagta 3480
   ggtgtcattc tattctgggg ggtggggtgg ggcaggacag caagggggag gattgggaag 3540
   acaatagcag gcatgctggg gatgcggtgg gctctatggg tacccagggc cgcataactt 3600
   cgtataatgt atgctatacg aagttataag atctgtactg aaatgtgtgg gcgtggctta 3660
   agggtgggaa agaatatata aggtgggggt cttatgtagt tttgtatctg ttttgcagca 3720
   gccgccgccg ccatgagcac caactcgttt gatggaagca ttgtgagctc atatttgaca 3780
   acgcgcatgc ccccatgggc cggggtgcgt cagaatgtga tgggctccag cattgatggt 3840
   cgccccgtcc tgcccgcaaa ctctactacc ttgacctacg agaccgtgtc tggaacgccg 3900
   ttggagactg cagcctccgc cgccgcttca gccgctgcag ccaccgcccg cgggattgtg 3960
   actgactttg ctttcctgag cccgcttgca agcagtgcag cttcccgttc atccgcccgc 4020
   gatgacaagt tgacggctct tttggcacaa ttggattctt tgacccggga acttaatgtc 4080
   gtttctcagc agctgttgga tctgcgccag caggtttctg ccctgaaggc ttcctcccct 4140
   cccaatgcgg tttaaaacat aaataaaaaa ccagactctg tttggatttg gatcaagcaa 4200
   gtgtcttgct gtctttattt aggggttttg cgcgcgcggt aggcccggga ccagcggtct 4260
   cggtcgttga gggtcctgtg tattttttcc aggacgtggt aaaggtgact ctggatgttc 4320
   agatacatgg gcataagccc gtctctgggg tggaggtagc accactgcag agcttcatgc 4380
   tgcggggtgg tgttgtagat gatccagtcg tagcaggagc gctgggcgtg gtgcctaaaa 4440
   atgtctttca gtagcaagct gattgccagg ggcaggccct tggtgtaagt gtttacaaag 4500
   cggttaagct gggatgggtg catacgtggg gatatgagat gcatcttgga ctgtattttt 4560
   aggttggcta tgttcccagc catatccctc cggggattca tgttgtgcag aaccaccagc 4620
   acagtgtatc cggtgcactt gggaaatttg tcatgtagct tagaaggaaa tgcgtggaag 4680
   aacttggaga cgcccttgtg acctccaaga ttttccatgc attcgtccat aatgatggca 4740
   atgggcccac gggcggcggc ctgggcgaag atatttctgg gatcactaac gtcatagttg 4800
   tgttccagga tgagatcgtc ataggccatt tttacaaagc gcgggcggag ggtgccagac 4860
   tgcggtataa tggttccatc cggcccaggg gcgtagttac cctcacagat ttgcatttcc 4920
   cacgctttga gttcagatgg ggggatcatg tctacctgcg gggcgatgaa gaaaacggtt 4980
   tccggggtag gggagatcag ctgggaagaa agcaggttcc tgagcagctg cgacttaccg 5040
   cagccggtgg gcccgtaaat cacacctatt accggctgca actggtagtt aagagagctg 5100
   cagctgccgt catccctgag caggggggcc acttcgttaa gcatgtccct gactcgcatg 5160
   ttttccctga ccaaatccgc cagaaggcgc tcgccgccca gcgatagcag ttcttgcaag 5220
   gaagcaaagt ttttcaacgg tttgagaccg tccgccgtag gcatgctttt gagcgtttga 5280
   ccaagcagtt ccaggcggtc ccacagctcg gtcacctgct ctacggcatc tcgatccagc 5340
   atatctcctc gtttcgcggg ttggggcggc tttcgctgta cggcagtagt cggtgctcgt 5400
   ccagacgggc cagggtcatg tctttccacg ggcgcagggt cctcgtcagc gtagtctggg 5460
   tcacggtgaa ggggtgcgct ccgggctgcg cgctggccag ggtgcgcttg aggctggtcc 5520
   tgctggtgct gaagcgctgc cggtcttcgc cctgcgcgtc ggccaggtag catttgacca 5580
   tggtgtcata gtccagcccc tccgcggcgt ggcccttggc gcgcagcttg cccttggagg 5640
   aggcgccgca cgaggggcag tgcagacttt tgagggcgta gagcttgggc gcgagaaata 5700
   ccgattccgg ggagtaggca tccgcgccgc aggccccgca gacggtctcg cattccacga 5760
   gccaggtgag ctctggccgt tcggggtcaa aaaccaggtt tcccccatgc tttttgatgc 5820
   gtttcttacc tctggtttcc atgagccggt gtccacgctc ggtgacgaaa aggctgtccg 5880
   tgtccccgta tacagacttg agaggcctgt cctcgagcgg tgttccgcgg tcctcctcgt 5940
   atagaaactc ggaccactct gagacaaagg ctcgcgtcca ggccagcacg aaggaggcta 6000
   agtgggaggg gtagcggtcg ttgtccacta gggggtccac tcgctccagg gtgtgaagac 6060
   acatgtcgcc ctcttcggca tcaaggaagg tgattggttt gtaggtgtag gccacgtgac 6120
   cgggtgttcc tgaagggggg ctataaaagg gggtgggggc gcgttcgtcc tcactctctt 6180
   ccgcatcgct gtctgcgagg gccagctgtt ggggtgagtc gacgcgaggc tggatggcct 6240
   tccccattat gattcttctc gcttccggcg gcatcgggat gcccgcgttg caggccatgc 6300
   tgtccaggca ggtagatgac gaccatcagg gacagcttca aggccagcaa aaggccagga 6360
   accgtaaaaa ggccgcgttg ctggcgtttt tccataggct ccgcccccct gacgagcatc 6420
   acaaaaatcg acgctcaagt cagaggtggc gaaacccgac aggactataa agataccagg 6480
   cgtttccccc tggaagctcc ctcgtgcgct ctcctgttcc gaccctgccg cttaccggat 6540
   acctgtccgc ctttctccct tcgggaagcg tggcgctttc tcatagctca cgctgtaggt 6600
   atctcagttc ggtgtaggtc gttcgctcca agctgggctg tgtgcacgaa ccccccgttc 6660
   agcccgaccg ctgcgcctta tccggtaact atcgtcttga gtccaacccg gtaagacacg 6720
   acttatcgcc actggcagca gccactggta acaggattag cagagcgagg tatgtaggcg 6780
   gtgctacaga gttcttgaag tggtggccta actacggcta cactagaagg acagtatttg 6840
   gtatctgcgc tctgctgaag ccagttacct tcggaaaaag agttggtagc tcttgatccg 6900
   gcaaacaaac caccgctggt agcggtggtt tttttgtttg caagcagcag attacgcgca 6960
   gaaaaaaagg atctcaagaa gatcctttga tcttttctac ggggtctgac gctcagtgga 7020
   acgaaaactc acgttaaggg attttggtca tgagattatc aaaaaggatc ttcacctaga 7080
   tccttttaaa ttaaaaatga agttttaaat caatctaaag tatatatgag taaacttggt 7140
   ctgacagtta ccaatgctta atcagtgagg cacctatctc agcgatctgt ctatttcgtt 7200
   catccatagt tgcctgactc cccgtcgtgt agataactac gatacgggag ggcttaccat 7260
   ctggccccag tgctgcaatg ataccgcgag acccacgctc accggctcca gatttatcag 7320
   caataaacca gccagccgga agggccgagc gcagaagtgg tcctgcaact ttatccgcct 7380
   ccatccagtc tattaattgt tgccgggaag ctagagtaag tagttcgcca gttaatagtt 7440
   tgcgcaacgt tgttgccatt gctgcaggca tcgtggtgtc acgctcgtcg tttggtatgg 7500
   cttcattcag ctccggttcc caacgatcaa ggcgagttac atgatccccc atgttgtgca 7560
   aaaaagcggt tagctccttc ggtcctccga tcgttgtcag aagtaagttg gccgcagtgt 7620
   tatcactcat ggttatggca gcactgcata attctcttac tgtcatgcca tccgtaagat 7680
   gcttttctgt gactggtgag tactcaacca agtcattctg agaatagtgt atgcggcgac 7740
   cgagttgctc ttgcccggcg tcaacacggg ataataccgc gccacatagc agaactttaa 7800
   aagtgctcat cattggaaaa cgttcttcgg ggcgaaaact ctcaaggatc ttaccgctgt 7860
   tgagatccag ttcgatgtaa cccactcgtg cacccaactg atcttcagca tcttttactt 7920
   tcaccagcgt ttctgggtga gcaaaaacag gaaggcaaaa tgccgcaaaa aagggaataa 7980
   gggcgacacg gaaatgttga atactcatac tcttcctttt tcaatattat tgaagcattt 8040
   atcagggtta ttgtctcatg agcggataca tatttgaatg tatttagaaa aataaacaaa 8100
   taggggttcc gcgcacattt ccccgaaaag tgccacctga cgtctaagaa accattatta 8160
   tcatgacatt aacctataaa aataggcgta tcacgaggcc ctttcgtctt caagaattgt 8220
   t                                                                 8221
<210> 28
   <211> 8439
   <212> DNA
   <213> Artificial Sequence
<220>
   <223> pAdApt Ebola GP(Z)
<400> 28
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ctctagacca ggccctggat 1320
   cgatccaaca acacaatggg cgttacagga atattgcagt tacctcgtga tcgattcaag 1380
   aggacatcat tctttctttg ggtaattatc cttttccaaa gaacattttc catcccactt 1440
   ggagtcatcc acaatagcac attacaggtt agtgatgtcg acaaactagt ttgtcgtgac 1500
   aaactgtcat ccacaaatca attgagatca gttggactga atctcgaagg gaatggagtg 1560
   gcaactgacg tgccatctgc aactaaaaga tggggcttca ggtccggtgt cccaccaaag 1620
   gtggtcaatt atgaagctgg tgaatgggct gaaaactgct acaatcttga aatcaaaaaa 1680
   cctgacggga gtgagtgtct accagcagcg ccagacggga ttcggggctt cccccggtgc 1740
   cggtatgtgc acaaagtatc aggaacggga ccgtgtgccg gagactttgc cttccataaa 1800
   gagggtgctt tcttcctgta tgatcgactt gcttccacag ttatctaccg aggaacgact 1860
   ttcgctgaag gtgtcgttgc atttctgata ctgccccaag ctaagaagga cttcttcagc 1920
   tcacacccct tgagagagcc ggtcaatgca acggaggacc cgtctagtgg ctactattct 1980
   accacaatta gatatcaggc taccggtttt ggaaccaatg agacagagta cttgttcgag 2040
   gttgacaatt tgacctacgt ccaacttgaa tcaagattca caccacagtt tctgctccag 2100
   ctgaatgaga caatatatac aagtgggaaa aggagcaata ccacgggaaa actaatttgg 2160
   aaggtcaacc ccgaaattga tacaacaatc ggggagtggg ccttctggga aactaaaaaa 2220
   aacctcacta gaaaaattcg cagtgaagag ttgtctttca cagttgtatc aaacggagcc 2280
   aaaaacatca gtggtcagag tccggcgcga acttcttccg acccagggac caacacaaca 2340
   actgaagacc acaaaatcat ggcttcagaa aattcctctg caatggttca agtgcacagt 2400
   caaggaaggg aagctgcagt gtcgcatcta acaacccttg ccacaatctc cacgagtccc 2460
   caatccctca caaccaaacc aggtccggac aacagcaccc ataatacacc cgtgtataaa 2520
   cttgacatct ctgaggcaac tcaagttgaa caacatcacc gcagaacaga caacgacagc 2580
   acagcctccg acactccctc tgccacgacc gcagccggac ccccaaaagc agagaacacc 2640
   aacacgagca agagcactga cttcctggac cccgccacca caacaagtcc ccaaaaccac 2700
   agcgagaccg ctggcaacaa caacactcat caccaagata ccggagaaga gagtgccagc 2760
   agcgggaagc taggcttaat taccaatact attgctggag tcgcaggact gatcacaggc 2820
   gggagaagaa ctcgaagaga agcaattgtc aatgctcaac ccaaatgcaa ccctaattta 2880
   cattactgga ctactcagga tgaaggtgct gcaatcggac tggcctggat accatatttc 2940
   gggccagcag ccgagggaat ttacatagag gggctaatgc acaatcaaga tggtttaatc 3000
   tgtgggttga gacagctggc caacgagacg actcaagctc ttcaactgtt cctgagagcc 3060
   acaactgagc tacgcacctt ttcaatcctc aaccgtaagg caattgattt cttgctgcag 3120
   cgatggggcg gcacatgcca cattctggga ccggactgct gtatcgaacc acatgattgg 3180
   accaagaaca taacagacaa aattgatcag attattcatg attttgttga taaaaccctt 3240
   ccggaccagg gggacaatga caattggtgg acaggatgga gacaatggat accggcaggt 3300
   attggagtta caggcgttgt aattgcagtt atcgctttat tctgtatatg caaatttgtc 3360
   ttttagtttt tcttcagatt gcttcatgga aaagctcagc ctcaaatcaa tgaaaccagg 3420
   atttaattat atggattact tgaatctaag attacttgac aaatgataat ataatacact 3480
   ggagctttaa acatagccaa tgtgattcta actcctttaa actcacagtt aatcataaac 3540
   aaggtttgag gtaccgagct cgaattgatc tgctgtgcct tctagttgcc agccatctgt 3600
   tgtttgcccc tcccccgtgc cttccttgac cctggaaggt gccactccca ctgtcctttc 3660
   ctaataaaat gaggaaattg catcgcattg tctgagtagg tgtcattcta ttctgggggg 3720
   tggggtgggg caggacagca agggggagga ttgggaagac aatagcaggc atgctgggga 3780
   tgcggtgggc tctatgggta cccagggccg cataacttcg tataatgtat gctatacgaa 3840
   gttataagat ctgtactgaa atgtgtgggc gtggcttaag ggtgggaaag aatatataag 3900
   gtgggggtct tatgtagttt tgtatctgtt ttgcagcagc cgccgccgcc atgagcacca 3960
   actcgtttga tggaagcatt gtgagctcat atttgacaac gcgcatgccc ccatgggccg 4020
   gggtgcgtca gaatgtgatg ggctccagca ttgatggtcg ccccgtcctg cccgcaaact 4080
   ctactacctt gacctacgag accgtgtctg gaacgccgtt ggagactgca gcctccgccg 4140
   ccgcttcagc cgctgcagcc accgcccgcg ggattgtgac tgactttgct ttcctgagcc 4200
   cgcttgcaag cagtgcagct tcccgttcat ccgcccgcga tgacaagttg acggctcttt 4260
   tggcacaatt ggattctttg acccgggaac ttaatgtcgt ttctcagcag ctgttggatc 4320
   tgcgccagca ggtttctgcc ctgaaggctt cctcccctcc caatgcggtt taaaacataa 4380
   ataaaaaacc agactctgtt tggatttgga tcaagcaagt gtcttgctgt ctttatttag 4440
   gggttttgcg cgcgcggtag gcccgggacc agcggtctcg gtcgttgagg gtcctgtgta 4500
   ttttttccag gacgtggtaa aggtgactct ggatgttcag atacatgggc ataagcccgt 4560
   ctctggggtg gaggtagcac cactgcagag cttcatgctg cggggtggtg ttgtagatga 4620
   tccagtcgta gcaggagcgc tgggcgtggt gcctaaaaat gtctttcagt agcaagctga 4680
   ttgccagggg caggcccttg gtgtaagtgt ttacaaagcg gttaagctgg gatgggtgca 4740
   tacgtgggga tatgagatgc atcttggact gtatttttag gttggctatg ttcccagcca 4800
   tatccctccg gggattcatg ttgtgcagaa ccaccagcac agtgtatccg gtgcacttgg 4860
   gaaatttgtc atgtagctta gaaggaaatg cgtggaagaa cttggagacg cccttgtgac 4920
   ctccaagatt ttccatgcat tcgtccataa tgatggcaat gggcccacgg gcggcggcct 4980
   gggcgaagat atttctggga tcactaacgt catagttgtg ttccaggatg agatcgtcat 5040
   aggccatttt tacaaagcgc gggcggaggg tgccagactg cggtataatg gttccatccg 5100
   gcccaggggc gtagttaccc tcacagattt gcatttccca cgctttgagt tcagatgggg 5160
   ggatcatgtc tacctgcggg gcgatgaaga aaacggtttc cggggtaggg gagatcagct 5220
   gggaagaaag caggttcctg agcagctgcg acttaccgca gccggtgggc ccgtaaatca 5280
   cacctattac cggctgcaac tggtagttaa gagagctgca gctgccgtca tccctgagca 5340
   ggggggccac ttcgttaagc atgtccctga ctcgcatgtt ttccctgacc aaatccgcca 5400
   gaaggcgctc gccgcccagc gatagcagtt cttgcaagga agcaaagttt ttcaacggtt 5460
   tgagaccgtc cgccgtaggc atgcttttga gcgtttgacc aagcagttcc aggcggtccc 5520
   acagctcggt cacctgctct acggcatctc gatccagcat atctcctcgt ttcgcgggtt 5580
   ggggcggctt tcgctgtacg gcagtagtcg gtgctcgtcc agacgggcca gggtcatgtc 5640
   tttccacggg cgcagggtcc tcgtcagcgt agtctgggtc acggtgaagg ggtgcgctcc 5700
   gggctgcgcg ctggccaggg tgcgcttgag gctggtcctg ctggtgctga agcgctgccg 5760
   gtcttcgccc tgcgcgtcgg ccaggtagca tttgaccatg gtgtcatagt ccagcccctc 5820
   cgcggcgtgg cccttggcgc gcagcttgcc cttggaggag gcgccgcacg aggggcagtg 5880
   cagacttttg agggcgtaga gcttgggcgc gagaaatacc gattccgggg agtaggcatc 5940
   cgcgccgcag gccccgcaga cggtctcgca ttccacgagc caggtgagct ctggccgttc 6000
   ggggtcaaaa accaggtttc cccatgcttt tttgatgcgt ttcttacctc tggtttccat 6060
   gagccggtgt ccacgctcgg tgacgaaaag gctgtccgtg tccccgtata cagacttgag 6120
   aggcctgtcc tcgagcggtg ttccgcggtc ctcctcgtat agaaactcgg accactctga 6180
   gacaaaggct cgcgtccagg ccagcacgaa ggaggctaag tgggaggggt agcggtcgtt 6240
   gtccactagg gggtccactc gctccagggt gtgaagacac atgtcgccct cttcggcatc 6300
   aaggaaggtg attggtttgt aggtgtaggc cacgtgaccg ggtgttcctg aaggggggct 6360
   ataaaagggg gtgggggcgc gttcgtcctc actctcttcc gcatcgctgt ctgcgagggc 6420
   cagctgttgg ggtgagtcga cgcgaggctg gatggccttc cccattatga ttcttctcgc 6480
   ttccggcggc atcgggatgc ccgcgttgca ggccatgctg tccaggcagg tagatgacga 6540
   ccatcaggga cagcttcaag gccagcaaaa ggccaggaac cgtaaaaagg ccgcgttgct 6600
   ggcgtttttc cataggctcc gcccccctga cgagcatcac aaaaatcgac gctcaagtca 6660
   gaggtggcga aacccgacag gactataaag ataccaggcg tttccccctg gaagctccct 6720
   cgtgcgctct cctgttccga ccctgccgct taccggatac ctgtccgcct ttctcccttc 6780
   gggaagcgtg gcgctttctc atagctcacg ctgtaggtat ctcagttcgg tgtaggtcgt 6840
   tcgctccaag ctgggctgtg tgcacgaacc ccccgttcag cccgaccgct gcgccttatc 6900
   cggtaactat cgtcttgagt ccaacccggt aagacacgac ttatcgccac tggcagcagc 6960
   cactggtaac aggattagca gagcgaggta tgtaggcggt gctacagagt tcttgaagtg 7020
   gtggcctaac tacggctaca ctagaaggac agtatttggt atctgcgctc tgctgaagcc 7080
   agttaccttc ggaaaaagag ttggtagctc ttgatccggc aaacaaacca ccgctggtag 7140
   cggtggtttt tttgtttgca agcagcagat tacgcgcaga aaaaaaggat ctcaagaaga 7200
   tcctttgatc ttttctacgg ggtctgacgc tcagtggaac gaaaactcac gttaagggat 7260
   tttggtcatg agattatcaa aaaggatctt cacctagatc cttttaaatt aaaaatgaag 7320
   ttttaaatca atctaaagta tatatgagta aacttggtct gacagttacc aatgcttaat 7380
   cagtgaggca cctatctcag cgatctgtct atttcgttca tccatagttg cctgactccc 7440
   cgtcgtgtag ataactacga tacgggaggg cttaccatct ggccccagtg ctgcaatgat 7500
   accgcgagac ccacgctcac cggctccaga tttatcagca ataaaccagc cagccggaag 7560
   ggccgagcgc agaagtggtc ctgcaacttt atccgcctcc atccagtcta ttaattgttg 7620
   ccgggaagct agagtaagta gttcgccagt taatagtttg cgcaacgttg ttgccattgc 7680
   tgcaggcatc gtggtgtcac gctcgtcgtt tggtatggct tcattcagct ccggttccca 7740
   acgatcaagg cgagttacat gatcccccat gttgtgcaaa aaagcggtta gctccttcgg 7800
   tcctccgatc gttgtcagaa gtaagttggc cgcagtgtta tcactcatgg ttatggcagc 7860
   actgcataat tctcttactg tcatgccatc cgtaagatgc ttttctgtga ctggtgagta 7920
   ctcaaccaag tcattctgag aatagtgtat gcggcgaccg agttgctctt gcccggcgtc 7980
   aacacgggat aataccgcgc cacatagcag aactttaaaa gtgctcatca ttggaaaacg 8040
   ttcttcgggg cgaaaactct caaggatctt accgctgttg agatccagtt cgatgtaacc 8100
   cactcgtgca cccaactgat cttcagcatc ttttactttc accagcgttt ctgggtgagc 8160
   aaaaacagga aggcaaaatg ccgcaaaaaa gggaataagg gcgacacgga aatgttgaat 8220
   actcatactc ttcctttttc aatattattg aagcatttat cagggttatt gtctcatgag 8280
   cggatacata tttgaatgta tttagaaaaa taaacaaata ggggttccgc gcacatttcc 8340
   ccgaaaagtg ccacctgacg tctaagaaac cattattatc atgacattaa cctataaaaa 8400
   taggcgtatc acgaggccct ttcgtcttca agaattgtt 8439
<210> 29
   <211> 8199
   <212> DNA
   <213> Artificial Sequence
<220>
   <223> pAdApt Ebola GP(Z)(dTM)
<400> 29
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ctctagacca ggccctggat 1320
   cgatccaaca acacaatggg cgttacagga atattgcagt tacctcgtga tcgattcaag 1380
   aggacatcat tctttctttg ggtaattatc cttttccaaa gaacattttc catcccactt 1440
   ggagtcatcc acaatagcac attacaggtt agtgatgtcg acaaactagt ttgtcgtgac 1500
   aaactgtcat ccacaaatca attgagatca gttggactga atctcgaagg gaatggagtg 1560
   gcaactgacg tgccatctgc aactaaaaga tggggcttca ggtccggtgt cccaccaaag 1620
   gtggtcaatt atgaagctgg tgaatgggct gaaaactgct acaatcttga aatcaaaaaa 1680
   cctgacggga gtgagtgtct accagcagcg ccagacggga ttcggggctt cccccggtgc 1740
   cggtatgtgc acaaagtatc aggaacggga ccgtgtgccg gagactttgc cttccataaa 1800
   gagggtgctt tcttcctgta tgatcgactt gcttccacag ttatctaccg aggaacgact 1860
   ttcgctgaag gtgtcgttgc atttctgata ctgccccaag ctaagaagga cttcttcagc 1920
   tcacacccct tgagagagcc ggtcaatgca acggaggacc cgtctagtgg ctactattct 1980
   accacaatta gatatcaggc taccggtttt ggaaccaatg agacagagta cttgttcgag 2040
   gttgacaatt tgacctacgt ccaacttgaa tcaagattca caccacagtt tctgctccag 2100
   ctgaatgaga caatatatac aagtgggaaa aggagcaata ccacgggaaa actaatttgg 2160
   aaggtcaacc ccgaaattga tacaacaatc ggggagtggg ccttctggga aactaaaaaa 2220
   aacctcacta gaaaaattcg cagtgaagag ttgtctttca cagttgtatc aaacggagcc 2280
   aaaaacatca gtggtcagag tccggcgcga acttcttccg acccagggac caacacaaca 2340
   actgaagacc acaaaatcat ggcttcagaa aattcctctg caatggttca agtgcacagt 2400
   caaggaaggg aagctgcagt gtcgcatcta acaacccttg ccacaatctc cacgagtccc 2460
   caatccctca caaccaaacc aggtccggac aacagcaccc ataatacacc cgtgtataaa 2520
   cttgacatct ctgaggcaac tcaagttgaa caacatcacc gcagaacaga caacgacagc 2580
   acagcctccg acactccctc tgccacgacc gcagccggac ccccaaaagc agagaacacc 2640
   aacacgagca agagcactga cttcctggac cccgccacca caacaagtcc ccaaaaccac 2700
   agcgagaccg ctggcaacaa caacactcat caccaagata ccggagaaga gagtgccagc 2760
   agcgggaagc taggcttaat taccaatact attgctggag tcgcaggact gatcacaggc 2820
   gggagaagaa ctcgaagaga agcaattgtc aatgctcaac ccaaatgcaa ccctaattta 2880
   cattactgga ctactcagga tgaaggtgct gcaatcggac tggcctggat accatatttc 2940
   gggccagcag ccgagggaat ttacatagag gggctaatgc acaatcaaga tggtttaatc 3000
   tgtgggttga gacagctggc caacgagacg actcaagctc ttcaactgtt cctgagagcc 3060
   acaactgagc tacgcacctt ttcaatcctc aaccgtaagg caattgattt cttgctgcag 3120
   cgatggggcg gcacatgcca cattctggga ccggactgct gtatcgaacc acatgattgg 3180
   accaagaaca taacagacaa aattgatcag attattcatg attttgttga taaaaccctt 3240
   ccggaccagg gggacaatga caattggtgg acaggatgga gacaatggat ggccgcatcg 3300
   tgactgactg acgatctgcc tcgcgagatc tgctgtgcct tctagttgcc agccatctgt 3360
   tgtttgcccc tcccccgtgc cttccttgac cctggaaggt gccactccca ctgtcctttc 3420
   ctaataaaat gaggaaattg catcgcattg tctgagtagg tgtcattcta ttctgggggg 3480
   tggggtgggg caggacagca agggggagga ttgggaagac aatagcaggc atgctgggga 3540
   tgcggtgggc tctatgggta cccagggccg cataacttcg tataatgtat gctatacgaa 3600
   gttataagat ctgtactgaa atgtgtgggc gtggcttaag ggtgggaaag aatatataag 3660
   gtgggggtct tatgtagttt tgtatctgtt ttgcagcagc cgccgccgcc atgagcacca 3720
   actcgtttga tggaagcatt gtgagctcat atttgacaac gcgcatgccc ccatgggccg 3780
   gggtgcgtca gaatgtgatg ggctccagca ttgatggtcg ccccgtcctg cccgcaaact 3840
   ctactacctt gacctacgag accgtgtctg gaacgccgtt ggagactgca gcctccgccg 3900
   ccgcttcagc cgctgcagcc accgcccgcg ggattgtgac tgactttgct ttcctgagcc 3960
   cgcttgcaag cagtgcagct tcccgttcat ccgcccgcga tgacaagttg acggctcttt 4020
   tggcacaatt ggattctttg acccgggaac ttaatgtcgt ttctcagcag ctgttggatc 4080
   tgcgccagca ggtttctgcc ctgaaggctt cctcccctcc caatgcggtt taaaacataa 4140
   ataaaaaacc agactctgtt tggatttgga tcaagcaagt gtcttgctgt ctttatttag 4200
   gggttttgcg cgcgcggtag gcccgggacc agcggtctcg gtcgttgagg gtcctgtgta 4260
   ttttttccag gacgtggtaa aggtgactct ggatgttcag atacatgggc ataagcccgt 4320
   ctctggggtg gaggtagcac cactgcagag cttcatgctg cggggtggtg ttgtagatga 4380
   tccagtcgta gcaggagcgc tgggcgtggt gcctaaaaat gtctttcagt agcaagctga 4440
   ttgccagggg caggcccttg gtgtaagtgt ttacaaagcg gttaagctgg gatgggtgca 4500
   tacgtgggga tatgagatgc atcttggact gtatttttag gttggctatg ttcccagcca 4560
   tatccctccg gggattcatg ttgtgcagaa ccaccagcac agtgtatccg gtgcacttgg 4620
   gaaatttgtc atgtagctta gaaggaaatg cgtggaagaa cttggagacg cccttgtgac 4680
   ctccaagatt ttccatgcat tcgtccataa tgatggcaat gggcccacgg gcggcggcct 4740
   gggcgaagat atttctggga tcactaacgt catagttgtg ttccaggatg agatcgtcat 4800
   aggccatttt tacaaagcgc gggcggaggg tgccagactg cggtataatg gttccatccg 4860
   gcccaggggc gtagttaccc tcacagattt gcatttccca cgctttgagt tcagatgggg 4920
   ggatcatgtc tacctgcggg gcgatgaaga aaacggtttc cggggtaggg gagatcagct 4980
   gggaagaaag caggttcctg agcagctgcg acttaccgca gccggtgggc ccgtaaatca 5040
   cacctattac cggctgcaac tggtagttaa gagagctgca gctgccgtca tccctgagca 5100
   ggggggccac ttcgttaagc atgtccctga ctcgcatgtt ttccctgacc aaatccgcca 5160
   gaaggcgctc gccgcccagc gatagcagtt cttgcaagga agcaaagttt ttcaacggtt 5220
   tgagaccgtc cgccgtaggc atgcttttga gcgtttgacc aagcagttcc aggcggtccc 5280
   acagctcggt cacctgctct acggcatctc gatccagcat atctcctcgt ttcgcgggtt 5340
   ggggcggctt tcgctgtacg gcagtagtcg gtgctcgtcc agacgggcca gggtcatgtc 5400
   tttccacggg cgcagggtcc tcgtcagcgt agtctgggtc acggtgaagg ggtgcgctcc 5460
   gggctgcgcg ctggccaggg tgcgcttgag gctggtcctg ctggtgctga agcgctgccg 5520
   gtcttcgccc tgcgcgtcgg ccaggtagca tttgaccatg gtgtcatagt ccagcccctc 5580
   cgcggcgtgg cccttggcgc gcagcttgcc cttggaggag gcgccgcacg aggggcagtg 5640
   cagacttttg agggcgtaga gcttgggcgc gagaaatacc gattccgggg agtaggcatc 5700
   cgcgccgcag gccccgcaga cggtctcgca ttccacgagc caggtgagct ctggccgttc 5760
   ggggtcaaaa accaggtttc ccccatgctt tttgatgcgt ttcttacctc tggtttccat 5820
   gagccggtgt ccacgctcgg tgacgaaaag gctgtccgtg tccccgtata cagacttgag 5880
   aggcctgtcc tcgagcggtg ttccgcggtc ctcctcgtat agaaactcgg accactctga 5940
   gacaaaggct cgcgtccagg ccagcacgaa ggaggctaag tgggaggggt agcggtcgtt 6000
   gtccactagg gggtccactc gctccagggt gtgaagacac atgtcgccct cttcggcatc 6060
   aaggaaggtg attggtttgt aggtgtaggc cacgtgaccg ggtgttcctg aaggggggct 6120
   ataaaagggg gtgggggcgc gttcgtcctc actctcttcc gcatcgctgt ctgcgagggc 6180
   cagctgttgg ggtgagtcga cgcgaggctg gatggccttc cccattatga ttcttctcgc 6240
   ttccggcggc atcgggatgc ccgcgttgca ggccatgctg tccaggcagg tagatgacga 6300
   ccatcaggga cagcttcaag gccagcaaaa ggccaggaac cgtaaaaagg ccgcgttgct 6360
   ggcgtttttc cataggctcc gcccccctga cgagcatcac aaaaatcgac gctcaagtca 6420
   gaggtggcga aacccgacag gactataaag ataccaggcg tttccccctg gaagctccct 6480
   cgtgcgctct cctgttccga ccctgccgct taccggatac ctgtccgcct ttctcccttc 6540
   gggaagcgtg gcgctttctc atagctcacg ctgtaggtat ctcagttcgg tgtaggtcgt 6600
   tcgctccaag ctgggctgtg tgcacgaacc ccccgttcag cccgaccgct gcgccttatc 6660
   cggtaactat cgtcttgagt ccaacccggt aagacacgac ttatcgccac tggcagcagc 6720
   cactggtaac aggattagca gagcgaggta tgtaggcggt gctacagagt tcttgaagtg 6780
   gtggcctaac tacggctaca ctagaaggac agtatttggt atctgcgctc tgctgaagcc 6840
   agttaccttc ggaaaaagag ttggtagctc ttgatccggc aaacaaacca ccgctggtag 6900
   cggtggtttt tttgtttgca agcagcagat tacgcgcaga aaaaaaggat ctcaagaaga 6960
   tcctttgatc ttttctacgg ggtctgacgc tcagtggaac gaaaactcac gttaagggat 7020
   tttggtcatg agattatcaa aaaggatctt cacctagatc cttttaaatt aaaaatgaag 7080
   ttttaaatca atctaaagta tatatgagta aacttggtct gacagttacc aatgcttaat 7140
   cagtgaggca cctatctcag cgatctgtct atttcgttca tccatagttg cctgactccc 7200
   cgtcgtgtag ataactacga tacgggaggg cttaccatct ggccccagtg ctgcaatgat 7260
   accgcgagac ccacgctcac cggctccaga tttatcagca ataaaccagc cagccggaag 7320
   ggccgagcgc agaagtggtc ctgcaacttt atccgcctcc atccagtcta ttaattgttg 7380
   ccgggaagct agagtaagta gttcgccagt taatagtttg cgcaacgttg ttgccattgc 7440
   tgcaggcatc gtggtgtcac gctcgtcgtt tggtatggct tcattcagct ccggttccca 7500
   acgatcaagg cgagttacat gatcccccat gttgtgcaaa aaagcggtta gctccttcgg 7560
   tcctccgatc gttgtcagaa gtaagttggc cgcagtgtta tcactcatgg ttatggcagc 7620
   actgcataat tctcttactg tcatgccatc cgtaagatgc ttttctgtga ctggtgagta 7680
   ctcaaccaag tcattctgag aatagtgtat gcggcgaccg agttgctctt gcccggcgtc 7740
   aacacgggat aataccgcgc cacatagcag aactttaaaa gtgctcatca ttggaaaacg 7800
   ttcttcgggg cgaaaactct caaggatctt accgctgttg agatccagtt cgatgtaacc 7860
   cactcgtgca cccaactgat cttcagcatc ttttactttc accagcgttt ctgggtgagc 7920
   aaaaacagga aggcaaaatg ccgcaaaaaa gggaataagg gcgacacgga aatgttgaat 7980
   actcatactc ttcctttttc aatattattg aagcatttat cagggttatt gtctcatgag 8040
   cggatacata tttgaatgta tttagaaaaa taaacaaata ggggttccgc gcacatttcc 8100
   ccgaaaagtg ccacctgacg tctaagaaac cattattatc atgacattaa cctataaaaa 8160
   taggcgtatc acgaggccct ttcgtcttca agaattgtt                        8199
<210> 30
   <211> 7778
   <212> DNA
   <213> Artificial Sequence
<220>
   <223> pVR1012 Marburg
<400> 30
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga gtcgaatgaa 1920
   gaacattaat tgctgggtaa aagtgattaa tttctttaaa tttgaccaga ataatatttt 1980
   gtcagtgaat atattctcat atcacttgat taaaaacaga aaattaccct aacatgaaga 2040
   ccacatgttt ccttatcagt cttatcttaa ttcaagggac aaaaaatctc cccattttag 2100
   agatagctag taataatcaa ccccaaaatg tggattcggt atgctccgga actctccaga 2160
   agacagaaga cgtccatctg atgggattca cactgagtgg gcaaaaagtt gctgattccc 2220
   ctttggaggc atccaagcga tgggctttca ggacaggtgt acctcccaag aatgttgagt 2280
   acacagaggg ggaggaagcc aaaacatgct acaatataag tgtaacggat ccctctggaa 2340
   aatccttgct gttagatcct cctaccaaca tccgtgacta tcctaaatgc aaaactatcc 2400
   atcatattca aggtcaaaac cctcatgcac aggggatcgc ccttcattta tggggagcat 2460
   tttttctgta tgatcgcatt gcctccacaa caatgtaccg aggcaaagtc ttcactgaag 2520
   ggaacatagc agctatgatt gtcaataaga cagtgcacaa aatgattttc tcgcggcaag 2580
   gacaagggta ccgtcatatg aatctgactt ctactaataa atattggaca agtagtaacg 2640
   gaacgcaaac gaatgacact ggatgtttcg gcgctcttca agaatacaat tctacaaaga 2700
   accaaacatg tgctccgtcc aaaatacctc caccactgcc cacagcccgt ccggagatca 2760
   aactcacaag caccccaact gatgccacca aactcaatac cacggaccca agcagtgatg 2820
   atgaggacct cgcaacatcc ggctcagggt ccggagaacg agaaccccac acaacttctg 2880
   atgcggtcac caagcaaggg ctttcatcaa caatgccacc cactccctca ccacaaccaa 2940
   gcacgccaca gcaaggagga aacaacacaa accattccca agatgctgtg actgaactag 3000
   acaaaaataa cacaactgca caaccgtcca tgccccctca taacactacc acaatctcta 3060
   ctaacaacac ctccaaacac aacttcagca ctctctctgc accattacaa aacaccacca 3120
   atgacaacac acagagcaca atcactgaaa atgagcaaac cagtgccccc tcgataacaa 3180
   ccctgcctcc aacgggaaat cccaccacag caaagagcac cagcagcaaa aaaggccccg 3240
   ccacaacggc accaaacacg acaaatgagc atttcaccag tcctcccccc acccccagct 3300
   cgactgcaca acatcttgta tatttcagaa gaaagcgaag tatcctctgg agggaaggcg 3360
   acatgttccc ttttctggat gggttaataa atgctccaat tgattttgac ccagttccaa 3420
   atacaaaaac aatctttgat gaatcctcta gttctggtgc ctcggctgag gaagatcaac 3480
   atgcctcccc caatattagt ttaactttat cttattttcc taatataaat gagaacactg 3540
   cctactctgg agaaaatgag aatgattgtg atgcagagtt aagaatttgg agcgttcagg 3600
   aggatgacct ggccgcaggg ctcagttgga taccgttttt tggccctgga attgaaggac 3660
   tttacactgc tgttttaatt aaaaatcaaa acaatttggt ctgcaggttg aggcgtctag 3720
   ccaatcaaac tgccaaatcc ttggaactct tattgagagt cacaactgag gaaagaacat 3780
   tctccttaat caatagacat gctattgact ttctactcac aagatgggga ggaacatgca 3840
   aagtgcttgg acctgattgt tgcatcggga tagaagactt gtccaaaaat atttcagagc 3900
   aaattgacca aattaaaaag gacgaacaaa aagaggggac tggttggggt ctgggtggta 3960
   aatggtggac atccgactgg ggtgttctta ctaacttggg cattttgcta ctattatcca 4020
   tagctgtctt gattgctcta tcctgtattt gtcgtatctt tactaaatat atcggataac 4080
   gttaaatgtg taatgattag gactttagga caattgctac tgagcccttt tctaatctac 4140
   tgaaatcaac ttgggagatt tttaagaagc tgataactta atgtgaatca atagtttatg 4200
   tattatcgat tattatggtt tgatattcaa ttgttattat tgtcaggagt gaccttttct 4260
   atttgatgca ttaatgtttt aaactacctc ttaagccttt gagggcgtcc caatatgtgc 4320
   gtaggggtta atttaaaggg atttcttatt gtacagtttt ctgtattact tatttgggct 4380
   tgaagacata gttaagattt gccgaaatgc tctccagtca attccatccc ctctcagaaa 4440
   agacgtgctg ttcaaagagt cttaatttat aaccaactat tgcaagaatt aatttacttt 4500
   ttccgttata cttagttaca ttaatctttt gactgttcag cattattaac gacttgtctt 4560
   aattcaatcg ttcggatgaa attcataagg aaaaatgagc ctccttcccc ctattctggg 4620
   ctgagaaaat ttctcttatc cgcctaaaat cagatctgtt aggtcatggg tccttcataa 4680
   tctgtttgag catgaatatt gatgaaatga ccaaatgata gtgcatttgt atagactcaa 4740
   ttatccttta ttaagaaaaa tcgacctgca ggcatgcaag cttcaggatc cagatctgct 4800
   gtgccttcta gttgccagcc atctgttgtt tgcccctccc ccgtgccttc cttgaccctg 4860
   gaaggtgcca ctcccactgt cctttcctaa taaaatgagg aaattgcatc gcattgtctg 4920
   agtaggtgtc attctattct ggggggtggg gtggggcagc acagcaaggg ggaggattgg 4980
   gaagacaata gcaggcatgc tggggatgcg gtgggctcta tgggtaccca ggtgctgaag 5040
   aattgacccg gttcctcctg ggccagaaag aagcaggcac atccccttct ctgtgacaca 5100
   ccctgtccac gcccctggtt cttagttcca gccccactca taggacactc atagctcagg 5160
   agggctccgc cttcaatccc acccgctaaa gtacttggag cggtctctcc ctccctcatc 5220
   agcccaccaa accaaaccta gcctccaaga gtgggaagaa attaaagcaa gataggctat 5280
   taagtgcaga gggagagaaa atgcctccaa catgtgagga agtaatgaga gaaatcatag 5340
   aatttcttcc gcttcctcgc tcactgactc gctgcgctcg gtcgttcggc tgcggcgagc 5400
   ggtatcagct cactcaaagg cggtaatacg gttatccaca gaatcagggg ataacgcagg 5460
   aaagaacatg tgagcaaaag gccagcaaaa ggccaggaac cgtaaaaagg ccgcgttgct 5520
   ggcgtttttc cataggctcc gcccccctga cgagcatcac aaaaatcgac gctcaagtca 5580
   gaggtggcga aacccgacag gactataaag ataccaggcg tttccccctg gaagctccct 5640
   cgtgcgctct cctgttccga ccctgccgct taccggatac ctgtccgcct ttctcccttc 5700
   gggaagcgtg gcgctttctc aatgctcacg ctgtaggtat ctcagttcgg tgtaggtcgt 5760
   tcgctccaag ctgggctgtg tgcacgaacc ccccgttcag cccgaccgct gcgccttatc 5820
   cggtaactat cgtcttgagt ccaacccggt aagacacgac ttatcgccac tggcagcagc 5880
   cactggtaac aggattagca gagcgaggta tgtaggcggt gctacagagt tcttgaagtg 5940
   gtggcctaac tacggctaca ctagaaggac agtatttggt atctgcgctc tgctgaagcc 6000
   agttaccttc ggaaaaagag ttggtagctc ttgatccggc aaacaaacca ccgctggtag 6060
   cggtggtttt tttgtttgca agcagcagat tacgcgcaga aaaaaaggat ctcaagaaga 6120
   tcctttgatc ttttctacgg ggtctgacgc tcagtggaac gaaaactcac gttaagggat 6180
   tttggtcatg agattatcaa aaaggatctt cacctagatc cttttaaatt aaaaatgaag 6240
   ttttaaatca atctaaagta tatatgagta aacttggtct gacagttacc aatgcttaat 6300
   cagtgaggca cctatctcag cgatctgtct atttcgttca tccatagttg cctgactccg 6360
   gggggggggg gcgctgaggt ctgcctcgtg aagaaggtgt tgctgactca taccaggcct 6420
   gaatcgcccc atcatccagc cagaaagtga gggagccacg gttgatgaga gctttgttgt 6480
   aggtggacca gttggtgatt ttgaactttt gctttgccac ggaacggtct gcgttgtcgg 6540
   gaagatgcgt gatctgatcc ttcaactcag caaaagttcg atttattcaa caaagccgcc 6600
   gtcccgtcaa gtcagcgtaa tgctctgcca gtgttacaac caattaacca attctgatta 6660
   gaaaaactca tcgagcatca aatgaaactg caatttattc atatcaggat tatcaatacc 6720
   atatttttga aaaagccgtt tctgtaatga aggagaaaac tcaccgaggc agttccatag 6780
   gatggcaaga tcctggtatc ggtctgcgat tccgactcgt ccaacatcaa tacaacctat 6840
   taatttcccc tcgtcaaaaa taaggttatc aagtgagaaa tcaccatgag tgacgactga 6900
   atccggtgag aatggcaaaa gcttatgcat ttctttccag acttgttcaa caggccagcc 6960
   attacgctcg tcatcaaaat cactcgcatc aaccaaaccg ttattcattc gtgattgcgc 7020
   ctgagcgaga cgaaatacgc gatcgctgtt aaaaggacaa ttacaaacag gaatcgaatg 7080
   caaccggcgc aggaacactg ccagcgcatc aacaatattt tcacctgaat caggatattc 7140
   ttctaatacc tggaatgctg ttttcccggg gatcgcagtg gtgagtaacc atgcatcatc 7200
   aggagtacgg ataaaatgct tgatggtcgg aagaggcata aattccgtca gccagtttag 7260
   tctgaccatc tcatctgtaa catcattggc aacgctacct ttgccatgtt tcagaaacaa 7320
   ctctggcgca tcgggcttcc catacaatcg atagattgtc gcacctgatt gcccgacatt 7380
   atcgcgagcc catttatacc catataaatc agcatccatg ttggaattta atcgcggcct 7440
   cgagcaagac gtttcccgtt gaatatggct cataacaccc cttgtattac tgtttatgta 7500
   agcagacagt tttattgttc atgatgatat atttttatct tgtgcaatgt aacatcagag 7560
   attttgagac acaacgtggc tttccccccc cccccattat tgaagcattt atcagggtta 7620
   ttgtctcatg agcggataca tatttgaatg tatttagaaa aataaacaaa taggggttcc 7680
   gcgcacattt ccccgaaaag tgccacctga cgtctaagaa accattatta tcatgacatt 7740
   aacctataaa aataggcgta tcacgaggcc ctttcgtc 7778
<210> 31
   <211> 7005
   <212> DNA
   <213> Artificial Sequence
<220>
   <223> pVR1012x/s Marburg GP(dTM)
<400> 31
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga gtcgaatgaa 1920
   gaacattaat tgctgggtaa aagtgattaa tttctttaaa tttgaccaga ataatatttt 1980
   gtcagtgaat atattctcat atcacttgat taaaaacaga aaattaccct aacatgaaga 2040
   ccacatgttt ccttatcagt cttatcttaa ttcaagggac aaaaaatctc cccattttag 2100
   agatagctag taataatcaa ccccaaaatg tggattcggt atgctccgga actctccaga 2160
   agacagaaga cgtccatctg atgggattca cactgagtgg gcaaaaagtt gctgattccc 2220
   ctttggaggc atccaagcga tgggctttca ggacaggtgt acctcccaag aatgttgagt 2280
   acacagaggg ggaggaagcc aaaacatgct acaatataag tgtaacggat ccctctggaa 2340
   aatccttgct gttagatcct cctaccaaca tccgtgacta tcctaaatgc aaaactatcc 2400
   atcatattca aggtcaaaac cctcatgcac aggggatcgc ccttcattta tggggagcat 2460
   tttttctgta tgatcgcatt gcctccacaa caatgtaccg aggcaaagtc ttcactgaag 2520
   ggaacatagc agctatgatt gtcaataaga cagtgcacaa aatgattttc tcgcggcaag 2580
   gacaagggta ccgtcatatg aatctgactt ctactaataa atattggaca agtagtaacg 2640
   gaacgcaaac gaatgacact ggatgtttcg gcgctcttca agaatacaat tctacaaaga 2700
   accaaacatg tgctccgtcc aaaatacctc caccactgcc cacagcccgt ccggagatca 2760
   aactcacaag caccccaact gatgccacca aactcaatac cacggaccca agcagtgatg 2820
   atgaggacct cgcaacatcc ggctcagggt ccggagaacg agaaccccac acaacttctg 2880
   atgcggtcac caagcaaggg ctttcatcaa caatgccacc cactccctca ccacaaccaa 2940
   gcacgccaca gcaaggagga aacaacacaa accattccca agatgctgtg actgaactag 3000
   acaaaaataa cacaactgca caaccgtcca tgccccctca taacactacc acaatctcta 3060
   ctaacaacac ctccaaacac aacttcagca ctctctctgc accattacaa aacaccacca 3120
   atgacaacac acagagcaca atcactgaaa atgagcaaac cagtgccccc tcgataacaa 3180
   ccctgcctcc aacgggaaat cccaccacag caaagagcac cagcagcaaa aaaggccccg 3240
   ccacaacggc accaaacacg acaaatgagc atttcaccag tcctcccccc acccccagct 3300
   cgactgcaca acatcttgta tatttcagaa gaaagcgaag tatcctctgg agggaaggcg 3360
   acatgttccc ttttctggat gggttaataa atgctccaat tgattttgac ccagttccaa 3420
   atacaaaaac aatctttgat gaatcctcta gttctggtgc ctcggctgag gaagatcaac 3480
   atgcctcccc caatattagt ttaactttat cttattttcc taatataaat gagaacactg 3540
   cctactctgg agaaaatgag aatgattgtg atgcagagtt aagaatttgg agcgttcagg 3600
   aggatgacct ggccgcaggg ctcagttgga taccgttttt tggccctgga attgaaggac 3660
   tttacactgc tgttttaatt aaaaatcaaa acaatttggt ctgcaggttg aggcgtctag 3720
   ccaatcaaac tgccaaatcc ttggaactct tattgagagt cacaactgag gaaagaacat 3780
   tctccttaat caatagacat gctattgact ttctactcac aagatgggga ggaacatgca 3840
   aagtgcttgg acctgattgt tgcatcggga tagaagactt gtccaaaaat atttcagagc 3900
   aaattgacca aattaaaaag gacgaacaaa aagaggggac tggttggggt ctgggtggta 3960
   aatggtggac atccgactgg ggttaagatc tgctgtgcct tctagttgcc agccatctgt 4020
   tgtttgcccc tcccccgtgc cttccttgac cctggaaggt gccactccca ctgtcctttc 4080
   ctaataaaat gaggaaattg catcgcattg tctgagtagg tgtcattcta ttctgggggg 4140
   tggggtgggg caggacagca agggggagga ttgggaagac aatagcaggc atgctgggga 4200
   tgcggtgggc tctatgggta cccaggtgct gaagaattga cccggttcct cctgggccag 4260
   aaagaagcag gcacatcccc ttctctgtga cacaccctgt ccacgcccct ggttcttagt 4320
   tccagcccca ctcataggac actcatagct caggagggct ccgccttcaa tcccacccgc 4380
   taaagtactt ggagcggtct ctccctccct catcagccca ccaaaccaaa cctagcctcc 4440
   aagagtggga agaaattaaa gcaagatagg ctattaagtg cagagggaga gaaaatgcct 4500
   ccaacatgtg aggaagtaat gagagaaatc atagaatttt aaggccatga tttaaggcca 4560
   tcatggcctt aatcttccgc ttcctcgctc actgactcgc tgcgctcggt cgttcggctg 4620
   cggcgagcgg tatcagctca ctcaaaggcg gtaatacggt tatccacaga atcaggggat 4680
   aacgcaggaa agaacatgtg agcaaaaggc cagcaaaagg ccaggaaccg taaaaaggcc 4740
   gcgttgctgg cgtttttcca taggctccgc ccccctgacg agcatcacaa aaatcgacgc 4800
   tcaagtcaga ggtggcgaaa cccgacagga ctataaagat accaggcgtt tccccctgga 4860
   agctccctcg tgcgctctcc tgttccgacc ctgccgctta ccggatacct gtccgccttt 4920
   ctcccttcgg gaagcgtggc gctttctcat agctcacgct gtaggtatct cagttcggtg 4980
   taggtcgttc gctccaagct gggctgtgtg cacgaacccc ccgttcagcc cgaccgctgc 5040
   gccttatccg gtaactatcg tcttgagtcc aacccggtaa gacacgactt atcgccactg 5100
   gcagcagcca ctggtaacag gattagcaga gcgaggtatg taggcggtgc tacagagttc 5160
   ttgaagtggt ggcctaacta cggctacact agaagaacag tatttggtat ctgcgctctg 5220
   ctgaagccag ttaccttcgg aaaaagagtt ggtagctctt gatccggcaa acaaaccacc 5280
   gctggtagcg gtggtttttt tgtttgcaag cagcagatta cgcgcagaaa aaaaggatct 5340
   caagaagatc ctttgatctt ttctacgggg tctgacgctc agtggaacga aaactcacgt 5400
   taagggattt tggtcatgag attatcaaaa aggatcttca cctagatcct tttaaattaa 5460
   aaatgaagtt ttaaatcaat ctaaagtata tatgagtaaa cttggtctga cagttaccaa 5520
   tgcttaatca gtgaggcacc tatctcagcg atctgtctat ttcgttcatc catagttgcc 5580
   tgactcgggg ggggggggcg ctgaggtctg cctcgtgaag aaggtgttgc tgactcatac 5640
   caggcctgaa tcgccccatc atccagccag aaagtgaggg agccacggtt gatgagagct 5700
   ttgttgtagg tggaccagtt ggtgattttg aacttttgct ttgccacgga acggtctgcg 5760
   ttgtcgggaa gatgcgtgat ctgatccttc aactcagcaa aagttcgatt tattcaacaa 5820
   agccgccgtc ccgtcaagtc agcgtaatgc tctgccagtg ttacaaccaa ttaaccaatt 5880
   ctgattagaa aaactcatcg agcatcaaat gaaactgcaa tttattcata tcaggattat 5940
   caataccata tttttgaaaa agccgtttct gtaatgaagg agaaaactca ccgaggcagt 6000
   tccataggat ggcaagatcc tggtatcggt ctgcgattcc gactcgtcca acatcaatac 6060
   aacctattaa tttcccctcg tcaaaaataa ggttatcaag tgagaaatca ccatgagtga 6120
   cgactgaatc cggtgagaat ggcaaaagct tatgcatttc tttccagact tgttcaacag 6180
   gccagccatt acgctcgtca tcaaaatcac tcgcatcaac caaaccgtta ttcattcgtg 6240
   attgcgcctg agcgagacga aatacgcgat cgctgttaaa aggacaatta caaacaggaa 6300
   tcgaatgcaa ccggcgcagg aacactgcca gcgcatcaac aatattttca cctgaatcag 6360
   gatattcttc taatacctgg aatgctgttt tcccggggat cgcagtggtg agtaaccatg 6420
   catcatcagg agtacggata aaatgcttga tggtcggaag aggcataaat tccgtcagcc 6480
   agtttagtct gaccatctca tctgtaacat cattggcaac gctacctttg ccatgtttca 6540
   gaaacaactc tggcgcatcg ggcttcccat acaatcgata gattgtcgca cctgattgcc 6600
   cgacattatc gcgagcccat ttatacccat ataaatcagc atccatgttg gaatttaatc 6660
   gcggcctcga gcaagacgtt tcccgttgaa tatggctcat aacacccctt gtattactgt 6720
   ttatgtaagc agacagtttt attgttcatg atgatatatt tttatcttgt gcaatgtaac 6780
   atcagagatt ttgagacaca acgtggcttt cccccccccc ccattattga agcatttatc 6840
   agggttattg tctcatgagc ggatacatat ttgaatgtat ttagaaaaat aaacaaatag 6900
   gggttccgcg cacatttccc cgaaaagtgc cacctgacgt ctaagaaacc attattatca 6960
   tgacattaac ctataaaaat aggcgtatca cgaggccctt tcgtc                 7005
<210> 32
   <211> 8256
   <212> DNA
   <213> Artificial Sequence
<220>
   <223> pAdApt Marburg GP(dTM)
<400> 32
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ctctagagtc gaatgaagaa 1320
   cattaattgc tgggtaaaag tgattaattt ctttaaattt gaccagaata atattttgtc 1380
   agtgaatata ttctcatatc acttgattaa aaacagaaaa ttaccctaac atgaagacca 1440
   catgtttcct tatcagtctt atcttaattc aagggacaaa aaatctcccc attttagaga 1500
   tagctagtaa taatcaaccc caaaatgtgg attcggtatg ctccggaact ctccagaaga 1560
   cagaagacgt ccatctgatg ggattcacac tgagtgggca aaaagttgct gattcccctt 1620
   tggaggcatc caagcgatgg gctttcagga caggtgtacc tcccaagaat gttgagtaca 1680
   cagaggggga ggaagccaaa acatgctaca atataagtgt aacggatccc tctggaaaat 1740
   ccttgctgtt agatcctcct accaacatcc gtgactatcc taaatgcaaa actatccatc 1800
   atattcaagg tcaaaaccct catgcacagg ggatcgccct tcatttatgg ggagcatttt 1860
   ttctgtatga tcgcattgcc tccacaacaa tgtaccgagg caaagtcttc actgaaggga 1920
   acatagcagc tatgattgtc aataagacag tgcacaaaat gattttctcg cggcaaggac 1980
   aagggtaccg tcatatgaat ctgacttcta ctaataaata ttggacaagt agtaacggaa 2040
   cgcaaacgaa tgacactgga tgtttcggcg ctcttcaaga atacaattct acaaagaacc 2100
   aaacatgtgc tccgtccaaa atacctccac cactgcccac agcccgtccg gagatcaaac 2160
   tcacaagcac cccaactgat gccaccaaac tcaataccac ggacccaagc agtgatgatg 2220
   aggacctcgc aacatccggc tcagggtccg gagaacgaga accccacaca acttctgatg 2280
   cggtcaccaa gcaagggctt tcatcaacaa tgccacccac tccctcacca caaccaagca 2340
   cgccacagca aggaggaaac aacacaaacc attcccaaga tgctgtgact gaactagaca 2400
   aaaataacac aactgcacaa ccgtccatgc cccctcataa cactaccaca atctctacta 2460
   acaacacctc caaacacaac ttcagcactc tctctgcacc attacaaaac accaccaatg 2520
   acaacacaca gagcacaatc actgaaaatg agcaaaccag tgccccctcg ataacaaccc 2580
   tgcctccaac gggaaatccc accacagcaa agagcaccag cagcaaaaaa ggccccgcca 2640
   caacggcacc aaacacgaca aatgagcatt tcaccagtcc tccccccacc cccagctcga 2700
   ctgcacaaca tcttgtatat ttcagaagaa agcgaagtat cctctggagg gaaggcgaca 2760
   tgttcccttt tctggatggg ttaataaatg ctccaattga ttttgaccca gttccaaata 2820
   caaaaacaat ctttgatgaa tcctctagtt ctggtgcctc ggctgaggaa gatcaacatg 2880
   cctcccccaa tattagttta actttatctt attttcctaa tataaatgag aacactgcct 2940
   actctggaga aaatgagaat gattgtgatg cagagttaag aatttggagc gttcaggagg 3000
   atgacctggc cgcagggctc agttggatac cgttttttgg ccctggaatt gaaggacttt 3060
   acactgctgt tttaattaaa aatcaaaaca atttggtctg caggttgagg cgtctagcca 3120
   atcaaactgc caaatccttg gaactcttat tgagagtcac aactgaggaa agaacattct 3180
   ccttaatcaa tagacatgct attgactttc tactcacaag atggggagga acatgcaaag 3240
   tgcttggacc tgattgttgc atcgggatag aagacttgtc caaaaatatt tcagagcaaa 3300
   ttgaccaaat taaaaaggac gaacaaaaag aggggactgg ttggggtctg ggtggtaaat 3360
   ggtggacatc cgactggggt taagatctgc tgtgccttct agttgccagc catctgttgt 3420
   ttgcccctcc cccgtgcctt ccttgaccct ggaaggtgcc actcccactg tcctttccta 3480
   ataaaatgag gaaattgcat cgcattgtct gagtaggtgt cattctattc tggggggtgg 3540
   ggtggggcag cacagcaagg gggaggattg ggaagacaat agcaggcatg ctggggatgc 3600
   ggtgggctct atgggtaccc agggccgcat aacttcgtat aatgtatgct atacgaagtt 3660
   ataagatctg tactgaaatg tgtgggcgtg gcttaagggt gggaaagaat atataaggtg 3720
   ggggtcttat gtagttttgt atctgttttg cagcagccgc cgccgccatg agcaccaact 3780
   cgtttgatgg aagcattgtg agctcatatt tgacaacgcg catgccccca tgggccgggg 3840
   tgcgtcagaa tgtgatgggc tccagcattg atggtcgccc cgtcctgccc gcaaactcta 3900
   ctaccttgac ctacgagacc gtgtctggaa cgccgttgga gactgcagcc tccgccgccg 3960
   cttcagccgc tgcagccacc gcccgcggga ttgtgactga ctttgctttc ctgagcccgc 4020
   ttgcaagcag tgcagcttcc cgttcatccg cccgcgatga caagttgacg gctcttttgg 4080
   cacaattgga ttctttgacc cgggaactta atgtcgtttc tcagcagctg ttggatctgc 4140
   gccagcaggt ttctgccctg aaggcttcct cccctcccaa tgcggtttaa aacataaata 4200
   aaaaaccaga ctctgtttgg atttggatca agcaagtgtc ttgctgtctt tatttagggg 4260
   ttttgcgcgc gcggtaggcc cgggaccagc ggtctcggtc gttgagggtc ctgtgtattt 4320
   tttccaggac gtggtaaagg tgactctgga tgttcagata catgggcata agcccgtctc 4380
   tggggtggag gtagcaccac tgcagagctt catgctgcgg ggtggtgttg tagatgatcc 4440
   agtcgtagca ggagcgctgg gcgtggtgcc taaaaatgtc tttcagtagc aagctgattg 4500
   ccaggggcag gcccttggtg taagtgttta caaagcggtt aagctgggat gggtgcatac 4560
   gtggggatat gagatgcatc ttggactgta tttttaggtt ggctatgttc ccagccatat 4620
   ccctccgggg attcatgttg tgcagaacca ccagcacagt gtatccggtg cacttgggaa 4680
   atttgtcatg tagcttagaa ggaaatgcgt ggaagaactt ggagacgccc ttgtgacctc 4740
   caagattttc catgcattcg tccataatga tggcaatggg cccacgggcg gcggcctggg 4800
   cgaagatatt tctgggatca ctaacgtcat agttgtgttc caggatgaga tcgtcatagg 4860
   ccatttttac aaagcgcggg cggagggtgc cagactgcgg tataatggtt ccatccggcc 4920
   caggggcgta gttaccctca cagatttgca tttcccacgc tttgagttca gatgggggga 4980
   tcatgtctac ctgcggggcg atgaagaaaa cggtttccgg ggtaggggag atcagctggg 5040
   aagaaagcag gttcctgagc agctgcgact taccgcagcc ggtgggcccg taaatcacac 5100
   ctattaccgg ctgcaactgg tagttaagag agctgcagct gccgtcatcc ctgagcaggg 5160
   gggccacttc gttaagcatg tccctgactc gcatgttttc cctgaccaaa tccgccagaa 5220
   ggcgctcgcc gcccagcgat agcagttctt gcaaggaagc aaagtttttc aacggtttga 5280
   gaccgtccgc cgtaggcatg cttttgagcg tttgaccaag cagttccagg cggtcccaca 5340
   gctcggtcac ctgctctacg gcatctcgat ccagcatatc tcctcgtttc gcgggttggg 5400
   gcggctttcg ctgtacggca gtagtcggtg ctcgtccaga cgggccaggg tcatgtcttt 5460
   ccacgggcgc agggtcctcg tcagcgtagt ctgggtcacg gtgaaggggt gcgctccggg 5520
   ctgcgcgctg gccagggtgc gcttgaggct ggtcctgctg gtgctgaagc gctgccggtc 5580
   ttcgccctgc gcgtcggcca ggtagcattt gaccatggtg tcatagtcca gcccctccgc 5640
   ggcgtggccc ttggcgcgca gcttgccctt ggaggaggcg ccgcacgagg ggcagtgcag 5700
   acttttgagg gcgtagagct tgggcgcgag aaataccgat tccggggagt aggcatccgc 5760
   gccgcaggcc ccgcagacgg tctcgcattc cacgagccag gtgagctctg gccgttcggg 5820
   gtcaaaaacc aggtttcccc catgcttttt gatgcgtttc ttacctctgg tttccatgag 5880
   ccggtgtcca cgctcggtga cgaaaaggct gtccgtgtcc ccgtatacag acttgagagg 5940
   cctgtcctcg agcggtgttc cgcggtcctc ctcgtataga aactcggacc actctgagac 6000
   aaaggctcgc gtccaggcca gcacgaagga ggctaagtgg gaggggtagc ggtcgttgtc 6060
   cactaggggg tccactcgct ccagggtgtg aagacacatg tcgccctctt cggcatcaag 6120
   gaaggtgatt ggtttgtagg tgtaggccac gtgaccgggt gttcctgaag gggggctata 6180
   aaagggggtg ggggcgcgtt cgtcctcact ctcttccgca tcgctgtctg cgagggccag 6240
   ctgttggggt gagtcgacgc gaggctggat ggccttcccc attatgattc ttctcgcttc 6300
   cggcggcatc gggatgcccg cgttgcaggc catgctgtcc aggcaggtag atgacgacca 6360
   tcagggacag cttcaaggcc agcaaaaggc caggaaccgt aaaaaggccg cgttgctggc 6420
   gtttttccat aggctccgcc cccctgacga gcatcacaaa aatcgacgct caagtcagag 6480
   gtggcgaaac ccgacaggac tataaagata ccaggcgttt ccccctggaa gctccctcgt 6540
   gcgctctcct gttccgaccc tgccgcttac cggatacctg tccgcctttc tcccttcggg 6600
   aagcgtggcg ctttctcata gctcacgctg taggtatctc agttcggtgt aggtcgttcg 6660
   ctccaagctg ggctgtgtgc acgaaccccc cgttcagccc gaccgctgcg ccttatccgg 6720
   taactatcgt cttgagtcca acccggtaag acacgactta tcgccactgg cagcagccac 6780
   tggtaacagg attagcagag cgaggtatgt aggcggtgct acagagttct tgaagtggtg 6840
   gcctaactac ggctacacta gaaggacagt atttggtatc tgcgctctgc tgaagccagt 6900
   taccttcgga aaaagagttg gtagctcttg atccggcaaa caaaccaccg ctggtagcgg 6960
   tggttttttt gtttgcaagc agcagattac gcgcagaaaa aaaggatctc aagaagatcc 7020
   tttgatcttt tctacggggt ctgacgctca gtggaacgaa aactcacgtt aagggatttt 7080
   ggtcatgaga ttatcaaaaa ggatcttcac ctagatcctt ttaaattaaa aatgaagttt 7140
   taaatcaatc taaagtatat atgagtaaac ttggtctgac agttaccaat gcttaatcag 7200
   tgaggcacct atctcagcga tctgtctatt tcgttcatcc atagttgcct gactccccgt 7260
   cgtgtagata actacgatac gggagggctt accatctggc cccagtgctg caatgatacc 7320
   gcgagaccca cgctcaccgg ctccagattt atcagcaata aaccagccag ccggaagggc 7380
   cgagcgcaga agtggtcctg caactttatc cgcctccatc cagtctatta attgttgccg 7440
   ggaagctaga gtaagtagtt cgccagttaa tagtttgcgc aacgttgttg ccattgctgc 7500
   aggcatcgtg gtgtcacgct cgtcgtttgg tatggcttca ttcagctccg gttcccaacg 7560
   atcaaggcga gttacatgat cccccatgtt gtgcaaaaaa gcggttagct ccttcggtcc 7620
   tccgatcgtt gtcagaagta agttggccgc agtgttatca ctcatggtta tggcagcact 7680
   gcataattct cttactgtca tgccatccgt aagatgcttt tctgtgactg gtgagtactc 7740
   aaccaagtca ttctgagaat agtgtatgcg gcgaccgagt tgctcttgcc cggcgtcaac 7800
   acgggataat accgcgccac atagcagaac tttaaaagtg ctcatcattg gaaaacgttc 7860
   ttcggggcga aaactctcaa ggatcttacc gctgttgaga tccagttcga tgtaacccac 7920
   tcgtgcaccc aactgatctt cagcatcttt tactttcacc agcgtttctg ggtgagcaaa 7980
   aacaggaagg caaaatgccg caaaaaaggg aataagggcg acacggaaat gttgaatact 8040
   catactcttc ctttttcaat attattgaag catttatcag ggttattgtc tcatgagcgg 8100
   atacatattt gaatgtattt agaaaaataa acaaataggg gttccgcgca catttccccg 8160
   aaaagtgcca cctgacgtct aagaaaccat tattatcatg acattaacct ataaaaatag 8220
   gcgtatcacg aggccctttc gtcttcaaga attgtt                           8256
<210> 33
   <211> 6447
   <212> DNA
   <213> Artificial Sequence
<220>
   <223> pVR1012x/s Lassa GP
<400> 33
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga atttaggatt 1920
   gcgcttttag agattcacta ctagttagga attcctaaat catggggcag attattacat 1980
   tctttcaaga agtgccacat gtaatagagg aagtcatgaa cattgtgcta attgcgcttt 2040
   ctctattggc aatcttgaag ggcttgtata acatcgctac atgtgggatt attggattgg 2100
   ttgccttttt attcttgtgt ggcaagtctt gttccctaac ccttaaaggg ggatatgagc 2160
   tgcaaacctt agaattaaat atggagaccc taaacatgac catgccctta tcatgcacca 2220
   agaacagcag tcatcattac ataagagtgg gcaatgagac tggattagaa ttgactttaa 2280
   ctaacaccag cattataaat cacaaatttt gcaacttatc cgatgctcac aaaaagaatc 2340
   tttatgatca tgctctcatg agcatcatct caacattcca tctatccatt ccaaacttca 2400
   atcagtatga agccatgagt tgtgatttca atggagggaa aatcagtgtg caatacaacc 2460
   tctctcattc ctatgctggg gatgcggccg aacactgtgg gacagttgcc aacggagtgt 2520
   tgcaaacatt tatgagaatg gcctggggtg gaagatacat tgcattagac tcaggaaagg 2580
   gaaactggga ctgtataatg accagctacc agtacctgat aattcaaaat acaacatggg 2640
   aggaccactg ccaattctca agaccgtctc ctatcgggta ccttggcctt ttgtcacaaa 2700
   ggacaagaga tatatatata agtaggaggc tcttggggac cttcacctgg acattgtcag 2760
   attctgaggg caatgaaaca ccaggtggtt attgtttaac caggtggatg ctaattgaag 2820
   cagaactcaa gtgttttggg aatacagctg tggcaaaatg caatgagaag catgatgagg 2880
   agttttgtga catgctgaga ttgtttgatt tcaacaagca agcaatccgt aggttgaagg 2940
   ctgaggccca gatgagtatt caattaataa ataaagccgt gaatgcctta atcaatgatc 3000
   aattaatcat gaagaaccat ttaagagaca tcatgggcat tccctactgc aattacagca 3060
   agtattggta ccttaatcat actagtagcg ggagaacatc actaccaaag tgttggctta 3120
   tatccaatgg gtcatatcta aatgaaaccc agttctctga tgacatagaa cagcaagccg 3180
   acaatatgat cacagagatg cttcagaaag aatacattga aagacaaggg aaaacgccct 3240
   tgggactagt ggacattttc atctttagca caagctttta tctgatcagc attttcttgc 3300
   atttaattaa aatccctaca catcgacaca tcgttgggaa accctgtccc aaaccccata 3360
   gactaaatca catgggagta tgttcctgtg gactgtacaa acaccctggt gttccaacaa 3420
   agtggaagag atagggatcc agatctgctg tgccttctag ttgccagcca tctgttgttt 3480
   gcccctcccc cgtgccttcc ttgaccctgg aaggtgccac tcccactgtc ctttcctaat 3540
   aaaatgagga aattgcatcg cattgtctga gtaggtgtca ttctattctg gggggtgggg 3600
   tggggcagga cagcaagggg gaggattggg aagacaatag caggcatgct ggggatgcgg 3660
   tgggctctat gggtacccag gtgctgaaga attgacccgg ttcctcctgg gccagaaaga 3720
   agcaggcaca tccccttctc tgtgacacac cctgtccacg cccctggttc ttagttccag 3780
   ccccactcat aggacactca tagctcagga gggctccgcc ttcaatccca cccgctaaag 3840
   tacttggagc ggtctctccc tccctcatca gcccaccaaa ccaaacctag cctccaagag 3900
   tgggaagaaa ttaaagcaag ataggctatt aagtgcagag ggagagaaaa tgcctccaac 3960
   atgtgaggaa gtaatgagag aaatcataga attttaaggc catcatggcc ttaatcttcc 4020
   gcttcctcgc tcactgactc gctgcgctcg gtcgttcggc tgcggcgagc ggtatcagct 4080
   cactcaaagg cggtaatacg gttatccaca gaatcagggg ataacgcagg aaagaacatg 4140
   tgagcaaaag gccagcaaaa ggccaggaac cgtaaaaagg ccgcgttgct ggcgtttttc 4200
   cataggctcc gcccccctga cgagcatcac aaaaatcgac gctcaagtca gaggtggcga 4260
   aacccgacag gactataaag ataccaggcg tttccccctg gaagctccct cgtgcgctct 4320
   cctgttccga ccctgccgct taccggatac ctgtccgcct ttctcccttc gggaagcgtg 4380
   gcgctttctc atagctcacg ctgtaggtat ctcagttcgg tgtaggtcgt tcgctccaag 4440
   ctgggctgtg tgcacgaacc ccccgttcag cccgaccgct gcgccttatc cggtaactat 4500
   cgtcttgagt ccaacccggt aagacacgac ttatcgccac tggcagcagc cactggtaac 4560
   aggattagca gagcgaggta tgtaggcggt gctacagagt tcttgaagtg gtggcctaac 4620
   tacggctaca ctagaagaac agtatttggt atctgcgctc tgctgaagcc agttaccttc 4680
   ggaaaaagag ttggtagctc ttgatccggc aaacaaacca ccgctggtag cggtggtttt 4740
   tttgtttgca agcagcagat tacgcgcaga aaaaaaggat ctcaagaaga tcctttgatc 4800
   ttttctacgg ggtctgacgc tcagtggaac gaaaactcac gttaagggat tttggtcatg 4860
   agattatcaa aaaggatctt cacctagatc cttttaaatt aaaaatgaag ttttaaatca 4920
   atctaaagta tatatgagta aacttggtct gacagttacc aatgcttaat cagtgaggca 4980
   cctatctcag cgatctgtct atttcgttca tccatagttg cctgactcgg gggggggggg 5040
   cgctgaggtc tgcctcgtga agaaggtgtt gctgactcat accaggcctg aatcgcccca 5100
   tcatccagcc agaaagtgag ggagccacgg ttgatgagag ctttgttgta ggtggaccag 5160
   ttggtgattt tgaacttttg ctttgccacg gaacggtctg cgttgtcggg aagatgcgtg 5220
   atctgatcct tcaactcagc aaaagttcga tttattcaac aaagccgccg tcccgtcaag 5280
   tcagcgtaat gctctgccag tgttacaacc aattaaccaa ttctgattag aaaaactcat 5340
   cgagcatcaa atgaaactgc aatttattca tatcaggatt atcaatacca tatttttgaa 5400
   aaagccgttt ctgtaatgaa ggagaaaact caccgaggca gttccatagg atggcaagat 5460
   cctggtatcg gtctgcgatt ccgactcgtc caacatcaat acaacctatt aatttcccct 5520
   cgtcaaaaat aaggttatca agtgagaaat caccatgagt gacgactgaa tccggtgaga 5580
   atggcaaaag cttatgcatt tctttccaga cttgttcaac aggccagcca ttacgctcgt 5640
   catcaaaatc actcgcatca accaaaccgt tattcattcg tgattgcgcc tgagcgagac 5700
   gaaatacgcg atcgctgtta aaaggacaat tacaaacagg aatcgaatgc aaccggcgca 5760
   ggaacactgc cagcgcatca acaatatttt cacctgaatc aggatattct tctaatacct 5820
   ggaatgctgt tttcccgggg atcgcagtgg tgagtaacca tgcatcatca ggagtacgga 5880
   taaaatgctt gatggtcgga agaggcataa attccgtcag ccagtttagt ctgaccatct 5940
   catctgtaac atcattggca acgctacctt tgccatgttt cagaaacaac tctggcgcat 6000
   cgggcttccc atacaatcga tagattgtcg cacctgattg cccgacatta tcgcgagccc 6060
   atttataccc atataaatca gcatccatgt tggaatttaa tcgcggcctc gagcaagacg 6120
   tttcccgttg aatatggctc ataacacccc ttgtattact gtttatgtaa gcagacagtt 6180
   ttattgttca tgatgatata tttttatctt gtgcaatgta acatcagaga ttttgagaca 6240
   caacgtggct ttcccccccc ccccattatt gaagcattta tcagggttat tgtctcatga 6300
   gcggatacat atttgaatgt atttagaaaa ataaacaaat aggggttccg cgcacatttc 6360
   cccgaaaagt gccacctgac gtctaagaaa ccattattat catgacatta acctataaaa 6420
   ataggcgtat cacgaggccc tttcgtc                                     6447
<210> 34
   <211> 6258
   <212> DNA
   <213> Artificial Sequence
<220>
   <223> pVR1012x/s Lassa GP(dTM)
<400> 34
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga atttaggatt 1920
   gcgcttttag agattcacta ctagttagga attcctaaat catggggcag attattacat 1980
   tctttcaaga agtgccacat gtaatagagg aagtcatgaa cattgtgcta attgcgcttt 2040
   ctctattggc aatcttgaag ggcttgtata acatcgctac atgtgggatt attggattgg 2100
   ttgccttttt attcttgtgt ggcaagtctt gttccctaac ccttaaaggg ggatatgagc 2160
   tgcaaacctt agaattaaat atggagaccc taaacatgac catgccctta tcatgcacca 2220
   agaacagcag tcatcattac ataagagtgg gcaatgagac tggattagaa ttgactttaa 2280
   ctaacaccag cattataaat cacaaatttt gcaacttatc cgatgctcac aaaaagaatc 2340
   tttatgatca tgctctcatg agcatcatct caacattcca tctatccatt ccaaacttca 2400
   atcagtatga agccatgagt tgtgatttca atggagggaa aatcagtgtg caatacaacc 2460
   tctctcattc ctatgctggg gatgcggccg aacactgtgg gacagttgcc aacggagtgt 2520
   tgcaaacatt tatgagaatg gcctggggtg gaagatacat tgcattagac tcaggaaagg 2580
   gaaactggga ctgtataatg accagctacc agtacctgat aattcaaaat acaacatggg 2640
   aggaccactg ccaattctca agaccgtctc ctatcgggta ccttggcctt ttgtcacaaa 2700
   ggacaagaga tatatatata agtaggaggc tcttggggac cttcacctgg acattgtcag 2760
   attctgaggg caatgaaaca ccaggtggtt attgtttaac caggtggatg ctaattgaag 2820
   cagaactcaa gtgttttggg aatacagctg tggcaaaatg caatgagaag catgatgagg 2880
   agttttgtga catgctgaga ttgtttgatt tcaacaagca agcaatccgt aggttgaagg 2940
   ctgaggccca gatgagtatt caattaataa ataaagccgt gaatgcctta atcaatgatc 3000
   aattaatcat gaagaaccat ttaagagaca tcatgggcat tccctactgc aattacagca 3060
   agtattggta ccttaatcat actagtagcg ggagaacatc actaccaaag tgttggctta 3120
   tatccaatgg gtcatatcta aatgaaaccc agttctctga tgacatagaa cagcaagccg 3180
   acaatatgat cacagagatg cttcagaaag aatacattga aagacaaggg aaaacgccct 3240
   tgtagggatc cagatctgct gtgccttcta gttgccagcc atctgttgtt tgcccctccc 3300
   ccgtgccttc cttgaccctg gaaggtgcca ctcccactgt cctttcctaa taaaatgagg 3360
   aaattgcatc gcattgtctg agtaggtgtc attctattct ggggggtggg gtggggcagg 3420
   acagcaaggg ggaggattgg gaagacaata gcaggcatgc tggggatgcg gtgggctcta 3480
   tgggtaccca ggtgctgaag aattgacccg gttcctcctg ggccagaaag aagcaggcac 3540
   atccccttct ctgtgacaca ccctgtccac gcccctggtt cttagttcca gccccactca 3600
   taggacactc atagctcagg agggctccgc cttcaatccc acccgctaaa gtacttggag 3660
   cggtctctcc ctccctcatc agcccaccaa accaaaccta gcctccaaga gtgggaagaa 3720
   attaaagcaa gataggctat taagtgcaga gggagagaaa atgcctccaa catgtgagga 3780
   agtaatgaga gaaatcatag aattttaagg ccatcatggc cttaatcttc cgcttcctcg 3840
   ctcactgact cgctgcgctc ggtcgttcgg ctgcggcgag cggtatcagc tcactcaaag 3900
   gcggtaatac ggttatccac agaatcaggg gataacgcag gaaagaacat gtgagcaaaa 3960
   ggccagcaaa aggccaggaa ccgtaaaaag gccgcgttgc tggcgttttt ccataggctc 4020
   cgcccccctg acgagcatca caaaaatcga cgctcaagtc agaggtggcg aaacccgaca 4080
   ggactataaa gataccaggc gtttccccct ggaagctccc tcgtgcgctc tcctgttccg 4140
   accctgccgc ttaccggata cctgtccgcc tttctccctt cgggaagcgt ggcgctttct 4200
   catagctcac gctgtaggta tctcagttcg gtgtaggtcg ttcgctccaa gctgggctgt 4260
   gtgcacgaac cccccgttca gcccgaccgc tgcgccttat ccggtaacta tcgtcttgag 4320
   tccaacccgg taagacacga cttatcgcca ctggcagcag ccactggtaa caggattagc 4380
   agagcgaggt atgtaggcgg tgctacagag ttcttgaagt ggtggcctaa ctacggctac 4440
   actagaagaa cagtatttgg tatctgcgct ctgctgaagc cagttacctt cggaaaaaga 4500
   gttggtagct cttgatccgg caaacaaacc accgctggta gcggtggttt ttttgtttgc 4560
   aagcagcaga ttacgcgcag aaaaaaagga tctcaagaag atcctttgat cttttctacg 4620
   gggtctgacg ctcagtggaa cgaaaactca cgttaaggga ttttggtcat gagattatca 4680
   aaaaggatct tcacctagat ccttttaaat taaaaatgaa gttttaaatc aatctaaagt 4740
   atatatgagt aaacttggtc tgacagttac caatgcttaa tcagtgaggc acctatctca 4800
   gcgatctgtc tatttcgttc atccatagtt gcctgactcg gggggggggg gcgctgaggt 4860
   ctgcctcgtg aagaaggtgt tgctgactca taccaggcct gaatcgcccc atcatccagc 4920
   cagaaagtga gggagccacg gttgatgaga gctttgttgt aggtggacca gttggtgatt 4980
   ttgaactttt gctttgccac ggaacggtct gcgttgtcgg gaagatgcgt gatctgatcc 5040
   ttcaactcag caaaagttcg atttattcaa caaagccgcc gtcccgtcaa gtcagcgtaa 5100
   tgctctgcca gtgttacaac caattaacca attctgatta gaaaaactca tcgagcatca 5160
   aatgaaactg caatttattc atatcaggat tatcaatacc atatttttga aaaagccgtt 5220
   tctgtaatga aggagaaaac tcaccgaggc agttccatag gatggcaaga tcctggtatc 5280
   ggtctgcgat tccgactcgt ccaacatcaa tacaacctat taatttcccc tcgtcaaaaa 5340
   taaggttatc aagtgagaaa tcaccatgag tgacgactga atccggtgag aatggcaaaa 5400
   gcttatgcat ttctttccag acttgttcaa caggccagcc attacgctcg tcatcaaaat 5460
   cactcgcatc aaccaaaccg ttattcattc gtgattgcgc ctgagcgaga cgaaatacgc 5520
   gatcgctgtt aaaaggacaa ttacaaacag gaatcgaatg caaccggcgc aggaacactg 5580
   ccagcgcatc aacaatattt tcacctgaat caggatattc ttctaatacc tggaatgctg 5640
   ttttcccggg gatcgcagtg gtgagtaacc atgcatcatc aggagtacgg ataaaatgct 5700
   tgatggtcgg aagaggcata aattccgtca gccagtttag tctgaccatc tcatctgtaa 5760
   catcattggc aacgctacct ttgccatgtt tcagaaacaa ctctggcgca tcgggcttcc 5820
   catacaatcg atagattgtc gcacctgatt gcccgacatt atcgcgagcc catttatacc 5880
   catataaatc agcatccatg ttggaattta atcgcggcct cgagcaagac gtttcccgtt 5940
   gaatatggct cataacaccc cttgtattac tgtttatgta agcagacagt tttattgttc 6000
   atgatgatat atttttatct tgtgcaatgt aacatcagag attttgagac acaacgtggc 6060
   tttccccccc cccccattat tgaagcattt atcagggtta ttgtctcatg agcggataca 6120
   tatttgaatg tatttagaaa aataaacaaa taggggttcc gcgcacattt ccccgaaaag 6180
   tgccacctga cgtctaagaa accattatta tcatgacatt aacctataaa aataggcgta 6240
   tcacgaggcc ctttcgtc 6258
<210> 35
   <211> 7711
   <212> DNA
   <213> Artificial Sequence
<220>
   <223> pAdApt Lassa GP
<400> 35
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ctctagaatt taggattgcg 1320
   cttttagaga ttcactacta gttaggaatt cctaaatcat ggggcagatt attacattct 1380
   ttcaagaagt gccacatgta atagaggaag tcatgaacat tgtgctaatt gcgctttctc 1440
   tattggcaat cttgaagggc ttgtataaca tcgctacatg tgggattatt ggattggttg 1500
   cctttttatt cttgtgtggc aagtcttgtt ccctaaccct taaaggggga tatgagctgc 1560
   aaaccttaga attaaatatg gagaccctaa acatgaccat gcccttatca tgcaccaaga 1620
   acagcagtca tcattacata agagtgggca atgagactgg attagaattg actttaacta 1680
   acaccagcat tataaatcac aaattttgca acttatccga tgctcacaaa aagaatcttt 1740
   atgatcatgc tctcatgagc atcatctcaa cattccatct atccattcca aacttcaatc 1800
   agtatgaagc catgagttgt gatttcaatg gagggaaaat cagtgtgcaa tacaacctct 1860
   ctcattccta tgctggggat gcggccgaac actgtgggac agttgccaac ggagtgttgc 1920
   aaacatttat gagaatggcc tggggtggaa gatacattgc attagactca ggaaagggaa 1980
   actgggactg tataatgacc agctaccagt acctgataat tcaaaataca acatgggagg 2040
   accactgcca attctcaaga ccgtctccta tcgggtacct tggccttttg tcacaaagga 2100
   caagagatat atatataagt aggaggctct tggggacctt cacctggaca ttgtcagatt 2160
   ctgagggcaa tgaaacacca ggtggttatt gtttaaccag gtggatgcta attgaagcag 2220
   aactcaagtg ttttgggaat acagctgtgg caaaatgcaa tgagaagcat gatgaggagt 2280
   tttgtgacat gctgagattg tttgatttca acaagcaagc aatccgtagg ttgaaggctg 2340
   aggcccagat gagtattcaa ttaataaata aagccgtgaa tgccttaatc aatgatcaat 2400
   taatcatgaa gaaccattta agagacatca tgggcattcc ctactgcaat tacagcaagt 2460
   attggtacct taatcatact agtagcggga gaacatcact accaaagtgt tggcttatat 2520
   ccaatgggtc atatctaaat gaaacccagt tctctgatga catagaacag caagccgaca 2580
   atatgatcac agagatgctt cagaaagaat acattgaaag acaagggaaa acgcccttgg 2640
   gactagtgga cattttcatc tttagcacaa gcttttatct gatcagcatt ttcttgcatt 2700
   taattaaaat ccctacacat cgacacatcg ttgggaaacc ctgtcccaaa ccccatagac 2760
   taaatcacat gggagtatgt tcctgtggac tgtacaaaca ccctggtgtt ccaacaaagt 2820
   ggaagagata gggatccaga tctgctgtgc cttctagttg ccagccatct gttgtttgcc 2880
   cctcccccgt gccttccttg accctggaag gtgccactcc cactgtcctt tcctaataaa 2940
   atgaggaaat tgcatcgcat tgtctgagta ggtgtcattc tattctgggg ggtggggtgg 3000
   ggcagcacag caagggggag gattgggaag acaatagcag gcatgctggg gatgcggtgg 3060
   gctctatggg tacccagggc cgcataactt cgtataatgt atgctatacg aagttataag 3120
   atctgtactg aaatgtgtgg gcgtggctta agggtgggaa agaatatata aggtgggggt 3180
   cttatgtagt tttgtatctg ttttgcagca gccgccgccg ccatgagcac caactcgttt 3240
   gatggaagca ttgtgagctc atatttgaca acgcgcatgc ccccatgggc cggggtgcgt 3300
   cagaatgtga tgggctccag cattgatggt cgccccgtcc tgcccgcaaa ctctactacc 3360
   ttgacctacg agaccgtgtc tggaacgccg ttggagactg cagcctccgc cgccgcttca 3420
   gccgctgcag ccaccgcccg cgggattgtg actgactttg ctttcctgag cccgcttgca 3480
   agcagtgcag cttcccgttc atccgcccgc gatgacaagt tgacggctct tttggcacaa 3540
   ttggattctt tgacccggga acttaatgtc gtttctcagc agctgttgga tctgcgccag 3600
   caggtttctg ccctgaaggc ttcctcccct cccaatgcgg tttaaaacat aaataaaaaa 3660
   ccagactctg tttggatttg gatcaagcaa gtgtcttgct gtctttattt aggggttttg 3720
   cgcgcgcggt aggcccggga ccagcggtct cggtcgttga gggtcctgtg tattttttcc 3780
   aggacgtggt aaaggtgact ctggatgttc agatacatgg gcataagccc gtctctgggg 3840
   tggaggtagc accactgcag agcttcatgc tgcggggtgg tgttgtagat gatccagtcg 3900
   tagcaggagc gctgggcgtg gtgcctaaaa atgtctttca gtagcaagct gattgccagg 3960
   ggcaggccct tggtgtaagt gtttacaaag cggttaagct gggatgggtg catacgtggg 4020
   gatatgagat gcatcttgga ctgtattttt aggttggcta tgttcccagc catatccctc 4080
   cggggattca tgttgtgcag aaccaccagc acagtgtatc cggtgcactt gggaaatttg 4140
   tcatgtagct tagaaggaaa tgcgtggaag aacttggaga cgcccttgtg acctccaaga 4200
   ttttccatgc attcgtccat aatgatggca atgggcccac gggcggcggc ctgggcgaag 4260
   atatttctgg gatcactaac gtcatagttg tgttccagga tgagatcgtc ataggccatt 4320
   tttacaaagc gcgggcggag ggtgccagac tgcggtataa tggttccatc cggcccaggg 4380
   gcgtagttac cctcacagat ttgcatttcc cacgctttga gttcagatgg ggggatcatg 4440
   tctacctgcg gggcgatgaa gaaaacggtt tccggggtag gggagatcag ctgggaagaa 4500
   agcaggttcc tgagcagctg cgacttaccg cagccggtgg gcccgtaaat cacacctatt 4560
   accggctgca actggtagtt aagagagctg cagctgccgt catccctgag caggggggcc 4620
   acttcgttaa gcatgtccct gactcgcatg ttttccctga ccaaatccgc cagaaggcgc 4680
   tcgccgccca gcgatagcag ttcttgcaag gaagcaaagt ttttcaacgg tttgagaccg 4740
   tccgccgtag gcatgctttt gagcgtttga ccaagcagtt ccaggcggtc ccacagctcg 4800
   gtcacctgct ctacggcatc tcgatccagc atatctcctc gtttcgcggg ttggggcggc 4860
   tttcgctgta cggcagtagt cggtgctcgt ccagacgggc cagggtcatg tctttccacg 4920
   ggcgcagggt cctcgtcagc gtagtctggg tcacggtgaa ggggtgcgct ccgggctgcg 4980
   cgctggccag ggtgcgcttg aggctggtcc tgctggtgct gaagcgctgc cggtcttcgc 5040
   cctgcgcgtc ggccaggtag catttgacca tggtgtcata gtccagcccc tccgcggcgt 5100
   ggcccttggc gcgcagcttg cccttggagg aggcgccgca cgaggggcag tgcagacttt 5160
   tgagggcgta gagcttgggc gcgagaaata ccgattccgg ggagtaggca tccgcgccgc 5220
   aggccccgca gacggtctcg cattccacga gccaggtgag ctctggccgt tcggggtcaa 5280
   aaaccaggtt tcccccatgc tttttgatgc gtttcttacc tctggtttcc atgagccggt 5340
   gtccacgctc ggtgacgaaa aggctgtccg tgtccccgta tacagacttg agaggcctgt 5400
   cctcgagcgg tgttccgcgg tcctcctcgt atagaaactc ggaccactct gagacaaagg 5460
   ctcgcgtcca ggccagcacg aaggaggcta agtgggaggg gtagcggtcg ttgtccacta 5520
   gggggtccac tcgctccagg gtgtgaagac acatgtcgcc ctcttcggca tcaaggaagg 5580
   tgattggttt gtaggtgtag gccacgtgac cgggtgttcc tgaagggggg ctataaaagg 5640
   gggtgggggc gcgttcgtcc tcactctctt ccgcatcgct gtctgcgagg gccagctgtt 5700
   ggggtgagtc gacgcgaggc tggatggcct tccccattat gattcttctc gcttccggcg 5760
   gcatcgggat gcccgcgttg caggccatgc tgtccaggca ggtagatgac gaccatcagg 5820
   gacagcttca aggccagcaa aaggccagga accgtaaaaa ggccgcgttg ctggcgtttt 5880
   tccataggct ccgcccccct gacgagcatc acaaaaatcg acgctcaagt cagaggtggc 5940
   gaaacccgac aggactataa agataccagg cgtttccccc tggaagctcc ctcgtgcgct 6000
   ctcctgttcc gaccctgccg cttaccggat acctgtccgc ctttctccct tcgggaagcg 6060
   tggcgctttc tcatagctca cgctgtaggt atctcagttc ggtgtaggtc gttcgctcca 6120
   agctgggctg tgtgcacgaa ccccccgttc agcccgaccg ctgcgcctta tccggtaact 6180
   atcgtcttga gtccaacccg gtaagacacg acttatcgcc actggcagca gccactggta 6240
   acaggattag cagagcgagg tatgtaggcg gtgctacaga gttcttgaag tggtggccta 6300
   actacggcta cactagaagg acagtatttg gtatctgcgc tctgctgaag ccagttacct 6360
   tcggaaaaag agttggtagc tcttgatccg gcaaacaaac caccgctggt agcggtggtt 6420
   tttttgtttg caagcagcag attacgcgca gaaaaaaagg atctcaagaa gatcctttga 6480
   tcttttctac ggggtctgac gctcagtgga acgaaaactc acgttaaggg attttggtca 6540
   tgagattatc aaaaaggatc ttcacctaga tccttttaaa ttaaaaatga agttttaaat 6600
   caatctaaag tatatatgag taaacttggt ctgacagtta ccaatgctta atcagtgagg 6660
   cacctatctc agcgatctgt ctatttcgtt catccatagt tgcctgactc cccgtcgtgt 6720
   agataactac gatacgggag ggcttaccat ctggccccag tgctgcaatg ataccgcgag 6780
   acccacgctc accggctcca gatttatcag caataaacca gccagccgga agggccgagc 6840
   gcagaagtgg tcctgcaact ttatccgcct ccatccagtc tattaattgt tgccgggaag 6900
   ctagagtaag tagttcgcca gttaatagtt tgcgcaacgt tgttgccatt gctgcaggca 6960
   tcgtggtgtc acgctcgtcg tttggtatgg cttcattcag ctccggttcc caacgatcaa 7020
   ggcgagttac atgatccccc atgttgtgca aaaaagcggt tagctccttc ggtcctccga 7080
   tcgttgtcag aagtaagttg gccgcagtgt tatcactcat ggttatggca gcactgcata 7140
   attctcttac tgtcatgcca tccgtaagat gcttttctgt gactggtgag tactcaacca 7200
   agtcattctg agaatagtgt atgcggcgac cgagttgctc ttgcccggcg tcaacacggg 7260
   ataataccgc gccacatagc agaactttaa aagtgctcat cattggaaaa cgttcttcgg 7320
   ggcgaaaact ctcaaggatc ttaccgctgt tgagatccag ttcgatgtaa cccactcgtg 7380
   cacccaactg atcttcagca tcttttactt tcaccagcgt ttctgggtga gcaaaaacag 7440
   gaaggcaaaa tgccgcaaaa aagggaataa gggcgacacg gaaatgttga atactcatac 7500
   tcttcctttt tcaatattat tgaagcattt atcagggtta ttgtctcatg agcggataca 7560
   tatttgaatg tatttagaaa aataaacaaa taggggttcc gcgcacattt ccccgaaaag 7620
   tgccacctga cgtctaagaa accattatta tcatgacatt aacctataaa aataggcgta 7680
   tcacgaggcc ctttcgtctt caagaattgt t                                7711
<210> 36
   <211> 7522
   <212> DNA
   <213> Artificial Sequence
<220>
   <223> pAdApt Lassa GP(dTM)
<400> 36
   ttaattaacc gcaattctca tgtttgacag cttatcatca tcaataatat accttatttt 60
   ggattgaagc caatatgata atgagggggt ggagtttgtg acgtggcgcg gggcgtggga 120
   acggggcggg tgacgtagta gtgtggcgga agtgtgatgt tgcaagtgtg gcggaacaca 180
   tgtaagcgac ggatgtggca aaagtgacgt ttttggtgtg cgccggtgta cacaggaagt 240
   gacaattttc gcgcggtttt aggcggatgt tgtagtaaat ttgggcgtaa ccgagtaaga 300
   tttggccatt ttcgcgggaa aactgaataa gaggaagtga aatctgaata attttgtgtt 360
   actcatagcg cgtaatattt gtctagggcc gcggggactt tgaccgttta cgtggagact 420
   cgcccaggtg tttttctcag gtgttttccg cgttccgggt caaagttggc gttttattat 480
   tatagtcagt acgtaccagt gcactggcct agagcggccc cattgcatac gttgtatcca 540
   tatcataata tgtacattta tattggctca tgtccaacat taccgccatg ttgacattga 600
   ttattgacta gttattaata gtaatcaatt acggggtcat tagttcatag cccatatatg 660
   gagttccgcg ttacataact tacggtaaat ggcccgcctg gctgaccgcc caacgacccc 720
   cgcccattga cgtcaataat gacgtatgtt cccatagtaa cgccaatagg gactttccat 780
   tgacgtcaat gggtggagta tttacggtaa actgcccact tggcagtaca tcaagtgtat 840
   catatgccaa gtacgccccc tattgacgtc aatgacggta aatggcccgc ctggcattat 900
   gcccagtaca tgaccttatg ggactttcct acttggcagt acatctacgt attagtcatc 960
   gctattacca tggtgatgcg gttttggcag tacatcaatg ggcgtggata gcggtttgac 1020
   tcacggggat ttccaagtct ccaccccatt gacgtcaatg ggagtttgtt ttggcaccaa 1080
   aatcaacggg actttccaaa atgtcgtaac aactccgccc cattgacgca aatgggcggt 1140
   aggcgtgtac ggtgggaggt ctatataagc agagctcgtt tagtgaaccg tcagatcgcc 1200
   tggagacgcc atccacgctg ttttgacctc catagaagac accgggaccg atccagcctc 1260
   cgtcaccgtc gtcgacacgt gtgatcagat atcgcggccg ctctagaatt taggattgcg 1320
   cttttagaga ttcactacta gttaggaatt cctaaatcat ggggcagatt attacattct 1380
   ttcaagaagt gccacatgta atagaggaag tcatgaacat tgtgctaatt gcgctttctc 1440
   tattggcaat cttgaagggc ttgtataaca tcgctacatg tgggattatt ggattggttg 1500
   cctttttatt cttgtgtggc aagtcttgtt ccctaaccct taaaggggga tatgagctgc 1560
   aaaccttaga attaaatatg gagaccctaa acatgaccat gcccttatca tgcaccaaga 1620
   acagcagtca tcattacata agagtgggca atgagactgg attagaattg actttaacta 1680
   acaccagcat tataaatcac aaattttgca acttatccga tgctcacaaa aagaatcttt 1740
   atgatcatgc tctcatgagc atcatctcaa cattccatct atccattcca aacttcaatc 1800
   agtatgaagc catgagttgt gatttcaatg gagggaaaat cagtgtgcaa tacaacctct 1860
   ctcattccta tgctggggat gcggccgaac actgtgggac agttgccaac ggagtgttgc 1920
   aaacatttat gagaatggcc tggggtggaa gatacattgc attagactca ggaaagggaa 1980
   actgggactg tataatgacc agctaccagt acctgataat tcaaaataca acatgggagg 2040
   accactgcca attctcaaga ccgtctccta tcgggtacct tggccttttg tcacaaagga 2100
   caagagatat atatataagt aggaggctct tggggacctt cacctggaca ttgtcagatt 2160
   ctgagggcaa tgaaacacca ggtggttatt gtttaaccag gtggatgcta attgaagcag 2220
   aactcaagtg ttttgggaat acagctgtgg caaaatgcaa tgagaagcat gatgaggagt 2280
   tttgtgacat gctgagattg tttgatttca acaagcaagc aatccgtagg ttgaaggctg 2340
   aggcccagat gagtattcaa ttaataaata aagccgtgaa tgccttaatc aatgatcaat 2400
   taatcatgaa gaaccattta agagacatca tgggcattcc ctactgcaat tacagcaagt 2460
   attggtacct taatcatact agtagcggga gaacatcact accaaagtgt tggcttatat 2520
   ccaatgggtc atatctaaat gaaacccagt tctctgatga catagaacag caagccgaca 2580
   atatgatcac agagatgctt cagaaagaat acattgaaag acaagggaaa acgcccttgt 2640
   agggatccag atctgctgtg ccttctagtt gccagccatc tgttgtttgc ccctcccccg 2700
   tgccttcctt gaccctggaa ggtgccactc ccactgtcct ttcctaataa aatgaggaaa 2760
   ttgcatcgca ttgtctgagt aggtgtcatt ctattctggg gggtggggtg gggcagcaca 2820
   gcaaggggga ggattgggaa gacaatagca ggcatgctgg ggatgcggtg ggctctatgg 2880
   gtacccaggg ccgcataact tcgtataatg tatgctatac gaagttataa gatctgtact 2940
   gaaatgtgtg ggcgtggctt aagggtggga aagaatatat aaggtggggg tcttatgtag 3000
   ttttgtatct gttttgcagc agccgccgcc gccatgagca ccaactcgtt tgatggaagc 3060
   attgtgagct catatttgac aacgcgcatg cccccatggg ccggggtgcg tcagaatgtg 3120
   atgggctcca gcattgatgg tcgccccgtc ctgcccgcaa actctactac cttgacctac 3180
   gagaccgtgt ctggaacgcc gttggagact gcagcctccg ccgccgcttc agccgctgca 3240
   gccaccgccc gcgggattgt gactgacttt gctttcctga gcccgcttgc aagcagtgca 3300
   gcttcccgtt catccgcccg cgatgacaag ttgacggctc ttttggcaca attggattct 3360
   ttgacccggg aacttaatgt cgtttctcag cagctgttgg atctgcgcca gcaggtttct 3420
   gccctgaagg cttcctcccc tcccaatgcg gtttaaaaca taaataaaaa accagactct 3480
   gtttggattt ggatcaagca agtgtcttgc tgtctttatt taggggtttt gcgcgcgcgg 3540
   taggcccggg accagcggtc tcggtcgttg agggtcctgt gtattttttc caggacgtgg 3600
   taaaggtgac tctggatgtt cagatacatg ggcataagcc cgtctctggg gtggaggtag 3660
   caccactgca gagcttcatg ctgcggggtg gtgttgtaga tgatccagtc gtagcaggag 3720
   cgctgggcgt ggtgcctaaa aatgtctttc agtagcaagc tgattgccag gggcaggccc 3780
   ttggtgtaag tgtttacaaa gcggttaagc tgggatgggt gcatacgtgg ggatatgaga 3840
   tgcatcttgg actgtatttt taggttggct atgttcccag ccatatccct ccggggattc 3900
   atgttgtgca gaaccaccag cacagtgtat ccggtgcact tgggaaattt gtcatgtagc 3960
   ttagaaggaa atgcgtggaa gaacttggag acgcccttgt gacctccaag attttccatg 4020
   cattcgtcca taatgatggc aatgggccca cgggcggcgg cctgggcgaa gatatttctg 4080
   ggatcactaa cgtcatagtt gtgttccagg atgagatcgt cataggccat ttttacaaag 4140
   cgcgggcgga gggtgccaga ctgcggtata atggttccat ccggcccagg ggcgtagtta 4200
   ccctcacaga tttgcatttc ccacgctttg agttcagatg gggggatcat gtctacctgc 4260
   ggggcgatga agaaaacggt ttccggggta ggggagatca gctgggaaga aagcaggttc 4320
   ctgagcagct gcgacttacc gcagccggtg ggcccgtaaa tcacacctat taccggctgc 4380
   aactggtagt taagagagct gcagctgccg tcatccctga gcaggggggc cacttcgtta 4440
   agcatgtccc tgactcgcat gttttccctg accaaatccg ccagaaggcg ctcgccgccc 4500
   agcgatagca gttcttgcaa ggaagcaaag tttttcaacg gtttgagacc gtccgccgta 4560
   ggcatgcttt tgagcgtttg accaagcagt tccaggcggt cccacagctc ggtcacctgc 4620
   tctacggcat ctcgatccag catatctcct cgtttcgcgg gttggggcgg ctttcgctgt 4680
   acggcagtag tcggtgctcg tccagacggg ccagggtcat gtctttccac gggcgcaggg 4740
   tcctcgtcag cgtagtctgg gtcacggtga aggggtgcgc tccgggctgc gcgctggcca 4800
   gggtgcgctt gaggctggtc ctgctggtgc tgaagcgctg ccggtcttcg ccctgcgcgt 4860
   cggccaggta gcatttgacc atggtgtcat agtccagccc ctccgcggcg tggcccttgg 4920
   cgcgcagctt gcccttggag gaggcgccgc acgaggggca gtgcagactt ttgagggcgt 4980
   agagcttggg cgcgagaaat accgattccg gggagtaggc atccgcgccg caggccccgc 5040
   agacggtctc gcattccacg agccaggtga gctctggccg ttcggggtca aaaaccaggt 5100
   ttcccccatg ctttttgatg cgtttcttac ctctggtttc catgagccgg tgtccacgct 5160
   cggtgacgaa aaggctgtcc gtgtccccgt atacagactt gagaggcctg tcctcgagcg 5220
   gtgttccgcg gtcctcctcg tatagaaact cggaccactc tgagacaaag gctcgcgtcc 5280
   aggccagcac gaaggaggct aagtgggagg ggtagcggtc gttgtccact agggggtcca 5340
   ctcgctccag ggtgtgaaga cacatgtcgc cctcttcggc atcaaggaag gtgattggtt 5400
   tgtaggtgta ggccacgtga ccgggtgttc ctgaaggggg gctataaaag ggggtggggg 5460
   cgcgttcgtc ctcactctct tccgcatcgc tgtctgcgag ggccagctgt tggggtgagt 5520
   cgacgcgagg ctggatggcc ttccccatta tgattcttct cgcttccggc ggcatcggga 5580
   tgcccgcgtt gcaggccatg ctgtccaggc aggtagatga cgaccatcag ggacagcttc 5640
   aaggccagca aaaggccagg aaccgtaaaa aggccgcgtt gctggcgttt ttccataggc 5700
   tccgcccccc tgacgagcat cacaaaaatc gacgctcaag tcagaggtgg cgaaacccga 5760
   caggactata aagataccag gcgtttcccc ctggaagctc cctcgtgcgc tctcctgttc 5820
   cgaccctgcc gcttaccgga tacctgtccg cctttctccc ttcgggaagc gtggcgcttt 5880
   ctcatagctc acgctgtagg tatctcagtt cggtgtaggt cgttcgctcc aagctgggct 5940
   gtgtgcacga accccccgtt cagcccgacc gctgcgcctt atccggtaac tatcgtcttg 6000
   agtccaaccc ggtaagacac gacttatcgc cactggcagc agccactggt aacaggatta 6060
   gcagagcgag gtatgtaggc ggtgctacag agttcttgaa gtggtggcct aactacggct 6120
   acactagaag gacagtattt ggtatctgcg ctctgctgaa gccagttacc ttcggaaaaa 6180
   gagttggtag ctcttgatcc ggcaaacaaa ccaccgctgg tagcggtggt ttttttgttt 6240
   gcaagcagca gattacgcgc agaaaaaaag gatctcaaga agatcctttg atcttttcta 6300
   cggggtctga cgctcagtgg aacgaaaact cacgttaagg gattttggtc atgagattat 6360
   caaaaaggat cttcacctag atccttttaa attaaaaatg aagttttaaa tcaatctaaa 6420
   gtatatatga gtaaacttgg tctgacagtt accaatgctt aatcagtgag gcacctatct 6480
   cagcgatctg tctatttcgt tcatccatag ttgcctgact ccccgtcgtg tagataacta 6540
   cgatacggga gggcttacca tctggcccca gtgctgcaat gataccgcga gacccacgct 6600
   caccggctcc agatttatca gcaataaacc agccagccgg aagggccgag cgcagaagtg 6660
   gtcctgcaac tttatccgcc tccatccagt ctattaattg ttgccgggaa gctagagtaa 6720
   gtagttcgcc agttaatagt ttgcgcaacg ttgttgccat tgctgcaggc atcgtggtgt 6780
   cacgctcgtc gtttggtatg gcttcattca gctccggttc ccaacgatca aggcgagtta 6840
   catgatcccc catgttgtgc aaaaaagcgg ttagctcctt cggtcctccg atcgttgtca 6900
   gaagtaagtt ggccgcagtg ttatcactca tggttatggc agcactgcat aattctctta 6960
   ctgtcatgcc atccgtaaga tgcttttctg tgactggtga gtactcaacc aagtcattct 7020
   gagaatagtg tatgcggcga ccgagttgct cttgcccggc gtcaacacgg gataataccg 7080
   cgccacatag cagaacttta aaagtgctca tcattggaaa acgttcttcg gggcgaaaac 7140
   tctcaaggat cttaccgctg ttgagatcca gttcgatgta acccactcgt gcacccaact 7200
   gatcttcagc atcttttact ttcaccagcg tttctgggtg agcaaaaaca ggaaggcaaa 7260
   atgccgcaaa aaagggaata agggcgacac ggaaatgttg aatactcata ctcttccttt 7320
   ttcaatatta ttgaagcatt tatcagggtt attgtctcat gagcggatac atatttgaat 7380
   gtatttagaa aaataaacaa ataggggttc cgcgcacatt tccccgaaaa gtgccacctg 7440
   acgtctaaga aaccattatt atcatgacat taacctataa aaataggcgt atcacgaggc 7500
   cctttcgtct tcaagaattg tt 7522
<210> 37
   <211> 6324
   <212> DNA
   <213> Artificial Sequence
<220>
   <223> CMV/R Ebola GP(Z) delta TM/h
<400> 37
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctcca tcggctcgca tctctccttc acgcgcccgc 1020
   cgccttacct gaggccgcca tccacgccgg ttgagtcgcg ttctgccgcc tcccgcctgt 1080
   ggtgcctcct gaactacgtc cgccgtctag gtaagtttag agctcaggtc gagaccgggc 1140
   ctttgtccgg cgctcccttg gagcctacct agactcagcc ggctctccac gctttgcctg 1200
   accctgcttg ctcaactcta gttaacggtg gagggcagtg tagtctgagc agtactcgtt 1260
   gctgccgcgc gcgccaccag acataatagc tgacagacta acagactgtt cctttccatg 1320
   ggtcttttct gcagtcaccg tcgtcgacga tatcgccgcc atgggcgtga ccggcatcct 1380
   gcagctgccc agggacaggt tcaagaggac cagcttcttc ctgtgggtga tcatcctgtt 1440
   ccagaggacc ttcagcatcc ccctgggcgt gatccacaac agcaccctgc aggtgagcga 1500
   cgtggacaag ctggtgtgca gggacaagct gagcagcacc aaccagctga ggagcgtggg 1560
   cctgaacctg gagggcaacg gcgtggccac cgacgtgccc agcgccacca agaggtgggg 1620
   cttcaggagc ggcgtgcctc ccaaggtggt gaactacgag gccggcgagt gggccgagaa 1680
   ctgctacaac ctggagatca agaagcccga cggcagcgag tgcctgcccg ccgcccctga 1740
   cggcatcagg ggcttcccca ggtgcaggta cgtgcacaag gtgagcggca ccggcccctg 1800
   cgccggcgac ttcgccttcc acaaggaggg cgccttcttc ctgtacgaca ggctggccag 1860
   caccgtgatc tacaggggca ccaccttcgc cgagggcgtg gtggccttcc tgatcctgcc 1920
   ccaggccaag aaggacttct tcagcagcca ccctctgagg gagcccgtga acgccaccga 1980
   ggaccccagc agcggctact acagcaccac catcaggtac caggccaccg gcttcggcac 2040
   caacgagacc gagtacctgt tcgaggtgga caacctgacc tacgtgcagc tggagtctag 2100
   attcacccct cagttcctgc tgcagctgaa cgagaccatc tacaccagcg gcaagaggag 2160
   caacaccacc ggcaagctga tctggaaggt gaaccccgag atcgacacca ccatcggcga 2220
   gtgggccttc tgggagacca agaagaacct gaccaggaag atcaggagcg aggagctgag 2280
   cttcaccgtc gtgagcaacg gggccaagaa catcagcggc cagagccccg ccaggaccag 2340
   cagcgacccc ggcaccaaca ccaccaccga ggaccacaag atcatggcca gcgagaacag 2400
   cagcgccatg gtgcaggtgc acagccaggg cagggaggcc gccgtgagcc acctgaccac 2460
   cctggccacc atcagcacca gccctcagtc tttaaccacc aagcccggcc ccgacaacag 2520
   cacccacaac acccctgtgt acaagctgga catcagcgag gccacccagg tggagcagca 2580
   ccacaggagg accgacaacg acagcaccgc cagcgacacc ccttccgcca ccaccgccgc 2640
   cggccctccg aaggccgaga acaccaacac cagcaagagc accgactttc tggatcccgc 2700
   caccaccacc agccctcaga accacagcga gaccgccggc aacaacaaca cccaccacca 2760
   ggacaccggc gaggagagcg ccagcagcgg caagctgggc ctgatcacca acaccatcgc 2820
   cggcgtggcc ggcctgatca ccggcggcag gaggaccagg agggaggcca tcgtgaacgc 2880
   ccagcccaag tgcaacccca acctgcacta ctggaccacc caggacgagg gcgccgccat 2940
   cggcctggcc tggattccct acttcggccc cgccgccgag ggcatctaca tcgagggcct 3000
   gatgcacaac caggacggcc tgatctgcgg cctgaggcag ctggccaacg agaccaccca 3060
   ggccctgcag ctgttcctga gggccaccac cgagctgagg accttcagca tcctgaacag 3120
   gaaggccatc gacttcctgc tgcagaggtg gggcggcacc tgccacatcc tgggccccga 3180
   ctgctgcatc gagccccacg actggaccaa gaacatcacc gacaagatcg accagatcat 3240
   ccacgacttc gtggacaaga ccctgcccga ccagggcgac aacgacaact ggtggaccgg 3300
   ctgaacacgt ggaattcaga tctgctgtgc cttctagttg ccagccatct gttgtttgcc 3360
   cctcccccgt gccttccttg accctggaag gtgccactcc cactgtcctt tcctaataaa 3420
   atgaggaaat tgcatcgcat tgtctgagta ggtgtcattc tattctgggg ggtggggtgg 3480
   ggcaggacag caagggggag gattgggaag acaatagcag gcatgctggg gatgcggtgg 3540
   gctctatggg tacccaggtg ctgaagaatt gacccggttc ctcctgggcc agaaagaagc 3600
   aggcacatcc ccttctctgt gacacaccct gtccacgccc ctggttctta gttccagccc 3660
   cactcatagg acactcatag ctcaggaggg ctccgccttc aatcccaccc gctaaagtac 3720
   ttggagcggt ctctccctcc ctcatcagcc caccaaacca aacctagcct ccaagagtgg 3780
   gaagaaatta aagcaagata ggctattaag tgcagaggga gagaaaatgc ctccaacatg 3840
   tgaggaagta atgagagaaa tcatagaatt ttaaggccat catggcctta atcttccgct 3900
   tcctcgctca ctgactcgct gcgctcggtc gttcggctgc ggcgagcggt atcagctcac 3960
   tcaaaggcgg taatacggtt atccacagaa tcaggggata acgcaggaaa gaacatgtga 4020
   gcaaaaggcc agcaaaaggc caggaaccgt aaaaaggccg cgttgctggc gtttttccat 4080
   aggctccgcc cccctgacga gcatcacaaa aatcgacgct caagtcagag gtggcgaaac 4140
   ccgacaggac tataaagata ccaggcgttt ccccctggaa gctccctcgt gcgctctcct 4200
   gttccgaccc tgccgcttac cggatacctg tccgcctttc tcccttcggg aagcgtggcg 4260
   ctttctcata gctcacgctg taggtatctc agttcggtgt aggtcgttcg ctccaagctg 4320
   ggctgtgtgc acgaaccccc cgttcagccc gaccgctgcg ccttatccgg taactatcgt 4380
   cttgagtcca acccggtaag acacgactta tcgccactgg cagcagccac tggtaacagg 4440
   attagcagag cgaggtatgt aggcggtgct acagagttct tgaagtggtg gcctaactac 4500
   ggctacacta gaagaacagt atttggtatc tgcgctctgc tgaagccagt taccttcgga 4560
   aaaagagttg gtagctcttg atccggcaaa caaaccaccg ctggtagcgg tggttttttt 4620
   gtttgcaagc agcagattac gcgcagaaaa aaaggatctc aagaagatcc tttgatcttt 4680
   tctacggggt ctgacgctca gtggaacgaa aactcacgtt aagggatttt ggtcatgaga 4740
   ttatcaaaaa ggatcttcac ctagatcctt ttaaattaaa aatgaagttt taaatcaatc 4800
   taaagtatat atgagtaaac ttggtctgac agttaccaat gcttaatcag tgaggcacct 4860
   atctcagcga tctgtctatt tcgttcatcc atagttgcct gactcggggg gggggggcgc 4920
   tgaggtctgc ctcgtgaaga aggtgttgct gactcatacc aggcctgaat cgccccatca 4980
   tccagccaga aagtgaggga gccacggttg atgagagctt tgttgtaggt ggaccagttg 5040
   gtgattttga acttttgctt tgccacggaa cggtctgcgt tgtcgggaag atgcgtgatc 5100
   tgatccttca actcagcaaa agttcgattt attcaacaaa gccgccgtcc cgtcaagtca 5160
   gcgtaatgct ctgccagtgt tacaaccaat taaccaattc tgattagaaa aactcatcga 5220
   gcatcaaatg aaactgcaat ttattcatat caggattatc aataccatat ttttgaaaaa 5280
   gccgtttctg taatgaagga gaaaactcac cgaggcagtt ccataggatg gcaagatcct 5340
   ggtatcggtc tgcgattccg actcgtccaa catcaataca acctattaat ttcccctcgt 5400
   caaaaataag gttatcaagt gagaaatcac catgagtgac gactgaatcc ggtgagaatg 5460
   gcaaaagctt atgcatttct ttccagactt gttcaacagg ccagccatta cgctcgtcat 5520
   caaaatcact cgcatcaacc aaaccgttat tcattcgtga ttgcgcctga gcgagacgaa 5580
   atacgcgatc gctgttaaaa ggacaattac aaacaggaat cgaatgcaac cggcgcagga 5640
   acactgccag cgcatcaaca atattttcac ctgaatcagg atattcttct aatacctgga 5700
   atgctgtttt cccggggatc gcagtggtga gtaaccatgc atcatcagga gtacggataa 5760
   aatgcttgat ggtcggaaga ggcataaatt ccgtcagcca gtttagtctg accatctcat 5820
   ctgtaacatc attggcaacg ctacctttgc catgtttcag aaacaactct ggcgcatcgg 5880
   gcttcccata caatcgatag attgtcgcac ctgattgccc gacattatcg cgagcccatt 5940
   tatacccata taaatcagca tccatgttgg aatttaatcg cggcctcgag caagacgttt 6000
   cccgttgaat atggctcata acaccccttg tattactgtt tatgtaagca gacagtttta 6060
   ttgttcatga tgatatattt ttatcttgtg caatgtaaca tcagagattt tgagacacaa 6120
   cgtggctttc cccccccccc cattattgaa gcatttatca gggttattgt ctcatgagcg 6180
   gatacatatt tgaatgtatt tagaaaaata aacaaatagg ggttccgcgc acatttcccc 6240
   gaaaagtgcc acctgacgtc taagaaacca ttattatcat gacattaacc tataaaaata 6300
   ggcgtatcac gaggcccttt cgtc                                         6324
<210> 38
   <211> 6868
   <212> DNA
   <213> Artificial Sequence
<220>
   <223> pVR1012x/s Ebola GP(Z) delta TM/h (P87666)
<400> 38
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgacg atatcgccgc catggagggc ctgagcctgc tgcagctgcc 1920
   cagggacaag ttcaggaaga gcagcttctt cgtgtgggtg atcatcctgt tccagaaggc 1980
   cttcagcatg cccctgggcg tggtgaccaa cagcaccctg gaggtgaccg agatcgacca 2040
   gctggtgtgc aaggaccacc tggccagcac cgaccagctg aagagcgtgg gcctgaacct 2100
   ggagggcagc ggcgtgagca ccgacatccc cagcgccacc aagaggtggg gcttcaggag 2160
   cggcgtgcct ccccaggtgg tgagctacga ggccggcgag tgggccgaga actgctacaa 2220
   cctggagatc aagaagcccg acggcagcga gtgcctgcct cctcctcctg acggcgtgag 2280
   gggcttcccc aggtgcaggt acgtgcacaa ggcccagggc accggcccct gccccggcga 2340
   ctacgccttc cacaaggacg gcgccttctt cctgtacgac aggctggcca gcaccgtgat 2400
   ctacaggggc gtgaacttcg ccgagggcgt gatcgccttc ctgatcctgg ccaagcccaa 2460
   ggagaccttc ctgcagagcc ctcccatcag ggaggccgcc aactacaccg agaacaccag 2520
   cagctactac gccaccagct atctagagta cgagatcgag aacttcggcg cccagcacag 2580
   caccaccctg ttcaagatca acaacaacac cttcgtgctg ctggacaggc cccacacccc 2640
   tcagttcctg ttccagctga acgacaccat ccagctgcac cagcagctga gcaacaccac 2700
   cggcaagctg atctggaccc tggacgccaa catcaacgcc gacatcggcg agtgggcctt 2760
   ctgggagaac aagaagaacc tgagcgagca gctgaggggc gaggagctga gcttcgagac 2820
   cctgagcctg aacgagaccg aggacgacga cgccaccagc agcaggacca ccaagggcag 2880
   gatcagcgac agggccacca ggaagtacag cgacctggtg cccaaggaca gccccggcat 2940
   ggtgagcctg cacgtgcccg agggcgagac caccctgccc agccagaaca gcaccgaggg 3000
   caggagggtg gacgtgaaca cccaggagac catcaccgag accaccgcca ccatcatcgg 3060
   caccaacggc aacaacatgc agatcagcac catcggcacc ggcctgagca gcagccagat 3120
   cctgagcagc agccccacca tggcccctag ccccgagacc cagaccagca ccacctacac 3180
   ccctaagctg cccgtgatga ccaccgagga gcccaccacc cctcccagga acagccccgg 3240
   atccaccacc gaggccccta ccctgaccac ccctgagaac atcaccaccg ccgtgaagac 3300
   cgtgtgggcc caggagagca ccagcaacgg cctgatcacc agcaccgtga ccggcatcct 3360
   gggcagcctg ggcctgagga agaggagcag gaggcaggtg aacaccaggg ccaccggcaa 3420
   gtgcaacccc aacctgcact actggaccgc ccaggagcag cacaacgccg ccggcatcgc 3480
   ctggattccc tacttcggcc ccggcgccga gggcatctac accgagggcc tgatgcacaa 3540
   ccagaacgcc ctggtgtgcg gcctgaggca gctggccaac gagaccaccc aggccctgca 3600
   gctgttcctg agggccacca ccgagctgag gacctacacc atcctgaaca ggaaggccat 3660
   cgacttcctg ctgaggaggt ggggcggcac ctgcaggatt ctgggccccg actgctgcat 3720
   cgagccccac gactggacca agaacatcac cgacaagatc aaccagatca tccacgactt 3780
   catcgacaac cctctgccca accaggacaa cgacgacaac tggtggaccg gctgaacacg 3840
   tggaattcag atctgctgtg ccttctagtt gccagccatc tgttgtttgc ccctcccccg 3900
   tgccttcctt gaccctggaa ggtgccactc ccactgtcct ttcctaataa aatgaggaaa 3960
   ttgcatcgca ttgtctgagt aggtgtcatt ctattctggg gggtggggtg gggcaggaca 4020
   gcaaggggga ggattgggaa gacaatagca ggcatgctgg ggatgcggtg ggctctatgg 4080
   gtacccaggt gctgaagaat tgacccggtt cctcctgggc cagaaagaag caggcacatc 4140
   cccttctctg tgacacaccc tgtccacgcc cctggttctt agttccagcc ccactcatag 4200
   gacactcata gctcaggagg gctccgcctt caatcccacc cgctaaagta cttggagcgg 4260
   tctctccctc cctcatcagc ccaccaaacc aaacctagcc tccaagagtg ggaagaaatt 4320
   aaagcaagat aggctattaa gtgcagaggg agagaaaatg cctccaacat gtgaggaagt 4380
   aatgagagaa atcatagaat tttaaggcca tgatttaagg ccatcatggc cttaatcttc 4440
   cgcttcctcg ctcactgact cgctgcgctc ggtcgttcgg ctgcggcgag cggtatcagc 4500
   tcactcaaag gcggtaatac ggttatccac agaatcaggg gataacgcag gaaagaacat 4560
   gtgagcaaaa ggccagcaaa aggccaggaa ccgtaaaaag gccgcgttgc tggcgttttt 4620
   ccataggctc cgcccccctg acgagcatca caaaaatcga cgctcaagtc agaggtggcg 4680
   aaacccgaca ggactataaa gataccaggc gtttccccct ggaagctccc tcgtgcgctc 4740
   tcctgttccg accctgccgc ttaccggata cctgtccgcc tttctccctt cgggaagcgt 4800
   ggcgctttct catagctcac gctgtaggta tctcagttcg gtgtaggtcg ttcgctccaa 4860
   gctgggctgt gtgcacgaac cccccgttca gcccgaccgc tgcgccttat ccggtaacta 4920
   tcgtcttgag tccaacccgg taagacacga cttatcgcca ctggcagcag ccactggtaa 4980
   caggattagc agagcgaggt atgtaggcgg tgctacagag ttcttgaagt ggtggcctaa 5040
   ctacggctac actagaagaa cagtatttgg tatctgcgct ctgctgaagc cagttacctt 5100
   cggaaaaaga gttggtagct cttgatccgg caaacaaacc accgctggta gcggtggttt 5160
   ttttgtttgc aagcagcaga ttacgcgcag aaaaaaagga tctcaagaag atcctttgat 5220
   cttttctacg gggtctgacg ctcagtggaa cgaaaactca cgttaaggga ttttggtcat 5280
   gagattatca aaaaggatct tcacctagat ccttttaaat taaaaatgaa gttttaaatc 5340
   aatctaaagt atatatgagt aaacttggtc tgacagttac caatgcttaa tcagtgaggc 5400
   acctatctca gcgatctgtc tatttcgttc atccatagtt gcctgactcg gggggggggg 5460
   gcgctgaggt ctgcctcgtg aagaaggtgt tgctgactca taccaggcct gaatcgcccc 5520
   atcatccagc cagaaagtga gggagccacg gttgatgaga gctttgttgt aggtggacca 5580
   gttggtgatt ttgaactttt gctttgccac ggaacggtct gcgttgtcgg gaagatgcgt 5640
   gatctgatcc ttcaactcag caaaagttcg atttattcaa caaagccgcc gtcccgtcaa 5700
   gtcagcgtaa tgctctgcca gtgttacaac caattaacca attctgatta gaaaaactca 5760
   tcgagcatca aatgaaactg caatttattc atatcaggat tatcaatacc atatttttga 5820
   aaaagccgtt tctgtaatga aggagaaaac tcaccgaggc agttccatag gatggcaaga 5880
   tcctggtatc ggtctgcgat tccgactcgt ccaacatcaa tacaacctat taatttcccc 5940
   tcgtcaaaaa taaggttatc aagtgagaaa tcaccatgag tgacgactga atccggtgag 6000
   aatggcaaaa gcttatgcat ttctttccag acttgttcaa caggccagcc attacgctcg 6060
   tcatcaaaat cactcgcatc aaccaaaccg ttattcattc gtgattgcgc ctgagcgaga 6120
   cgaaatacgc gatcgctgtt aaaaggacaa ttacaaacag gaatcgaatg caaccggcgc 6180
   aggaacactg ccagcgcatc aacaatattt tcacctgaat caggatattc ttctaatacc 6240
   tggaatgctg ttttcccggg gatcgcagtg gtgagtaacc atgcatcatc aggagtacgg 6300
   ataaaatgct tgatggtcgg aagaggcata aattccgtca gccagtttag tctgaccatc 6360
   tcatctgtaa catcattggc aacgctacct ttgccatgtt tcagaaacaa ctctggcgca 6420
   tcgggcttcc catacaatcg atagattgtc gcacctgatt gcccgacatt atcgcgagcc 6480
   catttatacc catataaatc agcatccatg ttggaattta atcgcggcct cgagcaagac 6540
   gtttcccgtt gaatatggct cataacaccc cttgtattac tgtttatgta agcagacagt 6600
   tttattgttc atgatgatat atttttatct tgtgcaatgt aacatcagag attttgagac 6660
   acaacgtggc tttccccccc cccccattat tgaagcattt atcagggtta ttgtctcatg 6720
   agcggataca tatttgaatg tatttagaaa aataaacaaa taggggttcc gcgcacattt 6780
   ccccgaaaag tgccacctga cgtctaagaa accattatta tcatgacatt aacctataaa 6840
   aataggcgta tcacgaggcc ctttcgtc 6868
<210> 39
   <211> 6322
   <212> DNA
   <213> Artificial Sequence
<220>
   <223> CMV/R-GP(S/G)(deltaTM)/h
<400> 39
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctcca tcggctcgca tctctccttc acgcgcccgc 1020
   cgccttacct gaggccgcca tccacgccgg ttgagtcgcg ttctgccgcc tcccgcctgt 1080
   ggtgcctcct gaactacgtc cgccgtctag gtaagtttag agctcaggtc gagaccgggc 1140
   ctttgtccgg cgctcccttg gagcctacct agactcagcc ggctctccac gctttgcctg 1200
   accctgcttg ctcaactcta gttaacggtg gagggcagtg tagtctgagc agtactcgtt 1260
   gctgccgcgc gcgccaccag acataatagc tgacagacta acagactgtt cctttccatg 1320
   ggtcttttct gcagtcaccg tcgtcgacga tatcgccgcc atggagggcc tgagcctgct 1380
   gcagctgccc agggacaagt tcaggaagag cagcttcttc gtgtgggtga tcatcctgtt 1440
   ccagaaggcc ttcagcatgc ccctgggcgt ggtgaccaac agcaccctgg aggtgaccga 1500
   gatcgaccag ctggtgtgca aggaccacct ggccagcacc gaccagctga agagcgtggg 1560
   cctgaacctg gagggcagcg gcgtgagcac cgacatcccc agcgccacca agaggtgggg 1620
   cttcaggagc ggcgtgcctc ccaaggtggt gagctacgag gccggcgagt gggccgagaa 1680
   ctgctacaac ctggagatca agaagcccga cggcagcgag tgcctgcctc ctcctcctga 1740
   cggcgtgagg ggcttcccca ggtgcaggta cgtgcacaag gcccagggca ccggcccctg 1800
   ccccggcgac tacgccttcc acaaggacgg cgccttcttc ctgtacgaca ggctggccag 1860
   caccgtgatc tacaggggcg tgaacttcgc cgagggcgtg atcgccttcc tgatcctggc 1920
   caagcccaag gagaccttcc tgcagagccc tcccatcagg gaggccgtga actacaccga 1980
   gaacaccagc agctactacg ccaccagcta tctagagtac gagatcgaga acttcggcgc 2040
   ccagcacagc accaccctgt tcaagatcga caacaacacc ttcgtgaggc tggacaggcc 2100
   ccacacccct cagttcctgt tccagctgaa cgacaccatc cacctgcacc agcagctgag 2160
   caacaccacc ggcaggctga tctggaccct ggacgccaac atcaacgccg acatcggcga 2220
   gtgggccttc tgggagaaca agaagaacct gagcgagcag ctgaggggcg aggagctgag 2280
   cttcgaggcc ctgagcctga acgagaccga ggacgacgac gccgccagca gcaggatcac 2340
   caagggcagg atcagcgaca gggccaccag gaagtacagc gacctggtgc ccaagaacag 2400
   ccccggcatg gtgcccctgc acatccccga gggcgagacc accctgccca gccagaacag 2460
   caccgagggc aggagggtgg gcgtgaacac ccaggagacc atcaccgaga ccgccgccac 2520
   catcatcggc accaacggca accacatgca gatcagcacc atcggcatca ggcccagcag 2580
   cagccagatc cccagcagca gccccaccac cgcccctagc cccgaggccc agacccccac 2640
   cacccacacc agcggaccca gcgtgatggc caccgaggag cccaccaccc ctcccggcag 2700
   cagccccgga cccaccaccg aggcccctac cctgaccacc cctgagaaca tcaccaccgc 2760
   cgtgaagacc gtgctgcccc aggagagcac cagcaacggc ctgatcacca gcaccgtgac 2820
   cggcatcctg ggcagcctgg gcctgaggaa gaggagcagg aggcagacca acaccaaggc 2880
   caccggcaag tgcaacccca acctgcacta ctggaccgcc caggagcagc acaacgccgc 2940
   cggcatcgcc tggattccct acttcggccc cggcgccgag ggcatctaca ccgagggcct 3000
   gatgcacaac cagaacgccc tggtgtgcgg cctgaggcag ctggccaacg agaccaccca 3060
   ggccctgcag ctgttcctga gggccaccac cgagctgagg acctacacca tcctgaacag 3120
   gaaggccatc gacttcctgc tgaggaggtg gggcggcacc tgcaggattc tgggccccga 3180
   ctgctgcatc gagccccacg actggaccaa gaacatcacc gacaagatca accagatcat 3240
   ccacgacttc atcgacaacc ctctgcccaa ccaggacaac gacgacaact ggtggaccgg 3300
   ctgaacacgt ggaattgatc tgctgtgcct tctagttgcc agccatctgt tgtttgcccc 3360
   tcccccgtgc cttccttgac cctggaaggt gccactccca ctgtcctttc ctaataaaat 3420
   gaggaaattg catcgcattg tctgagtagg tgtcattcta ttctgggggg tggggtgggg 3480
   caggacagca agggggagga ttgggaagac aatagcaggc atgctgggga tgcggtgggc 3540
   tctatgggta cccaggtgct gaagaattga cccggttcct cctgggccag aaagaagcag 3600
   gcacatcccc ttctctgtga cacaccctgt ccacgcccct ggttcttagt tccagcccca 3660
   ctcataggac actcatagct caggagggct ccgccttcaa tcccacccgc taaagtactt 3720
   ggagcggtct ctccctccct catcagccca ccaaaccaaa cctagcctcc aagagtggga 3780
   agaaattaaa gcaagatagg ctattaagtg cagagggaga gaaaatgcct ccaacatgtg 3840
   aggaagtaat gagagaaatc atagaatttt aaggccatca tggccttaat cttccgcttc 3900
   ctcgctcact gactcgctgc gctcggtcgt tcggctgcgg cgagcggtat cagctcactc 3960
   aaaggcggta atacggttat ccacagaatc aggggataac gcaggaaaga acatgtgagc 4020
   aaaaggccag caaaaggcca ggaaccgtaa aaaggccgcg ttgctggcgt ttttccatag 4080
   gctccgcccc cctgacgagc atcacaaaaa tcgacgctca agtcagaggt ggcgaaaccc 4140
   gacaggacta taaagatacc aggcgtttcc ccctggaagc tccctcgtgc gctctcctgt 4200
   tccgaccctg ccgcttaccg gatacctgtc cgcctttctc ccttcgggaa gcgtggcgct 4260
   ttctcatagc tcacgctgta ggtatctcag ttcggtgtag gtcgttcgct ccaagctggg 4320
   ctgtgtgcac gaaccccccg ttcagcccga ccgctgcgcc ttatccggta actatcgtct 4380
   tgagtccaac ccggtaagac acgacttatc gccactggca gcagccactg gtaacaggat 4440
   tagcagagcg aggtatgtag gcggtgctac agagttcttg aagtggtggc ctaactacgg 4500
   ctacactaga agaacagtat ttggtatctg cgctctgctg aagccagtta ccttcggaaa 4560
   aagagttggt agctcttgat ccggcaaaca aaccaccgct ggtagcggtg gtttttttgt 4620
   ttgcaagcag cagattacgc gcagaaaaaa aggatctcaa gaagatcctt tgatcttttc 4680
   tacggggtct gacgctcagt ggaacgaaaa ctcacgttaa gggattttgg tcatgagatt 4740
   atcaaaaagg atcttcacct agatcctttt aaattaaaaa tgaagtttta aatcaatcta 4800
   aagtatatat gagtaaactt ggtctgacag ttaccaatgc ttaatcagtg aggcacctat 4860
   ctcagcgatc tgtctatttc gttcatccat agttgcctga ctcggggggg gggggcgctg 4920
   aggtctgcct cgtgaagaag gtgttgctga ctcataccag gcctgaatcg ccccatcatc 4980
   cagccagaaa gtgagggagc cacggttgat gagagctttg ttgtaggtgg accagttggt 5040
   gattttgaac ttttgctttg ccacggaacg gtctgcgttg tcgggaagat gcgtgatctg 5100
   atccttcaac tcagcaaaag ttcgatttat tcaacaaagc cgccgtcccg tcaagtcagc 5160
   gtaatgctct gccagtgtta caaccaatta accaattctg attagaaaaa ctcatcgagc 5220
   atcaaatgaa actgcaattt attcatatca ggattatcaa taccatattt ttgaaaaagc 5280
   cgtttctgta atgaaggaga aaactcaccg aggcagttcc ataggatggc aagatcctgg 5340
   tatcggtctg cgattccgac tcgtccaaca tcaatacaac ctattaattt cccctcgtca 5400
   aaaataaggt tatcaagtga gaaatcacca tgagtgacga ctgaatccgg tgagaatggc 5460
   aaaagcttat gcatttcttt ccagacttgt tcaacaggcc agccattacg ctcgtcatca 5520
   aaatcactcg catcaaccaa accgttattc attcgtgatt gcgcctgagc gagacgaaat 5580
   acgcgatcgc tgttaaaagg acaattacaa acaggaatcg aatgcaaccg gcgcaggaac 5640
   actgccagcg catcaacaat attttcacct gaatcaggat attcttctaa tacctggaat 5700
   gctgttttcc cggggatcgc agtggtgagt aaccatgcat catcaggagt acggataaaa 5760
   tgcttgatgg tcggaagagg cataaattcc gtcagccagt ttagtctgac catctcatct 5820
   gtaacatcat tggcaacgct acctttgcca tgtttcagaa acaactctgg cgcatcgggc 5880
   ttcccataca atcgatagat tgtcgcacct gattgcccga cattatcgcg agcccattta 5940
   tacccatata aatcagcatc catgttggaa tttaatcgcg gcctcgagca agacgtttcc 6000
   cgttgaatat ggctcataac accccttgta ttactgttta tgtaagcaga cagttttatt 6060
   gttcatgatg atatattttt atcttgtgca atgtaacatc agagattttg agacacaacg 6120
   tggctttccc ccccccccca ttattgaagc atttatcagg gttattgtct catgagcgga 6180
   tacatatttg aatgtattta gaaaaataaa caaatagggg ttccgcgcac atttccccga 6240
   aaagtgccac ctgacgtcta agaaaccatt attatcatga cattaaccta taaaaatagg 6300
   cgtatcacga ggccctttcg tc                                          6322
<210> 40
   <211> 6324
   <212> DNA
   <213> Artificial Sequence
<220>
   <223> CMV/R-GP(S, Q66798)(dTM)/h
<400> 40
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctcca tcggctcgca tctctccttc acgcgcccgc 1020
   cgccttacct gaggccgcca tccacgccgg ttgagtcgcg ttctgccgcc tcccgcctgt 1080
   ggtgcctcct gaactacgtc cgccgtctag gtaagtttag agctcaggtc gagaccgggc 1140
   ctttgtccgg cgctcccttg gagcctacct agactcagcc ggctctccac gctttgcctg 1200
   accctgcttg ctcaactcta gttaacggtg gagggcagtg tagtctgagc agtactcgtt 1260
   gctgccgcgc gcgccaccag acataatagc tgacagacta acagactgtt cctttccatg 1320
   ggtcttttct gcagtcaccg tcgtcgacga tatcgccgcc atggagggcc tgagcctgct 1380
   gcagctgccc agggacaagt tcaggaagag cagcttcttc gtgtgggtga tcatcctgtt 1440
   ccagaaggcc ttcagcatgc ccctgggcgt ggtgaccaac agcaccctgg aggtgaccga 1500
   gatcgaccag ctggtgtgca aggaccacct ggccagcacc gaccagctga agagcgtggg 1560
   cctgaacctg gagggcagcg gcgtgagcac cgacatcccc agcgccacca agaggtgggg 1620
   cttcaggagc ggcgtgcctc cccaggtggt gagctacgag gccggcgagt gggccgagaa 1680
   ctgctacaac ctggagatca agaagcccga cggcagcgag tgcctgcctc ctcctcctga 1740
   cggcgtgagg ggcttcccca ggtgcaggta cgtgcacaag gcccagggca ccggcccctg 1800
   ccccggcgac tacgccttcc acaaggacgg cgccttcttc ctgtacgaca ggctggccag 1860
   caccgtgatc tacaggggcg tgaacttcgc cgagggcgtg atcgccttcc tgatcctggc 1920
   caagcccaag gagaccttcc tgcagagccc tcccatcagg gaggccgcca actacaccga 1980
   gaacaccagc agctactacg ccaccagcta tctagagtac gagatcgaga acttcggcgc 2040
   ccagcacagc accaccctgt tcaagatcaa caacaacacc ttcgtgctgc tggacaggcc 2100
   ccacacccct cagttcctgt tccagctgaa cgacaccatc cagctgcacc agcagctgag 2160
   caacaccacc ggcaagctga tctggaccct ggacgccaac atcaacgccg acatcggcga 2220
   gtgggccttc tgggagaaca agaagaacct gagcgagcag ctgaggggcg aggagctgag 2280
   cttcgagacc ctgagcctga acgagaccga ggacgacgac gccaccagca gcaggaccac 2340
   caagggcagg atcagcgaca gggccaccag gaagtacagc gacctggtgc ccaaggacag 2400
   ccccggcatg gtgagcctgc acgtgcccga gggcgagacc accctgccca gccagaacag 2460
   caccgagggc aggagggtgg acgtgaacac ccaggagacc atcaccgaga ccaccgccac 2520
   catcatcggc accaacggca acaacatgca gatcagcacc atcggcaccg gcctgagcag 2580
   cagccagatc ctgagcagca gccccaccat ggcccctagc cccgagaccc agaccagcac 2640
   cacctacacc cctaagctgc ccgtgatgac caccgaggag cccaccaccc ctcccaggaa 2700
   cagccccgga tccaccaccg aggcccctac cctgaccacc cctgagaaca tcaccaccgc 2760
   cgtgaagacc gtgtgggccc aggagagcac cagcaacggc ctgatcacca gcaccgtgac 2820
   cggcatcctg ggcagcctgg gcctgaggaa gaggagcagg aggcaggtga acaccagggc 2880
   caccggcaag tgcaacccca acctgcacta ctggaccgcc caggagcagc acaacgccgc 2940
   cggcatcgcc tggattccct acttcggccc cggcgccgag ggcatctaca ccgagggcct 3000
   gatgcacaac cagaacgccc tggtgtgcgg cctgaggcag ctggccaacg agaccaccca 3060
   ggccctgcag ctgttcctga gggccaccac cgagctgagg acctacacca tcctgaacag 3120
   gaaggccatc gacttcctgc tgaggaggtg gggcggcacc tgcaggattc tgggccccga 3180
   ctgctgcatc gagccccacg actggaccaa gaacatcacc gacaagatca accagatcat 3240
   ccacgacttc atcgacaacc ctctgcccaa ccaggacaac gacgacaact ggtggaccgg 3300
   ctgaacacgt ggaattcaga tctgctgtgc cttctagttg ccagccatct gttgtttgcc 3360
   cctcccccgt gccttccttg accctggaag gtgccactcc cactgtcctt tcctaataaa 3420
   atgaggaaat tgcatcgcat tgtctgagta ggtgtcattc tattctgggg ggtggggtgg 3480
   ggcaggacag caagggggag gattgggaag acaatagcag gcatgctggg gatgcggtgg 3540
   gctctatggg tacccaggtg ctgaagaatt gacccggttc ctcctgggcc agaaagaagc 3600
   aggcacatcc ccttctctgt gacacaccct gtccacgccc ctggttctta gttccagccc 3660
   cactcatagg acactcatag ctcaggaggg ctccgccttc aatcccaccc gctaaagtac 3720
   ttggagcggt ctctccctcc ctcatcagcc caccaaacca aacctagcct ccaagagtgg 3780
   gaagaaatta aagcaagata ggctattaag tgcagaggga gagaaaatgc ctccaacatg 3840
   tgaggaagta atgagagaaa tcatagaatt ttaaggccat catggcctta atcttccgct 3900
   tcctcgctca ctgactcgct gcgctcggtc gttcggctgc ggcgagcggt atcagctcac 3960
   tcaaaggcgg taatacggtt atccacagaa tcaggggata acgcaggaaa gaacatgtga 4020
   gcaaaaggcc agcaaaaggc caggaaccgt aaaaaggccg cgttgctggc gtttttccat 4080
   aggctccgcc cccctgacga gcatcacaaa aatcgacgct caagtcagag gtggcgaaac 4140
   ccgacaggac tataaagata ccaggcgttt ccccctggaa gctccctcgt gcgctctcct 4200
   gttccgaccc tgccgcttac cggatacctg tccgcctttc tcccttcggg aagcgtggcg 4260
   ctttctcata gctcacgctg taggtatctc agttcggtgt aggtcgttcg ctccaagctg 4320
   ggctgtgtgc acgaaccccc cgttcagccc gaccgctgcg ccttatccgg taactatcgt 4380
   cttgagtcca acccggtaag acacgactta tcgccactgg cagcagccac tggtaacagg 4440
   attagcagag cgaggtatgt aggcggtgct acagagttct tgaagtggtg gcctaactac 4500
   ggctacacta gaagaacagt atttggtatc tgcgctctgc tgaagccagt taccttcgga 4560
   aaaagagttg gtagctcttg atccggcaaa caaaccaccg ctggtagcgg tggttttttt 4620
   gtttgcaagc agcagattac gcgcagaaaa aaaggatctc aagaagatcc tttgatcttt 4680
   tctacggggt ctgacgctca gtggaacgaa aactcacgtt aagggatttt ggtcatgaga 4740
   ttatcaaaaa ggatcttcac ctagatcctt ttaaattaaa aatgaagttt taaatcaatc 4800
   taaagtatat atgagtaaac ttggtctgac agttaccaat gcttaatcag tgaggcacct 4860
   atctcagcga tctgtctatt tcgttcatcc atagttgcct gactcggggg gggggggcgc 4920
   tgaggtctgc ctcgtgaaga aggtgttgct gactcatacc aggcctgaat cgccccatca 4980
   tccagccaga aagtgaggga gccacggttg atgagagctt tgttgtaggt ggaccagttg 5040
   gtgattttga acttttgctt tgccacggaa cggtctgcgt tgtcgggaag atgcgtgatc 5100
   tgatccttca actcagcaaa agttcgattt attcaacaaa gccgccgtcc cgtcaagtca 5160
   gcgtaatgct ctgccagtgt tacaaccaat taaccaattc tgattagaaa aactcatcga 5220
   gcatcaaatg aaactgcaat ttattcatat caggattatc aataccatat ttttgaaaaa 5280
   gccgtttctg taatgaagga gaaaactcac cgaggcagtt ccataggatg gcaagatcct 5340
   ggtatcggtc tgcgattccg actcgtccaa catcaataca acctattaat ttcccctcgt 5400
   caaaaataag gttatcaagt gagaaatcac catgagtgac gactgaatcc ggtgagaatg 5460
   gcaaaagctt atgcatttct ttccagactt gttcaacagg ccagccatta cgctcgtcat 5520
   caaaatcact cgcatcaacc aaaccgttat tcattcgtga ttgcgcctga gcgagacgaa 5580
   atacgcgatc gctgttaaaa ggacaattac aaacaggaat cgaatgcaac cggcgcagga 5640
   acactgccag cgcatcaaca atattttcac ctgaatcagg atattcttct aatacctgga 5700
   atgctgtttt cccggggatc gcagtggtga gtaaccatgc atcatcagga gtacggataa 5760
   aatgcttgat ggtcggaaga ggcataaatt ccgtcagcca gtttagtctg accatctcat 5820
   ctgtaacatc attggcaacg ctacctttgc catgtttcag aaacaactct ggcgcatcgg 5880
   gcttcccata caatcgatag attgtcgcac ctgattgccc gacattatcg cgagcccatt 5940
   tatacccata taaatcagca tccatgttgg aatttaatcg cggcctcgag caagacgttt 6000
   cccgttgaat atggctcata acaccccttg tattactgtt tatgtaagca gacagtttta 6060
   ttgttcatga tgatatattt ttatcttgtg caatgtaaca tcagagattt tgagacacaa 6120
   cgtggctttc cccccccccc cattattgaa gcatttatca gggttattgt ctcatgagcg 6180
   gatacatatt tgaatgtatt tagaaaaata aacaaatagg ggttccgcgc acatttcccc 6240
   gaaaagtgcc acctgacgtc taagaaacca ttattatcat gacattaacc tataaaaata 6300
   ggcgtatcac gaggcccttt cgtc                                        6324
<210> 41
   <211> 6236
   <212> DNA
   <213> Artificial Sequence
<220>
   <223> pVR1012x/s Lassa (codon optimized)
<400> 41
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga gatatcgccg 1920
   ccatgggcca gatcgtgacc ttcttccagg aggtgcccca tgtgatcgag gaggtgatga 1980
   acatcgtgct gatcgccctg agcgtgctgg ccgtgctgaa gggcctgtac aacttcgcca 2040
   cctgcggcct ggtgggcctg gtgaccttcc tgctgctgtg cggcaggagc tgcaccacca 2100
   gcctgtacaa gggcgtgtac gagctgcaga ccctggagct gaacatggag accctgaaca 2160
   tgaccatgcc cctgagctgc accaagaaca acagccacca ctacatcatg gtgggcaacg 2220
   agaccggcct ggagctaacc ctgaccaaca ccagcatcat caaccacaag ttctgcaacc 2280
   tgagcgacgc ccacaagaag aacctgtacg accacgccct gatgagcatc atcagcacct 2340
   tccacctgag catccccaac ttcaaccagt acgaggccat gagctgcgac ttcaacggcg 2400
   gcaagatcag cgtgcagtac aacctgagcc acagctacgc cggcgacgcc gccaaccact 2460
   gcggcaccgt ggccaacggc gtgctgcaga ccttcatgag gatggcctgg ggcggcagct 2520
   acatcgccct ggacagcggc aggggcaact gggactgcat catgaccagc taccagtacc 2580
   tgatcatcca gaacaccacc tgggaggacc actgccagtt cagcaggccc agccccatcg 2640
   gctacctggg cctgctgagc cagaggacca gggacatcta catcagcagg aggctgctgg 2700
   gcaccttcac ctggaccctg agcgacagcg agggcaagga cacacccggc ggctactgcc 2760
   tgaccaggtg gatgctgatc gaggccgagc tgaagtgctt cggcaacacc gccgtggcca 2820
   agtgcaacga gaagcacgac gaggagttct gcgacatgct gaggctgttc gacttcaaca 2880
   agcaggccat ccagaggctg aaggccgagg cccagatgag catccagctg atcaacaagg 2940
   ccgtgaacgc cctgatcaac gaccagctga tcatgaagaa ccacctgagg gacatcatgg 3000
   gcatccccta ctgcaactac agcaagtact ggtacctgaa ccacaccacc accggcagga 3060
   ccagcctgcc caagtgctgg ctggtgagca acggcagcta cctgaacgag acccacttca 3120
   gcgacgacat cgagcagcag gccgacaaca tgatcaccga gatgctgcag aaggagtaca 3180
   tggagaggca gggcaagacc tgaacacgtg ggatccagat ctgctgtgcc ttctagttgc 3240
   cagccatctg ttgtttgccc ctcccccgtg ccttccttga ccctggaagg tgccactccc 3300
   actgtccttt cctaataaaa tgaggaaatt gcatcgcatt gtctgagtag gtgtcattct 3360
   attctggggg gtggggtggg gcaggacagc aagggggagg attgggaaga caatagcagg 3420
   catgctgggg atgcggtggg ctctatgggt acccaggtgc tgaagaattg acccggttcc 3480
   tcctgggcca gaaagaagca ggcacatccc cttctctgtg acacaccctg tccacgcccc 3540
   tggttcttag ttccagcccc actcatagga cactcatagc tcaggagggc tccgccttca 3600
   atcccacccg ctaaagtact tggagcggtc tctccctccc tcatcagccc accaaaccaa 3660
   acctagcctc caagagtggg aagaaattaa agcaagatag gctattaagt gcagagggag 3720
   agaaaatgcc tccaacatgt gaggaagtaa tgagagaaat catagaattt taaggccatg 3780
   atttaaggcc atcatggcct taatcttccg cttcctcgct cactgactcg ctgcgctcgg 3840
   tcgttcggct gcggcgagcg gtatcagctc actcaaaggc ggtaatacgg ttatccacag 3900
   aatcagggga taacgcagga aagaacatgt gagcaaaagg ccagcaaaag gccaggaacc 3960
   gtaaaaaggc cgcgttgctg gcgtttttcc ataggctccg cccccctgac gagcatcaca 4020
   aaaatcgacg ctcaagtcag aggtggcgaa acccgacagg actataaaga taccaggcgt 4080
   ttccccctgg aagctccctc gtgcgctctc ctgttccgac cctgccgctt accggatacc 4140
   tgtccgcctt tctcccttcg ggaagcgtgg cgctttctca tagctcacgc tgtaggtatc 4200
   tcagttcggt gtaggtcgtt cgctccaagc tgggctgtgt gcacgaaccc cccgttcagc 4260
   ccgaccgctg cgccttatcc ggtaactatc gtcttgagtc caacccggta agacacgact 4320
   tatcgccact ggcagcagcc actggtaaca ggattagcag agcgaggtat gtaggcggtg 4380
   ctacagagtt cttgaagtgg tggcctaact acggctacac tagaagaaca gtatttggta 4440
   tctgcgctct gctgaagcca gttaccttcg gaaaaagagt tggtagctct tgatccggca 4500
   aacaaaccac cgctggtagc ggtggttttt ttgtttgcaa gcagcagatt acgcgcagaa 4560
   aaaaaggatc tcaagaagat cctttgatct tttctacggg gtctgacgct cagtggaacg 4620
   aaaactcacg ttaagggatt ttggtcatga gattatcaaa aaggatcttc acctagatcc 4680
   ttttaaatta aaaatgaagt tttaaatcaa tctaaagtat atatgagtaa acttggtctg 4740
   acagttacca atgcttaatc agtgaggcac ctatctcagc gatctgtcta tttcgttcat 4800
   ccatagttgc ctgactcggg gggggggggc gctgaggtct gcctcgtgaa gaaggtgttg 4860
   ctgactcata ccaggcctga atcgccccat catccagcca gaaagtgagg gagccacggt 4920
   tgatgagagc tttgttgtag gtggaccagt tggtgatttt gaacttttgc tttgccacgg 4980
   aacggtctgc gttgtcggga agatgcgtga tctgatcctt caactcagca aaagttcgat 5040
   ttattcaaca aagccgccgt cccgtcaagt cagcgtaatg ctctgccagt gttacaacca 5100
   attaaccaat tctgattaga aaaactcatc gagcatcaaa tgaaactgca atttattcat 5160
   atcaggatta tcaataccat atttttgaaa aagccgtttc tgtaatgaag gagaaaactc 5220
   accgaggcag ttccatagga tggcaagatc ctggtatcgg tctgcgattc cgactcgtcc 5280
   aacatcaata caacctatta atttcccctc gtcaaaaata aggttatcaa gtgagaaatc 5340
   accatgagtg acgactgaat ccggtgagaa tggcaaaagc ttatgcattt ctttccagac 5400
   ttgttcaaca ggccagccat tacgctcgtc atcaaaatca ctcgcatcaa ccaaaccgtt 5460
   attcattcgt gattgcgcct gagcgagacg aaatacgcga tcgctgttaa aaggacaatt 5520
   acaaacagga atcgaatgca accggcgcag gaacactgcc agcgcatcaa caatattttc 5580
   acctgaatca ggatattctt ctaatacctg gaatgctgtt ttcccgggga tcgcagtggt 5640
   gagtaaccat gcatcatcag gagtacggat aaaatgcttg atggtcggaa gaggcataaa 5700
   ttccgtcagc cagtttagtc tgaccatctc atctgtaaca tcattggcaa cgctaccttt 5760
   gccatgtttc agaaacaact ctggcgcatc gggcttccca tacaatcgat agattgtcgc 5820
   acctgattgc ccgacattat cgcgagccca tttataccca tataaatcag catccatgtt 5880
   ggaatttaat cgcggcctcg agcaagacgt ttcccgttga atatggctca taacacccct 5940
   tgtattactg tttatgtaag cagacagttt tattgttcat gatgatatat ttttatcttg 6000
   tgcaatgtaa catcagagat tttgagacac aacgtggctt tccccccccc cccattattg 6060
   aagcatttat cagggttatt gtctcatgag cggatacata tttgaatgta tttagaaaaa 6120
   taaacaaata ggggttccgc gcacatttcc ccgaaaagtg ccacctgacg tctaagaaac 6180
   cattattatc atgacattaa cctataaaaa taggcgtatc acgaggccct ttcgtc     6236
<210> 42
   <211> 6902
   <212> DNA
   <213> Artificial Sequence
<220>
   <223> pVR1012x/s Marburg (codon optimized)
<400> 42
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctccg cggccgggaa cggtgcattg gaacgcggat 1020
   tccccgtgcc aagagtgacg taagtaccgc ctatagactc tataggcaca cccctttggc 1080
   tcttatgcat gctatactgt ttttggcttg gggcctatac acccccgctt ccttatgcta 1140
   taggtgatgg tatagcttag cctataggtg tgggttattg accattattg accactcccc 1200
   tattggtgac gatactttcc attactaatc cataacatgg ctctttgcca caactatctc 1260
   tattggctat atgccaatac tctgtccttc agagactgac acggactctg tatttttaca 1320
   ggatggggtc ccatttatta tttacaaatt cacatataca acaacgccgt cccccgtgcc 1380
   cgcagttttt attaaacata gcgtgggatc tccacgcgaa tctcgggtac gtgttccgga 1440
   catgggctct tctccggtag cggcggagct tccacatccg agccctggtc ccatgcctcc 1500
   agcggctcat ggtcgctcgg cagctccttg ctcctaacag tggaggccag acttaggcac 1560
   agcacaatgc ccaccaccac cagtgtgccg cacaaggccg tggcggtagg gtatgtgtct 1620
   gaaaatgagc gtggagattg ggctcgcacg gctgacgcag atggaagact taaggcagcg 1680
   gcagaagaag atgcaggcag ctgagttgtt gtattctgat aagagtcaga ggtaactccc 1740
   gttgcggtgc tgttaacggt ggagggcagt gtagtctgag cagtactcgt tgctgccgcg 1800
   cgcgccacca gacataatag ctgacagact aacagactgt tcctttccat gggtcttttc 1860
   tgcagtcacc gtcgtcgaca cgtgtgatca gatatcgcgg ccgctctaga gatatcgccg 1920
   ccatgaagac cacctgcctg ttcatcagcc tgatcctgat ccagggcatc aagaccctgc 1980
   ccatcctgga gatcgccagc aacaaccagc cccagaacgt ggacagcgtg tgcagcggca 2040
   ccctgcagaa gaccgaggac gtgcacctga tgggcttcac cctgagcggc cagaaggtgg 2100
   ccgacagccc tctggaggcc agcaagaggt gggccttcag gaccggcgtg ccccccaaga 2160
   acgtggagta caccgagggc gaggaggcca agacctgcta caacatcagc gtgaccgacc 2220
   ccagcggcaa gagcctgctg ctggaccctc ccaccaacat cagggactac cctaagtgca 2280
   agaccatcca ccacatccag ggccagaacc ctcacgccca gggcatcgcc ctgcacctgt 2340
   ggggcgcctt cttcctgtac gacaggatcg ccagcaccac catgtacagg ggcagggtgt 2400
   tcaccgaggg caacatcgcc gccatgatcg ttaacaagac cgtgcacaag atgatcttca 2460
   gcaggcaggg ccagggctac aggcacatga acctgaccag caccaacaag tactggacca 2520
   gcaacaacgg cacccagacc aacgacaccg gctgcttcgg cgccctgcag gagtacaaca 2580
   gcaccaagaa ccagacctgc gcccccagca agatccccag ccccctgccc accgccaggc 2640
   ccgagatcaa gcccaccagc acccccaccg acgccaccac cctgaacacc accgacccca 2700
   acaacgacga cgaggacctg atcaccagcg gcagcggcag cggcgagcag gagccctaca 2760
   ccaccagcga cgccgtgacc aagcagggcc tgagcagcac catgcctcct acccctagcc 2820
   ctcagcccag cacccctcag caggagggca acaacaccga ccacagccag ggcaccgtga 2880
   ccgagcccaa caagaccaac accaccgccc agcccagcat gcctcctcac aacaccaccg 2940
   ccatcagcac caacaacacc agcaagaaca acttcagcac cctgagcgtg agcctgcaga 3000
   acaccaccaa ctacgacacc cagagcaccg ccaccgagaa cgagcagacc agcgccccta 3060
   gcaagaccac cctgcctccc accggcaacc tgaccaccgc caagagcacc aacaacacca 3120
   agggccccac caccaccgcc cctaacatga ccaacggcca cctgaccagc cccagcccca 3180
   cccccaaccc caccacccag cacctggtgt acttcaggaa gaagaggagc atcctgtgga 3240
   gggagggcga tatgttcccc ttcctggacg gcctgatcaa cgcccctatc gacttcgacc 3300
   ccgtgcccaa caccaagacc atcttcgacg agagcagcag cagcggcgcc agcgccgagg 3360
   aggaccagca cgccagcccc aacatcagcc tgaccctgag ctacttcccc aacatcaacg 3420
   agaacaccgc ctacagcggc gagaacgaga acgactgcga cgccgagctg aggatctgga 3480
   gcgtgcagga ggacgacctg gccgccggcc tgagctggat tcccttcttc ggccccggca 3540
   tcgagggcct gtacaccgcc ggcctgatca agaaccagaa caacctggtg tgcaggctga 3600
   ggaggctggc caaccagacc gccaagagcc tggagctgct gctgagggtg accaccgagg 3660
   agaggacctt cagcctgatc aacaggcacg ccatcgactt cctgctgacc aggtggggcg 3720
   gcacctgcaa ggtgctgggc cccgactgct gcatcggcat cgaggacctg agcaggaaca 3780
   tcagcgagca gatcgaccag atcaagaagg acgagcagaa ggagggcacc ggctggggcc 3840
   tgggcggcaa gtggtggacc agcgactgaa cacgtgggat ccagatctgc tgtgccttct 3900
   agttgccagc catctgttgt ttgcccctcc cccgtgcctt ccttgaccct ggaaggtgcc 3960
   actcccactg tcctttccta ataaaatgag gaaattgcat cgcattgtct gagtaggtgt 4020
   cattctattc tggggggtgg ggtggggcag gacagcaagg gggaggattg ggaagacaat 4080
   agcaggcatg ctggggatgc ggtgggctct atgggtaccc aggtgctgaa gaattgaccc 4140
   ggttcctcct gggccagaaa gaagcaggca catccccttc tctgtgacac accctgtcca 4200
   cgcccctggt tcttagttcc agccccactc ataggacact catagctcag gagggctccg 4260
   ccttcaatcc cacccgctaa agtacttgga gcggtctctc cctccctcat cagcccacca 4320
   aaccaaacct agcctccaag agtgggaaga aattaaagca agataggcta ttaagtgcag 4380
   agggagagaa aatgcctcca acatgtgagg aagtaatgag agaaatcata gaattttaag 4440
   gccatgattt aaggccatca tggccttaat cttccgcttc ctcgctcact gactcgctgc 4500
   gctcggtcgt tcggctgcgg cgagcggtat cagctcactc aaaggcggta atacggttat 4560
   ccacagaatc aggggataac gcaggaaaga acatgtgagc aaaaggccag caaaaggcca 4620
   ggaaccgtaa aaaggccgcg ttgctggcgt ttttccatag gctccgcccc cctgacgagc 4680
   atcacaaaaa tcgacgctca agtcagaggt ggcgaaaccc gacaggacta taaagatacc 4740
   aggcgtttcc ccctggaagc tccctcgtgc gctctcctgt tccgaccctg ccgcttaccg 4800
   gatacctgtc cgcctttctc ccttcgggaa gcgtggcgct ttctcatagc tcacgctgta 4860
   ggtatctcag ttcggtgtag gtcgttcgct ccaagctggg ctgtgtgcac gaaccccccg 4920
   ttcagcccga ccgctgcgcc ttatccggta actatcgtct tgagtccaac ccggtaagac 4980
   acgacttatc gccactggca gcagccactg gtaacaggat tagcagagcg aggtatgtag 5040
   gcggtgctac agagttcttg aagtggtggc ctaactacgg ctacactaga agaacagtat 5100
   ttggtatctg cgctctgctg aagccagtta ccttcggaaa aagagttggt agctcttgat 5160
   ccggcaaaca aaccaccgct ggtagcggtg gtttttttgt ttgcaagcag cagattacgc 5220
   gcagaaaaaa aggatctcaa gaagatcctt tgatcttttc tacggggtct gacgctcagt 5280
   ggaacgaaaa ctcacgttaa gggattttgg tcatgagatt atcaaaaagg atcttcacct 5340
   agatcctttt aaattaaaaa tgaagtttta aatcaatcta aagtatatat gagtaaactt 5400
   ggtctgacag ttaccaatgc ttaatcagtg aggcacctat ctcagcgatc tgtctatttc 5460
   gttcatccat agttgcctga ctcggggggg gggggcgctg aggtctgcct cgtgaagaag 5520
   gtgttgctga ctcataccag gcctgaatcg ccccatcatc cagccagaaa gtgagggagc 5580
   cacggttgat gagagctttg ttgtaggtgg accagttggt gattttgaac ttttgctttg 5640
   ccacggaacg gtctgcgttg tcgggaagat gcgtgatctg atccttcaac tcagcaaaag 5700
   ttcgatttat tcaacaaagc cgccgtcccg tcaagtcagc gtaatgctct gccagtgtta 5760
   caaccaatta accaattctg attagaaaaa ctcatcgagc atcaaatgaa actgcaattt 5820
   attcatatca ggattatcaa taccatattt ttgaaaaagc cgtttctgta atgaaggaga 5880
   aaactcaccg aggcagttcc ataggatggc aagatcctgg tatcggtctg cgattccgac 5940
   tcgtccaaca tcaatacaac ctattaattt cccctcgtca aaaataaggt tatcaagtga 6000
   gaaatcacca tgagtgacga ctgaatccgg tgagaatggc aaaagcttat gcatttcttt 6060
   ccagacttgt tcaacaggcc agccattacg ctcgtcatca aaatcactcg catcaaccaa 6120
   accgttattc attcgtgatt gcgcctgagc gagacgaaat acgcgatcgc tgttaaaagg 6180
   acaattacaa acaggaatcg aatgcaaccg gcgcaggaac actgccagcg catcaacaat 6240
   attttcacct gaatcaggat attcttctaa tacctggaat gctgttttcc cggggatcgc 6300
   agtggtgagt aaccatgcat catcaggagt acggataaaa tgcttgatgg tcggaagagg 6360
   cataaattcc gtcagccagt ttagtctgac catctcatct gtaacatcat tggcaacgct 6420
   acctttgcca tgtttcagaa acaactctgg cgcatcgggc ttcccataca atcgatagat 6480
   tgtcgcacct gattgcccga cattatcgcg agcccattta tacccatata aatcagcatc 6540
   catgttggaa tttaatcgcg gcctcgagca agacgtttcc cgttgaatat ggctcataac 6600
   accccttgta ttactgttta tgtaagcaga cagttttatt gttcatgatg atatattttt 6660
   atcttgtgca atgtaacatc agagattttg agacacaacg tggctttccc ccccccccca 6720
   ttattgaagc atttatcagg gttattgtct catgagcgga tacatatttg aatgtattta 6780
   gaaaaataaa caaatagggg ttccgcgcac atttccccga aaagtgccac ctgacgtcta 6840
   agaaaccatt attatcatga cattaaccta taaaaatagg cgtatcacga ggccctttcg 6900
   tc 6902
<210> 43
   <211> 6625
   <212> DNA
   <213> Artificial Sequence
<220>
   <223> CMV/R Ebola NP
<400> 43
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcagattgg 240
   ctattggcca ttgcatacgt tgtatccata tcataatatg tacatttata ttggctcatg 300
   tccaacatta ccgccatgtt gacattgatt attgactagt tattaatagt aatcaattac 360
   ggggtcatta gttcatagcc catatatgga gttccgcgtt acataactta cggtaaatgg 420
   cccgcctggc tgaccgccca acgacccccg cccattgacg tcaataatga cgtatgttcc 480
   catagtaacg ccaataggga ctttccattg acgtcaatgg gtggagtatt tacggtaaac 540
   tgcccacttg gcagtacatc aagtgtatca tatgccaagt acgcccccta ttgacgtcaa 600
   tgacggtaaa tggcccgcct ggcattatgc ccagtacatg accttatggg actttcctac 660
   ttggcagtac atctacgtat tagtcatcgc tattaccatg gtgatgcggt tttggcagta 720
   catcaatggg cgtggatagc ggtttgactc acggggattt ccaagtctcc accccattga 780
   cgtcaatggg agtttgtttt ggcaccaaaa tcaacgggac tttccaaaat gtcgtaacaa 840
   ctccgcccca ttgacgcaaa tgggcggtag gcgtgtacgg tgggaggtct atataagcag 900
   agctcgttta gtgaaccgtc agatcgcctg gagacgccat ccacgctgtt ttgacctcca 960
   tagaagacac cgggaccgat ccagcctcca tcggctcgca tctctccttc acgcgcccgc 1020
   cgccttacct gaggccgcca tccacgccgg ttgagtcgcg ttctgccgcc tcccgcctgt 1080
   ggtgcctcct gaactacgtc cgccgtctag gtaagtttag agctcaggtc gagaccgggc 1140
   ctttgtccgg cgctcccttg gagcctacct agactcagcc ggctctccac gctttgcctg 1200
   accctgcttg ctcaactcta gttaacggtg gagggcagtg tagtctgagc agtactcgtt 1260
   gctgccgcgc gcgccaccag acataatagc tgacagacta acagactgtt cctttccatg 1320
   ggtcttttct gcagtcaccg tcgtcgacac gtgtgatcag atatcgcggc cgctctagac 1380
   caggccctgg atccagatcg atccgagtat ggattctcgt cctcagaaaa tctggatggc 1440
   gccgagtctc actgaatctg acatggatta ccacaagatc ttgacagcag gtctgtccgt 1500
   tcaacagggg attgttcggc aaagagtcat cccagtgtat caagtaaaca atcttgaaga 1560
   aatttgccaa cttatcatac aggcctttga agcaggtgtt gattttcaag agagtgcgga 1620
   cagtttcctt ctcatgcttt gtcttcatca tgcgtaccag ggagattaca aacttttctt 1680
   ggaaagtggc gcagtcaagt atttggaagg gcacgggttc cgttttgaag tcaagaagcg 1740
   tgatggagtg aagcgccttg aggaattgct gccagcagta tctagtggaa aaaacattaa 1800
   gagaacactt gctgccatgc cggaagagga gacaactgaa gctaatgccg gtcagtttct 1860
   ctcctttgca agtctattcc ttccgaaatt ggtagtagga gaaaaggctt gccttgagaa 1920
   ggttcaaagg caaattcaag tacatgcaga gcaaggactg atacaatatc caacagcttg 1980
   gcaatcagta ggacacatga tggtgatttt ccgtttgatg cgaacaaatt ttctgatcaa 2040
   atttctccta atacaccaag ggatgcacat ggttgccggg catgatgcca acgatgctgt 2100
   gatttcaaat tcagtggctc aagctcgttt ttcaggctta ttgattgtca aaacagtact 2160
   tgatcatatc ctacaaaaga cagaacgagg agttcgtctc catcctcttg caaggaccgc 2220
   caaggtaaaa aatgaggtga actcctttaa ggctgcactc agctccctgg ccaagcatgg 2280
   agagtatgct cctttcgccc gacttttgaa cctttctgga gtaaataatc ttgagcatgg 2340
   tcttttccct caactatcgg caattgcact cggagtcgcc acagcacacg ggagtaccct 2400
   cgcaggagta aatgttggag aacagtatca acaactcaga gaggctgcca ctgaggctga 2460
   gaagcaactc caacaatatg cagagtctcg cgaacttgac catcttggac ttgatgatca 2520
   ggaaaagaaa attcttatga acttccatca gaaaaagaac gaaatcagct tccagcaaac 2580
   aaacgctatg gtaactctaa gaaaagagcg cctggccaag ctgacagaag ctatcactgc 2640
   tgcgtcactg cccaaaacaa gtggacatta cgatgatgat gacgacattc cctttccagg 2700
   acccatcaat gatgacgaca atcctggcca tcaagatgat gatccgactg actcacagga 2760
   tacgaccatt cccgatgtgg tggttgatcc cgatgatgga agctacggcg aataccagag 2820
   ttactcggaa aacggcatga atgcaccaga tgacttggtc ctattcgatc tagacgagga 2880
   cgacgaggac actaagccag tgcctaatag atcgaccaag ggtggacaac agaagaacag 2940
   tcaaaagggc cagcatatag agggcagaca gacacaatcc aggccaattc aaaatgtccc 3000
   aggccctcac agaacaatcc accacgccag tgcgccactc acggacaatg acagaagaaa 3060
   tgaaccctcc ggctcaacca gccctcgcat gctgacacca attaacgaag aggcagaccc 3120
   actggacgat gccgacgacg agacgtctag ccttccgccc ttggagtcag atgatgaaga 3180
   gcaggacagg gacggaactt ccaaccgcac acccactgtc gccccaccgg ctcccgtata 3240
   cagagatcac tctgaaaaga aagaactccc gcaagacgag caacaagatc aggaccacac 3300
   tcaagaggcc aggaaccagg acagtgacaa cacccagtca gaacactctt ttgaggagat 3360
   gtatcgccac attctaagat cacaggggcc atttgatgct gttttgtatt atcatatgat 3420
   gaaggatgag cctgtagttt tcagtaccag tgatggcaaa gagtacacgt atccagactc 3480
   ccttgaagag gaatatccac catggctcac tgaaaaagag gctatgaatg aagagaatag 3540
   atttgttaca ttggatggtc aacaatttta ttggccggtg atgaatcaca agaataaatt 3600
   catggcaatc ctgcaacatc atcagctgtg ccttctagtt gccagccatc tgttgtttgc 3660
   ccctcccccg tgccttcctt gaccctggaa ggtgccactc ccactgtcct ttcctaataa 3720
   aatgaggaaa ttgcatcgca ttgtctgagt aggtgtcatt ctattctggg gggtggggtg 3780
   gggcaggaca gcaaggggga ggattgggaa gacaatagca ggcatgctgg ggatgcggtg 3840
   ggctctatgg gtacccaggt gctgaagaat tgacccggtt cctcctgggc cagaaagaag 3900
   caggcacatc cccttctctg tgacacaccc tgtccacgcc cctggttctt agttccagcc 3960
   ccactcatag gacactcata gctcaggagg gctccgcctt caatcccacc cgctaaagta 4020
   cttggagcgg tctctccctc cctcatcagc ccaccaaacc aaacctagcc tccaagagtg 4080
   ggaagaaatt aaagcaagat aggctattaa gtgcagaggg agagaaaatg cctccaacat 4140
   gtgaggaagt aatgagagaa atcatagaat tttaaggcca tcatggcctt aatcttccgc 4200
   ttcctcgctc actgactcgc tgcgctcggt cgttcggctg cggcgagcgg tatcagctca 4260
   ctcaaaggcg gtaatacggt tatccacaga atcaggggat aacgcaggaa agaacatgtg 4320
   agcaaaaggc cagcaaaagg ccaggaaccg taaaaaggcc gcgttgctgg cgtttttcca 4380
   taggctccgc ccccctgacg agcatcacaa aaatcgacgc tcaagtcaga ggtggcgaaa 4440
   cccgacagga ctataaagat accaggcgtt tccccctgga agctccctcg tgcgctctcc 4500
   tgttccgacc ctgccgctta ccggatacct gtccgccttt ctcccttcgg gaagcgtggc 4560
   gctttctcat agctcacgct gtaggtatct cagttcggtg taggtcgttc gctccaagct 4620
   gggctgtgtg cacgaacccc ccgttcagcc cgaccgctgc gccttatccg gtaactatcg 4680
   tcttgagtcc aacccggtaa gacacgactt atcgccactg gcagcagcca ctggtaacag 4740
   gattagcaga gcgaggtatg taggcggtgc tacagagttc ttgaagtggt ggcctaacta 4800
   cggctacact agaagaacag tatttggtat ctgcgctctg ctgaagccag ttaccttcgg 4860
   aaaaagagtt ggtagctctt gatccggcaa acaaaccacc gctggtagcg gtggtttttt 4920
   tgtttgcaag cagcagatta cgcgcagaaa aaaaggatct caagaagatc ctttgatctt 4980
   ttctacgggg tctgacgctc agtggaacga aaactcacgt taagggattt tggtcatgag 5040
   attatcaaaa aggatcttca cctagatcct tttaaattaa aaatgaagtt ttaaatcaat 5100
   ctaaagtata tatgagtaaa cttggtctga cagttaccaa tgcttaatca gtgaggcacc 5160
   tatctcagcg atctgtctat ttcgttcatc catagttgcc tgactcgggg ggggggggcg 5220
   ctgaggtctg cctcgtgaag aaggtgttgc tgactcatac caggcctgaa tcgccccatc 5280
   atccagccag aaagtgaggg agccacggtt gatgagagct ttgttgtagg tggaccagtt 5340
   ggtgattttg aacttttgct ttgccacgga acggtctgcg ttgtcgggaa gatgcgtgat 5400
   ctgatccttc aactcagcaa aagttcgatt tattcaacaa agccgccgtc ccgtcaagtc 5460
   agcgtaatgc tctgccagtg ttacaaccaa ttaaccaatt ctgattagaa aaactcatcg 5520
   agcatcaaat gaaactgcaa tttattcata tcaggattat caataccata tttttgaaaa 5580
   agccgtttct gtaatgaagg agaaaactca ccgaggcagt tccataggat ggcaagatcc 5640
   tggtatcggt ctgcgattcc gactcgtcca acatcaatac aacctattaa tttcccctcg 5700
   tcaaaaataa ggttatcaag tgagaaatca ccatgagtga cgactgaatc cggtgagaat 5760
   ggcaaaagct tatgcatttc tttccagact tgttcaacag gccagccatt acgctcgtca 5820
   tcaaaatcac tcgcatcaac caaaccgtta ttcattcgtg attgcgcctg agcgagacga 5880
   aatacgcgat cgctgttaaa aggacaatta caaacaggaa tcgaatgcaa ccggcgcagg 5940
   aacactgcca gcgcatcaac aatattttca cctgaatcag gatattcttc taatacctgg 6000
   aatgctgttt tcccggggat cgcagtggtg agtaaccatg catcatcagg agtacggata 6060
   aaatgcttga tggtcggaag aggcataaat tccgtcagcc agtttagtct gaccatctca 6120
   tctgtaacat cattggcaac gctacctttg ccatgtttca gaaacaactc tggcgcatcg 6180
   ggcttcccat acaatcgata gattgtcgca cctgattgcc cgacattatc gcgagcccat 6240
   ttatacccat ataaatcagc atccatgttg gaatttaatc gcggcctcga gcaagacgtt 6300
   tcccgttgaa tatggctcat aacacccctt gtattactgt ttatgtaagc agacagtttt 6360
   attgttcatg atgatatatt tttatcttgt gcaatgtaac atcagagatt ttgagacaca 6420
   acgtggcttt cccccccccc ccattattga agcatttatc agggttattg tctcatgagc 6480
   ggatacatat ttgaatgtat ttagaaaaat aaacaaatag gggttccgcg cacatttccc 6540
   cgaaaagtgc cacctgacgt ctaagaaacc attattatca tgacattaac ctataaaaat 6600
   aggcgtatca cgaggccctt tcgtc                                      6625
<210> 44
   <211> 22
   <212> DNA
   <213> Artificial Sequence
<220>
   <223> Ebola Sudan GP primer
<400> 44
   atcttcagga tctcgccatg ga 22
<210> 45
   <211> 24
   <212> DNA
   <213> Artificial Sequence
<220>
   <223> Ebola Sudan GP primer
<400> 45
   gatattcaac aaagcagctt gcag 24
<210> 46
   <211> 21
   <212> DNA
   <213> Artificial Sequence
<220>
   <223> Ebola Ivory Coast GP primer
<400> 46
   ctaatcacag tcaccatggg a 21
<210> 47
   <211> 24
   <212> DNA
   <213> Artificial Sequence
<220>
   <223> Ebola Ivory Coast GP primer
<400> 47
   aaagtatgat gctatattag ttca 24
<210> 48
   <211> 10
   <212> PRT
   <213> Ebola Virus
<400> 48
   Gln Arg Thr Phe Ser Ile Pro Leu Gly Val
   1 5 10
<210> 49
   <211> 10
   <212> PRT
   <213> Ebola Virus
<400> 49
   Arg Arg Thr Arg Arg Glu Ala Ile Val Asn
   1 5 10
<210> 50
   <211> 16
   <212> PRT
   <213> Ebola Virus
<400> 50
   Gly Ala Ala Ile Gly Leu Ala Trp Ile Pro Tyr Phe Gly Pro Ala Ala
   1 5 10 15
<210> 51
   <211> 5
   <212> PRT
   <213> Ebola Virus
<400> 51
   Arg Arg Thr Arg Arg
   1 5
<210> 52
   <211> 1087
   <212> DNA
   <213> Artificial Sequence
<220>
   <223> The CMV Enhancer/Promoter, R Region (HTVL-1), CMV IE Splicing Acceptor sequence
<400> 52
   ccattgcata cgttgtatcc atatcataat atgtacattt atattggctc atgtccaaca 60
   ttaccgccat gttgacattg attattgact agttattaat agtaatcaat tacggggtca 120
   ttagttcata gcccatatat ggagttccgc gttacataac ttacggtaaa tggcccgcct 180
   ggctgaccgc ccaacgaccc ccgcccattg acgtcaataa tgacgtatgt tcccatagta 240
   acgccaatag ggactttcca ttgacgtcaa tgggtggagt atttacggta aactgcccac 300
   ttggcagtac atcaagtgta tcatatgcca agtacgcccc ctattgacgt caatgacggt 360
   aaatggcccg cctggcatta tgcccagtac atgaccttat gggactttcc tacttggcag 420
   tacatctacg tattagtcat cgctattacc atggtgatgc ggttttggca gtacatcaat 480
   gggcgtggat agcggtttga ctcacgggga tttccaagtc tccaccccat tgacgtcaat 540
   gggagtttgt tttggcacca aaatcaacgg gactttccaa aatgtcgtaa caactccgcc 600
   ccattgacgc aaatgggcgg taggcgtgta cggtgggagg tctatataag cagagctcgt 660
   ttagtgaacc gtcagatcgc ctggagacgc catccacgct gttttgacct ccatagaaga 720
   caccgggacc gatccagcct ccatcggctc gcatctctcc ttcacgcgcc cgccgcctta 780
   cctgaggccg ccatccacgc cggttgagtc gcgttctgcc gcctcccgcc tgtggtgcct 840
   cctgaactac gtccgccgtc taggtaagtt tagagctcag gtcgagaccg ggcctttgtc 900
   cggcgctccc ttggagccta cctagactca gccggctctc cacgctttgc ctgaccctgc 960
   ttgctcaact ctagttaacg gtggagggca gtgtagtctg agcagtactc gttgctgccg 1020
   cgcgcgccac cagacataat agctgacaga ctaacagact gttcctttcc atgggtcttt 1080
   tctgcag 1087

## Description

### Field of the Invention

The present invention relates to an expression vector comprising SEQ ID NO: 52 or analog thereof having at least 95, 96, 97, 98, 99 or 100% sequence identity thereto that mediates expression, wherein the percentage of identity is calculated over the full length of the reference nucleotide sequence.

### Background of the Invention

The Ebola viruses, and the genetically-related Marburg virus, are filoviruses associated with outbreaks of highly lethal hemorrhagic fever in humans and primates in North America, Europe, and Africa (Peters, C.J. et al. in: Fields Virology, eds. Fields, B.N. et al. 1161-1176, Philadelphia, Lippincott-Raven, 1996; Peters, C.J. et al. 1994 Semin Virol 5:147-154). Ebola viruses are negative-stranded RNA viruses comprised of four subtypes, including those described in the Zaire, Sudan, Reston, and Ivory Coast episodes (Sanchez, A. et al. 1996 PNAS USA 93:3602-3607). Although several subtypes have been defined, the genetic organization of these viruses is similar, each containing seven linearly arrayed genes. Among the viral proteins, the envelope glycoprotein exists in two alternative forms, a 50-70 kilodalton (kDa) secreted protein of unknown function encoded by the viral genome and a 130 kDa transmembrane glycoprotein generated by RNA editing that mediates viral entry (Peters, C.J. et al. in: Fields Virology, eds. Fields, B.N. et al. 1161-1176, Philadelphia, Lippincott-Raven, 1996; Sanchez, A. et al. 1996 PNAS USA 93:3602-3607). Other structural gene products include the nucleoprotein (NP), matrix proteins VP24 and VP40, presumed nonstructural proteins VP30 and VP35, and the viral polymerase (reviewed in Peters, C.J. et al. in: Fields Virology, eds. Fields, B.N. et al. 1161-1176, Philadelphia, Lippincott-Raven, 1996). Although spontaneous variation of its RNA sequence does occur in nature, there appears to be less nucleotide polymorphism within Ebola subtypes than among other RNA viruses (Sanchez, A. et al. 1996 PNAS USA 93:3602-3607), suggesting that immunization may be useful in protecting against this disease. Previous attempts to elicit protective immune responses against Ebola virus using traditional active and passive immunization approaches have, however, not succeeded in primates (Peters, C.J. et al. in: Fields Virology, eds. Fields, B.N. et al. 1161-1176, Philadelphia, Lippincott-Raven, 1996; Clegg, J.C.S. et al. 1997 New Generation Vaccines, eds.: Levine, M.M. et al. 749-765, New York, NY. Marcel Dekker, Inc.; Jahrling, P.B. et al. 1996 Arch Virol Suppl 11:135-140). It would thus be desirable to provide a vaccine to elicit an immune response against a filovirus or disease caused by infection with filovirus. It would further be desirable to provide methods of making and using said vaccine.

Outbreaks of hemorrhagic fever caused by the Ebola virus are associated with high mortality rates that are a distinguishing feature of this human pathogen. The highest lethality is associated with the Zaire subtype, one of four strains identified to date (Feldmann, H. et al. 1994 Virology 199:469-473; Sanchez, A. et al. 1996 PNAS USA 93:3602-3607). Its rapid progression allows little opportunity to develop natural immunity, and there is currently no effective anti-viral therapy. Therefore, vaccination offers a promising intervention to prevent infection and limit spread. Here we describe a highly effective vaccine strategy for Ebola virus infection in primates. A combination of DNA immunization and boosting with adenoviral vectors that encode viral proteins generated cellular and humoral immunity in cynomolgus macaques. Challenge with a lethal dose of the highly pathogenic, wild-type, 1976 Mayinga strain of Ebola Zaire virus resulted in uniform infection in controls, who progressed to a moribund state and death in less than one week. In contrast, all vaccinated animals were asymptomatic for more than six months, with no detectable virus after the initial challenge. These findings demonstrate that it is possible to develop a preventive vaccine against Ebola virus infection in primates.

### Brief Description of the Drawings

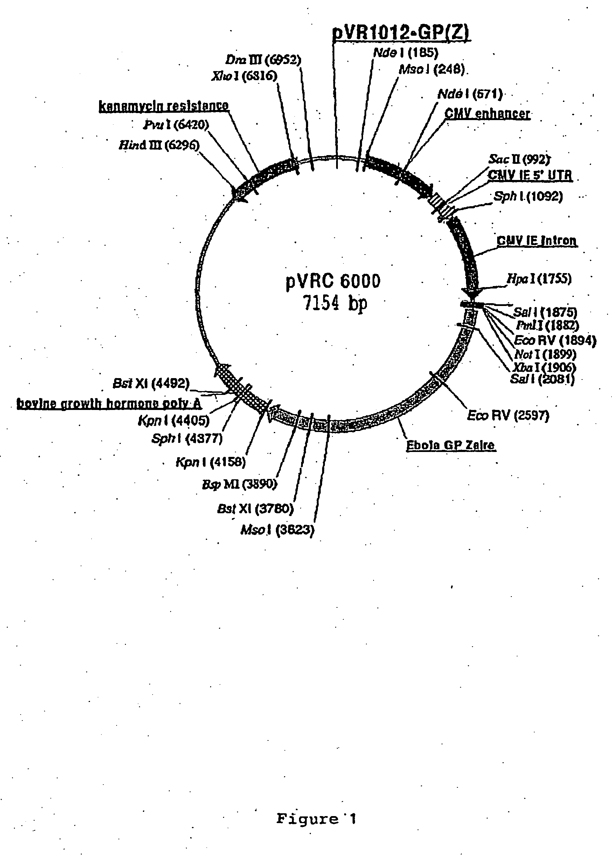

**Figure 1** shows VRC6000 (pVR1012-GP(Z)) construct map (see, Ebola/Marburg/Lassa Plasmids, and Recombinant Adenoviruses in Table 2).

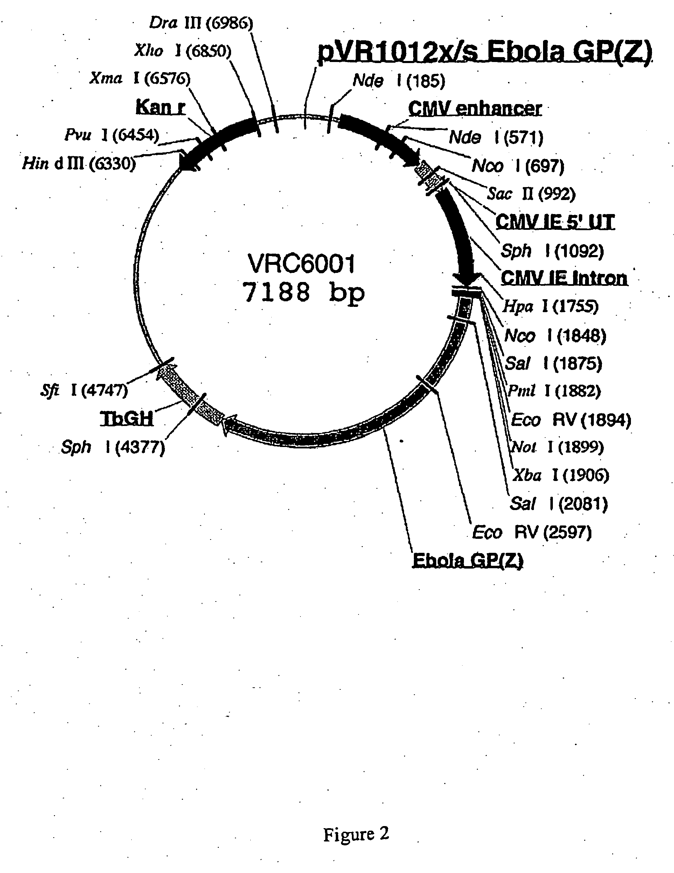

**Figure 2** shows VRC6001 (pVR1012x/s-GP(Z)) construct map (see, Ebola/Marburg/Lassa Plasmids, and Recombinant Adenoviruses in Table 2).

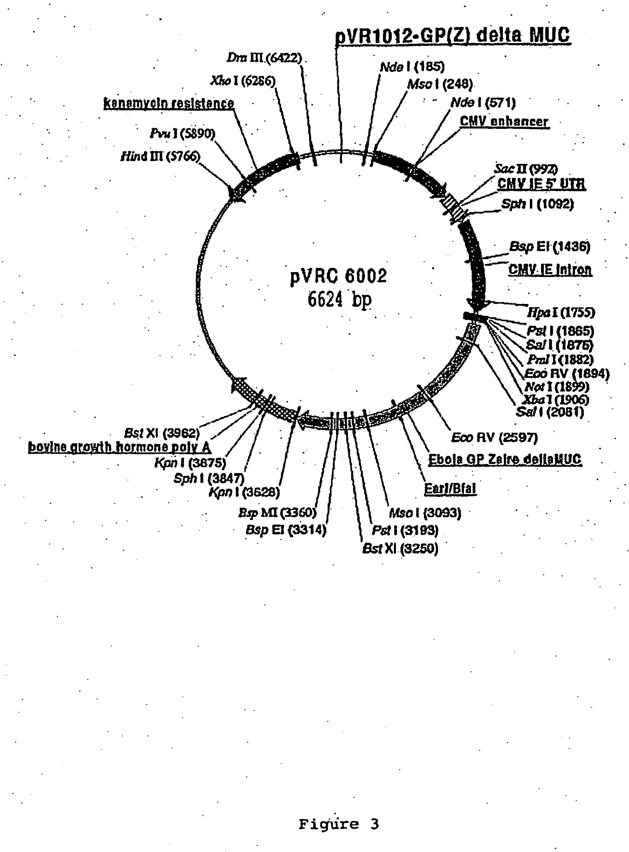

**Figure 3** shows VRC6002 (pVR1012-GP(Z) delta MUC) construct map (see, Ebola/Marburg/Lassa Plasmids, and Recombinant Adenoviruses in Table 2).

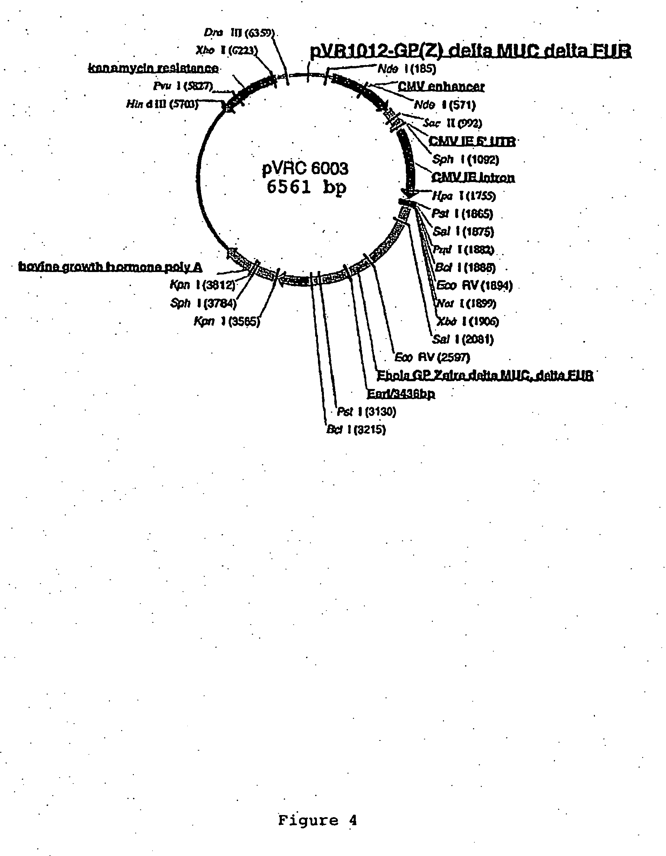

**Figure 4** shows VRC6003 (pVR1012-GP(Z) delta MUC delta FUR) construct map (see, Ebola/Marburg/Lassa Plasmids, and Recombinant Adenoviruses in Table 2).

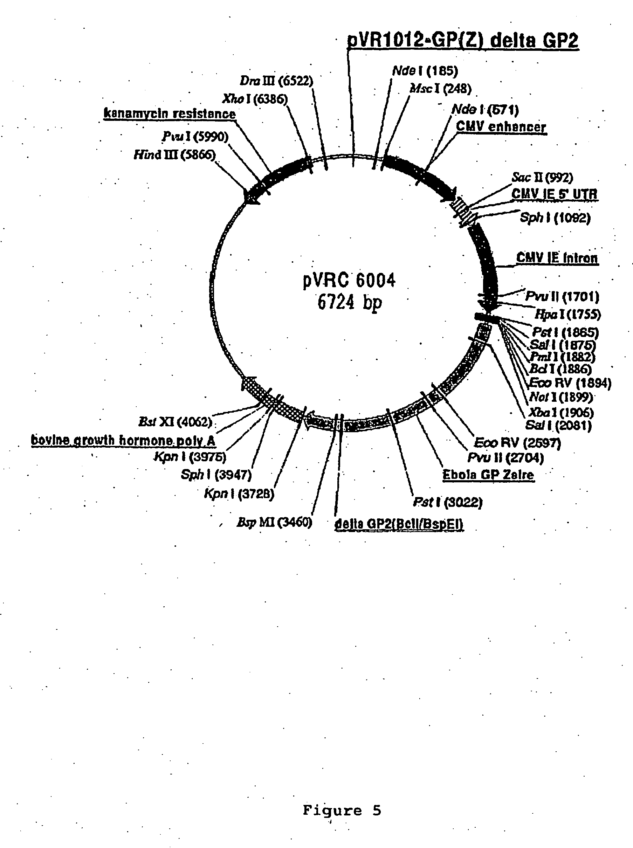

**Figure 5** shows VRC6004 (pVR1012-GP(Z) delta GP2) construct map (see, Ebola/Marburg/Lassa Plasmids, and Recombinant Adenoviruses in Table 2).

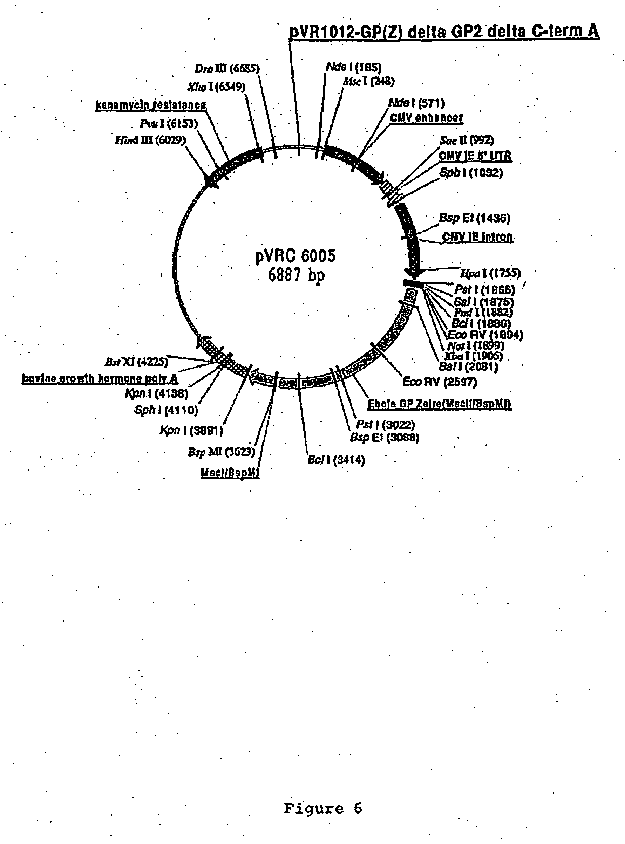

**Figure 6** shows VRC6005 (pVR1012-GP(Z) delta GP2 delta C-term A) construct map (see, Ebola/Marburg/Lassa Plasmids, and Recombinant Adenoviruses in Table 2).

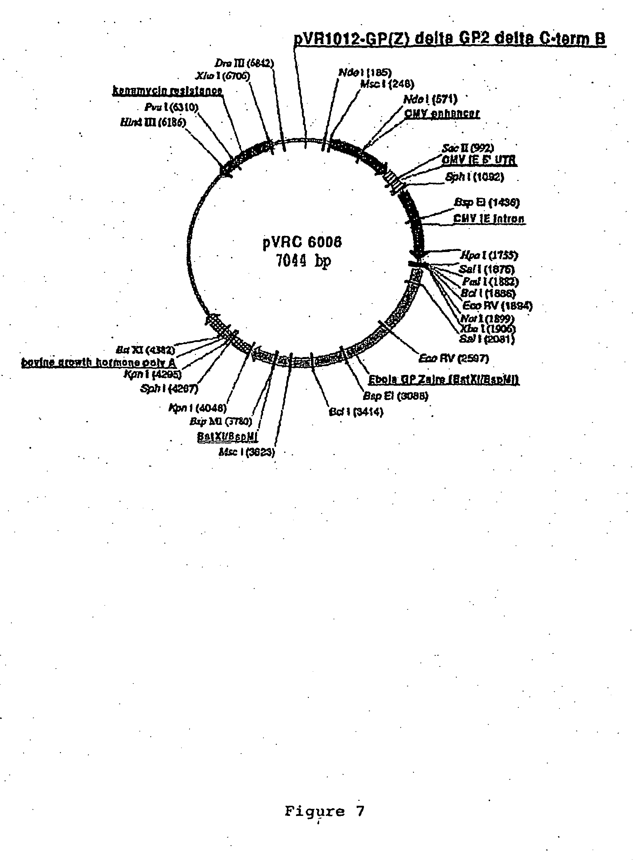

**Figure 7** shows VRC6006 (pVR1012-GP(Z) delta GP2 delta C-term B) construct map (see, Ebola/Marburg/Lassa Plasmids, and Recombinant Adenoviruses in Table 2).

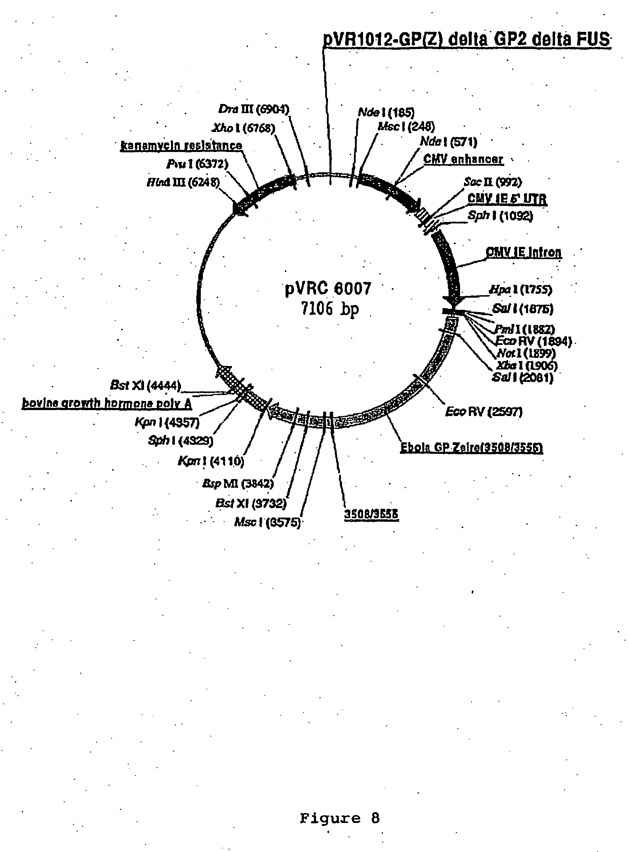

**Figure 8** shows VRC6007 (pVR1012-GP(Z) delta GP2 delta FUS) construct map (see, Ebola/Marburg/Lassa Plasmids, and Recombinant Adenoviruses in Table 2).

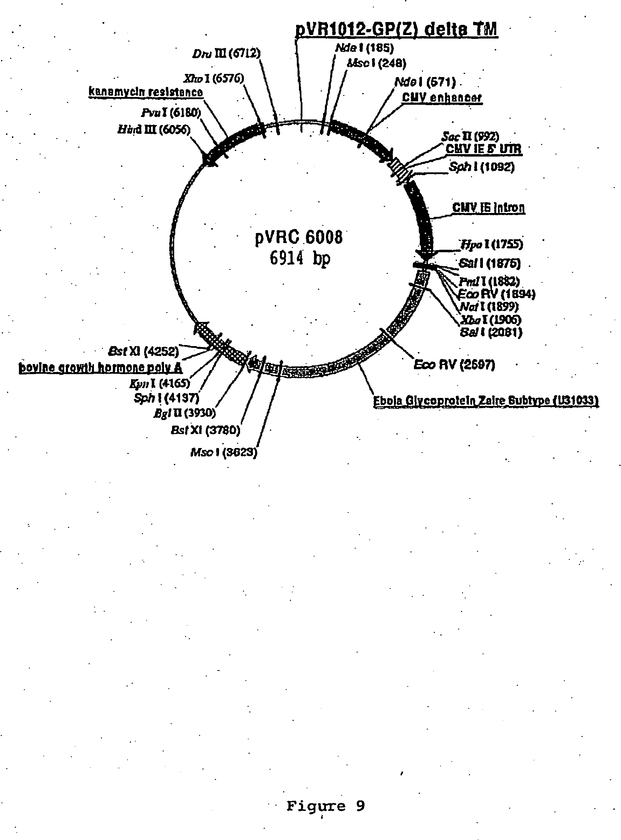

**Figure 9** shows VRC6008 (pVR1012-GP(Z) delta TM) construct map (see, Ebola/Marburg/Lassa Plasmids, and Recombinant Adenoviruses in Table 2).

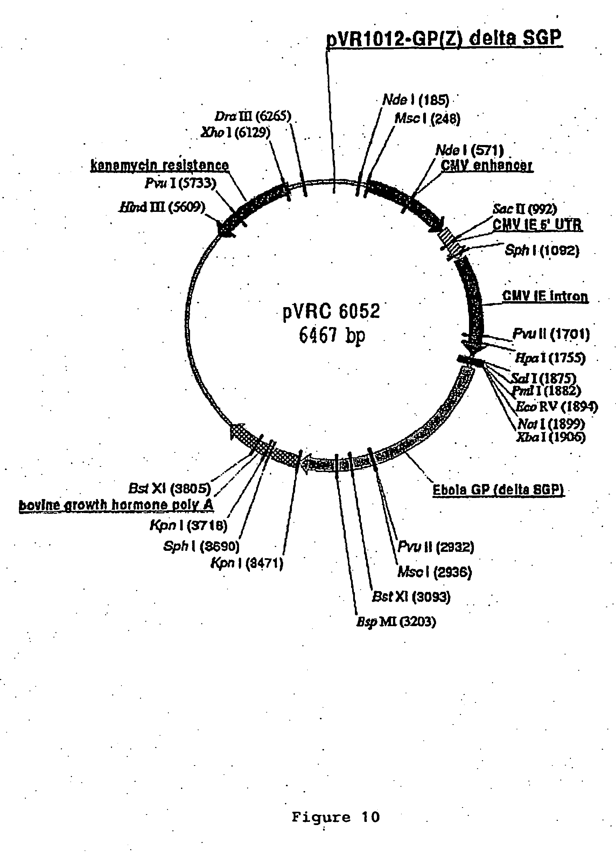

**Figure 10** shows VRC 6052 (pVR1012-GP(Z) delta SGP) construct map (see, Ebola/Marburg/Lassa Plasmids, and Recombinant Adenoviruses in Table 2).

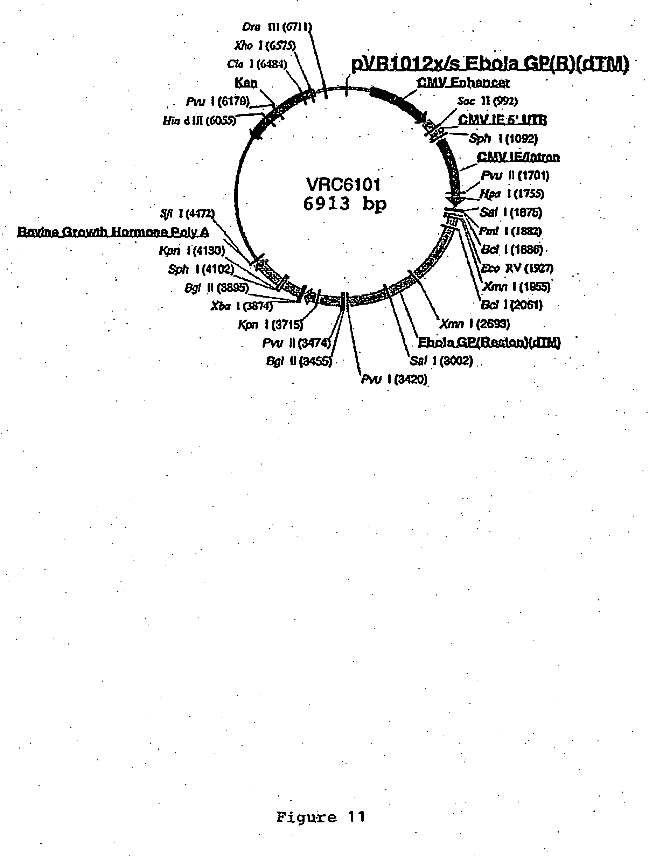

**Figure 11** shows VRC 6101 (pVR1012x/s Ebola GP(R) (dTM)) construct map (see, Ebola/Marburg/Lassa Plasmids, and Recombinant Adenoviruses in Table 2).

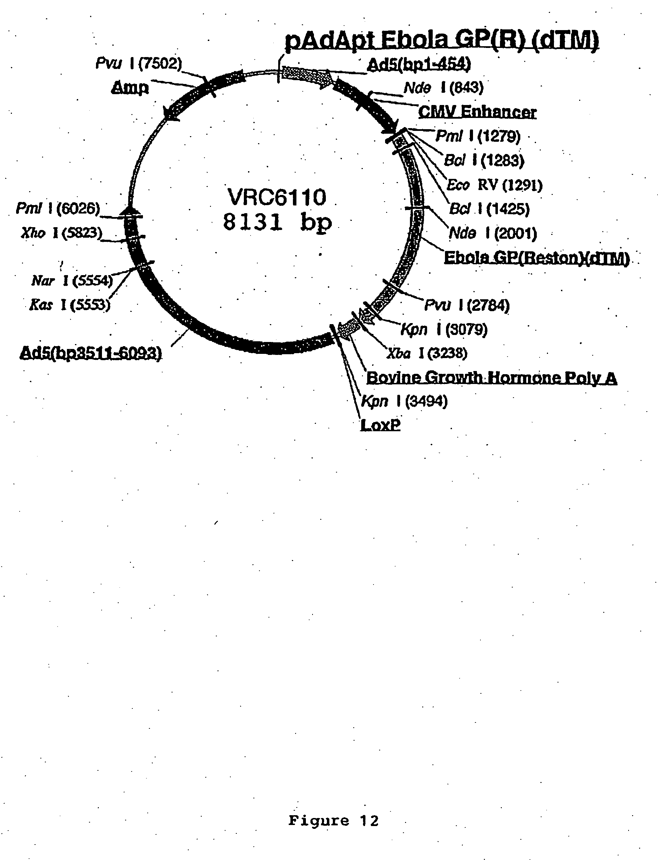

**Figure 12** shows VRC 6110 (pAdApt Ebola GP(R) (dTM)) construct map (see, Ebola/Marburg/Lassa Plasmids, and Recombinant Adenoviruses in Table 2).

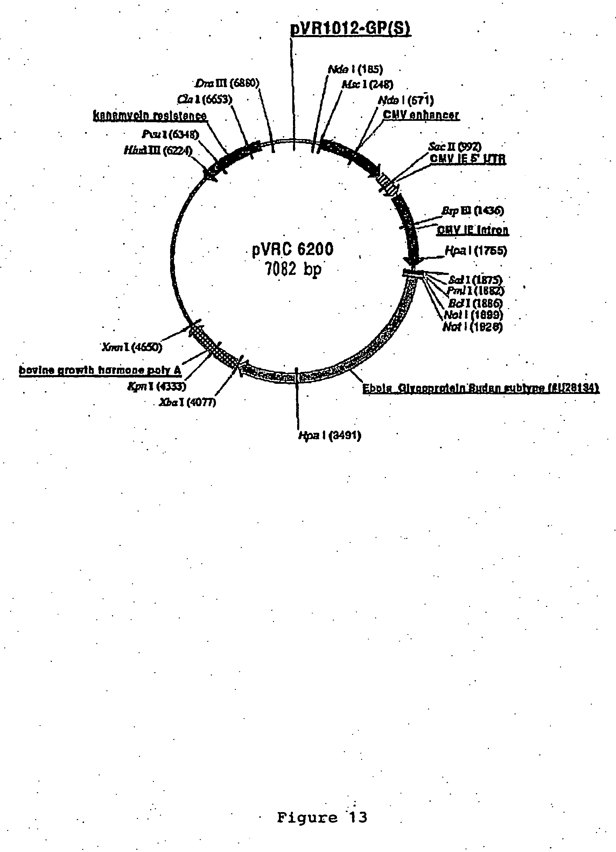

**Figure 13** shows VRC6200 (pVR1012-GP(S)) construct map (see, Ebola/Marburg/Lassa Plasmids, and Recombinant Adenoviruses in Table 2).

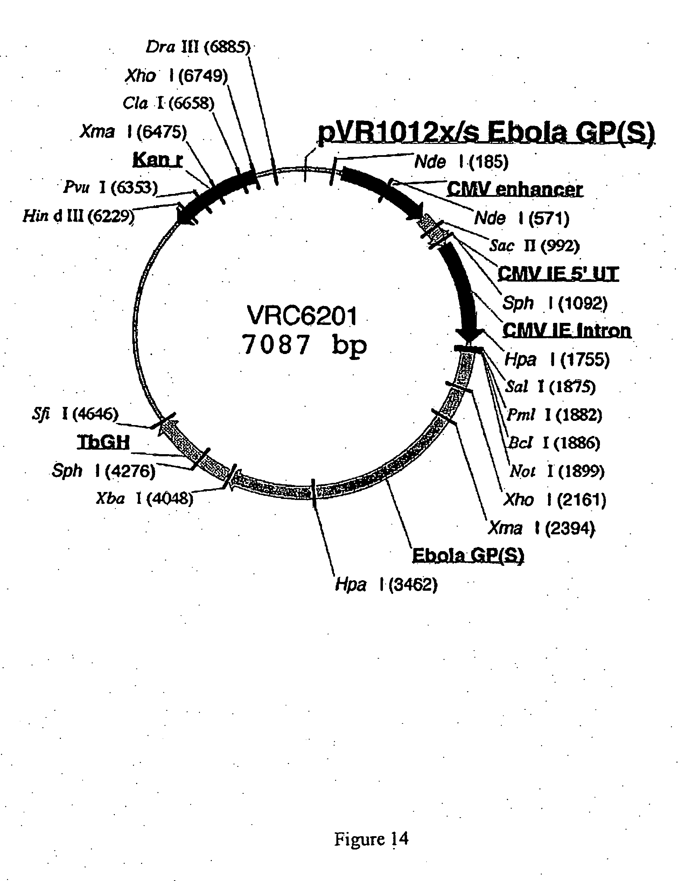

**Figure 14** shows VRC 6201 (pVR1012x/s Ebola GP(S)) construct map (see, Ebola/Marburg/Lassa Plasmids, and Recombinant Adenoviruses in Table 2).

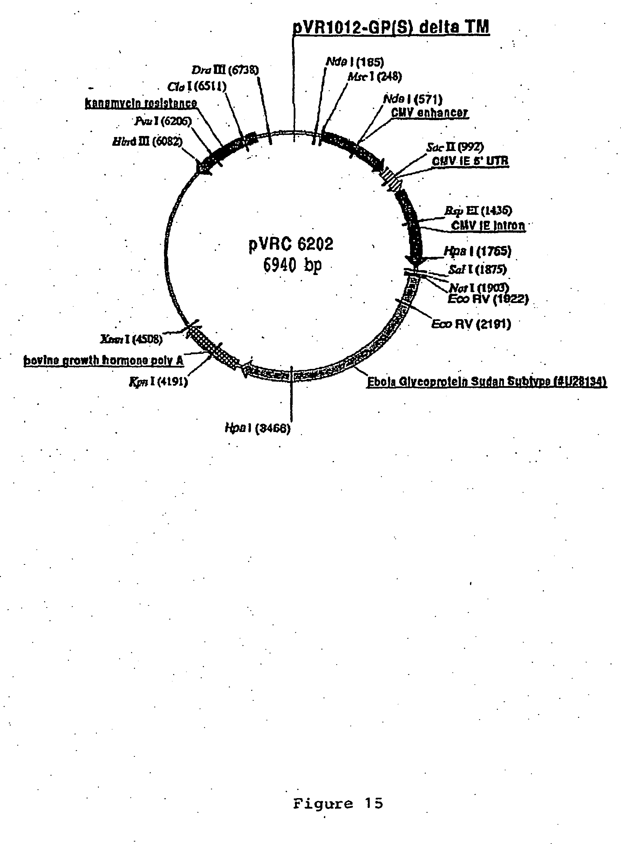

**Figure 15** shows VRC6202 (pVR1012-GP(S) delta TM) construct map (see, Ebola/Marburg/Lassa Plasmid, and Recombinant Adenoviruses in Table 2).

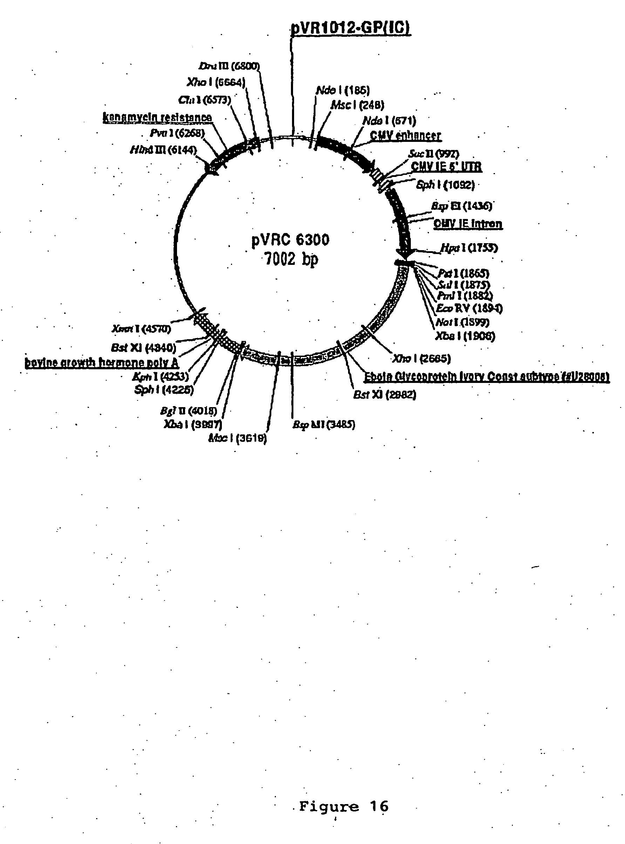

**Figure 16** shows VRC6300 (pVR1012-GP(IC)) construct map (see, Ebola/Marburg/Lassa Plasmids, and Recombinant Adenoviruses in Table 2).

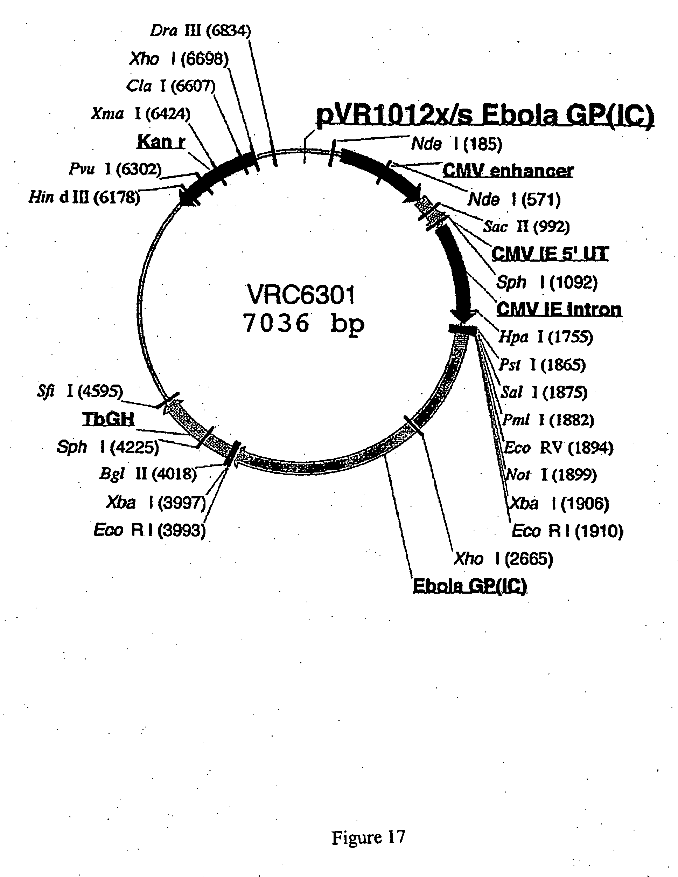

**Figure 17** shows VRC6301 (pVR1012x/s-GP(IC)) construct map (see, Ebola/Marburg/Lassa Plasmids, and Recombinant Adenoviruses in Table 2).

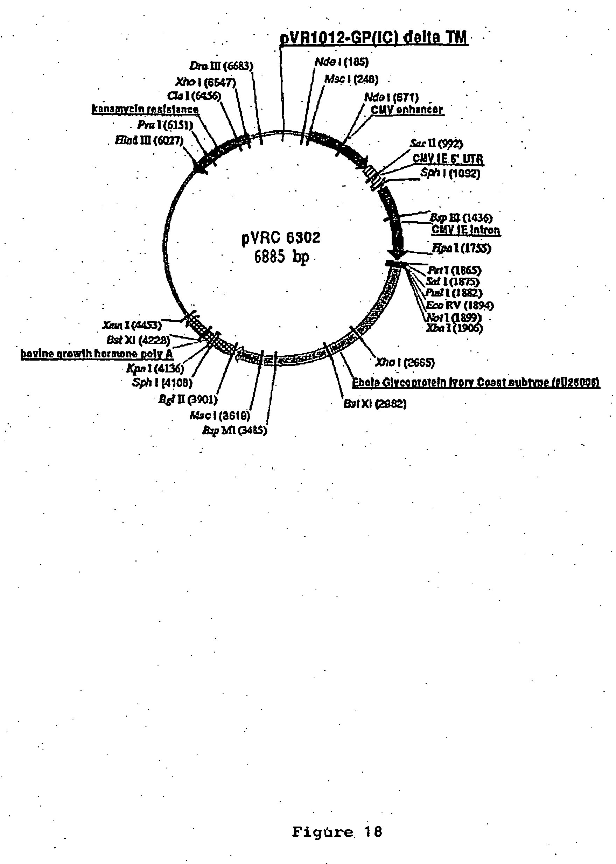

**Figure 18** shows VRC6302 (pVR1012-GP(IC) delta TM) construct map (see, Eboia/Marburg/Lassa Plasmids, and Recombinant Adenoviruses in Table 2).

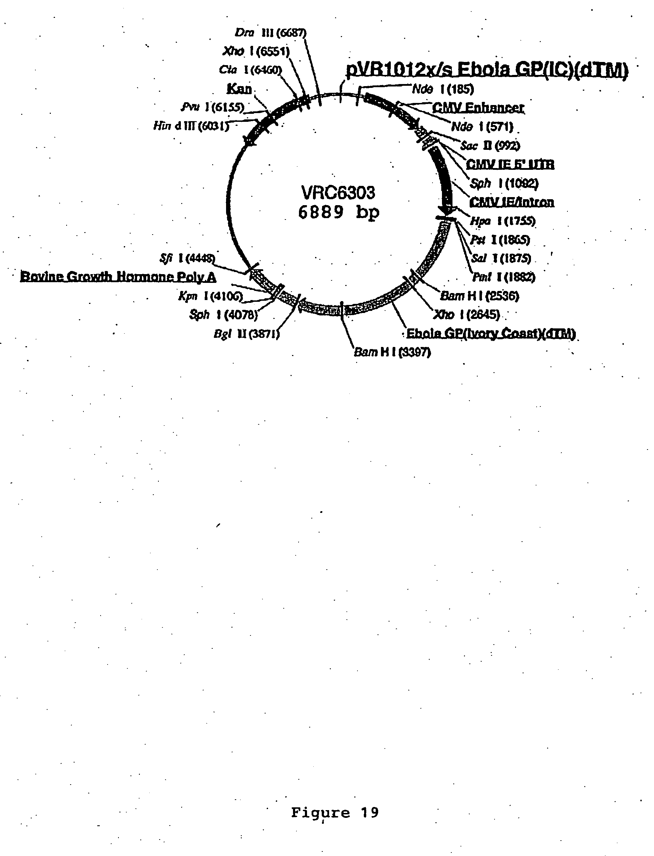

**Figure 19** shows VRC 6303 (pVR1012x/s Ebola GP (IC) (dTM)) construct map (see, Ebola/Marburg/Lassa Plasmids, and Recombinant Adenoviruses in Table 2).

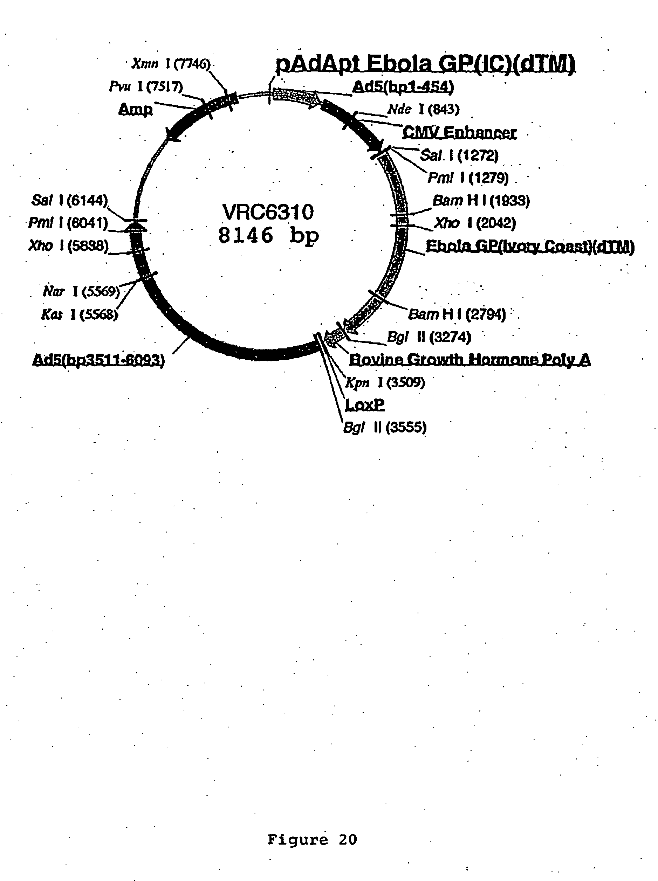

**Figure 20** shows VRC 6310 (pAdApt Ebola GP (IC) (dTM)) construct map (see, Ebola/Marburg/Lassa Plasmids, and Recombinant Adenoviruses in Table 2).

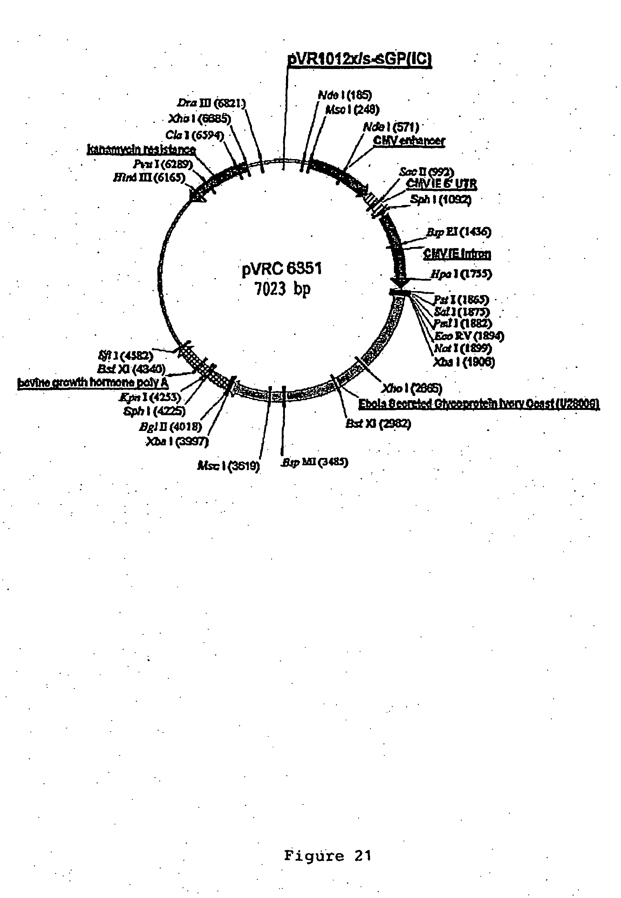

**Figure 21** shows VRC6351 (pVR1012x/s-SGP(IC)) construct map (see, Ebola/Marburg/Lassa Plasmids, and Recombinant Adenoviruses in Table 2).

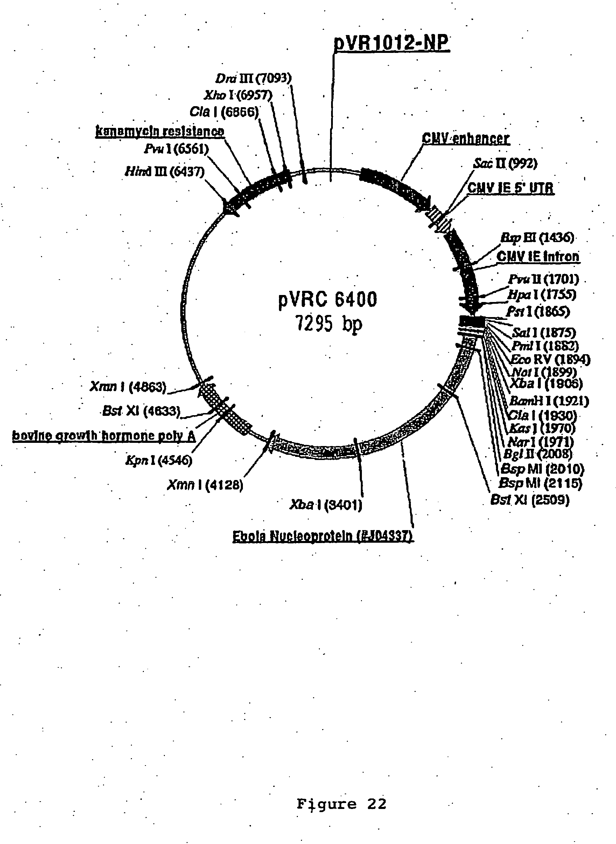

**Figure 22** shows VRC6400 (pVR1012-NP) construct map (see, Ebola/Marburg/Lassa Plasmids, and Recombinant Adenoviruses in Table 2).

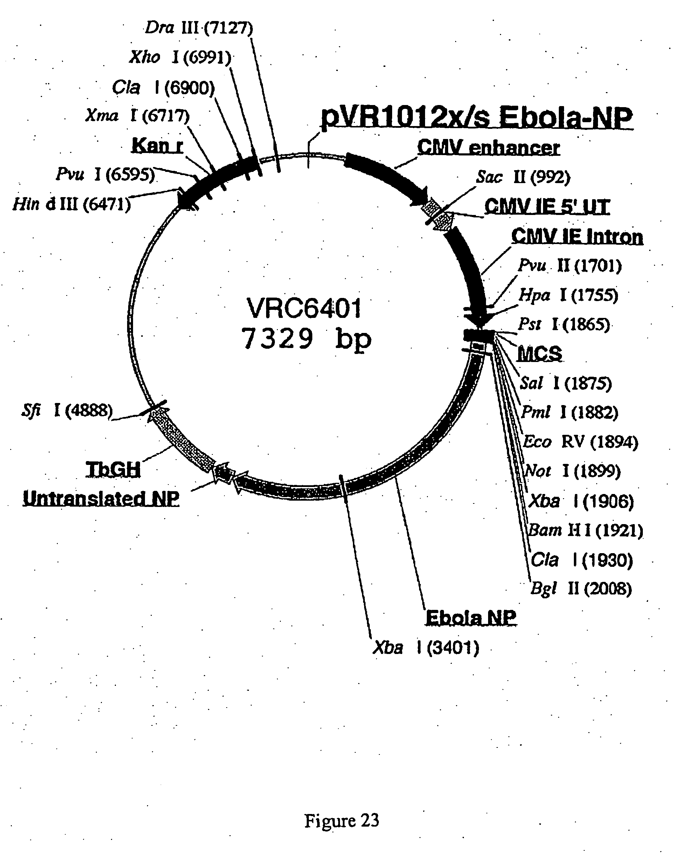

**Figure 23** shows VRC6401 (pVR1012x/s=NP) construct map (see, Ebola/Marburg/Lassa Plasmids, and Recombinant Adenoviruses in Table 2).

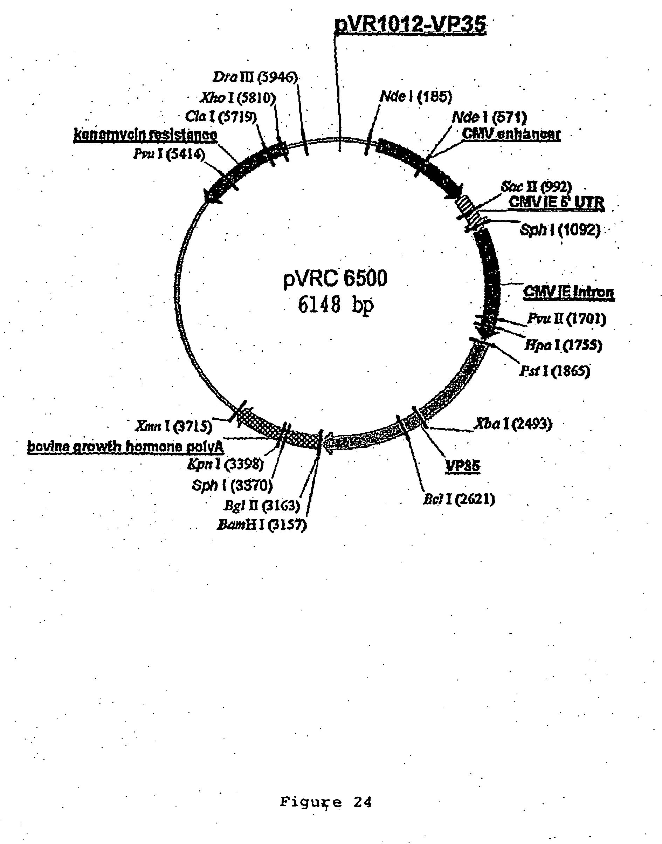

**Figure 24** shows VRC6500 (pVR1012-VP35) construct map (see, Ebola/Marburg/Lassa Plasmids, and Recombinant Adenoviruses in Table 2).

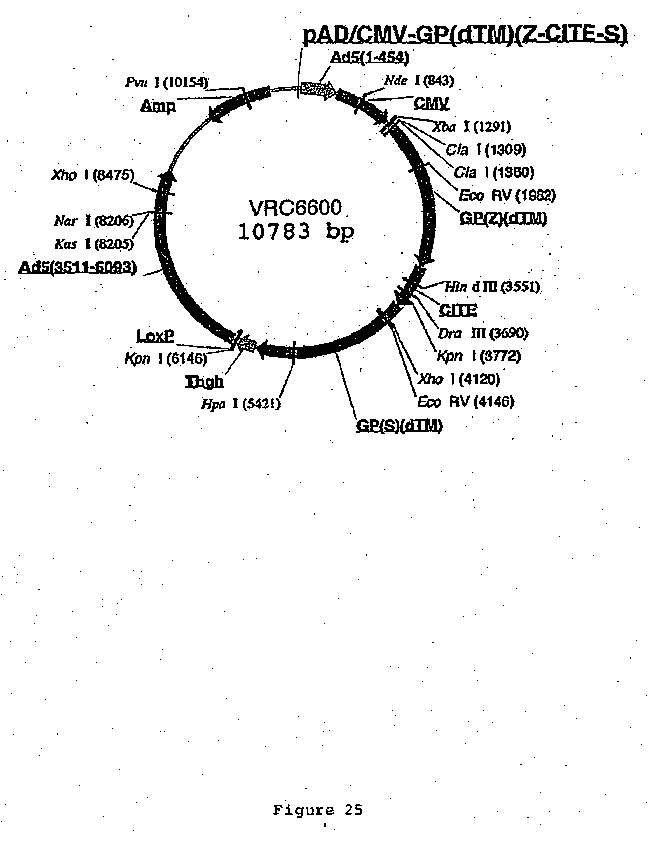

**Figure 25** shows VRC6600 (pAD/CMV-GP(dTM)(Z-CITE-S) construct map (see, Ebola/Marburg/Lassa Plasmids, and Recombinant Adenoviruses in Table 2).

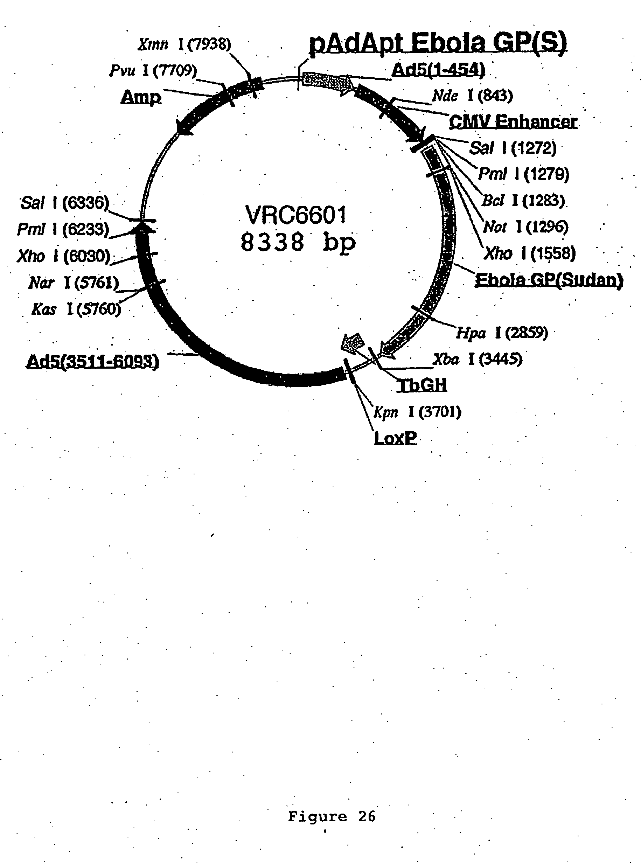

**Figure 26** shows VRC6601 (pAdApt Ebola GP(S)) construct map (see, Ebola/Marburg/Lassa Plasmids, and Recombinant Adenoviruses in Table 2).

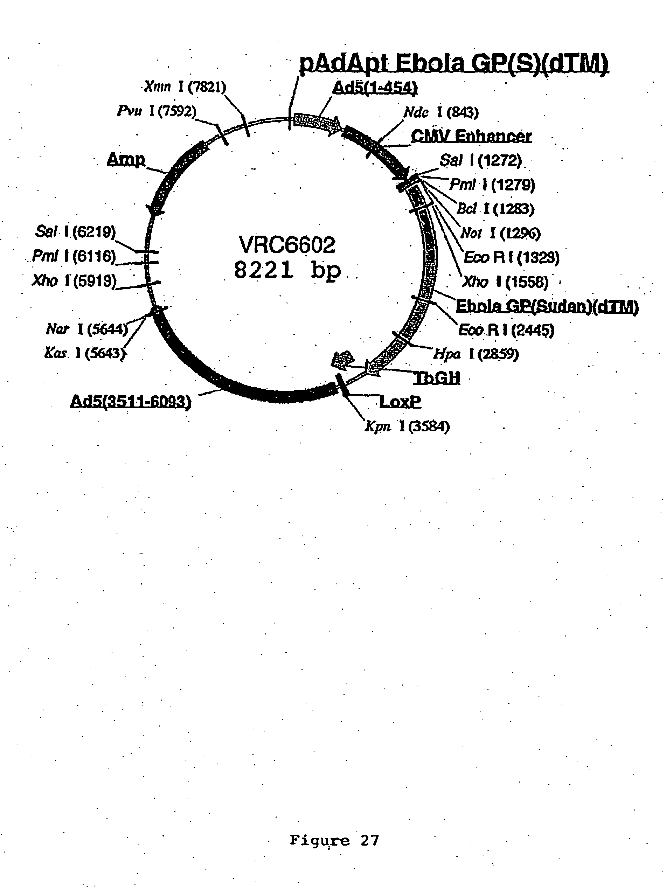

**Figure 27** shows VRC 6602 (pAdApt Ebola GP(S)(dTM)) construct map (see, Ebola/Marburg/Lassa Plasmids, and Recombinant Adenoviruses in Table 2).

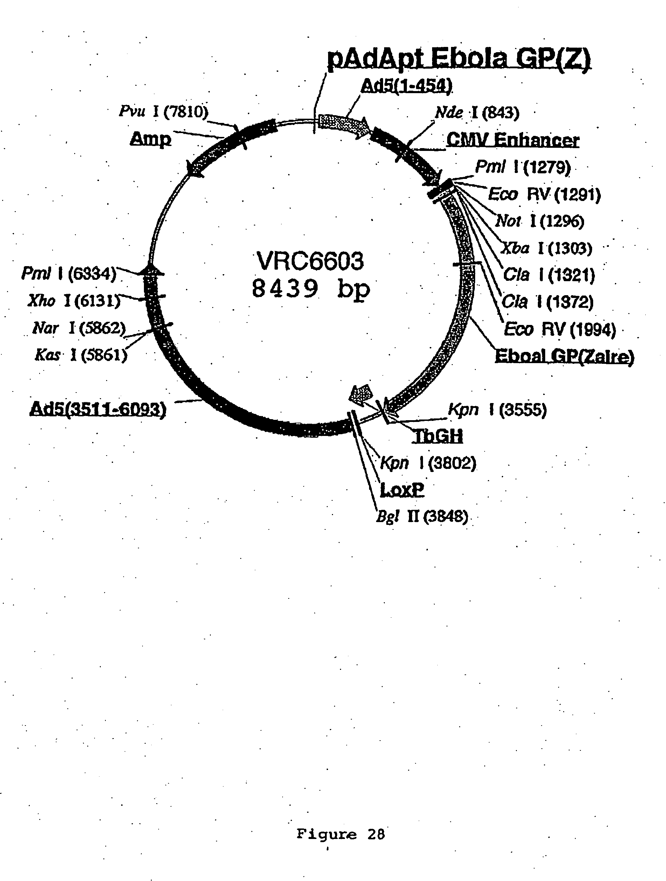

**Figure 28** shows VRC6603 (pAdApt Ebola GP(Z)) construct map (see, Ebola/Marburg/Lassa Plasmids, and Recombinant Adenoviruses in Table 2).

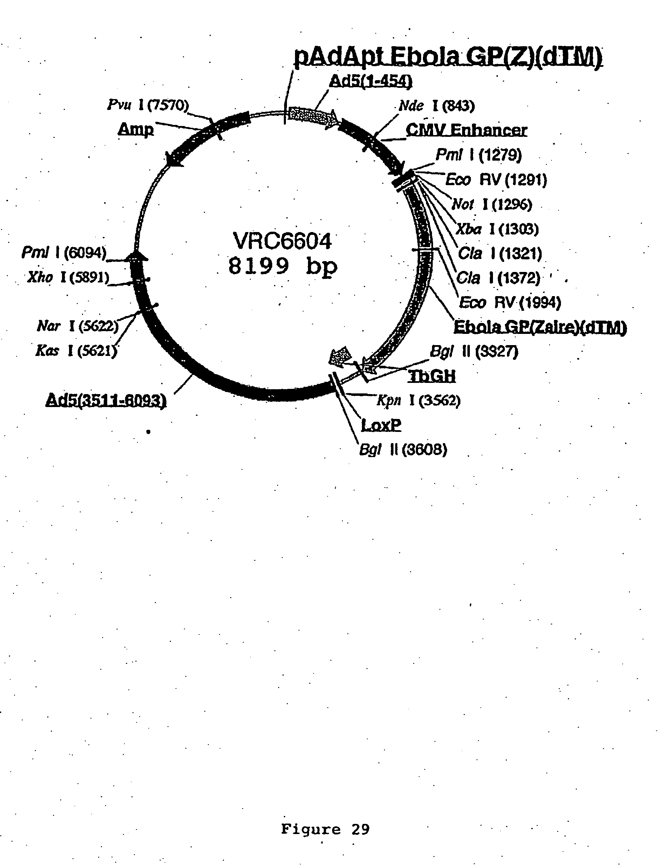

**Figure 29** shows VRC 6604 (pAdApt Ebola GP(Z)(dTM)) construct map (see, Ebola/Marburg/Lassa Plasmids, and Recombinant Adenoviruses in Table 2).

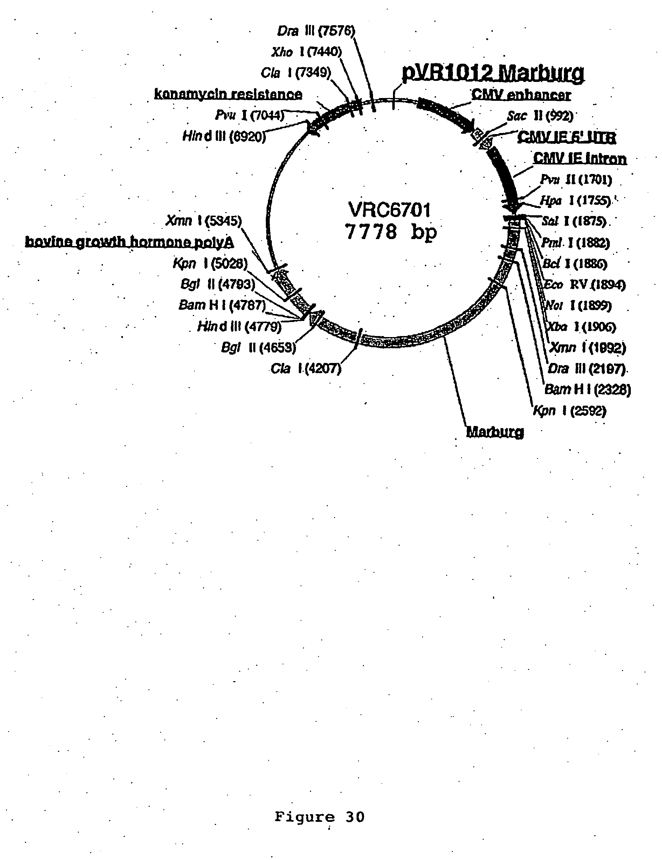

**Figure 30** shows VRC6701 (pVR1012-Marburg) construct map (see, Ebola/Marburg/Lassa Plasmids, and Recombinant Adenoviruses in Table 2).

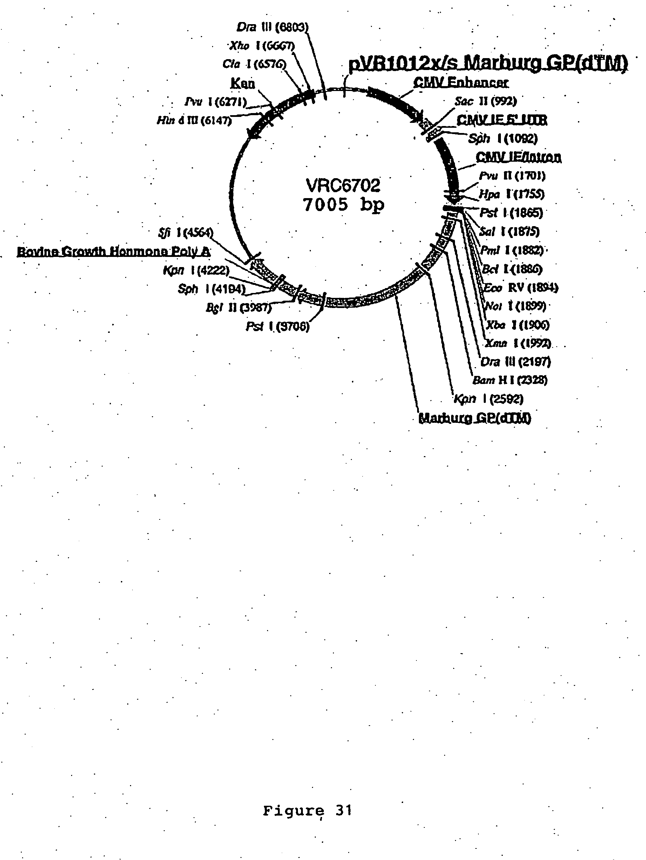

**Figure 31** shows VRC 6702 (pVR1012x/s Marburg GP (dTM)) construct map (see, Ebola/Marburg/Lassa Plasmids, and Recombinant Adenoviruses in Table 2).

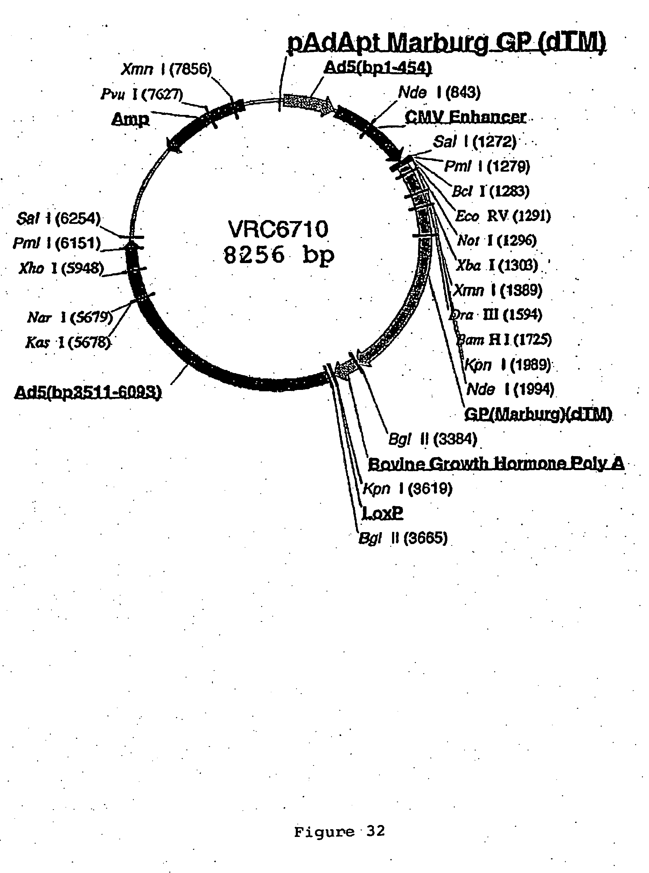

**Figure 32** shows VRC 6710 (pAdApt Marburg GP (dTM)) construct map (see, Ebola/Marburg/Lassa Plasmids, and Recombinant Adenoviruses in Table 2).

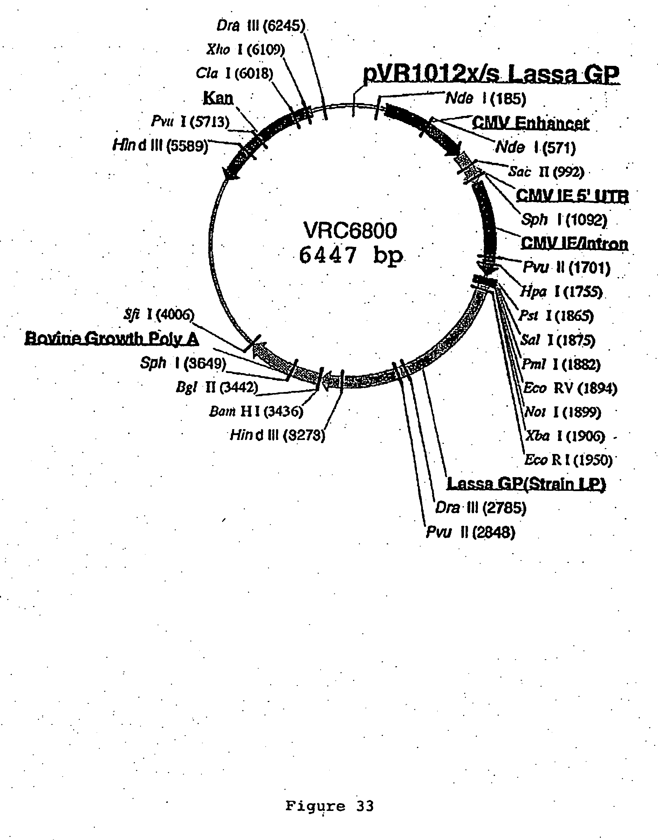

**Figure 33** shows VRC6800 (pVR1012x/s Lassa GP) construct map (see, Ebola/Marburg/Lassa Plasmids, and Recombinant Adenoviruses in Table 2).

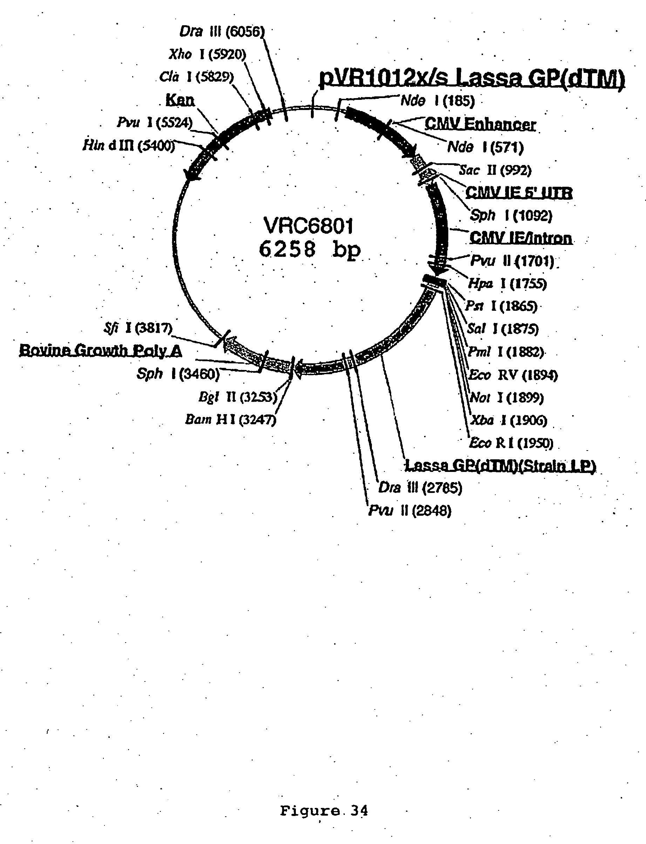

**Figure 34** shows VRC6801 (pVR1012x/s Lassa GP (dTM) construct map (see, Ebola/Marburg/Lassa Plasmids, and Recombinant Adenoviruses in Table 2).

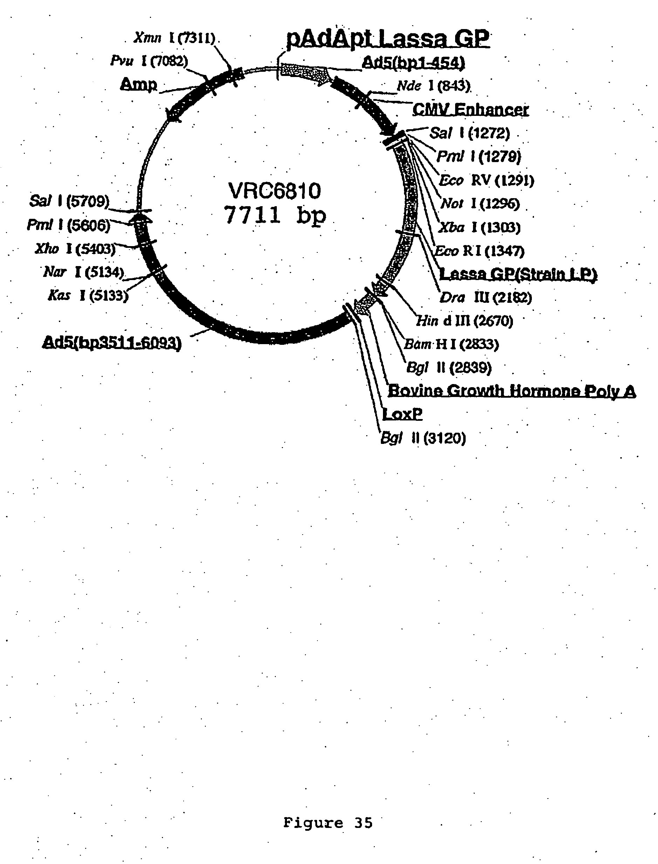

**Figure 35** shows VRC6810 (pAdApt Lassa GP) construct map (see, Ebola/Marburg/Lassa Plasmids, and Recombinant Adenoviruses in Table 2).

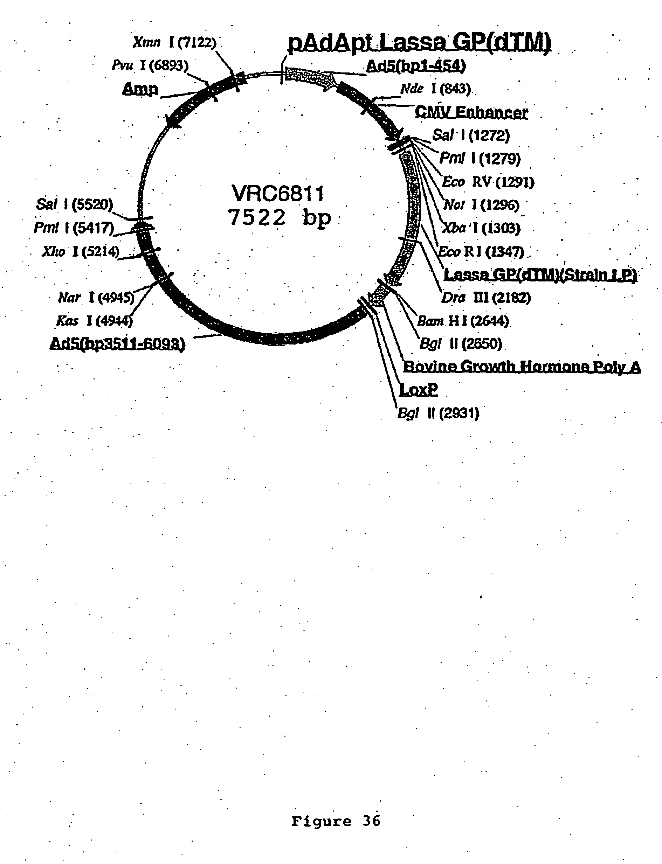

**Figure 36** shows VRC6811 (pAdApt Lassa GP (dTM)) construct map (see, Ebola/Marburg/Lassa Plasmids, and Recombinant Adenoviruses in Table 2).

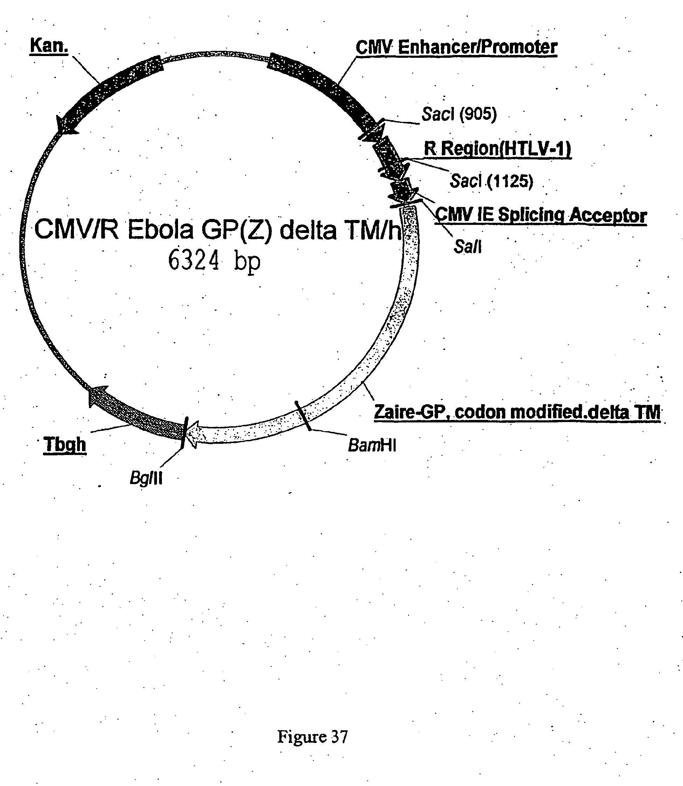

**Figure 37** shows CMV/R Ebola GP (Z) deltaTM/h (codon optimized) construct map (see, Ebola/Marburg/Lassa Plasmids, and Recombinant Adenoviruses in Table 2).

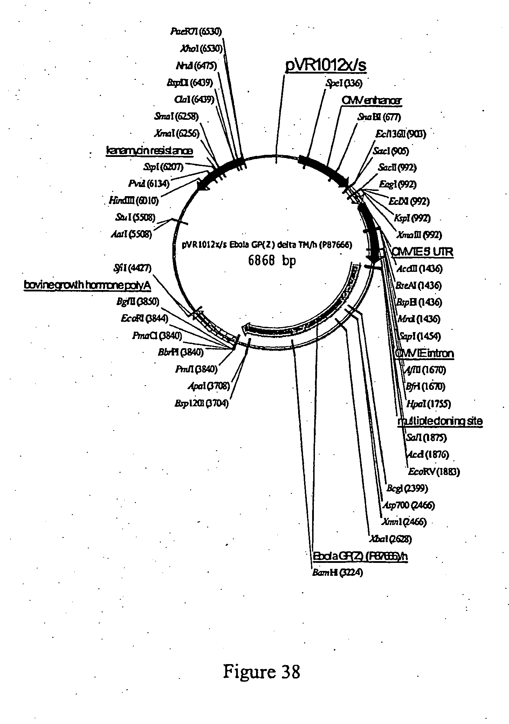

**Figure 38** shows pVR1012 Ebola GP (Z, P87666) delta TM/h (codon optimized) construct map (see, Ebola/Marburg/Lassa Plasmids, and Recombinant Adenoviruses in Table 2).

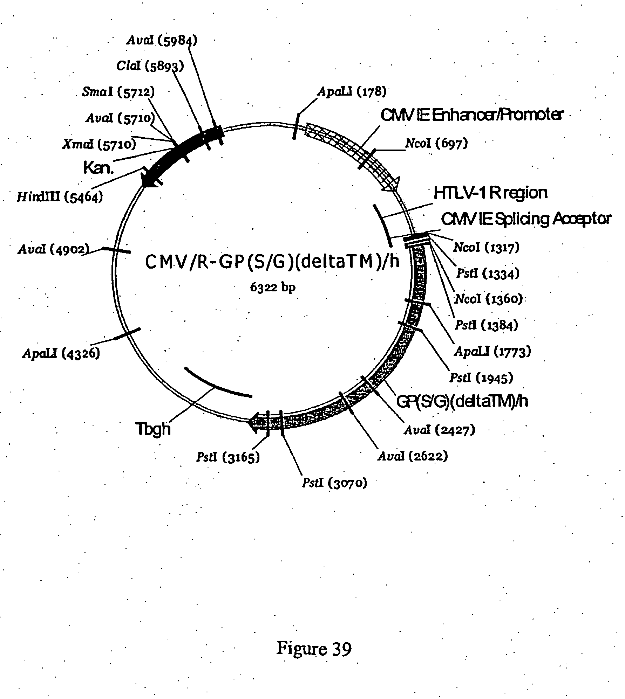

**Figure 39** shows CMV/R Ebola GP .(S/Gulu) delta TM/h (codon optimized) construct map (see, Ebola/Marburg/Lassa Plasmids, and Recombinant Adenoviruses in Table 2).

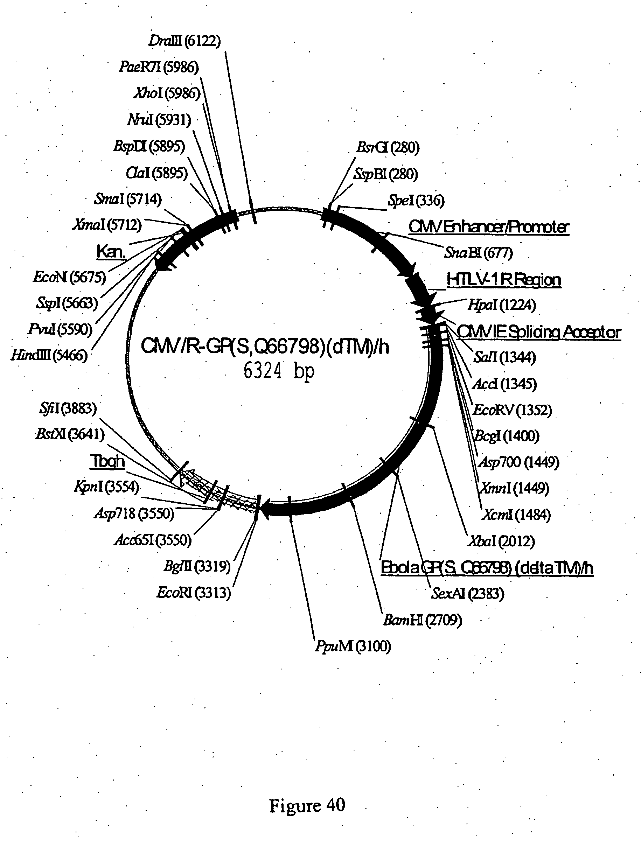

**Figure 40** shows CMV/R Ebola GP (S,Q66798) delta TM/h (codon optimized) construct map (see, Ebola/Marburg/Lassa Plasmids, and Recombinant Adenoviruses in Table 2).

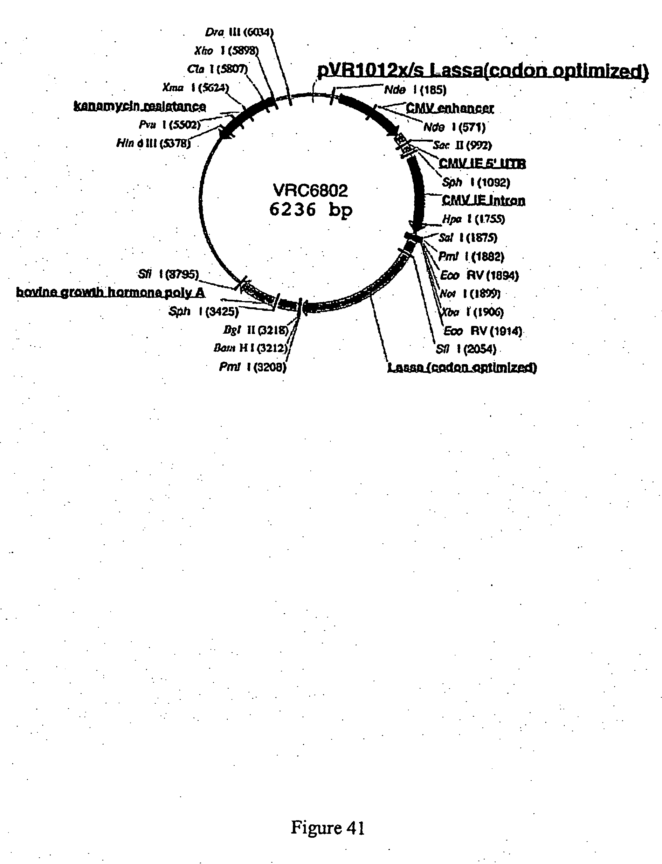

**Figure 41** shows VRC6802, pVR1012x/s Lassa delta TM/h (codon optimized) construct map (see, Ebola/Marburg/Lassa Plasmids, and Recombinant Adenoviruses in Table 2).

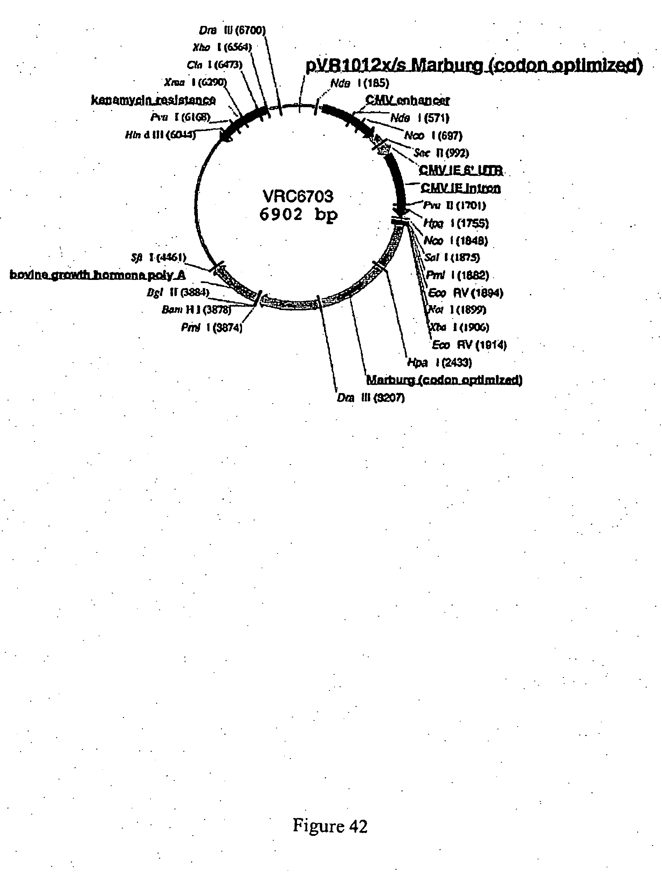

**Figure 42** shows VRC6703, pVR1012x/s Marburg delta TM/h (codon optimized) construct map (see, Ebola/Marburg/Lassa Plasmids, and Recombinant Adenoviruses in Table 2).

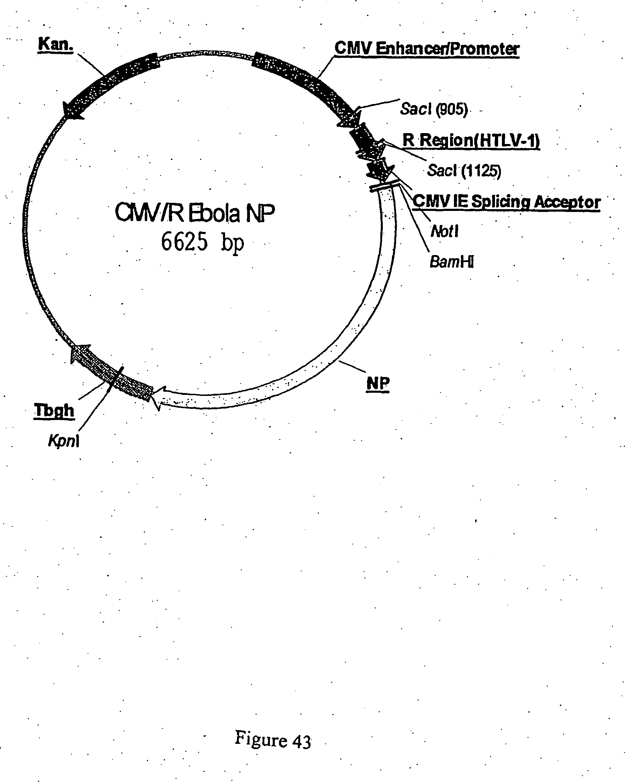

**Figure 43** shows CMV/R Ebola NP construct map (see, Ebola/Marburg/Lassa Plasmids, and Recombinant Adenoviruses in Table 2).

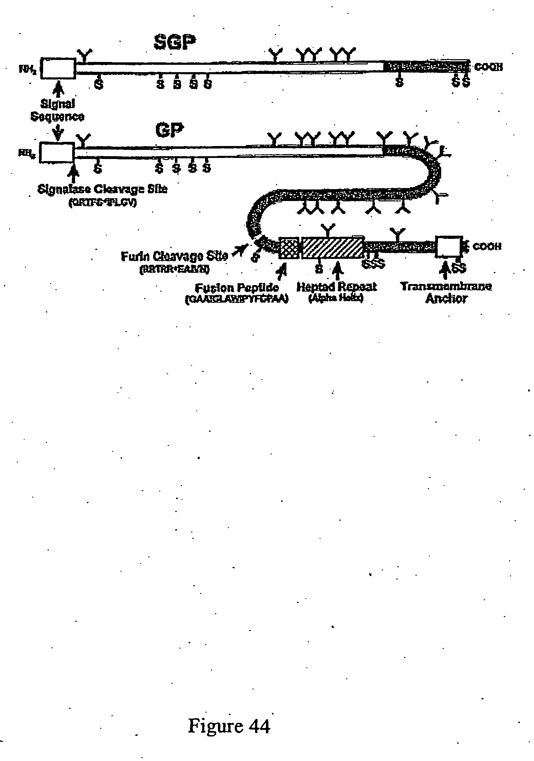

**Figure 44** is a diagrammatic representation of secreted glycoprotein (SGP) and glycoprotein (GP) molecules of Ebola virus (Zaire species isolated in 1976) showing important structural features. The white N-terminal regions of SGP and GP correspond to identical (shared) sequences, while the black C termini identify sequences unique to GP or SGP molecules. The common signalase cleavage sites for both SGP and GP and the furin cleavage site for GP0 (uncleaved form of GP) (↓) were determined by N-terminal sequencing. Also shown are cysteine residues (S), predicted N-linked glycosylation sites (Y-shaped projections), a predicted fusion peptide, a heptad repeat sequence, and a transmembrane anchor sequence. In Ebola viruses, the positions of these structures are conserved and their sequences are very similar or, in the case of N-linked glycosylation sites, are at least concentrated in the central region of GP. Signalase cleavage site is **SEQ ID NO: 48,** Furin cleavage site is **SEQ ID NO: 49,** and Fusion peptide is **SEQ ID NO: 50.**
**Figure 45** is a diagrammatic representation of the structural GP. Shown is the predicted orientation of the GP1-GP2 heterodimer linked by undetermined disulfide bonding (indicated by the question mark). Intramolecular disulfide bonds that are shown come from prior predictions based on similarities to retrovirus glycoprotein structures. See Fig. 44 for other features of the amino acid sequence.
**Figure 46** shows induction of the cytopathic effects by Ebola virus glycoproteins and mapping of the molecular determinants of cytopathicity.
**Figure 47** shows Ebola-specific antibody responses generated by different DNA/adenovirus prime-boost combinations: Data are the means of the reciprocal endpoint dilution for each group of mice and error bars represent the standard deviation.

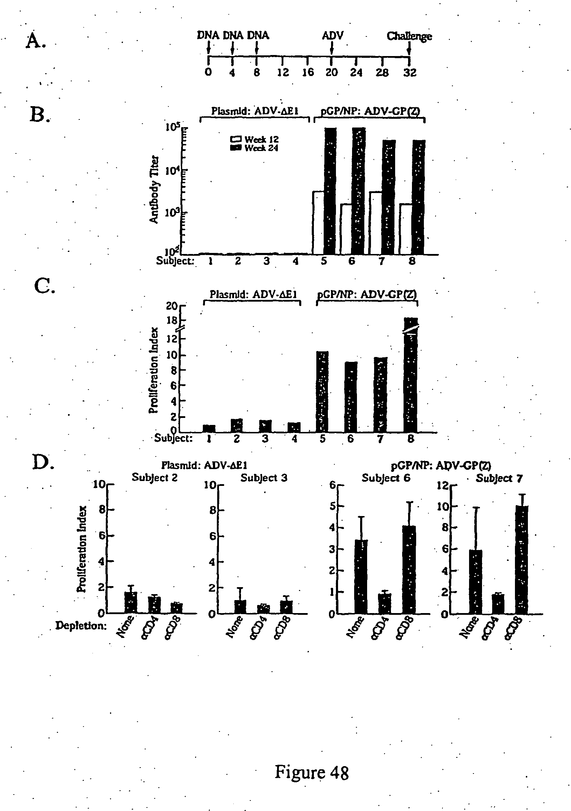

**Figure 48** shows DNA-Adenovirus immunization of cynomolgus macaques. **A)** Immunization schedule for DNA and/or adenovirus injections, and challenge with the wild-type Mayinga strain of the Zaire subtype of Ebola virus. **B)** Elisa titers of Ebola-specific antibodies in serum. Serum was collected at week 12 (open bar) and 2 days before the immunization at week 24 (closed bar). **C)** Lymphoproliferative responses to Ebola-secreted glycoprotein (SGP) following immunization. Bars represent the average fold-proliferation of all four blood samples for each subject. The standard deviation is not shown because the baseline level of induction varied between experiments. However, PBMC from all 8 animals were assayed within the same experiment for each time point, and the averages displayed in the figure are representative of the results obtained for any single time point. **D)** Lymphoproliferative responses to Ebola SGP in bulk PBMC following depletion of lymphocyte subsets. PBMC from week 24 were treated with Dynal magnetic beads coated with the indicated antibody to deplete CD4⁺ or CD8⁺ cell subsets. Cells remaining after depletion were normalized for input cell number and stimulated as described in the Example. Results are shown for two control (Subjects 2 and 3) and two vaccinated (Subjects 6 and 7) monkeys.

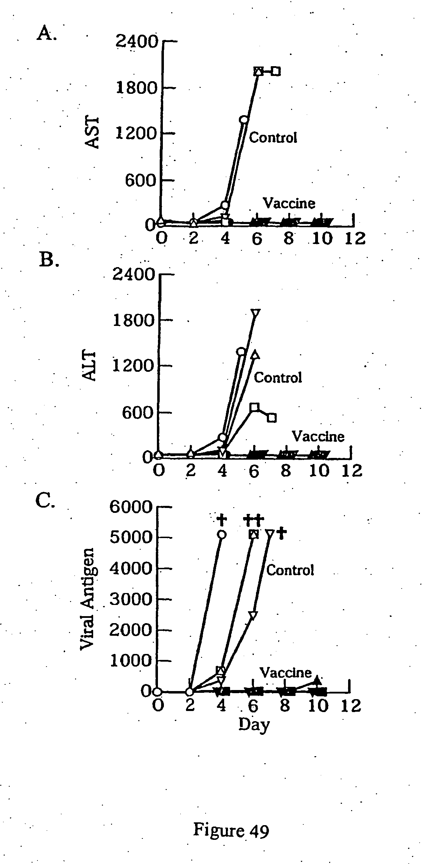

**Figure 49** shows protection of cynomolgus macaques against lethal challenge with Ebola virus after DNA-adenovirus immunization. **A, B)** Hepatic enzyme levels in monkeys after challenge with Ebola virus. Liver enzymes [alanine aminotransferase (ALT) and aspartate aminotransferase (AST)] levels in the non-human primate sera were measured by standard recommended procedures using General chemistry 12 reagent disk for the Piccolo^{™} Analyzer (Abaxis, Inc., Sunnyvale, CA). Results are shown for four immunized (closed symbols) and four control (open symbols) monkeys. **C)** Plasma viraemia in monkeys following infection with Ebola virus. Crosses represent time of death in control animals [days 5 (subject 1) and 6 (subjects 2 and 4)]. One control animal, subject 3, was euthanized on day 7 when it was moribund. One vaccinated animal that was resistant to infection, subject 5, was euthanized on day 10 for histological examination of tissues. By day 17, none of the animals had detectable viraemia, and they remained aviraemic for the duration of the observation period (6 months). Data are the reciprocal endpoint dilution of serum for each monkey. Results are shown for four immunized (closed symbols) and four control (open symbols) monkeys.
**Figure 50** shows enhanced expression of modified CMV expression vector, CMV/R.
**Figure 51** shows enhanced immunogenicity of modified CMV expression vector, CMV/R, in mice.

**Table 1 Ebola/Marburg/Lassa GenBank Accession Numbers.**

| **Gene** | **GenBank Accession number** |
|---|---|
| Ebola Zaire GP | U23187, P87666 |
| Ebola Zaire NP | J04337 |
| Ebola Sudan GP | U28134, Q66798 |
| Ebola Sudan NP | AF173836 |
| Ebola Ivory Coast GP | U28006 |
| Ebola Ivory Coast NP | JO4336 |
| Ebola Reston GP | U23152 |
| Ebola Reston NP | |
| Marburg GP | Z12132 |
| Marburg NP | X68495 |
| Lassa GP | AF181853 |
| Lassa NP | AF246121 |

**Table 2. Ebola/Marburg/Lassa Plasmids, and Recombinant Adenoviruses**

| **Construct** | **Construct Name/Description** | **Construct Map Name** | **SEQ ID NO** | **Figure** |
|---|---|---|---|---|
| VRC6000 | VRC6000 (pVR1012-GP(Z)) | pVR1012-GP(Z) | 1 | 1 |
| VRC6001 | VRC6001 (pVR1012x/s-GP(Z)) | pVR1012x/s Ebola GP(Z) | 2 | 2 |
| VRC6002 | VRC6002 (pVR1012-GP(Z) delta MUC) | pVR1012-GP(Z) delta MUC | 3 | 3 |
| VRC6003 | VRC6003 (pVR1012-GP(Z) delta MUC delta FUR) | pVR1012-GP(Z) delta MUC delta FUR | 4 | 4 |
| VRC6004 | VRC6004 (pVR1012-GP(Z) delta GP2) | pVR1012-GP(Z) delta GP2 | 5 | 5 |
| VRC6005 | VRC6005 (pVR1012-GP(Z) delta GP2 delta C-term A) | pVR1012-GP(Z) delta GP2 delta C-term A | 6 | 6 |
| VRC6006 | VRC6006 (pVR1012-GP(Z) delta GP2 delta C-term B) | pVR1012-GP(Z) delta GP2 delta C-term B | 7 | 7 |
| VRC6007 | VRC6007 (pVR1012-GP(Z) delta GP2 delta FUS) | pVR1012-GP(Z) delta GP2 delta FUS | 8 | 8 |
| VRC6008 | VRC6008 (pVR1012-GP(Z) delta TM) | pVR1012-GP(Z) delta TM | 9 | 9 |
| VRC6052 | VRC 6052 (pVR1012-GP(Z) delta SGP) | pVR1012-GP(Z) delta SGP | 10 | 10 |
| VRC6101 | VRC 6101 (pVR1012x/s Ebola GP(R) (dTM)) | pVR1012x/s Ebola GP(R)(dTM) | 11 | 11 |
| VRC6110 | VRC 6110 (pAdApt Ebola GP(R) (dTM)) | pAdApt Ebola GP(R) (dTM) | 12 | 12 |
| VRC6200 | VRC6200 (pVR1012-GP(S)) | pVR1012-GP(S) | 13 | 13 |
| VRC6201 | VRC 6201 (pVR1012x/s Ebola GP(S)) | pVR1012x/s Ebola GP(S) | 14 | 14 |
| VRC6202 | VRC6202 (pVR1012-GP(S) delta TM) | pVR1012-GP(S) delta TM | 15 | 15 |
| VRC6300 | VRC6300 (pVR1012-GP(IC)) | pVR1012-GP(IC). | 16 | 16 |
| VRC6301 | VRC6301 (pVR1012x/s-GP(IC)) | pVR1012x/s Ebola GP(IC) | 17 | 17 |
| VRC6302 | VRC6302 (pVR1012-GP(IC) delta TM) | pVR1012-GP(IC) delta TM | 18 | 18 |
| VRC6303 | VRC 6303 (pVR1012x/s Ebola GP (IC) (dTM)) | pVR1012x/s Ebola GP(IC)(dTM) | 19 | 19 |
| VRC6310 | VRC 6310 (pAdApt Ebola GP (IC) (dTM)) | pAdApt Ebola GP(IC)(dIM) | 20 | 20 |
| VRC6351 | VRC6351 (pVR1012x/s-sGP(IC)) | pVR1012x/s-sGP(IC) | 21 | 21 |
| VRC6400 | VRC6400 (pVR1012-NP) | pVR1012-NP | 22 | 22 |
| VRC6401 | VRC6401 (pVR1012x/s-NP) | pVR1012x/s Ebola-NP | 23 | 23 |
| VRC6500 | VRC6500 (pVR1012-VP35) | pVR1012-VP35 | 24 | 24 |
| VRC6600 | VRC6600 (pAD/CMV-GP(dTM)(Z-CITE-S) | pAD/CMV-GP(dTM)(Z-CITE-S) | 25 | 25 |
| VRC6601 | VRC6601 (pAdApt Ebola GP(S)) | pAdApt Ebola GP(S) | 26 | 26 |
| VRC6602 | VRC 6602 (pAdApt Ebola GP(S)(dTM)) | pAdApt Ebola GP(S)(dTM) | 27 | 27 |
| VRC6603 | VRC6603 (pAdApt Ebola GP(Z)) | pAdApt Ebola GP(Z) | 28 | 28 |
| VRC6604 | VRC 6604 (pAdApt Ebola GP(Z)(dTM)) | pAdApt Ebola GP(Z)(dTM) | 29 | 29 |
| VRC6701 | VRC6701 (pVR1012-Marburg) | pVR1012 Marburg | 30 | 30 |
| VRC6702 | VRC 6702 (pVR1012x/s Marburg GP (dTM)) | pVR1012x/s Marburg GP(dTM) | 31 | 31 |
| VRC6710 | VRC 6710 (pAdApt Marburg GP (dTM)) | pAdApt Marburg GP (dTM) | 32 | 32 |
| VRC6800 | VRC6800 (pVR1012x/s Lassa GP) | pVR1012x/s Lassa GP | 33 | 33 |
| VRC6801 | VRC6801 (pVR1012x/s Lassa GP (dTM) | pVR1012x/s Lassa GP (dTM) | 34 | 34 |
| VRC6810 | VRC6810 (pAdApt Lassa GP) | pAdApt Lassa GP | 35 | 35 |
| VRC6811 | VRC6811 (pAdApt Lassa GP (dTM)) | pAdApt Lassa GP (dTM) | 36 | 36 |
| | CMV/R Ebola GP (Z) deltaTM/h (codon optimized) | CMV/R Ebola GP(Z) delta TM/h | 37 | 37 |
| | pVR1012 EbolaGP(Z, P87666)delta TM/h (codon optimized) | pVR1012x/s Ebola GP(Z) delta TM/h (P87666) | 38 | 38 |
| | CMV/R Ebola GP (S/Gulu) delta TM/h (codon optimized) | CMV/R-GP(S/G)(deltaTM)/h | 39 | 39 |
| | CMV/R Ebola GP (S,Q66798) delta TM/h (codon optimized) | CMV/R-GP(S,Q66798)(dTM)/h | 40 | 40 |
| VRC6802 | VRC6802, pVR1012x/s Lassa delta TM/h (codon optimized) | pVR1012x/s Lassa (codon optimized) | 41 | 41 |
| VRC6703 | VRC6703, pVR1012x/sMarburgdeltaTM/h (codon optimized) | PVR1012x/s Marburg (codon optimized) | 42 | 42 |
| | CMV/R Ebola NP | CMV/R Ebola NP | 43 | 43 |

### Detailed Description of the Invention

The present invention provides an expression vector comprising SEQ ID NO: 52 or analog thereof having at least 95, 96, 97, 98, 99 or 100% sequence identity thereto that mediates expression, wherein the percentage of identity is calculated over the full length of the reference nucleotide sequence. Further described herein are filovirus vaccines comprising a nucleic acid molecule encoding a filoviral structural protein operatively-linked to a control sequence in a pharmaceutically acceptable excipient. In one aspect described herein, the nucleic acid molecule encodes the transmembrane form of the viral glycoprotein (GP). In the alternative, the nucleic acid molecule encodes the secreted form of the viral glycoprotein (SGP). In another alternative, the nucleic acid molecule encodes the viral nucleoprotein (NP).

Further described herein are vaccines comprising nucleic acid molecules encoding filoviral structural proteins other than GP, SGP, and NP, e.g., other structural gene products which elicit an immune response against a filovirus or disease caused by infection with filovirus. The nucleic acid molecules of the vaccines described herein encode structural gene products of any Ebola viral strain including the Zaire, Sudan, Ivory Coast and Reston strains. Nucleic acid molecules encoding structural gene products of the genetically-related Marburg virus strains may also be employed. Moreover, the nucleic acid molecules described herein may be modified, e.g., the nucleic acid molecules set forth herein may be mutated, as long as the modified expressed protein elicits an immune response against a pathogen or disease. For example, the nucleic acid molecule may be mutated so that the expressed protein is less toxic to cells. Also described herein are vaccines comprising a combination of nucleic acid molecules. For example, and without limitation, nucleic acid molecules encoding GP, SGP and NP of the Zaire, Sudan and Ivory Coast Ebola strains may be combined in any combination, in one vaccine composition.

Also described herein are methods for immunizing a subject against disease caused by infection with filovirus comprising administering to the subject an immunoeffective amount of a filovirus vaccine. Methods of making and using filovirus vaccines are also described herein including the preparation of pharmaceutical compositions.

### Biochemical Analysis of Secreted and Virion Glycoproteins of Ebola Virus.

Ebola (EBO) viruses are members of the *Filoviridae* and cause a severe, often fatal form of hemorrhagic fever disease in human and/or non-human primates. The glycoprotein (GP) gene of filoviruses is the fourth gene (of seven) from the 3' end of the negative-strand RNA genome. All EBO viruses characterized thus far have the same unconventional type of GP gene organization that results in the expression of a secreted, non structural glycoprotein (SGP) in preference to the structural GP. The SGP is encoded in a single frame (0 frame), while the GP is encoded in two frames (0 and -1 frames). Expression of the GP occurs when the two frames are connected through a transcriptional editing event that results in the insertion of a single extra adenosine (added to a run of seven adenosines).

Referring to Figure 44, for Zaire species of EBO virus, the N-terminal 295 residues (including signal sequence) of the SGP (364 total residues) and GP (676 total residues) are identical, but the length and composition of their C-terminal sequences are unique. The GP, a type 1 transmembrane protein, is found on the surface of the infectious virion and functions in attachment structure in the binding and entry of the virus into susceptible cells. Comparisons of GP predicted amino acid sequences for all species of EBO virus show a general conservation in the N-terminal and C-terminal regions (each approximately one-third of the total sequence) and are separated by a highly variable middle section. This protein is highly glycosylated, containing large amounts of N- and O-linked glycans, and for Marburg (MBG) virus (another type of filovirus) has been shown to form trimers. Just N terminal to the transmembrane anchor sequence of the GP (residues 650 to 672) is a motif (residues 585 to 609) that is highly conserved in filoviruses. This sequence also has a high degree of homology with a motif in the glycoproteins of oncogenic retroviruses that has been shown to be immunosuppressive in *vitro.* Partially overlapping this motif is a heptad repeat sequence (53 residues; positions 541 to 593) that is thought to function in the formation of intermolecular coiled coils in the assembly of trimers, similar to structures predicted for the surface glycoproteins of other viruses. Immediately N terminal to this sequence is a predicted fusion peptide followed closely by a putative multibasic cleavage site for a subtilisin/kexin-like convertase, furin. Cleavage by furin has been indirectly demonstrated by use of specific inhibitors and is predicted to occur at the last arginine in the sequence RRTRR↓ (position 501 from the beginning of the open reading frame [ORF]). Although the role of the SGP is less defined, recent studies have shown that SGP can bind to neutrophils, while GP binds to endothelial cells. The different binding patterns of SGP and GP suggest that despite having identical N-terminal amino acid sequences (-280 residues), these glycoproteins are structurally very distinct from one another.

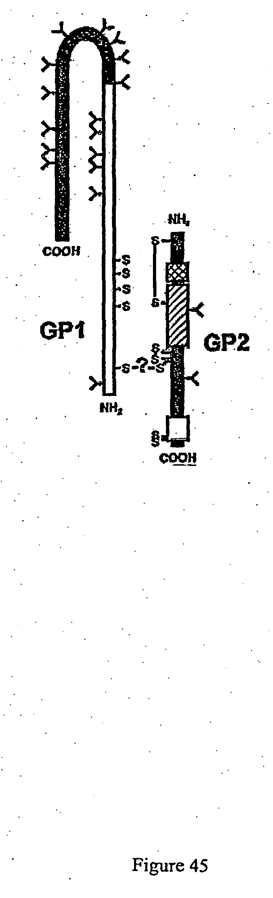

Referring to Figure 45, the glycoproteins expressed by a Zaire species of Ebola virus were analyzed for cleavage, oligomerization, and other structural properties to better define their functions. The 50- to 70-kDa secreted and 150-kDa virion/structural glycoproteins (SGP and GP, respectively), which share the 295 N-terminal residues, are cleaved near the N terminus by signalase. A second cleavage event, occurring in GP at a multibasic site (RRTRR↓) **(SEQ ID NO: 51)** that is likely mediated by furin, results in two glycoproteins (GP1 and GP2) linked by disulfide bonding. This furin cleavage site is preset in the same position in the GPs of all Ebola viruses (R[R/K]X[R/K]R↓), and one is predicted for Marburg viruses (R[R/K)KR↓), although in a different location. Based on the results of cross-linking studies, investigators were able to determined that Ebola virion peplomers are composed of trimers of GP1-GP2 heterodimers and that aspects of their structure are similar to those of retroviruses (including lentiviruses like HIV-1 and HIV-2), paramyxoviruses, and influenza viruses. Investigators also determined that SGP is secreted from infected cells almost exclusively in the form of a homodimer that is joined by disulfide bonding.

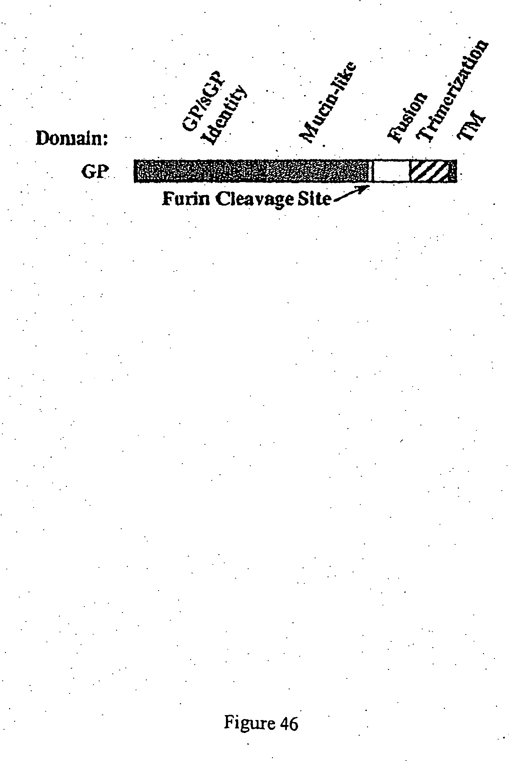

Referring to Figure 46, investigators defined the main viral determinant of Ebola virus pathogenicity; synthesis of the virion glycoprotein (GP) of Ebola virus Zaire induced cytotoxic effects in human endothelial cells *in vitro* and *in vivo.* This effect mapped to a serine-threonine-rich, mucin-like domain of this type I transmembrane glycoprotein, one of seven gene products of the virus. Gene transfer of GP into explanted human or porcine blood vessels caused massive endothelial cell loss within 48 hours that led to a substantial increase in vascular permeability. Deletion of the mucin-like region of GP abolished these effects without affecting protein expression or function. GP derived from the Reston strain of virus, which causes disease in non-human primates but not in man, did not disrupt the vasculature of human blood vessels. In contrast, the Zaire GP induced endothelial cell disruption and cytotoxicity in both non-human primate and human blood vessels, and the mucin domain was required for this effect. These findings indicate that GP, through its mucin domain, is the viral determinant of Ebola pathogenicity and likely contributes to hemorrhage during infection.

### Nucleic Acid Molecules

As indicated herein, nucleic acid molecules of the present invention may be in the form of RNA or in the form of DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment. For example, recombinant DNA molecules contained in a vector are considered isolated for the purposes further described herein. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules described herein. Isolated nucleic acid molecules described herein further include such molecules produced synthetically.

Nucleic acid molecules described herein include DNA molecules comprising an open reading frame (ORF) encoding a wild-type filovirus structural gene product; and DNA molecules which comprise a sequence substantially different from those described above but which, due to the degeneracy of the genetic code, still encode an ORF of a wild-type filovirus structural gene product. Of course, the genetic code is well known in the art.

Also described herein are fragments of the nucleic acid molecules described herein. By a fragment of a nucleic acid molecule having the nucleotide sequence of an ORF encoding a wild-type filovirus structural gene product is intended fragments at least about 15 nt., and more preferably at least about 20 nt., still more preferably at least about 30 nt., and even more preferably, at least about 40 nt. in length. Of course, larger fragments 50, 100, 150, 200, 250, 300, 350, 400, 450, or 500 nt. in length are also intended to be described herein as are fragments corresponding to most, if not all, of the nucleotide sequence of the ORF encoding a wild-type filovirus structural gene product. By a fragment at least 20 nt. in length, for example, is intended fragments which include 20 or more contiguous bases from the nucleotide sequence of the ORF of a wild-type filovirus structural gene product.

Preferred nucleic acid fragments described herein include nucleic acid molecules encoding epitope-bearing portions of the filovirus structural protein. In particular, such nucleic acid fragments described herein include nucleic acid molecules encoding epitope-bearing domains of a filovirus structural protein, where the domain is the GP/SGP identity domain, the mucin-like domain, the furin cleavage site, the fusion peptide domain, the heptad repeat domain, the transmembrane anchor domain, and the intracellular domain, and any combination thereof, for example, a filovirus glycoprotein having a truncation at the carboxy terminus to delete the transmembrane anchor and intracellular domain, a filovirus glycoprotein having a truncation at the carboxy terminus to delete the heptad repeat domain and transmembrane anchor and intracellular domain, a filovirus glycoprotein having a truncation at the carboxy terminus to delete the fusion peptide domain, heptad repeat domain, and transmembrane anchor and intracellular domain, a filovirus glycoprotein having a truncation at the carboxy terminus to delete the furin cleavage site, fusion peptide domain, heptad repeat domain, and transmembrane anchor and intracellular domain, a filovirus glycoprotein having a truncation at the carboxy terminus to delete the mucin-like domain, furin cleavage site, fusion peptide domain, heptad repeat domain, and transmembrane anchor and intracellular domain. Another example is a filovirus glycoprotein having an amino, internal, or carboxy deletion to delete the mucin-like domain, the furin cleavage site, the fusion peptide domain, the heptad repeat domain, the transmembrane anchor domain, or the intracellular domain.

In another aspect, described herein is a nucleic acid molecule comprising a polynucleotide which hybridizes under stringent hybridization conditions to a portion of the polynucleotide in a nucleic acid molecule as described above. By "stringent hybridization conditions" is intended overnight incubation at 42°C in a solution comprising: 50% formamide, 5 x SSC (750 mM NaCl, 75 mM trisodium citrate), 50 mM sodium phosphate (pH 7.6), 5 x Denhardt's solution, 10% dextran sulfate, and 20 µg/ml denatured, sheared salmon sperm DNA, followed by washing the filters in 0.1 x SSC at about 65°C.

By a polynucleotide which hybridizes to a "portion" of a polynucleotide is intended a polynucleotide (either DNA or RNA) hybridizing to at least about 15 nucleotides (nt.), and more preferably at least about 20 nt., still more preferably at least about 30 nt., and even more preferably about 30-70 nt. of the reference polynucleotide.

By a portion of a polynucleotide of "at least 20 nt. in length," for example, is intended 20 or more contiguous nucleotides from the nucleotide sequence of the reference polynucleotide. Of course, a polynucleotide which hybridizes only to a poly A sequence or a complementary stretch of T (or U) residues, would not be included in a polynucleotide of the invention used to hybridize to a portion of a nucleic acid of the invention, since such a polynucleotide would hybridize to any nucleic acid molecule containing a poly A stretch or the complement thereof (e.g., practically any double-stranded cDNA clone).

As indicated herein, nucleic acid molecules described herein which encode a filovirus structural gene product may include, but are not limited to those encoding the amino acid sequence of the full-length polypeptide, by itself, the coding sequence for the full-length polypeptide and additional sequences, such as those encoding a leader or secretory sequence, such as a pre-, or pro- or prepro-protein sequence, the coding sequence of the full-length polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing, including splicing and polyadenylation signals, for example, ribosome binding and stability of mRNA; and additional coding sequence which codes for additional amino acids, such as those which provide additional functionalities.

Further described herein are variants of the nucleic acid molecules described herein, which encode portions, analogs or derivatives of the filovirus structural gene product. Variants may occur naturally, such as a natural allelic variant. By an "allelic variant" is intended one of several alternate forms of a gene occupying a given locus on a genome of an organism. (Genes II, Lewin, B., ed., John Wiley & Sons, 1985 New York). Non-naturally occurring variants may be produced using art-known mutagenesis techniques.

Such variants include those produced by nucleotide substitutions, deletions or additions, which may involve one or more nucleotides. The variants may be altered in coding regions, non-coding regions, or both. Alterations in the coding regions may produce conservative or non-conservative amino acid substitutions, deletions or additions. Especially preferred among these are silent substitutions, additions and deletions, which do not alter the properties and activities of the filovirus structural gene product or portions thereof. Also especially preferred in this regard are conservative substitutions.

Further described herein are nucleic acid molecules comprising a polynucleotide having a nucleotide sequence at least 95% identical, and more preferably at least 96%, 97%, 98% or 99% identical to a nucleotide sequence encoding a polypeptide having the amino acid sequence of a wild-type filovirus structural gene product or fragment thereof or a nucleotide sequence complementary thereto.

By a polynucleotide having a nucleotide sequence at least, for example, 95% "identical" to a reference nucleotide sequence encoding a filovirus structural gene product is intended that the nucleotide sequence of the polynucleotide is identical to the reference sequence except that the polynucleotide sequence may include up to five point mutations per each 100 nucleotides of the reference nucleotide sequence encoding the Ebola virus structural gene product. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 95%, 96%, 97%, 98% or 99% identical to the reference nucleotide sequence can be determined conventionally using known computer programs such as the Bestfit program (Wisconsin Sequence Analysis Package, Version 8 for Unix, Genetics Computer Group, University Research Park, 575 Science Drive, Madison, Wis. 53711). Bestfit uses the local homology algorithm of Smith and Waterman, 1981 Advances in Applied Mathematics 2:482-489, to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

Described herein are nucleic acid molecules at least 95%, 96%, 97%, 98% or 99% identical to the nucleic acid sequences shown herein in the Sequence Listing which encode a polypeptide having Ebola, Marburg, or Lassa virus polypeptide activity. By "a polypeptide having Ebola, Marburg, or Lassa virus polypeptide activity" is intended polypeptides exhibiting Ebola, Marburg, or Lassa virus polypeptide activity in a particular biological assay. For example, GP, SGP or NP protein activity can be measured for changes in immunological character by an appropriate immunological assay.

Of course, due to the degeneracy of the genetic code, one of ordinary skill in the art will immediately recognize that a large number of the nucleic acid molecules having a sequence at least 95%, 96%, 97%, 98%, or 99% identical to a nucleic acid sequences shown herein in the Sequence Listing will encode a polypeptide "having Ebola, Marburg, or Lassa virus polypeptide activity". In fact, since degenerate variants of these nucleotide sequences all encode the same polypeptide, this will be clear to the skilled artisan even without performing the above described comparison assay. It will be further recognized in the art that, for such nucleic acid molecules that are not degenerate variants, a reasonable number will also encode a polypeptide having Ebola, Marburg, or Lassa virus polypeptide activity. This is because the skilled artisan is fully aware of amino acid substitutions that are either less likely or not likely to significantly effect protein function (e.g., replacing one aliphatic amino acid with a second aliphatic amino acid).

For example, guidance concerning how to make phenotypically silent amino acid substitutions is provided in Bowie, J. U. et al. 1990 Science 247:1306-1310, wherein the authors indicate that proteins are surprisingly tolerant of amino acid substitutions.

### Polypeptides and Fragments

Further described herein is a filovirus polypeptide having the amino acid sequence encoded by an open reading frame (ORF) of a wild-type filovirus structural gene, or a peptide or polypeptide comprising a portion thereof (e.g., SGP).

It will be recognized in the art that some amino acid sequences of the filovirus polypeptides can be varied without significant effect of the structure or function of the protein. If such differences in sequence are contemplated, it should be remembered that there will be critical areas on the protein which determine activity.

Thus, further described herein are variations of the filovirus polypeptide which show substantial filovirus polypeptide activity or which include regions of filovirus protein such as the protein portions discussed below. Such mutants include deletions, insertions, inversions, repeats, and type substitutions. As indicated, guidance concerning which amino acid changes are likely to be phenotypically silent can be found in Bowie, J.U. et al. 1990 Science 247:1306-1310.

Thus, the fragment, derivative or analog of the polypeptide described herein may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues include a substituent group, or (iii) one in which additional amino acids are fused to the mature polypeptide, such as an IgG Fc fusion region peptide or leader or secretory sequence or a sequence which is employed for purification of the mature polypeptide or a proprotein sequence. Such fragments, derivatives and analogs are deemed to be within the scope of those skilled in the art from the teachings herein.

As indicated, changes are preferably of a minor nature, such as conservative amino acid substitutions that do not significantly affect the folding or activity of the protein (see Table A).

**Table A. Conservative Amino Acid Substitutions**

| | |
|---|---|
| **Aromatic** | Phenylalanine |
| | Tryptophan |
| | Tyrosine |
| **Ionizable: Acidic** | Aspartic Acid |
| | Glutamic Acid |
| **Ionizable: Basic** | Arginine |
| | Histidine |
| | Lysine |
| **Nonionizable Polar** | Asparagine |
| | Glutamine |
| | Selenocystine |
| | Serine |
| | Threonine |
| **Nonpolar (Hydrophobic)** | Alanine |
| | Glycine |
| | Isoleucine |
| | Leucine |
| | Proline |
| | Valine |
| **Sulfur Containing** | Cysteine |
| | Methionine |

Of course, the number of amino acid substitutions a skilled artisan would make depends on many factors, including those described above. Generally speaking, the number of amino acid substitutions for any given filovirus polypeptide will not be more than 50, 40, 30, 20, 10, 5 or 3.

Amino acids in the filovirus polypeptides described herein that are essential for function can be identified by methods known in the art, such as site-directed mutagenesis or alanine-scanning mutagenesis (Cunningham & Wells 1989 Science 244:1081-1085). The latter procedure introduces single alanine mutations at every residue in the molecule. The resulting mutant molecules are then tested for biological activity such as changes in immunological character.

The polypeptides described herein are conveniently provided in an isolated form. By "isolated polypeptide" is intended a polypeptide removed from its native environment. Thus, a polypeptide produced and/or contained within a recombinant host cell is considered isolated for purposes described herein. Also intended as an "isolated polypeptide" are polypeptides that have been purified, partially or substantially, from a recombinant host cell or a native source. For example, a recombinantly produced version of the filovirus polypeptide can be substantially purified by the one-step method described in Smith and Johnson 1988 Gene 67:31-40.

The polypeptides described herein include a polypeptide comprising a polypeptide having the amino acid sequence of a wild-type filovirus structural gene product or portion thereof or encoded by a nucleic acid sequence shown herein in the Sequence Listing; as well as polypeptides which are at least 95% identical, and more preferably at least 96%, 97%, 98%, or 99% identical to those described above.

By a polypeptide having an amino acid sequence at least, for example, 95% "identical" to a reference amino acid sequence of an filovirus polypeptide is intended that the amino acid sequence of the polypeptide is identical to the reference sequence except that the polypeptide sequence may include up to five amino acid alterations per each 100 amino acids of the reference amino acid of the filovirus polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 95%, 96%, 97%, 98%, or 99% identical to a reference amino acid sequence can be determined conventionally using known computer programs such the Bestfit program (Wisconsin Sequence Analysis Package, Version 8 for Unix, Genetics Computer Group, University Research Park, 575 Science Drive, Madison, Wis. 53711). When using Bestfit or any other sequence alignment program to determine whether a particular sequence is, for instance, 95% identical to a reference sequence described herein, the parameters are set, of course, such that the percentage of identity is calculated over the full length of the reference amino acid sequence and that gaps in homology of up to 5% of the total number of amino acid residues in the reference sequence are allowed.

In another aspect, described herein are portions of the polypeptides described herein with at least 30 amino acids and more preferably at least 50 amino acids. Preferred portions described herein include polypeptides comprising an epitope-bearing portion of a filovirus structural protein. In particular, preferred portions described herein include polypeptides comprising an epitope-bearing domain of a filovirus structural protein, where the domain is the GP/SGP identity domain, the mucin-like domain, the furin cleavage site, the fusion peptide domain, the heptad repeat domain, the transmembrane anchor domain, and the intracellular domain, and any combination thereof, for example, a filovirus glycoprotein having a truncation at the carboxy terminus to delete the transmembrane anchor and intracellular domain, a filovirus glycoprotein having a truncation at the carboxy terminus to delete the heptad repeat domain and transmembrane anchor and intracellular domain, a filovirus glycoprotein having a truncation at the carboxy terminus to delete the fusion peptide domain, heptad repeat domain, and transmembrane anchor and intracellular domain, a filovirus glycoprotein having a truncation at the carboxy terminus to delete the furin cleavage site, fusion peptide domain, heptad repeat domain, and transmembrane anchor and intracellular domain, and a filovirus glycoprotein having a truncation at the carboxy terminus to delete the mucin-like domain, furin cleavage site, fusion peptide domain, heptad repeat domain, and transmembrane anchor and intracellular domain. Another example is a filovirus glycoprotein having an amino, internal, or carboxy deletion to delete the mucin-like domain, the furin cleavage site, the fusion peptide domain, the heptad repeat domain, the transmembrane anchor domain, or the intracellular domain.

The polypeptides described herein may be produced by any conventional means (Houghten, R.A. 1985 PNAS USA 82:5131-5135). The "Simultaneous Multiple Peptide Synthesis (SMPS)" process is described in U.S. Pat. No. 4,631,211 to Houghten et al. (1986).

The present invention provides an expression vector comprising SEQ ID NO: 52 or analog thereof having at least 95, 96, 97, 98, 99 or 100% sequence identity thereto that mediates expression, wherein the percentage of identity is calculated over the full length of the reference nucleotide sequence. Further described herein are vectors which include the nucleic acid molecules further described herein, host cells which are genetically engineered with the recombinant vectors, and the production of filovirus polypeptides or fragments thereof by recombinant techniques.

Further described herein are "prime and boost" immunization regimes in which the immune response induced by administration of a priming composition is boosted by administration of a boosting composition. Experimental demonstration described herein shows that effective boosting can be achieved using replication-defective adenovirus vectors, following priming with any of a variety of different types of priming compositions. As described herein, replication-deficient adenovirus which, as the experiments described below show, has been found to be an effective means for providing a boost to an immune response primed to antigen using any of a variety of different priming compositions.

Replication-deficient adenovirus derived from human serotype 5 has been developed as a live viral vector by Graham and colleagues (Graham & Prevec 1995 Mol Biotechnol 3:207-20; Bett et al. 1994 PNAS USA 91:8802-6). Adenoviruses are non-enveloped viruses containing a linear double-stranded DNA genome of around 3600 bp. Recombinant viruses can be constructed by *in vitro* recombination between an adenovirus genome plasmid and a shuttle vector containing the gene of interest together with a strong eukaryotic promoter, in a permissive cell line which allows viral replication. High viral titres can be obtained from the permissive cell line, but the resulting viruses, although capable of infecting a wide range of cell types, do not replicate in any cells other than the permissive line, and are therefore a safe antigen delivery system. Recombinant adenoviruses have been shown to elicit protective immune responses against a number of antigens including tick-borne encephalitis virus NS1 protein (Jacobs et al. 1992 J Virol 66:2086-95) and measles virus nucleoprotein (Fooks et al. 1995 Virology 210:456-65).

Remarkably, the experimental work described below demonstrates that use of embodiments of the present invention allows for recombinant replication-defective adenovirus expressing an antigen to boost an immune response primed by a DNA vaccine. The replication-defective adenovirus was found to induce an immune response after intramuscular immunization. In prime/boost vaccination regimes the replication-defective adenovirus is also envisioned as being able to prime a response that can be boosted by a different recombinant virus or recombinantly produced antigen.

Non-human primates immunized with plasmid DNA and boosted with replication-defective adenovirus were protected against challenge. Both recombinant replication-deficient adenovirus and plasmid DNA are vaccines that are safe for use in humans. Advantageously, the inventors found that a vaccination regime used intramuscular immunization for both prime and boost can be employed, constituting a general immunization regime suitable for inducing an immune response, e.g., in humans.

In various aspects described herein is a replication-deficient adenovirus vector encoding an antigen for boosting an immune response to the antigen primed by previous administration of the antigen or nucleic acid encoding the antigen.

A general aspect described herein is the use of a replication-deficient adenoviral vector for boosting an immune response to an antigen.

One aspect described herein is a method of boosting an immune response to an antigen in an individual, the method including provision in the individual of a replication-deficient adenoviral vector including nucleic acid encoding the antigen operably linked to regulator sequences for production of antigen in the individual by expression from the nucleic acid, whereby an immune response to the antigen previously primed in the individual is boosted.

An immune response to an antigen may be primed by genetic immunization, by infection with an infectious agent, or by recombinantly produced antigen.

A further aspect described herein is a method of inducing an immune response to an antigen in an individual, the method comprising administering to the individual a priming composition comprising the antigen or nucleic acid encoding the antigen and then administering a boosting composition which comprises a replication-deficient adenoviral vector including nucleic acid encoding the antigen operably linked to regulatory sequences for production of antigen in the individual by expression from the nucleic acid.

A further aspect describes the use of a replication-deficient adenoviral vector, as disclosed, in the manufacture of a medicament for administration to a mammal to boost an immune response to an antigen. Such a medicament is generally for administration following prior administration of a priming composition comprising the antigen.

The priming composition may comprise any viral vector, including adenoviral, or other than adenoviral, such as a vaccinia virus vector such as a replication-deficient strain such as modified virus Ankara (MVA) (Mayr et al. 1978 Zentralbl Bakteriol 167:375-90; Sutter and Moss 1992 PNAS USA 89:10847-51; Sutter et al. 1994 Vaccine 12:1032-40) or NYVAC (Tartaglia et al. 1992 Virology 118:217-32), an avipox vector such as fowlpox or canarypox, e.g., the strain known as ALVAC (Kanapox, Paoletti et al. 1994 Dev Biol Stand 1994 82:65-9), or a herpes virus vector.

The priming composition may comprise DNA encoding the antigen, such DNA preferably being in the form of a circular plasmid that is not capable of replicating in mammalian cells. Any selectable marker should not be resistant to an antibiotic used clinically, so for example Kanamycin resistance is preferred to Ampicillin resistance. Antigen expression should be driven by a promoter which is active in mammalian cells, for instance the cytomegalovirus immediate early (CMV IE) promoter.

In particular embodiments of the various aspects described herein, administration of a priming composition is followed by boosting with first and second boosting compositions, the first and second boosting compositions being the same or different from one another, e.g., as exemplified below. Still further boosting compositions may be employed. In one embodiment, a triple immunization regime employs DNA, then adenovirus (Ad) as a first boosting composition, and then MVA as a second boosting composition, optionally followed by a further (third) boosting composition or subsequent boosting administration of one or other or both of the same or different vectors. Another option is DNA then MVA then Ad, optionally followed by subsequent boosting administration of one or other or both of the same or different vectors.

The antigen to be included in respective priming and boosting compositions (however many boosting compositions are employed) need not be identical, but should share epitopes. The antigen may correspond to a complete antigen in a target pathogen or cell, or a fragment thereof. Peptide epitopes or artificial strings of epitopes may be employed, more efficiently cutting out unnecessary protein sequence in the antigen and encoding sequence in the vector or vectors. One or more additional epitopes may be included, for instance epitopes which are recognized by T helper cells, especially epitopes recognized in individuals of different HLA types.

Within the replication-deficient adenoviral vector, regulatory sequences for expression of the encoded antigen will include a promoter. By "promoter" is meant a sequence of nucleotides from which transportation may be initiated of DNA operably linked downstream (i.e. in the 3' direction on the sense strand of double-stranded DNA). "Operably linked" means joined as part of the same nucleic acid molecule, suitably positioned and oriented for transcription to be initiated from the promoter. DNA operably linked to a promoter is "under transcriptional initiation regulation" of the promoter. Other regulatory sequences including terminator fragments, polyadenylation sequences, enhancer sequences, marker genes, internal ribosome entry site (IRES) and other sequences may be included as appropriate, in accordance with the knowledge and practice of the ordinary person skilled in the art: see, for example, Molecular Cloning: a Laboratory Manual, 2nd edition, Sambrook et al. 1989 Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1994.

Suitable promoters for use in aspects and embodiments described herein include the cytomegalovirus immediate early (CMV IE) promoter, with or without intron A, and any other promoter that is active in mammalian cells.

Either or both of the priming and boosting compositions may include an adjuvant or cytokine, such as alpha-interferon, gamma-interferon, platelet-derived growth factor (PDGF), granulocyte macrophage-colony stimulating factor (GM-CSF) granulocyte-colony stimulating factor (gCSF), tumor necrosis factor (TNF), epidermal growth factor (EGF), IL-1, IL-2, IL-4, IL-6, IL-8, IL-10 and IL-12, or encoding nucleic acid therefor.

Administration of the boosting composition is generally weeks or months after administration of the priming composition, preferably about 2-3 weeks or 4 weeks, or 8 weeks, or 16 weeks, or 20 weeks, or 24 weeks, or 28 weeks, or 32 weeks.

Preferably, administration of priming composition, boosting' composition, or both priming and boosting compositions, is intramuscular immunization.

Intramuscular administration of adenovirus vaccines or plasmid DNA may be achieved by using a needle to inject a suspension of the virus or plasmid DNA. An alternative is the use of a needless injection device to administer a virus or plasmid DNA suspension (using, e.g., Biojector^{™}) or a freeze-dried powder containing the vaccine (e.g., in accordance with techniques and products of Powderject), providing for manufacturing individually prepared doses that do not need cold storage. This would be a great advantage for a vaccine that is needed in rural areas of Africa.

Adenovirus is a virus with an excellent safety record in human immunizations. The generation of recombinant viruses can be accomplished simply, and they can be manufactured reproducibly in large quantities. Intramuscular administration of recombinant replication-deficient adenovirus is therefore highly suitable for prophylactic or therapeutic vaccination of humans against diseases which can be controlled by an immune response.

The individual may have a disease or disorder such that delivery of the antigen and generation of an immune response to the antigen is of benefit or has a therapeutically beneficial effect.

Most likely, administration will have prophylactic aim to generate an immune response against a pathogen or disease before infection or development of symptoms.

Diseases and disorders that may be treated or prevented include those in which an immune response may play a protective or therapeutic role.

Components to be administered may be formulated in pharmaceutical compositions. These compositions may comprise a pharmaceutically acceptable excipient, carrier, buffer, stabilizer or other materials well known to those skilled in the art. Such materials should be non-toxic and should not interfere with the efficacy of the active ingredient. The precise nature of the carrier or other material may depend on the route of administration, e.g., intravenous, cutaneous or subcutaneous, intramucosal (e.g., gut), intranasal, intramuscular, or intraperitoneal routes.

As noted, administration is preferably intradermal, subcutaneous or intramuscular.

Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required.

A slow-release formulation may be employed.

Following production of replication-deficient adenoviral particles and optional formulation of such particles into compositions, the particles may be administered to an individual, particularly human or other primate.

Administration may be to anther mammal, e.g., rodent such as mouse, rat or hamster, guinea pig, rabbit, sheep, goat, pig, horse, cow, donkey, dog or cat.

Administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g., decisions on dosage etc., is within the responsibility of general practitioners and other medical doctors, or in a veterinary context a veterinarian, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. ed., 1980.

In one preferred regimen, DNA is administered (preferably intramuscularly) at a dose of 10 micrograms to 50 milligrams/injection, followed by adenovirus (preferably intramuscularly) at a dose of 5 x 10⁷ - 1 x 10¹² particles/injection.

The composition may, if desired, be presented in a kit, pack or dispenser, which may contain one or more unit dosage forms containing the active ingredient. The kit, for example, may comprise metal or plastic foil, such as a blister pack. The kit, pack, or dispenser may be accompanied by instructions for administration.

A composition may be administered alone or in combination with other treatments, either simultaneously or sequentially dependent upon the condition to be treated.

Delivery to a non-human mammal need not be for a therapeutic purpose, but may be for use in an experimental context; for instance in investigation of mechanisms of immune responses to an antigen of interest, e.g., protection against disease.

Further aspects described herein will be apparent to those of ordinary skill in the art, in view of the above disclosure and following experimental exemplification, included by way of illustration and not limitation, and with reference to the attached figures.

### Development of a Preventive Vaccine for Ebola Virus Infection in Primates

Genetic immunization has been shown to influence both humoral and cellular immune activation pathways and to protect against infection by human pathogens (Tang, D.C. et al. 1992 Nature 356:152-154; Ulmer, J.B. et al. 1993 Science 259:1745-1749; Wang, B. et al. 1993 PNAS USA 90:4156-4160; Sedegah, M. et al. 1994 PNAS USA 91:9866-9870). The effectiveness of plasmid vaccines is thought to result from host cell protein synthesis and endogenous presentation of the immunogen, and possibly to immunostimulatory effects of plasmid DNA itself (Krieg, A.M. et al. 1995 Nature 374:546-549; Sato, Y. et al. 1996 Science 273:352-354). DNA vaccines have been shown to elicit specific immune responses to Ebola virus antigens and to protect guinea pigs (Xu, L. et al. 1998 Nat Med 4:7-42) and mice (Vanderzanden, L. et al. 1998 Virology 246:134-144) against challenge with Ebola virus adapted to produce lethal infection in rodents (Connolly, B.M. et al. 1999 J Infect Dis 179:S203-S217; Bray, M. et al. 1998 J Infect Dis 178:651-661). Although both cell-mediated and humoral immune responses were elicited, antibody titer correlated with the degree of protection in animals immunized with plasmids encoding proteins from the Zaire subtype of Ebola virus.

A broadly effective vaccine would need to provide immunity to the multiple Ebola subtypes isolated in human infections (Zaire, Sudan and Ivory Coast), but a multivalent vaccine might dilute the specific immune response demonstrated for the single subtype vaccine. To address this concern, we analyzed the efficacy of the original Ebola Zaire DNA vaccine in comparison to its use in combination with DNA from Ebola subtypes Sudan and Ivory Coast. As in a previous study (Xu, L. et al. 1998 Nat Med 4:7-42), immunization with a single plasmid encoding Zaire subtype virion glycoprotein, GP(Z), generated a substantial virus-specific antibody response and conferred protective immunity in guinea pigs (Table I). Inclusion of a plasmid expressing Ebola nucleoprotein, NP, did not affect the antibody titer to Ebola GP(Z) or diminish its protective efficacy. Further broadening of the vaccine components to include NP and three subtypes of Ebola glycoprotein, Zaire, Ivory Coast and Sudan, GP(Z,IC,S)+NP, yielded a pre-challenge immune response comparable to the single-plasmid vaccine. Moreover, complete protection from infection with Ebola Zaire was achieved in guinea pigs that received the multivalent vaccine (Table I, subjects 13-16). Anamnestic antibody was not induced by the virus challenge, indicating that the vaccine itself provided an immune response sufficient to efficiently clear the virus. These findings show that multivalent plasmid immunization did not substantially diminish glycoprotein (GP)-specific antibody production and its protective efficacy in a rodent model.

**Table I. Multivalent genetic immunization in guinea pigs**

| **ID** | **Immunization** | **ELISA IgG** | **Survival** |
|---|---|---|---|
| 1 | Plasmid | 0 | No |
| 2 | Plasmid | 0 | No |
| 3 | Plasmid | 0 | No |
| 4 | Plasmid | 0 | No |
| | | | |
| 5 | GP(Z) | 6400 | Yes |
| 6 | GP(Z) | 6400 | Yes |
| 7 | GP(Z) | 6400 | Yes |
| 8 | GP(Z) | 3200 | Yes |
| | | | |
| 9 | GP(Z) + NP | 6400 | Yes |
| 10 | GP(Z) + NP | 6400 | Yes |
| 11 | GP(Z) + NP | 6400 | Yes |
| 12 | GP(Z) + NP | 6400 | Yes |
| | | | |
| 13 | GP(Z,IC,S) + NP | 6400 | Yes |
| 14 | GP(Z,IC,S) + NP | 1600 | Yes |
| 15 | GP(Z,IC,S) + NP | 6400 | Yes |
| 16 | GP(Z,IC,S) + NP | 6400 | Yes |

**Table I.** Comparison of multivalent *vs*. monovalent genetic immunization in guinea pigs. Guinea pigs were immunized intramuscularly three times at two-week intervals with 100 µg of DNA (Plasmid, 100 µg p1012; GP(Z), 100 µg pGP(Z); GP(Z) + NP, 75 µg pGP(Z) and 25 µg pNP; GP(Z, IC, S) + NP, 25 µg each of pGP(Z), pGP(IC), pGP(S) and pNP). Serum was collected 6 weeks after the first injection and pre-challenge titers for antibody to Ebola GP (ELISA IgG) were measured by ELISA (Ksiazek, T.G. *et al.* 1992 *J Clin Microbiol* **30**:947-950) and are displayed as the reciprocal endpoint dilution. Three months after the final immunization the animals were challenged as described (Xu, L. et al. 1998 Nat Med 4:37-42).

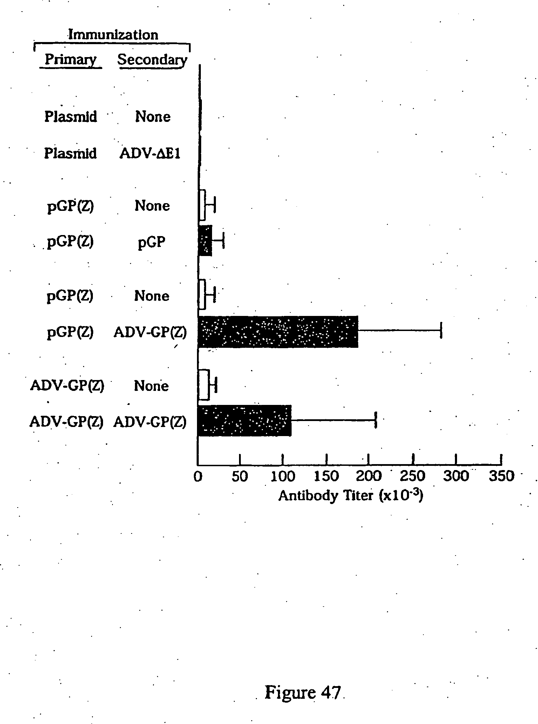

Because protection in the rodent model of Ebola virus infection correlated with antibody titers, and efficient humoral responses may influence clinical outcome in human disease (Baize, S. et al. 1999 Nat Med 5:423-426; Maruyama, T. et al. 1999 J Virol 73:6024-6030), we considered it important to elicit a strong humoral response for vaccines tested in primates, although cell-mediated immunity is coordinately induced and likely contributes to protection (Xu, L. et al. 1998 Nat Med 4:37-42). Recently, regimens of DNA priming followed by administration of viral vectors have demonstrated enhanced immune responses compared to vaccines using DNA alone (Sedegah, M. et al. 1998 PNAS USA 95:7648-7653; Hanke, T. et al. 1998 Vaccine 16:439-445; Robinson, H.L. et al. 1999 Nat Med 5:526-534; Schneider, J. et al. 1998 Nat Med 4:397-402). Recombinant, replication-deficient adenoviruses can be grown to high titer, infect antigen-presenting cells, and induce potent immune responses (Davis, A.R et al. 1985 PNAS USA 82:7560-7564; Natuk, R.J. et al. 1992 PNAS USA 89:7777-7781; Xiang, Z.Q. et al. 1996 Virology 219:220-227). Adenoviruses have shown a boosting effect in mice (Xiang, Z.Q. et al. 1999 J Immunol 162:6716-6723), but the combination of DNA and adenovirus has not been tested for efficacy in an infectious challenge model, and the success of this approach in primates is yet unknown. We therefore developed a recombinant adenoviral vector that directs high level GP expression ADV-GP(Z) and used this vector to test whether a modified prime-boost strategy would augment the antibody response to Ebola virus obtained with naked DNA alone. Mice were injected with DNA and adenovirus vectors either singly or in combinations, and cell-mediated and humoral immune responses were assessed. A 10- to 100-fold increase in antibody titer was found in mice injected with DNA followed by an adenovirus boost, compared to DNA immunization alone (Fig. 47). An increase in cytotoxic T cell responses was also observed with this combination. Immunization with ADV-GP(Z) alone yielded antibody titers that were not significantly different from those obtained with the DNA prime, adenovirus boost immunization. These data suggest that immunogenicity of the Ebola GP DNA vaccine in mice is improved by boosting with recombinant adenovirus and that this strategy might represent a useful approach to enhance immune responses in non-human primates.

Whereas the rodent model has been useful in the development of a vaccine strategy, Ebola virus isolated directly from humans must first be adapted by multiple, sequential passage in rodents in order to produce a lethal infection in mice or guinea pigs (Connolly, B.M. et al. 1999 J Infect Dis 179:S203-S217; Bray, M. et al. 1998 J Infect Dis 178:651-661). Primate models of Ebola infection are thought to have a stronger predictive value for human disease and immune protection. We therefore conducted studies in non-human primates using a bimodal DNA/ADV vaccine and the multiple plasmid strategy that correlated with protection in guinea pigs. Cynomolgus macaques (*Macaca fascicularis*) received 3 injections of naked DNA vectors at 4-week intervals (Fig. 48A) and, after several months of rest which has been shown to boost immune responses (Letvin, N.L. et al. 1997 PNAS USA 94:9378-9383), were boosted with recombinant adenovirus expressing only the Zaire glycoprotein (Fig. 48A). Control animals received empty vectors (plasmid DNA and ADV-ΔE1 recombinant adenovirus), and vaccinated animals received the multicomponent DNA vaccine containing NP and three subtypes of Ebola GP (pGP/NP), followed by ADV-GP(Z). As expected, anti-Ebola serum antibodies could not be detected in control animals, but in animals receiving the Ebola vaccine, an antigen-specific antibody response was detected at week 12, one month after the third DNA injection (Fig. 48B). After boosting with recombinant adenovirus, antibody titers increased 10- to 20-fold over the levels obtained with DNA alone. Three months after the final immunization, antibody levels remained high, except for one animal (subject 8) whose titer dropped slightly from 5 x 10⁴ to 1.3 x 10⁴.

Primate cellular responses to Ebola antigens were next examined with an *in vitro* lymphocyte proliferation assay. In control monkeys, antigen-specific lymphocyte proliferation, measured by ³H-thymidine uptake, was equivalent to that in matched, unstimulated cells, resulting in a proliferation index near 1.0 for each animal (Fig. 48C). In contrast, peripheral blood mononuclear cells (PBMC) from animals immunized with the multivalent vaccine showed 9- to 20-fold increased stimulation, remonstrating a robust immune response to Ebola antigen at the cellular level. Depletion of CD4-positive lymphocytes reduced the antigen-stimulated proliferative response of PBMC from vaccinated monkeys to the level observed in control animals (Fig. 48D). Depletion of CD8-positive lymphocytes, however, did not affect Ebola antigen-specific lymphocyte proliferation. Therefore, the CD4-positive subset of lymphocytes, which provide the T cell help required for high antibody titers, contributes to the vaccine-induced cellular immune response.

To determine the protective efficacy of this vaccination regimen, monkeys were challenged with a lethal dose of the wild-type Mayinga strain from the Zaire subtype of Ebola virus. In the control monkeys, blood chemistry revealed an increase in hepatic enzymes (Figure 49A, B) that is characteristic for Ebola virus infection (Fisher-Hoch, S.P. et al. 1985 J Infect Dis 152:887-894). No such increase was observed in vaccinated subjects. The elevation of serum alanine aminotransferase (ALT) and aspartate aminotransferase (AST) was parallel to a dramatic increase in viraemia in all of the control animals (Figure 49C). In contrast, no substantial increase in viral load was observed in vaccinated monkeys. The kinetics of disease progression was similar among the control animals, and the disease incidence was 100% in this group. Death occurred between days 5 and 6 for 3 animals, and the last monkey, moribund, was euthanized on day 7. In contrast, 4 out of 4 monkeys immunized with the combination DNA-adenovirus vaccine survived this lethal challenge of Ebola virus, and sterilizing immunity was achieved in 3 out of 4 subjects. The remaining animal showed a small transient rise in viral antigen; however, when followed long-term, all vaccinated animals showed no signs or symptoms of infection, and there was no detectable viraemia for more than 6 months after infection, as measured by ELISA detection of viral antigen (Fig. 49A) and end point titration analysis of cultured virus. The vaccine recipient (subject 8) that exhibited a transient low level of viraemia on day 10 returned to undetectable levels by day 17.

As the natural reservoir for Ebola virus is unknown, the potential for traditional public health measures to prevent future outbreaks is limited, thus increasing the urgency for the development of a vaccine and therapeutics in humans. The present findings demonstrate that primates can be immunized against the lethal effects of Ebola virus infection, and that sterilizing immunity can be achieved using a heterologous prime-boost strategy. A multicomponent genetic vaccine expressing Ebola virus structural proteins from diverse geographic isolates generated a strong antigen-specific immune response and resulted in the survival of immunized primates after challenge with a lethal dose of Ebola Zaire, the subtype of this virus associated with the highest number of deaths in human infections. The results of this study suggest that T-cell mediated and humoral immunity contribute to virus clearance in non-human primates, consistent with previous studies in rodents (Xu, L. et al. 1998 Nat Med 4:37-42; Wilson, J. et al. 2000 Science 287:1664-1666). Two immune parameters, antibody titer (1:75,000 *vs.* <1:100, *P* = 0.001) and the cellular proliferative response (∼12-fold *vs.* 1.4-fold, *P* = 0.0014), provided highly significant immune correlates of protection. Studies investigating the correlates of immune protection from Ebola virus infection in humans are hampered by the aggressive nature of the virus and necessarily high level of biosafety containment. With the model of primate immunity presented here, it is envisioned as now being possible to elucidate the mechanisms of immune protection from Ebola virus infection, to advance immune-based anti-viral therapies, and to develop a human vaccine for this pathogen and even other infectious causes of hemorrhagic fever.

### DESCRIPTIONS OF EBOLA, MARBURG, AND LASSA CONSTRUCTS

| | |
|---|---|
| **VRC 6000** | VRC6000 (pVR1012-GP(Z)). |
| | Backbone, pVR1012 (#450) expressing Ebola Glycoprotein of Zaire Subtype. Orientation is *BamH*I/*EcoR*I*lEcoR*V/*BcoR*I*lBg*/II) |
| **VRC 6001** | VRC6001 (pVR1012x/s-GP(Z)) No other description. |
| | This is the same as 6000, with the addition of an *Sfi* restriction site to the pVR1012 backbone. |
| **VRC 6002** | VRC6002 (pVR1012-GP(Z) delta MUC). |
| | The mucin-like domain of GP(Z) was deleted. 530 bp in the backbone, pVR1012 GP(Z) were deleted from *Ear*I(2844) to *Bfa*I(3374). This mutant can bind to the Ebola receptor. |
| **VRC 6003** | VRC6003 (pVR1012-GP(Z) delta MUC delta FUR). |
| | The mucin-like domain and furin-cleavage site of GP(Z) were deleted. 593 bp in the backbone, pVR1012 GP (Z) were deleted, from *Ear*I(2844) to *Ear*I(3437). The protein has properties similar to pVR1012-GP(Z) delta MUC. |
| **VRC 6004** | VRC6004 (pVR1012-GP(Z) delta GP2). |
| | A majority of the GP2 region in GP(Z) was deleted. 430 bp from the backbone, pVR1012-GP (Z) were deleted from *Bcl*I(3414) to *Bsp*EI(3844). The TM (transmembrane) region was retained. |
| **VRC 6005** | VRC6005 (pVR1012-GP(Z) delta GP2 delta C-term A). |
| | This is a C-terminal deletion of GP2. 267 bp were deleted from the pVR1012-GP (Z) backbone, from *Msc*I(3623) to *BspM*I(3890). |
| **VRC 6006** | VRC6006 (pVR1012-GP(Z) delta GP2 delta C-term B). |
| | This is a smaller deletion of GP2 C-terminal. 110 bp of backbone pVR1012-GP(Z) were deleted from *BstX*I(3780) to *BspM*I(3890)*.* |
| **VRC 6007** | VRC6007 (pVR1012-GP(Z) delta GP2 delta FUS). |
| | The fusion peptide in GP2 of GP(Z) was deleted in this mutant, using PCR. 47 bp from the backbone, pVR1012-GP(Z), was deleted from (3508-3555). |
| **VRC 6008** | VRC6008 (pVR1012-GP(Z) delta TM). |
| | The TM region of GP(Z) was truncated in this mutant. A stop codon (TGA) was added downstream of the *BspM*I site(3889). This protein is secreted and doesn't form a trimer. |
| **VRC 6052** | VRC 6052 (pVR1012-GP(Z) delta sGP). |
| | The majority of the SGP/GP homology region was deleted. 687 bp from the backbone, pVR1012-GP(Z), were deleted from *Hinc*II(2083) to *Hinc*II(2270). |
| **VRC 6101** | VRC 6101 (pVR1012x/s Ebola GP(R) (dTM)). |
| | The vector expresses Ebola glycoprotein (subtype Reston) without its transmembrane and intracellular domains. Using PCR, a stop codon was generated downstream of a.a. 650 of GP(R), followed by an *Xba*I site. This protein can be secreted and is termed GP(R)(dTM). |
| **VRC 6110** | VRC 6110 (pAdApt Ebola GP(R) (dTM)). |
| | An adenoviral shuttle vector expressing Ebola virus glycoprotein (Reston subtype) without its transmembrane and intracellular domains. Using PCR, a stop codon was generated downstream of a.a. 651 of GP(Reston), followed by an *Xba*I site. The resulting recombinant adenovirus expresses a 651 a.a. secreted glycoprotein termed GP(R)(dTM). |
| **VRC 6200** | VRC6200 (pVR1012-GP(S)) |
| | Backbone, pVR1012(#450), expressing Ebola Glycoprotein of the Sudan Subtype. Orientation is *EcoR*I/*EcoR*V/*BamH*I*lBamH*I*lBamH*I/*Xba*I*.* |
| **VRC 620**1 | VAC 6201 (pVR1012x/s Ebola GP(S)). |
| | No other description, but this is the same as 6200 with the addition of an Sfi site to the 1012 backbone. |
| **VRC 6202** | VRC6202 (pVR1012-GP(S) delta TM). |
| | The TM region of GP(S) was truncated in this mutant. A stop codon (TGA) was added downstream of the *BspM*I site(xxx). this protein is secreted and doesn't form a trimer. |
| **VRC 6300** | VRC6300 (pVR1012-GP(IC)). |
| | Backbone, pVR1012(#450), expressing Ebola Glycoprotein of the Ivory Coast Subtype. Orientation is *EcoR*I/*EcoR*V/*BamH*I/*BamH*I*lBamH*I/*Xba*I*.* |
| **VRC 6301** | VRC6301 (pVR1012x/s-GP(IC)). |
| | No other description, but this is the same as 6300 with the addition of an Sfi site to the 1012 backbone. |
| **VRC 6302** | VRC6302 (pVR1012-GP(IC) delta TM). |
| | The TM region of GP(IC) was truncated in this mutant. A stop codon (TGA) was added downstream of the *BspM*I site. This protein is secreted and doesn't form a timer. |
| **VRC 6303** | VRC 6303 (pVR1012x/s Ebola GP (IC) (dTM)). |
| | A pVRC2000 based vector expressing Ebola glycoprotein (Ivory Coast subtype) without transmembrane and intracellular domains. Using PCR, a stop codon was generated downstream of a.a. 650, followed by a *Bgl*II site. The vector expresses a 650 a.a. secreted glycoprotein (a.a. 1 - a.a. 650). |
| **VRC 6310** | VRC 6310 (pAdApt Ebola GP (IC) (dTM)). |
| | An adenoviral shuttle vector expressing Ebola glycoprotein (subtype Ivory Coast) without its transmembrane and intracellular domains. Using PCR, a stop codon was generated downstream of a.a. 651 of GP(IC). The resulting recombinant adenovirus expresses a 651 a.a secreted glycoprotein termed as GP(IC)(dTM). |
| **VRC 6351** | VRC6351 (pVR1012x/s-sGP(IC)). No other description. |
| **VRC 6400** | VRC6400 (pVR1012-NP). |
| | Backbone, pVR1012(#450) expressing Ebola Nucleoprotein of the Ivory Coast Subtype. |
| **VRC 6401** | VRC6401 (pVR1012x/s-NP). |
| | No other description, but this is the same as 6400 with the addition of an Sfi site to the 1012 backbone. |
| **VRC 6500** | VRC6500 (pVR1012-VP35). |
| | The backbone is pVR1012(#450). The insert is VP35 from Ebola cloned from pGEM 3Zf(+)VP35(#1213). |
| **VRC 6600** | VRC6600 (pAD/CMV-GP(dTM)(Z-CITE-S). No other description. |
| **VRC 6601** | VRC6601 (pAdApt Ebola GP(S)). No other description. |
| **VRC 6602** | VRC 6602 (pAdApt Ebola GP(S)(dTM)). |
| | An adenoviral shuttle vector expressing Ebola glycoprotein (Sudan subtype) without its transmembrane and intracellular domains. A stop codon was fused downstream of a.a. 650 of GP(S). The resulting recombinant adenovirus expresses a 654 a.a. secreted glycoprotein, termed as GP(S)(dTM). |
| **VRC 6603** | VRC6603 (pAdApt Ebola GP(Z)). No other description. |
| **VRC 6604** | VRC 6604 (pAdApt Ebola GP(Z)(dTM)). |
| | Adenoviral shuttle vector expressing Ebola glycoprotein (subtype Zaire) without its transmembrane and intracellular domains. A stop codon was fused downstream of a.a. 651 of GP(Z). The resulting recombinant adenovirus expresses a 655 a.a. secreted glycoprotein termed as GP(Z)(dTM). |
| **VRC 6701** | VRC6701 (pVR1012-Marburg). |
| | Marburg glycoprotein (GP) open reading frame, Musoke strain. Marburg was cloned into backbone #450(*Bam*(blunt)/*Xba*I) from VRC6700 (*Xba*/*Pvu*II)*.* |
| **VRC 6702** | VRC 6702 (pVR1012x/s Marburg GP (dTM)). |
| | This vector expresses the Marburg virus glycoprotein without its transmembrane and intracellular domains. Using PCR, a stop codon was generated downstream of a.a. 650 of GP(Marburg), followed by a *Bgl*II site. This protein can be secreted and termed as GP(Marburg)(dTM). |
| **VRC 6710** | VRC 6710 (pAdApt Marburg GP (dTM)). |
| | Adenoviral shuttle vector (pVRC1290) expressing Marburg virus glycoprotein without transmembrane and intracellular domains. Using PCR, a terminator codon was generated downstream of a.a. 650, followed by a *Bgl*II site. The resulting recombinant adenovirus expresses a 650 a.a. secreted protein (a.a. 1 - a.a. 650). |
| **VRC 6800** | VRC6800 (pVR1012x/s Lassa GP). No other description.. |
| **VRC 6801** | VRC6801 (pVR1012x/s Lassa GP (dTM). No other description. |
| **VRC 6810** | VRC6810 (pAdApt Lassa GP). No other description. |
| **VRC 6811** | VRC6811 (pAdApt Lassa GP (dTM)). No other description. |

### EXAMPLE 1

**Vector construction.** The construction of DNA vectors expressing Ebola Zaire glycoprotein (GP), secreted GP (SGP), and nucleoprotein (NP) has been described in Xu, L. et al. 1998 Nat Med 4:37-42. The GP Sudan and Ivory Coast expression vectors were constructed similarly. Briefly, GP open reading frames were generated from polymerase chain reaction after reverse transcription of RNA (RT-PCR) products of infected cell RNA using the following primers: 5' ATC TTC AGG ATC TCG CCA TGG A 3' (Sudan GP gene; *Nco*I > ATG; **SEQ ID NO: 44**), 5' GAT ATT CAA CAA AGC AGC TTG CAG 3' (Sudan GP gene; C-terminus GP stop; **SEQ ID NO: 45),** 5' CTA ATC ACA GTC ACC ATG GGA 3' (Ivory Coast GP gene; *Nco*I *>* ATG; **SEQ ID NO: 46**), 5' AAA GTA TGA TGC TAT ATT AGT TCA 3' (Ivory Coast GP gene; C-terminus GP stop; **SEQ ID NO: 47**) yielding the TA clones PCR2.1 Sudan and PCR2.1 Ivory Coast. The Sudan glycoprotein was digested from plasmid PCR2.1 with *Xba*I/*Hind*III*,* Klenow treated, and cloned into the *Xba*I site of p1012 (Xu, L. et al. 1998 Nat Med 4:37-42). Ivory Coast GP was digested from plasmid PCR2.1 with *EcoR*I*,* Klenow treated, and cloned into the *Xba*I site of p1012 (Xu, L. et al. 1998 Nat Med 4:37-42).

To make ADV-GP, the *BamH*I/*EcoR*I fragment of GP(Z) was digested from pGEM-3Zf(-)-GP, treated with Klenow, and inserted into *Hind*III/*Xba*I/Kle/CIP treated pRc/CMV plasmid. The resulting plasmid (PRC/CMV-GP(Z)) was digested by *Nru*I/*Dra*III and treated with Klenow. The *Nru*I/*Dra*III/Kle fragment containing the CMV enhancer, GP(Z) DNA and bovine growth hormone polyadenylation signal was inserted into the *Bg*/II site of the adenoviral shuttle plasmid pAdBgIII (Ohno, T. et al. 1994 Science 265:781-784). The adenovirus, a first generation dl 309-based Ad5 vector, contained a deletion in E1 to render the vector replication-defective and a partial deletion/substitution in E3, which disrupts the' coding sequences for the E3 proteins with a relative molecular mass of 14.7 kD, 14.5 kD and 10.4 kD, respectively. The recombinant adenovirus expressing Zaire GP, ADV-GP(Z), was made according to previously published methods (Aoki, K. et al. 1999 Mol Med 5:224-231). The dose of adenovirus administered, 10¹⁰ plaque-forming units (PFU) per animal (approximately 3 × 10⁹ PFU/kg), is within the range used safely in human gene therapy trials.

**Animal study and safety**. Eight cynomolgus macaques (*Macaca fascicularis*), 3 years of age and weighing 2-3 kg, obtained from Covance (Princeton, NJ), were used for the immunization and challenge experiment. To obtain blood specimens and administer vaccines, the monkeys were anesthetized with Ketamine. The animals were housed singly and received regular enrichment according to the Guide for the Care and Use of Laboratory Animals (DHEW No. NIH 86-23). Just before the Ebola virus challenge and up to the end of the experiment, the animals were maintained in the Maximum Containment Laboratory (BSL-4) and fed and checked daily. One animal was euthanized that appeared moribund and was subsequently necropsied for pathologic examination. In addition, a single asymptomatic vaccinated animal was euthanized for pathologic and virologic analysis.

**Mouse immunization.** DNA and adenovirus vectors expressing Ebola Zaire GP or NP were constructed as described previously (Xu, L. et al. 1998 Nat Med 4:37-42; Ohno, T. et al. 1994 Science 265:781-784), with gene expression under the control of the cytomegalovirus enhancer and promoter. Mice were immunized intramuscularly) with 100 µg of DNA (pGP or a p1012 plasmid control) or 10⁸ PFU of adenovirus (ADV-GP or ADV-ΔE1 control virus) on days 0, 14, and 28 and blood was collected on day 28. On day 42, mice received an intramuscular boost with DNA or adenovirus and titers were remeasured on day 56. ELISA IgG titers were determined using 96-well plates coated with a preparation of Ebola virus antigen derived from purified virions and enriched for membrane-associated proteins (GP, VP40 and VP24) (Ksiazek, T.G. et al. 1992 J Clin Microbiol 30:947-950). Specific antigen binding was detected using a goat anti-human IgG(H+L)-horseradish peroxidase conjugate and ABTS/Peroxide (substrate/indicator).

**Macaque immunization.** For the DNA immunizations, animals received 1 mg each of DNA expressing GP(Zaire) [GP(Z)], GP(Ivory Coast) [pGP(IC)], GP(Sudan) [pGP(S)] and NP(Zaire) administered as a mixture [pGP/NP], or 4 mg empty [pGP(Z)] control plasmid bilaterally (2 mg per side) in the deltoid muscle. Immunization at weeks 0 and 4 were by IM injection, and at week 8 by Biojector. For the adenovirus boost, animals received 10¹⁰ PFU of ADV-GP (Zaire subtype) or ADV-ΔE1 (empty vector) divided into two doses administered bilaterally in the deltoid muscle. At week 32, all animals received an intraperitoneal injection of approximately 6 PFUs of Ebola virus (Zaire 1976 isolate; Mayinga strain) (Kiley, M.P. et al. 1980 J Gen Virol 49:333-341) in 1 ml Hanks' buffered salt solution. The virus was isolated directly from patient blood and used after a single passage in Vero cells.

ELISA IgG titers were determined as above for control (Plasmid: ADV-AE1) and immunized [pGP/NP: ADV-GP(Z)] monkeys. The reciprocal endpoint of dilution for each subject was at week 12 and week 24. Serum antibody levels were measured by ELISA as described (Ksiazek, T.G. et al. 1992 J Clin Microbiol 30:947-950).

Blood was collected from control (plasmid: ADV-ΔE1) or immunized [pGP/NP: ADV-GP(Z)] animals 1-3 days prior to the immunizations at weeks 4, 8 and 20, and at week 24. Blood was separated over a Percoll gradient to obtain the lymphocyte enriched population. Lymphocytes were stimulated as described (Xu, L. et al. 1998 Nat Med 4:37-42) for 5 *days in vitro* using supernatant from cells transfected with either Ebola secreted glycoprotein (SGP) or empty plasmid, and proliferation was measured by ³H-thymidine uptake. The proliferation index was calculated as the proliferation in wells receiving SGP divided by proliferation in wells receiving control supernatant.

**Viral detection in macaques.** The presence of circulating Ebola virus antigen was detected as described (Ksiazek, T.G. et al. 1992 J Clin Microbiol 30:947-950) by capturing VP40 protein from serial dilutions of monkey plasma. 96-well plates coated with antiVP40 mAb were used to capture antigen, and detection was-with a rabbit anti-Ebola virus serum.

### EXAMPLE 2

The amino acid sequences of Ebola GP(Zaire) and NP (Zaire) were obtained from Genbank: GP(Zaire), Genbank accession no. P87666; NP(Zaire), Genbank accession no. NC_002549; while GP(Sudan/Gulu) was obtained from the CDC. The amino acid sequences were then back-translated to DNA sequences using mammalian referred codons. Serial 75 bp oligos with 25 bp overlapping were prepared to cover the entire gene. The oligos were then assembled into intact mammalian genes containing preferred codons using PCR. In the design, a stop codon was introduced in front of the predicted transmembrane domains of GP(Zaire) (a.a. 648-676) and GP (Sudan/Gulu) (a.a. 648-676) so that this region was excluded from these synthetically created genes. The deletions also led to the loss of a 4 a.a. cytoplasmic region in both constructs. Final sequencing of the Ebola GP (Zaire) sequence revealed 10 divergent amino acids from the laboratory GP sequence, which was used in our animal studies and these were corrected by site-directed mutagenesis. These inserts were cloned into p1012 x/s by *Xba*I/*Sal*I.

### Construction of CMV/R-GP(S/G)(ΔTM)/h

The codon-modified, transmembrane domain deleted form of the Ebola GP (Sudan/ Gulu) gene was excised from p1012 (x/s)-GP(S/G)(ΔTM)/h using *Sal*I/*Kpn*I*,* and inserted into the *Sal*I/*Kpn*I digested CMV/R/MCS plasmid.

### Construction of CMV/R GP(Z) (ΔTM)/h

The codon-modified, transmembrane domain deleted form of the Ebola GP (Zaire) gene was excised from p1012 x/s-GP (Z)(dTM)/h *Sal*I/*Bgl*II sites and cloned into the *Sal*I/*Bgl*I sites of the CMV/R plasmid.

### Construction of CMV/R Ebola NP

The *Arot*I*-Kpn*I fragment from VRC6400 (pVR1012-NP) expressing Ebola nucleoprotein of Zaire Subtype was excised and cloned into the *Not*I/*Kpn*I sites of the CMV/R plasmid.

### EXAMPLE 3

### IMPROVED NON-VIRAL MAMMALIAN EXPRESSION VECTOR

This invention provides an improved mammalian expression vector which generates a higher level of protein expression than vectors currently in use.

Initially, 3 new vectors, each containing a different enhancer, were developed and tested. The RSV enhancer, the mouse ubiquitin enhancer (mUBB), and the CMV enhancer (Xu et al. 1998 Nature Med. 4:37-42) were each combined with the HTL V-1 R region (Takebe et al. 1988 Mol Cell Biol 8:466-472) to create separate vectors. When these 3 vectors were compared to the backbone containing the CMV enhancer in combination with the CMV translational enhance and intron (CMVint), which is currently the most effective vector, *in vitro* data showed that expression with the vector containing the CMV/R was increased 5-10 fold compared to CMV/int, and immunological studies showed induction of significantly higher CD4 and CD8 T cell responses compared to CMVint. Both *in vivo* and *in vitro* responses were markedly higher with this new vector. Neither of the other two vectors produced comparable results.

The expression vector is unique in that it uses a specific translational enhancer in combination with specific enhancer/promoters to yield high levels of expression and enhanced immunogenicity for DNA vaccines. This is particularly important because the potency of these vaccines in humans is marginal and generic improvements can serve as important platforms to make the technology practical for human use. The expression vector cassettes can be used in other gene based vaccines as well, or for production of recombinant proteins from eukaryotic expression vectors. The invention is useful in the production of genetic vaccines and gene therapies for a wide variety of diseases, including HIV and other viral diseases and cancer.

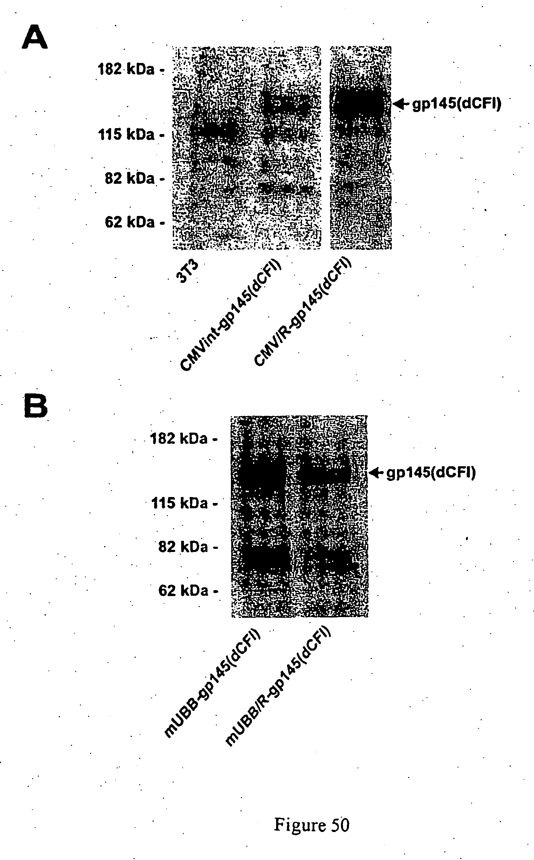

### Figure 50. Enhanced expression of modified CMV expression vector, CMV/R.

Mouse fibroblast 3T3 cells were transfected with **(A)** vector alone (lane 1), CMVint-gp-145(dCFI) (lane 2), CMV/R-gp145(dCFI) (lane 3) or **(B)** mUBB-gp145(dCFI) (lane 4), mUBB/R-gp145(dCFI) (lane 5) in 6-well tissue culture dishes with 0.5 ug of the corresponding plasmids using calcium phosphate. 24 hours after transfection, cells were harvested and lysed in lysis buffer (50 mM HEPES, 150 mM NaCl, 1% NP-40, Mini Complete protease inhibitor cocktail (Roche)). 10 µg of total protein of each sample were separated on a 4-15% gradient gel using SDS-PAGE, followed by protein transfer and Western blot analysis. Human IDV-IgG (1:5000) was used as the primary antibody, and HRP-conjugated goat anti-human IgG (1:5000) as the secondary antibody. The membrane was developed using the ECL Western blot developing system. The arrow indicates the specific band for the HIV Env gp 145(ΔCFI) polyprotein.

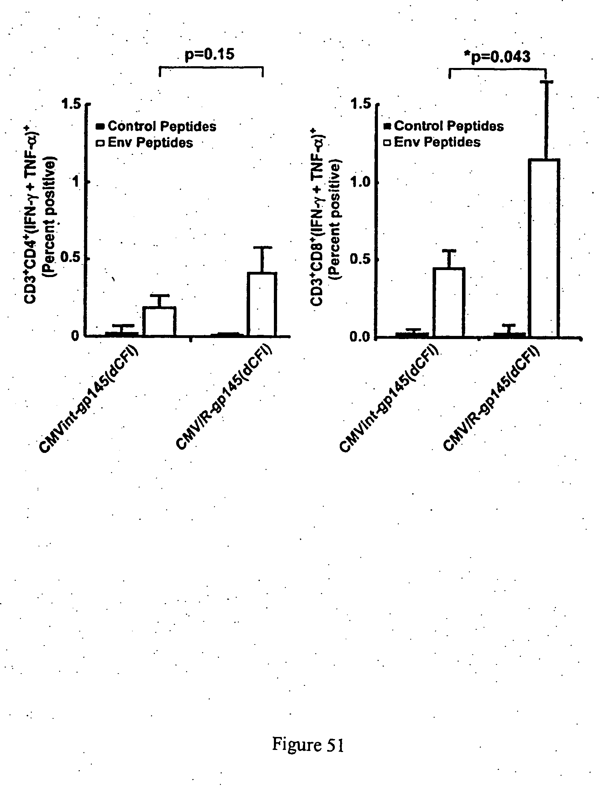

### Figure 51. Enhanced immunogenicity of modified CMV expression vector, CMV/R, in mice.

Five mice in each group were immunized with 50 µg of the indicated plasmid DNA at weeks 0, 2, and 6. 10 days after the last injection, splenocytes from each mouse were harvested and stimulated using a pool of control peptides (15 mer), or a pool of HIV Env peptides (15 mer) for 6 hours. The stimulated splenocytes were stained using a cocktail of antibodies containing PE-anti-mouse CD3, PeiCP-anti-mouse CD4, APC-anti-mouse CD8, FITC-anti-mouse IFN-γ and FITC-anti-mouse TNF-α.. The samples were analyzed by flow cytometry. CD3/CD4/IFN-γ/TNF-α and CD3/CD8/IFN-γ/TNF-α positive cell numbers were measured using FloJo software (Treestar).

### The CMV Enhancer/Promoter, R Region (HTVL-1), CMV IE Splicing Acceptor sequence (SEO ID NO: 52):

1-741: CMV Enhancer/Promoter
742-972: HTLV-1 R region
973-1095: CMV/ IE Splicing Acceptor

The aspects further described herein are further characterized by the following items:
1. A bimodal priming composition and boosting composition for priming and boosting an immune response to an antigen in an individual comprising (1) a priming composition comprised of a DNA plasmid comprising a nucleic acid molecule encoding Ebola, Marburg, Lassa, retrovirus, paramyxovirus, or influenza virus glycoprotein or nucleoprotein or epitope-bearing domain thereof, or a DNA plasmid taken from Table 2 or its insert, or analog thereof having at least 95% identity thereto, and (2) a boosting' composition comprised of a replication-deficient adenovirus comprising a nucleic acid molecule encoding Ebola, Marburg, Lassa, retrovirus, paramyxovirus, or influenza virus glycoprotein or nucleoprotein or epitope-bearing domain thereof, or a replication-deficient adenovirus taken from Table 2 or its insert, or analog thereof having at least 95% identity thereto, for production of said antigen by expression from said DNA plasmid and replication-deficient adenovirus, whereby an immune response to the antigen previously primed in the individual is boosted.
2. A bimodal priming composition and boosting composition for priming and boosting an immune response to an antigen in an individual comprising (1) a priming composition comprised of a DNA plasmid comprising a nucleic acid molecule encoding Ebola, Marburg, or Lassa glycoprotein or nucleoprotein or epitope-bearing domain thereof, or a DNA plasmid taken from Table 2 or its insert, or analog thereof having at least 95% identity thereto, and (2) a boosting composition comprised of a replication-deficient adenovirus comprising a nucleic acid molecule encoding Ebola, Marburg, or Lassa glycoprotein or nucleoprotein or epitope-bearing domain thereof, or a replication-deficient adenovirus taken from Table 2 or its insert, or analog thereof having at least 95% identity thereto, for production of said antigen by expression from said DNA plasmid and replication-deficient adenovirus, whereby an immune response to the antigen previously primed in the individual is boosted.
3. A bimodal priming composition and boosting composition for priming and boosting an immune response to an antigen in an individual comprising (1) a priming composition comprised of a first genetic construct comprising a nucleic acid molecule encoding Ebola, Marburg, or Lassa glycoprotein or nucleoprotein or epitope-bearing domain thereof or analog thereof having at least 95% identity thereto, and (2) a boosting composition comprised of a second genetic construct comprising a nucleic acid molecule encoding Ebola, Marburg, or Lassa glycoprotein or nucleoprotein or epitope-bearirig domain thereof or analog thereof having at least 95% identity thereto, or recombinantly produced Ebola, Marburg, or Lassa glycoprotein or nucleoprotein or epitope-bearing domain thereof or analog thereof having at least 95% identity thereto, wherein the first genetic construct is taken from the group consisting of plasmid DNA, replication-deficient adenovirus, replication-deficient vaccinia virus, recombinant avipox virus, and recombinant herpes virus, and wherein the second genetic construct is taken from the group consisting of replication-deficient adenovirus, replication-deficient vaccinia virus, recombinant avipox virus, and recombinant herpes virus, for production of said antigen by expression from said first genetic construct and second genetic construct, whereby an immune response to the antigen previously primed in the individual is boosted.
4. A method of boosting an immune response to an antigen in an individual comprising providing to said individual a boosting composition comprised of a replication-deficient adenovirus comprising a nucleic acid molecule encoding Ebola, Marburg, Lassa, retrovirus, paramyxovirus, or influenza virus glycoprotein or nucleoprotein or epitope-bearing domain thereof, or a replication-deficient adenovirus taken from Table 2 or its insert, or analog thereof having at least 95% identity thereto, for production of said antigen by expression from said replication-deficient adenovirus, whereby an immune response to the antigen previously primed in the individual is boosted.
5. A method of boosting an immune response to an antigen in an individual comprising providing to said individual a boosting composition comprised of a replication-deficient adenovirus comprising a nucleic acid molecule encoding Ebola, Marburg, or Lassa glycoprotein or nucleoprotein or epitope-bearing domain thereof, or a replication-deficient adenovirus taken from Table 2 or its insert, or analog thereof having at least 95% identity thereto, for production of said antigen by expression from said replication-deficient adenovirus, whereby an immune response to the antigen previously primed in the individual is boosted.
6. A method of boosting an immune response to an antigen in an individual comprising providing to said individual a boosting composition comprised of a second genetic construct comprising a nucleic acid molecule encoding Ebola, Marburg, or Lassa glycoprotein or nucleoprotein or epitope-bearing domain thereof or analog thereof having at least 95% identity thereto, or recombinantly produced Ebola, Marburg, or Lassa glycoprotein or nucleoprotein or epitope-bearing domain thereof or analog thereof having at least 95% identity thereto, for production of said antigen by expression from said replication-deficient adenovirus, wherein the second genetic construct is taken from the group consisting of replication-deficient adenovirus, replication-deficient vaccinia virus, recombinant avipox virus, and recombinant herpes virus, whereby an immune response to the antigen previously primed in the individual during production of said antigen by expression from a first genetic construct is boosted, and wherein the first genetic construct is taken from the group consisting of plasmid DNA, replication-deficient adenovirus, replication-deficient vaccinia virus, recombinant avipox virus, and recombinant herpes virus.
7. A method of inducing an immune response to an antigen in an individual comprising providing to said individual a priming composition comprising of a DNA plasmid comprising a nucleic acid molecule encoding Ebola, Marburg, Lassa, retrovirus, paramyxovirus, or influenza virus glycoprotein or nucleoprotein or epitope-bearing domain thereof, or a DNA plasmid taken from Table 2 or its insert, or analog thereof having at least 95% identity thereto, and then providing to said individual a boosting composition comprised of a replication-deficient adenovirus comprising a nucleic acid molecule encoding Ebola, Marburg, Lassa, retrovirus, paramyxovirus, or influenza virus glycoprotein or nucleoprotein or epitope-bearing domain thereof, or a replication-deficient adenovirus taken from Table 2 or its insert, or analog thereof having at least 95% identity thereto, for production of said antigen by expression from said DNA plasmid and replication-deficient adenovirus, whereby an immune response to the antigen previously primed in the individual is boosted.
8. A method of inducing an immune response to an antigen in an individual comprising providing to said individual a priming composition comprising of a DNA plasmid comprising a nucleic acid molecule encoding Ebola, Marburg, or Lassa, glycoprotein or nucleoprotein or epitope-bearing domain thereof, or a DNA plasmid taken from Table 2 or its insert, or analog thereof having at least 95% identity thereto, and then providing to said individual a boosting composition comprised of a replication-deficient adenovirus comprising a nucleic acid molecule encoding Ebola, Marburg, or Lassa, retrovirus glycoprotein or nucleoprotein or epitope-bearing domain thereof, or a replication-deficient adenovirus taken from Table 2 or its insert, or analog thereof having at least 95% identity thereto, for production of said antigen by expression from said DNA plasmid and replication-deficient adenovirus, whereby an immune response to the antigen previously primed in the individual is boosted.
9. A method of inducing an immune response to an antigen in an individual comprising providing to said individual a priming composition comprising of a first genetic construct comprising a nucleic acid molecule encoding Ebola, Marburg, or Lass glycoprotein or nucleoprotein or epitope-bearing domain thereof or analog thereof having at least 95% identity thereto, and then providing to said individual a boosting composition comprised of a second genetic construct comprising a nucleic acid molecule encoding Ebola, Marburg, or Lassa glycoprotein or nucleoprotein or epitope-bearing domain thereof or analog thereof having at least 95% identity thereto, or recombinantly produced Ebola, Marburg, or Lassa glycoprotein or nucleoprotein or epitope-bearing domain thereof or analog thereof having at least 95% identity thereto, wherein the first genetic construct is taken from the group consisting of plasmid DNA, replication-deficient adenovirus, replication-deficient vaccinia virus, recombinant avipox virus, and recombinant herpes virus, and wherein the second genetic construct is taken from the group consisting of replication-deficient adenovirus, replication-deficient vaccinia virus, recombinant avipox virus, and recombinant herpes virus, for production of said antigen by expression from said first genetic construct and second genetic construct, whereby an immune response to the antigen previously primed in the individual is boosted.
10. A composition or method of any of items 1-9 wherein the DNA plasmid or first genetic construct comprises a nucleic acid molecule encoding Ebola Zaire glycoprotein or epitope-bearing domain thereof or analog thereof having at least 95% identity thereto.
11. A composition or method of any of items 1-9 wherein the DNA plasmid or first genetic construct comprises a nucleic acid molecule encoding Ebola Sudan glycoprotein or epitope-bearing domain thereof or analog thereof having at least 95% identity thereto.
12. A composition or method of any of items 1-9 wherein the DNA plasmid or first genetic construct comprises a nucleic acid molecule encoding Ebola Ivory Coast glycoprotein or epitope-bearing domain thereof or analog thereof having at least 95% identity thereto.
13. A composition or method of any of items 1-9 wherein the DNA plasmid or first genetic construct comprises a nucleic acid molecule encoding Ebola Reston glycoprotein or epitope-bearing domain thereof or analog thereof having at least 95% identity thereto.
14. A composition or method of any of items 1-9 wherein the DNA plasmid or first genetic construct comprises a nucleic acid molecule encoding Marburg glycoprotein or epitope-bearing domain thereof or analog thereof having at least 95% identity thereto.
15. A composition or method of any of items 1-9 wherein the DNA plasmid or first genetic construct comprises a nucleic acid molecule encoding Ebola Zaire nucleoprotein or epitope-bearing domain thereof or analog thereof having at least 95% identity thereto.
16. A composition or method of any of items 1-9 wherein the DNA plasmid or first genetic construct comprises a nucleic acid molecule encoding Ebola Sudan nucleoprotein or epitope-bearing domain thereof or analog thereof having at least 95% identity thereto.
17. A composition or method of any of items 1-9 wherein the DNA plasmid or first genetic construct comprises a nucleic acid molecule encoding Ebola Ivory Coast nucleoprotein or epitope-bearing domain thereof or analog thereof having at least 95% identity thereto.
18. A composition or method of any of items 1-9 wherein the DNA plasmid or first genetic construct comprises a nucleic acid molecule encoding Ebola Reston nucleoprotein or epitope-bearing domain thereof or analog thereof having at least 95% identity thereto.
19. A composition or method of any of items 1-9 wherein the DNA plasmid or first genetic construct comprises a nucleic acid molecule encoding Marburg nucleoprotein or epitope-bearing domain thereof or analog thereof having at least 95% identity thereto.
20. A composition or method of any of items 1-9 wherein the DNA plasmid or first genetic construct comprises a nucleic acid molecule encoding Ebola Zaire glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
21. A composition or method of any of items 1-9 wherein the DNA plasmid or first genetic construct comprises a nucleic acid molecule encoding Ebola Sudan glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
22. A composition or method of any of items 1-9 wherein the DNA plasmid or first genetic construct comprises a nucleic acid molecule encoding Ebola Ivory Coast glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
23. A composition or method of any of items 1-9 wherein the DNA plasmid or first genetic construct comprises a nucleic acid molecule encoding Ebola Reston glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
24. A composition or method of any of items 1-9 wherein the DNA plasmid or first genetic construct comprises a nucleic acid molecule encoding Marburg glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
25. A composition or method of any of items 1-9 wherein the DNA plasmid or first genetic construct comprises a nucleic acid molecule encoding Ebola Zaire nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
26. A composition or method of any of items 1-9 wherein the DNA plasmid or first genetic construct comprises a nucleic acid molecule encoding Ebola Sudan nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
27. A composition or method of any of items 1-9 wherein the DNA plasmid or first genetic construct comprises a nucleic acid molecule encoding Ebola Ivory Coast nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
28. A composition or method of any of items 1-9 wherein the DNA plasmid or first genetic construct comprises a nucleic acid molecule encoding Ebola Reston nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
29. A composition or method of any of items 1-9 wherein the DNA plasmid or first genetic construct comprises a nucleic acid molecule encoding Marburg nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
30. A composition or method of any of items 1-9 wherein the replication-defective adenovirus or second genetic construct comprises a nucleic acid molecule encoding Ebola Zaire glycoprotein or epitope-bearing domain thereof or analog thereof having at least 95% identity thereto.
31. A composition or method of any of items 1-9 wherein the replication-defective adenovirus or second genetic construct comprises a nucleic acid molecule encoding Ebola Sudan glycoprotein or epitope-bearing domain thereof or analog thereof having at least 95% identity thereto.
32. A composition or method of any of items 1-9 wherein the replication-defective adenovirus or second genetic construct comprises a nucleic acid molecule encoding Ebola Ivory Coast glycoprotein or epitope-bearing domain thereof or analog thereof having at least 95% identity thereto.
33. A composition or method of any of items 1-9 wherein the replication-defective adenovirus or second genetic construct comprises a nucleic acid molecule encoding Ebola Reston glycoprotein or epitope-bearing domain thereof or analog thereof having at least 95% identity thereto.
34. A composition or method of any of items 1-9 wherein the replication-defective adenovirus or second genetic construct comprises a nucleic acid molecule encoding Marburg glycoprotein or epitope-bearing domain thereof or analog thereof having at least 95% identity thereto.
35. A composition or method of any of items 1-9 wherein the replication-defective adenovirus or second genetic construct comprises a nucleic acid molecule encoding Ebola Zaire nucleoprotein or epitope-bearing domain thereof or analog thereof having at least 95% identity thereto.
36. A composition or method of any of items 1-9 wherein the replication-defective adenovirus or second genetic construct comprises a nucleic acid molecule encoding Ebola Sudan nucleoprotein or epitope-bearing domain thereof or analog thereof having at least 95% identity thereto.
37. A composition or method of any of items 1-9 wherein the replication-defective adenovirus or second genetic construct comprises a nucleic acid molecule encoding Ebola Ivory Coast nucleoprotein or epitope-bearing domain thereof or analog thereof having at least 95% identity thereto.
38. A composition or method of any of items 1-9 wherein the replication-defective adenovirus or second genetic construct comprises a nucleic acid molecule encoding Ebola Reston nucleoprotein or epitope-bearing domain thereof or analog thereof having at least 95% identity thereto.
39. A composition or method of any of items 1-9 wherein the replication-defective adenovirus or second genetic construct comprises a nucleic acid molecule encoding Marburg nucleoprotein or epitope-bearing domain thereof or analog thereof having at least 95% identity thereto.
40. A composition or method of any of items 1-9 wherein the replication-defective adenovirus or second genetic construct comprises a nucleic acid molecule encoding Ebola Zaire glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
41. A composition or method of any of items 1-9 wherein the replication-defective adenovirus or second genetic construct comprises a nucleic acid molecule encoding Ebola Sudan glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
42. A composition or method of any of items 1-9 wherein the replication-defective adenovirus or second genetic construct comprises a nucleic acid molecule encoding Ebola Ivory Coast glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
43. A composition or method of any of items 1-9 wherein the replication-defective adenovirus or second genetic construct comprises a nucleic acid molecule encoding Ebola Reston glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
44. A composition or method of any of items 1-9 wherein the replication-defective adenovirus or second genetic construct comprises a nucleic acid molecule encoding Marburg glycoprotein or epitope-bearing domain thereof or analog thereof having at least 95% identity thereto, wherein the glycoprotein has an internal or carboxy deletion to delete the mucin-like domain, the furin cleavage site, the fusion peptide domain, the heptad repeat domain, the transmembrane anchor domain, or the intracellular domain.
45. A composition or method of any of items 1-9 wherein the replication-defective adenovirus or second genetic construct comprises a nucleic acid molecule encoding Ebola Zaire nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
46. A composition or method of any of items 1-9 wherein the replication-defective adenovirus or second genetic construct comprises a nucleic acid molecule encoding Ebola Sudan nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
47. A composition or method of any of items 1-9 wherein the replication-defective adenovirus or second genetic construct comprises a nucleic acid molecule encoding Ebola Ivory Coast nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
48. A composition or method of any of items 1-9 wherein the replication-defective adenovirus or second genetic construct comprises a nucleic acid molecule encoding Ebola Reston nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
49. A composition or method of any of items 1-9 wherein the replication-defective adenovirus or second genetic construct comprises a nucleic acid molecule encoding Marburg nucleoprotein or epitope-bearing domain thereof or analog thereof having at least 95% identity thereto, wherein the glycoprotein has a truncation at the carboxy terminus to delete the transmembrane anchor and intracellular domain, or a truncation at the carboxy terminus to delete the heptad repeat domain and transmembrane anchor and intracellular domain, or a truncation at the carboxy terminus to delete the fusion peptide domain, heptad repeat domain, and transmembrane anchor and intracellular domain, or a truncation at the carboxy terminus to delete the furin cleavage site, fusion peptide domain, heptad repeat domain, and transmembrane anchor and intracellular domain, or a truncation at the carboxy terminus to delete the mucin-like domain, furin cleavage site, fusion peptide domain, heptad repeat domain, and transmembrane anchor and intracellular domain.
50. VRC6000 or a composition or method of any of items 1-9 wherein the DNA plasmid or replication-deficient adenovirus taken from Table 2 is VRC6000.
51. VRC6001 or a composition or method of any of items 1-9 wherein the DNA plasmid or replication-deficient adenovirus taken from Table 2 is VRC6001.
52. VRC6002 or a composition or method of any of items 1-9 wherein the DNA plasmid or replication-deficient adenovirus taken from Table 2 is VRC6002.
53. VRC6003 or a composition or method of any of items 1-9 wherein the DNA plasmid or replication-deficient adenovirus taken from Table 2 is VRC6003.
54. VRC6004 or a composition or method of any of items 1-9 wherein the DNA plasmid or replication-deficient adenovirus taken from Table 2 is VRC6004.
55. VRC6005 or a composition or method of any of items 1-9 wherein the DNA plasmid or replication-deficient adenovirus taken from Table 2 is VRC6005.
56. VRC6006 or a composition or method of any of items 1-9 wherein the DNA plasmid or replication-deficient adenovirus taken from Table 2 is VRC6006.
57. VRC6007 or a composition or method of any of items 1-9 wherein the DNA plasmid or replication-deficient adenovirus taken from Table 2 is VRC6007.
58. VRC6008 or a composition or method of any of items 1-9 wherein the DNA plasmid or replication-deficient adenovirus taken from Table 2 is VRC6008.
59. VRC6052 or a composition or method of any of items 1-9 wherein the DNA plasmid or replication-deficient adenovirus taken from Table 2 is VRC6052.
60. VRC6101 or a composition or method of any of items 1-9 wherein the DNA plasmid or replication-deficient adenovirus taken from Table 2 is VRC6101.
61. VRC6110 or a composition or method of any of items 1-9 wherein the DNA plasmid or replication-deficient adenovirus taken from Table 2 is VRC6110.
62. VRC6200 or a composition or method of any of items 1-9 wherein the DNA plasmid or replication-deficient adenovirus taken from Table 2 is VRC6200.
63. VRC6201 or a composition or method of any of items 1-9 wherein the DNA plasmid or replication-deficient adenovirus taken from Table 2 is VRC6201.
64. VRC6202 or a composition or method of any of items 1-9 wherein the DNA plasmid or replication-deficient adenovirus taken from Table 2 is VRC6202.
65. VRC6300 or a composition or method of any of items 1-9 wherein the DNA plasmid or replication-deficient adenovirus taken from Table 2 is VRC6300.
66. VRC6301 or a composition or method of any of items 1-9 wherein the DNA plasmid or replication-deficient adenovirus, taken from Table 2 is VRC6301.
67. VRC6302 or a composition or method of any of items 1-9 wherein the DNA plasmid or replication-deficient adenovirus taken from Table 2 is VRC6302.
68. VRC6303 or a composition or method of any of items 1-9 wherein the DNA plasmid or replication-deficient adenovirus taken from Table 2 is VRC6303.
69. VRC6310 or a composition or method of any of items 1-9 wherein the DNA plasmid or replication-deficient adenovirus taken from Table 2 is VRC6310.
70. VRC6351 or a composition or method of any of items 1-9 wherein the DNA plasmid or replication-deficient adenovirus taken from Table 2 is VRC6351
71. VRC6400 or a composition or method of any of items1-9 wherein the DNA plasmid or replication-deficient adenovirus taken from Table 2 is VRC6400.
72. VRC6401 or a composition or method of any of items 1-9 wherein the DNA plasmid or replication-deficient adenovirus taken from Table 2 is VRC6401.
73. VRC6500 or a composition or method of any of items 1-9 wherein the DNA plasmid or replication-deficient adenovirus taken from Table 2 is VRC6500.
74. VRC6600 or a composition or method of any of items 1-9 wherein the DNA plasmid or replication-deficient adenovirus taken from Table 2 is VRC6600.
75. VRC6601 or a composition or method of any of items 1-9 wherein the DNA plasmid or replication-deficient adenovirus taken from Table 2 is VRC6601.
76. VRC6602 or a composition or method of any of items 1-9 wherein the DNA plasmid or replication-deficient adenovirus taken from Table 2 is VRC6602.
77. VRC6603 or a composition or method of any of items 1-9 wherein the DNA plasmid or replication-deficient adenovirus taken from Table 2 is VRC6603.
78. VRC6604 or a composition or method of any of items 1-9 wherein the DNA plasmid or replication-deficient adenovirus taken from Table 2 is VRC6604.
79. VRC6701 or a composition or method of any of items 1-9 wherein the DNA plasmid or replication-deficient adenovirus taken from Table 2 is VRC6701.
80. VRC6702 or a composition or method of any of items 1-9 wherein the DNA plasmid or replication-deficient adenovirus taken from Table 2 is VRC6702.
81. VRC6710 or a composition or method of any of items 1-9 wherein the DNA plasmid or replication-deficient adenovirus taken from Tab!e 2 is VRC6710.
82. VRC6800 or a composition or method of any of items 1-9 wherein the DNA plasmid or replication-deficient adenovirus taken from Table 2 is VRC6800.
83. VRC6801 or a composition or method of any of items 1-9 wherein the DNA plasmid or replication-deficient adenovirus taken from Table 2 is VRC6801.
84. VRC6810 or a composition or method of any of items 1-9 wherein the DNA plasmid or replication-deficient adenovirus taken from Table 2 is VRC6810.
85. VRC6811 or a composition or method of any of items 1-9 wherein the DNA plasmid or replication-deficient adenovirus taken from Table 2 is VRC6811.
86. CMV/R Ebola GP (Z) deltaTM/h (codon optimized) or a composition or method of any of items 1-9 wherein the DNA plasmid or replication-deficient adenovirus taken from Table 2 is CMV/R Ebola GP (Z) deltaTM/h (codon optimized).
87. pVR1012 Ebola GP (Z, P87666) delta TM/h (codon optimized) or a composition or method of any of items 1-9 wherein the DNA plasmid or replication-deficient adenovirus taken from Table 2 is pVR1012 Ebola GP (Z, P87666) delta TM/h (codon optimized).
88. CMV/R Ebola GP (S/Gulu) delta TM/h (codon optimized) or a composition or method of any of items 1-9 wherein the DNA plasmid or replication-deficient adenovirus taken from Table 2 is CMV/R Ebola GP (S/Gulu) delta TM/h (codon optimized).
89. CMV/R Ebola GP (S,Q66798) delta TM/h (codon optimized) or a composition or method of any of items 1-9 wherein the DNA plasmid or replication-deficient adenovirus taken from Table 2 is CMV/R Ebola GP (S,Q66798) delta TM/h (codon optimized).
90. VRC6802, pVR1012x/s Lassa delta TM/h (codon optimized) or a composition or method of any of items 1-9 wherein the DNA plasmid or replication-deficient adenovirus taken from Table 2 is VRC6802, pVR1012x/s Lassa (codon optimized).
91. VRC6703, pVR1012x/s Marburg delta TM/h (codon optimized) or a composition or method of any of items 1-9 wherein the DNA plasmid or replication-deficient adenovirus taken from Table 2 is VRC6703, pVR1012x/s Marburg (codon optimized).
92. CMV/R Ebola NP or a composition or method of any of items 1-9 wherein the DNA plasmid or replication-deficient adenovirus taken from Table 2 is CMV/R Ebola NP.
93. A composition or method of any of items 1-9 wherein the priming or boosting composition is administered intramuscularly.
94. A composition or method of any of items 1-9 wherein the boosting composition is administered about 2-3 weeks, or 4 weeks, or 8 weeks, or 16 weeks, or 20 weeks, or 24 weeks, or 28 weeks, or 32 weeks after administration of the priming composition.
95. A composition or method of any of items 1-9 wherein the priming or boosting composition is administered in combination with an adjuvant.
96. A composition or method of any of items 1-9 wherein the priming or boosting composition is administered in combination with a cytokine or nucleic acid encoding therefor.
97. A composition or method of any of items 1-9 wherein the DNA plasmid is administered at a dose of 10 micrograms to 50 milligrams/injection.
98. A composition or method of any of items 1-9 wherein the replication-deficient adenovirus is administered at a dose of 5 x 10 7 to 1 x 10 12 particles/injection.
99. A composition or method of any of items 1-9 wherein the priming or boosting composition is further comprised of either the same or a different DNA plasmid or replication-deficient adenovirus comprising a nucleic acid molecule encoding a second Ebola glycoprotein or nucleoprotein or epitope-bearing domain thereof or analog thereof having at least 95% identity thereto, and wherein the second Ebola glycoprotein or nucleoprotein is different from the first.

## Claims

1. An expression vector comprising SEQ ID NO:52 or analog thereof having at least 95, 96, 97, 98, 99, or 100% sequence identity thereto that mediates expression, wherein the percentage of identity is calculated over the full length of the reference nucleotide sequence.

2. The expression vector of claim 1, wherein the % identity is 95.

3. The expression vector of claim 1, wherein the % identity is 96.

4. The expression vector of claim 1, wherein the % identity is 97.

5. The expression vector of claim 1, wherein the % identity is 98.

6. The expression vector of claim 1, wherein the % identity is 99.

7. The expression vector of claim 1, wherein the % identity is 100.

## Patentansprüche

1. Expressionsvektor, umfassend SEQ ID NO:52 oder deren Analogon, das zumindest 95, 96, 97, 98, 99 oder 100% Sequenzidentität dazu hat, wobei der Prozentsatz der Identität über die volle Länge der Referenz-Nucleotidsequenz berechnet wird.

2. Expressionsvektor nach Anspruch 1, wobei der Prozentsatz der Identität 95 beträgt.

3. Expressionsvektor nach Anspruch 1, wobei der Prozentsatz der Identität 96 beträgt.

4. Expressionsvektor nach Anspruch 1, wobei der Prozentsatz der Identität 97 beträgt.

5. Expressionsvektor nach Anspruch 1, wobei der Prozentsatz der Identität 98 beträgt.

6. Expressionsvektor nach Anspruch 1, wobei der Prozentsatz der Identität 99 beträgt.

7. Expressionsvektor nach Anspruch 1, wobei der Prozentsatz der Identität 100 beträgt.

## Revendications

1. Vecteur d'expression comprenant SEQ ID NO :52 ou un analogue de celle-ci ayant au moins 95, 96, 97, 98, 99, ou 100% d'identité de séquence avec celle-ci qui médie l'expression, dans lequel le pourcentage d'identité est calculé sur la longueur totale de la séquence nucléotidique de référence.

2. Vecteur d'expression selon la revendication 1, dans lequel le pourcentage d'identité est de 95.

3. Vecteur d'expression selon la revendication 1, dans lequel le pourcentage d'identité est de 96.

4. Vecteur d'expression selon la revendication 1, dans lequel le pourcentage d'identité est de 97.

5. Vecteur d'expression selon la revendication 1, dans lequel le pourcentage d'identité est de 98.

6. Vecteur d'expression selon la revendication 1, dans lequel le pourcentage d'identité est de 99.

7. Vecteur d'expression selon la revendication 1, dans lequel le pourcentage d'identité est de 100.
